(19) Europäisches Patentamt
European Patent Office
Office européen des brevets

(11) EP 3 543 324 B1

(12) EUROPÄISCHE PATENTSCHRIFT

(45) Veröffentlichungstag und Bekanntmachung des Hinweises auf die Patenterteilung:
**30.11.2022 Patentblatt 2022/48**

(21) Anmeldenummer: **19155597.8**

(22) Anmeldetag: **23.02.2005**

(51) Internationale Patentklassifikation (IPC):
***C11B 1/02*** *(2006.01)* ***C12N 9/02*** *(2006.01)*
***C12N 9/10*** *(2006.01)* ***A01K 67/027*** *(2006.01)*
***C12N 15/82*** *(2006.01)*

(52) Gemeinsame Patentklassifikation (CPC):
**C12N 9/0071; A23D 9/00; A61K 8/922; A61K 31/202; A61P 3/02; A61P 3/04; A61P 3/06; A61P 3/10; A61P 9/00; A61P 9/12; A61P 19/02; A61P 25/00; A61P 27/02; A61P 29/00; A61P 35/00;** (Forts.)

(54) **VERFAHREN ZUR HERSTELLUNG MEHRFACH UNGESÄTTIGTEN FETTSÄUREN IN TRANSGENEN PFLANZEN**

METHOD FOR PRODUCING POLYUNSATURATED FATTY ACIDS IN TRANSGENIC PLANTS

PROCÉDÉ DE FABRICATION D'UNE PLURALITÉ D'ACIDES GRAS INSATURÉS DANS LES PLANTES TRANSGÉNIQUES

(84) Benannte Vertragsstaaten:
**AT BE BG CH CY CZ DE DK EE ES FI FR GB GR HU IE IS IT LI LT LU MC NL PL PT RO SE SI SK TR**

(30) Priorität:
**14.05.2004 DE 102004024014**
**13.03.2004 DE 102004012370**
**08.04.2004 DE 102004017518**
**24.12.2004 DE 102004062543**
**16.07.2004 PCT/EP2004/007957**
**27.02.2004 DE 102004009457**

(43) Veröffentlichungstag der Anmeldung:
**25.09.2019 Patentblatt 2019/39**

(62) Dokumentnummer(n) der früheren Anmeldung(en) nach Art. 76 EPÜ:
**13162907.3 / 2 623 584**
**05715462.7 / 1 723 220**

(73) Patentinhaber: **BASF Plant Science GmbH**
**67056 Ludwigshafen (DE)**

(72) Erfinder:
- **CIRPUS, Petra**
  **67165 Waldsee (DE)**
- **BAUER, Joerg**
  **Durham NC 27713 (US)**
- **QUI, Xiao**
  **Saskatoon Saskatchewan S7N 3S5 (CA)**
- **WU, Guohai**
  **Saskatoon Saskatchewan S7N 4A9 (CA)**
- **DATLA, Nagamani**
  **Saskatoon Saskatchewan S7V 1B6 (CA)**

(74) Vertreter: **BASF IP Association**
**BASF SE**
**G-FLP-C006**
**67056 Ludwigshafen (DE)**

(56) Entgegenhaltungen:
**WO-A2-2005/012316 DE-A1- 10 219 203**

- **BEAUDOIN FREDERIC ET AL: "Heterologous reconstitution in yeast of the polyunsaturated fatty acid biosynthetic pathway", PROCEEDINGS OF THE NATIONAL ACADEMY OF SCIENCES OF USA, NATIONAL ACADEMY OF SCIENCE. WASHINGTON, US, Bd. 97, Nr. 12, 6. Juni 2000 (2000-06-06), Seiten 6421-6426, XP002200201, ISSN: 0027-8424**
- **DOMERGUE F ET AL: "Cloning and functional characterization of Phaeodactylum tricornutum front-end desaturases involved in eicosapentaenoic acid biosynthesis", EUROPEAN JOURNAL OF BIOCHEMISTRY, BERLIN, DE, Bd. 269, Nr. 16, August 2002 (2002-08), Seiten 4105-4113, XP002228745, ISSN: 0014-2956**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

- **ZANK T K ET AL: "Cloning and functional expression of the first plant fatty acid elongase specific for DELTA6-polyunsaturated fatty acids", BIOCHEMICAL SOCIETY TRANSACTIONS, COLCHESTER, ESSEX, GB, Bd. 28, Nr. 6, Dezember 2000 (2000-12), Seiten 654-658, XP002174836, ISSN: 0300-5127**
- **DREXLER H ET AL: "Metabolic engineering of fatty acids for breeding of new oilseed crops: Strategies, problems and first results", JOURNAL OF PLANT PHYSIOLOGY, FISCHER, STUTTGART, DE, Bd. 160, Nr. 7, Juli 2003 (2003-07), Seiten 779-802, XP002266491, ISSN: 0176-1617**
- **MEYER ASTRID ET AL: "Novel fatty acid elongases and their use for the reconstitution of docosahexaenoic acid biosynthesis.", JOURNAL OF LIPID RESEARCH. OCT 2004, Bd. 45, Nr. 10, Oktober 2004 (2004-10), Seiten 1899-1909, XP009046591, ISSN: 0022-2275**
- **Drummond: "Re. GRAS Notification - Tuna Oil", , 15. Februar 2002 (2002-02-15), XP55389590, Gefunden im Internet: URL:https://www.fda.gov/downloads/Food/IngredientsPackagingLabeling/GRAS/NoticeInventory/ucm258378.pdf [gefunden am 2017-07-11]**


Bemerkungen:
Das gesamte Dokument mit Referenztabelle(n) und Sequenzliste(n) kann von der Webseite des EPA heruntergeladen werden

(52) Gemeinsame Patentklassifikation (CPC): (Forts.)
**A61Q 19/00; C12N 9/0083; C12N 9/1029; C12N 15/8247;** A61K 2800/86; C12Y 114/19

## Beschreibung

Die vorliegende Erfindung betrifft ein Verfahren zur Herstellung von mehrfach ungesättigten Fettsäuren im Samen transgener Pflanzen, indem Nukleinsäuren in den Organismus eingebracht werden, die für Polypeptide mit ω-3-Desaturase-, Δ-12-Desaturase-, Δ-6-Desaturase-, Δ-6-Elongase-, Δ-5-Desaturase-, Δ-5-Elongase-und/oder Δ-4-Desaturaseaktivität bevorzugt für Polypeptide mit Δ-6-Desaturase-, Δ-6-Elongase- und Δ-5-Desaturaseaktivität codieren.

Bei den Nukleinsäuresequenzen handelt es sich um die in SEQ ID NO: 11, SEQ ID NO: 27, SEQ ID NO: 193, SEQ ID NO: 197, SEQ ID NO: 199 und SEQ ID NO: 201 dargestellten Sequenzen. Bevorzugt wird neben diesen Nukleinsäuresequenzen eine weitere Nukleinsäuresequenz, die für ein Polypeptid mit einer Δ-12-Desaturaseaktivität kodiert, in die Pflanze eingebracht und ebenfalls gleichzeitig exprimiert. Besonders bevorzugt handelt es sich dabei um die in SEQ ID NO: 195 dargestellte Nukleinsäuresequenz.

Vorteilhaft können diese Nukleinsäuresequenzen gegebenenfalls zusammen mit weiteren Nukleinsäuresequenzen, die für Polypeptide der Biosynthese des Fettsäureoder Lipidstoffwechels codieren, in dem Organismus exprimiert werden. Besonders vorteilhaft sind Nukleinsäuresequenzen, die für eine Δ-6-Desaturase-, eine Δ-5-Desaturase-, Δ-4-Desaturase-, Δ-12-Desaturase- und/oder Δ-6-Elongaseaktivität codieren. Vorteilhaft stammen diese Desaturasen und Elongasen aus Thalassiosira, Euglena oder Ostreococcus. Weiterhin betrifft die Erfindung ein Verfahren zur Herstellung von Ölen und/oder Triacylglyceriden mit einem erhöhten Gehalt an langkettigen mehrfach ungesättigten Fettsäuren.

Die Erfindung betrifft in einer bevorzugten Ausführungsform außerdem ein Verfahren zur Herstellung von Arachidonsäure, Eicosapentaensäure oder Docosahexaensäure sowie ein Verfahren zur Herstellung von Triglyceriden mit einem erhöhten Gehalt an ungesättigten Fettsäuren, insbesondere Arachidonsäure, Eicosapentaensäure und/oder Docosahexaensäure, in transgenen Pflanzen vorteilhaft im Samen der transgenen Pflanze. Die Erfindung betrifft die Herstellung einer transgenen Pflanze mit erhöhtem Gehalt an mehrfach ungesättigten Fettsäuren, insbesondere Arachidonsäure, Eicosapentaensäure und/oder Docosahexaensäure, aufgrund der Expression der im erfindungsgemäßen Verfahren verwendeten Elongasen und Desaturasen.

Die Erfindung betrifft weiterhin rekombinante Nukleinsäuremoleküle, die die Nukleinsäuresequenzen, die für die Polypeptide mit Δ-6-Desaturase-, Δ-6-Elongase-, Δ-5-Desaturase- und Δ-5-Elongaseaktivität kodieren, gemeinsam oder einzeln enthalten, sowie transgene Pflanzen, die die vorgenannten rekombinanten Nukleinsäuremoleküle enthalten.

Ein weiterer Teil der Erfindung betrifft Öle, Lipide und/oder Fettsäuren hergestellt nach dem erfindungsgemäßen Verfahren und deren Verwendung. Außerdem betrifft die Erfindung ungesättigte Fettsäuren sowie Triglyceride mit einem erhöhten Gehalt an ungesättigten Fettsäuren und deren Verwendung.

Die Lipidsynthese lässt sich in zwei Abschnitte unterteilen: die Synthese von Fettsäuren und ihre Bindung an sn-Glycerin-3-Phosphat sowie die Addition oder Modifikation einer polaren Kopfgruppe. Übliche Lipide, die in Membranen verwendet werden, umfassen Phospholipide, Glycolipide, Sphingolipide und Phosphoglyceride. Die Fettsäuresynthese beginnt mit der Umwandlung von Acetyl-CoA in Malonyl-CoA durch die Acetyl-CoA-Carboxylase oder in Acetyl-ACP durch die Acetyltransacylase. Nach einer Kondensationsreaktion bilden diese beiden Produktmoleküle zusammen Acetoacetyl-ACP, das über eine Reihe von Kondensations-, Reduktions- und Dehydratisierungsreaktionen umgewandelt wird, so dass ein gesättigtes Fettsäuremolekül mit der gewünschten Kettenlänge erhalten wird. Die Produktion der ungesättigten Fettsäuren aus diesen Molekülen wird durch spezifische Desaturasen katalysiert, und zwar entweder aerob mittels molekularem Sauerstoff oder anaerob (bezüglich der Fettsäuresynthese in Mikroorganismen siehe F.C. Neidhardt et al. (1996) E. coli und Salmonella. ASM Press: Washington, D.C., S. 612-636 und darin enthaltene Literaturstellen; Lengeler et al. (Hrsgb.) (1999) Biology of Procaryotes. Thieme: Stuttgart, New York, und die enthaltene Literaturstellen, sowie Magnuson, K., et al. (1993) Microbiological Reviews 57:522-542 und die enthaltenen Literaturstellen). Die so hergestellten an Phospholipide gebundenen Fettsäuren müssen anschließend für die weiteren Elongationen aus den Phospholipiden wieder in den FettsäureCoA-Ester-Pool überführt werden. Dies ermöglichen Acyl-CoA:Lysophospholipid-Acyltransferasen. Weiterhin können diese Enzyme die elongierten Fettsäuren wieder von den CoA-Estern auf die Phospholipide übertragen. Diese Reaktionsabfolge kann gegebenenfalls mehrfach durchlaufen werden.

Ferner müssen Fettsäuren anschließend an verschiedene Modifikationsorte transportiert und in das Triacylglycerin-Speicherlipid eingebaut werden. Ein weiterer wichtiger Schritt bei der Lipidsynthese ist der Transfer von Fettsäuren auf die polaren Kopfgruppen, beispielsweise durch Glycerin-Fettsäure-Acyltransferase (siehe Frentzen, 1998, Lipid, 100(4-5):161-166).

Veröffentlichungen über die Pflanzen-Fettsäurebiosynthese, Desaturierung, den Lipidstoffwechsel und Membrantransport von fetthaltigen Verbindungen, die Betaoxidation, Fettsäuremodifikation und Cofaktoren, Triacylglycerin-Speicherung und - Assemblierung einschließlich der Literaturstellen darin siehe in den folgenden Artikeln: Kinney, 1997, Genetic Engineering, Hrsgb.: JK Setlow, 19:149-166; Ohlrogge und Browse, 1995, Plant Cell 7:957-970; Shanklin und Cahoon, 1998, Annu. Rev. Plant Physiol. Plant Mol. Biol. 49:611-641; Voelker, 1996, Genetic Engineering, Hrsgb.: JK Setlow, 18:111-13; Gerhardt, 1992, Prog. Lipid R. 31:397-417; Gühnemann-Schäfer & Kindl, 1995, Biochim. Biophys

Acta 1256:181-186; Kunau et al., 1995, Prog. Lipid Res. 34:267-342; Stymne et al., 1993, in: Biochemistry and Molecular Biology of Membrane and Storage Lipids of Plants, Hrsgb.: Murata und Somerville, Rockville, American Society of Plant Physiologists, 150-158, Murphy & Ross 1998, Plant Journal. 13(1):1-16.

Im folgenden werden mehrfach ungesättigte Fettsäuren als PUFA, PUFAs, LCPUFA oder LCPUFAs bezeichnet (**p**oly **u**nsaturated **f**atty **a**cids, **PUFA,** mehrfach ungesättigte Fettsäuren; **l**ong **c**hain **p**oly **u**nsaturated **f**atty **a**cids, **LCPUFA,** langkettige mehrfach ungesättigte Fettsäuren).

Fettsäuren und Triacylglyceride haben eine Vielzahl von Anwendungen in der Lebensmittelindustrie, der Tierernährung, der Kosmetik und im Pharmabereich.

Je nachdem, ob es sich um freie gesättigte und ungesättigte Fettsäuren oder um Triacylglyceride mit einem erhöhten Gehalt an gesättigten oder ungesättigten Fettsäuren handelt, sind sie für die unterschiedlichsten Anwendungen geeignet. Mehrfachungesättigte Fettsäuren wie Linol- und Linolensäure sind für Säugetiere essentiell, da sie nicht von diesen selbst hergestellt werden können. Deshalb stellen mehrfach ungesättigte ω-3-Fettsäuren und ω-6-Fettsäuren einen wichtigen Bestandteil der tierischen und menschlichen Nahrung dar. So werden z.B. in der humanen Ernährung Lipide mit ungesättigten Fettsäuren, speziell mehrfach ungesättigten, Fettsäuren bevorzugt. Den mehrfach ungesättigten ω-3-Fettsäuren wird dabei ein positiver Effekt auf den Cholesterinspiegel im Blut und damit auf die Prävention einer Herzerkrankung zugeschrieben. Durch Zugabe dieser ω-3-Fettsäuren zur Nahrung kann das Risiko einer Herzerkrankung, eines Schlaganfalls oder von Bluthochdruck deutlich verringert werden (Shimikawa 2001, World Rev. Nutr. Diet. 88, 100-108).

Auch entzündliche, speziell chronisch entzündliche, Prozesse im Rahmen immunologischer Erkrankungen wie rheumatoider Arthritis lassen sich durch ω-3-Fettsäuren positiv beeinflussen (Calder 2002, Proc. Nutr. Soc. 61, 345-358; Cleland und James 2000, J. Rheumatol. 27, 2305-2307). Sie werden deshalb Lebensmitteln, speziell diätetischen Lebensmitteln, zugegeben oder finden in Medikamenten Anwendung. ω-6-Fettsäuren wie Arachidonsäure üben bei diesen rheumatischen Erkrankungen eher einen negativen Effekt aus.

ω-3- und ω-6-Fettsäuren sind Vorläufer von Gewebshormonen, den sogenannten Eicosanoiden wie den Prostaglandinen, die sich von der Dihomo-γ-linolensäure, der Arachidonsäure und der Eicosapentaensäure ableiten, und den Thromboxanen und Leukotrienen, die sich von der Arachidonsäure und der Eicosapentaensäure ableiten. Eicosanoide (sog. $PG_2$-Serie), die aus ω-6-Fettsäuren gebildet werden, fördern in der Regel Entzündungsreaktionen, während Eicosanoide (sog. $PG_3$-Serie) aus ω-3-Fettsäuren geringe oder keine entzündungsfördernde Wirkung haben.

Mehrfach ungesättigte langkettige ω-3-Fettsäuren wie Eicosapentaensäure (= EPA, $C20{:}5^{\Delta5,8,11,14,17}$) oder Docosahexaensäure (= DHA, $C22{:}6^{\Delta4,7,10,13,16,19}$) sind wichtige Komponenten der menschlichen Ernährung aufgrund ihrer verschiedenen Rollen in der Gesundheit, die Aspekte wie die Entwicklung des kindlichen Gehirns, der Funktionalität des Auges, der Synthese von Hormonen und anderer Signalstoffe, sowie die Vorbeugung von Herz-Kreislauf-Beschwerden, Krebs und Diabetes umfassen (Poulos, A Lipids 30:1-14, 1995; Horrocks, LA und Yeo YK Pharmacol Res 40:211-225, 1999). Es besteht aus diesem Grund ein Bedarf an der Produktion mehrfach ungesättigter langkettiger Fettsäuren.

Aufgrund der heute üblichen Zusammensetzung der menschlichen Nahrung ist ein Zusatz von mehrfach ungesättigten ω-3-Fettsäuren, die bevorzugt in Fischölen vorkommen, zur Nahrung besonders wichtig. So werden beispielsweise mehrfach ungesättigte Fettsäuren wie Docosahexaensäure (= DHA, $C22{:}6^{\Delta4,7,10,13,16,19}$) oder Eisosapentaensäure (= EPA, $C20{:}5^{\Delta5,8,11,14,17}$) Babynahrung zur Erhöhung des Nährwertes zugesetzt. Der ungesättigten Fettsäure DHA wird dabei ein positiver Effekt auf die Entwicklung und Aufrechterhaltung von Gehirnfunktionen zugeschrieben. Es besteht aus diesem Grund ein Bedarf an der Produktion mehrfach ungesättigter langkettiger Fettsäuren.

Hauptsächlich werden die verschiedenen Fettsäuren und Triglyceride aus Mikroorganismen wie Mortierella oder Schizochytrium oder aus Öl-produzierenden Pflanzen wie Soja, Raps, Algen wie Crypthecodinium oder Phaeodactylum und weiteren gewonnen, wobei sie in der Regel in Form ihrer Triacylglyceride (= Triglyceride = Triglycerole) anfallen. Sie können aber auch aus Tieren wie z.B. Fischen gewonnen werden. Die freien Fettsäuren werden vorteilhaft durch Verseifung hergestellt. Sehr langkettige mehrfach ungesättigte Fettsäuren wie DHA, EPA, Arachidonsäure (= ARA, $C20{:}4^{\Delta5,8,11,14}$), Dihomo-y-linolensäure ($C20{:}3^{\Delta8,11,14}$) oder Docosapentaensäure (DPA, $C22{:}5^{\Delta7,10,13,16,19}$) werden in Ölfruchtpflanzen wie Raps, Soja, Sonnenblume, Färbersaflor nicht synthetisiert. Übliche natürliche Quellen für diese Fettsäuren sind Fische wie Hering, Lachs, Sardine, Goldbarsch, Aal, Karpfen, Forelle, Heilbutt, Makrele, Zander oder Thunfisch oder Algen.

Je nach Anwendungszweck werden Öle mit gesättigten oder ungesättigten Fettsäuren bevorzugt. So werden z.B. in der humanen Ernährung Lipide mit ungesättigten Fettsäuren speziell mehrfach ungesättigten Fettsäuren bevorzugt. Den mehrfach ungesättigten ω-3-Fettsäuren wird dabei ein positiver Effekt auf den Cholesterinspiegel im Blut und damit auf die Möglichkeit der Prävention einer Herzerkrankung zugeschrieben. Durch Zugabe dieser ω-3-Fettsäuren zur Nahrung kann das Risiko einer Herzerkrankung, eines Schlaganfalls oder von Bluthochdruck deutlich verringert werden. Auch entzündliche speziell chronisch entzündliche Prozesse im Rahmen immunologischer Erkrankungen wie rheumatroider Arthritis lassen sich durch ω-3-Fettsäuren positiv beeinflussen. Sie werden deshalb Lebensmitteln speziell diätischen Lebensmitteln zugegeben oder finden in Medikamenten Anwendung. ω-6-Fettsäuren wie Arachidonsäure

haben bei diesen rheumatischen Erkrankungen aufgrund unserer üblichen Nahrungsmittelzusammensetzung eher einen negativen Effekt auf diese Krankheiten.

Aufgrund ihrer positiven Eigenschaften hat es in der Vergangenheit nicht an Ansätzen gefehlt, Gene, die an der Synthese von Fettsäuren bzw. Triglyceriden beteiligt sind, für die Herstellung von Ölen in verschiedenen Organismen mit geändertem Gehalt an ungesättigten Fettsäuren verfügbar zu machen. So wird in WO 91/13972 und seinem US-Äquivalent eine Δ-9-Desaturase beschrieben. In WO 93/11245 wird eine Δ-15-Desaturase in WO 94/11516 wird eine Δ-12-Desaturase beansprucht. Weitere Desaturasen werden beispielsweise in EP-A-0 550 162, WO 94/18337, WO 97/30582, WO 97/21340, WO 95/18222, EP-A-0 794 250, Stukey et al., J. Biol. Chem., 265, 1990: 20144-20149, Wada et al., Nature 347, 1990: 200-203 oder Huang et al., Lipids 34, 1999: 649-659 beschrieben. Die biochemische Charakterisierung der verschiedenen Desaturasen ist jedoch bisher nur unzureichend erfolgt, da die Enzyme als membrangebundene Proteine nur sehr schwer zu isolieren und zu charakterisieren sind (McKeon et al., Methods in Enzymol. 71, 1981: 12141-12147, Wang et al., Plant Physiol. Biochem., 26, 1988: 777-792). In der Regel erfolgt die Charakterisierung membrangebundener Desaturasen durch Einbringung in einen geeigneten Organismus, der anschließend auf Enzymaktivität mittels Edukt- und Produktanalyse untersucht wird. Δ-6-Desaturasen werden in WO 93/06712, US 5,614,393, US5614393, WO 96/21022, WO00/21557 und WO 99/27111 beschrieben. Die Anwendung zur Produktion in transgenen Organismen wird in WO98/46763 WO98/46764, WO9846765 beschrieben. Die Expression verschiedener Desaturasen wird in WO99/64616 oder WO98/46776 beschrieben und beansprucht. Bzgl. der Effektivität der Expression von Desaturasen und ihrem Einfluss auf die Bildung mehrfach ungesättigter Fettsäuren ist anzumerken, dass durch Expression einer einzelnen Desaturase wie bisher beschrieben lediglich geringe Gehalte an ungesättigten Fettsäuren/Lipiden wie z.B. γ-Linolensäure und Stearidonsäure erreicht wurden.

In der Vergangenheit wurden zahlreiche Versuche unternommen, Elongase-Gene zu erhalten. Millar and Kunst, 1997 (Plant Journal 12:121-131) und Millar et al., 1999 (Plant Cell 11:825-838) beschreiben die Charakterisierung von pflanzlichen Elongasen zur Synthese von einfach ungesättigten langkettigen Fettsäuren (C22:1) bzw. zur Synthese von sehr langkettigen Fettsäuren für die Wachsbildung in Pflanzen ($C_{28}$-$C_{32}$). Beschreibungen zur Synthese von Arachidonsäure und EPA finden sich beispielsweise in WO 01/59128, WO 00/12720, WO 02/077213 und WO 02/08401. Die Synthese von mehrfach ungesättigter C24-Fettsäuren ist beispielsweise in Tvrdik et al. 2000, J. Cell Biol. 149:707-718 oder WO 02/44320 beschrieben.

Besonders geeignete Mikroorganismen zur Herstellung von PUFAs sind Mikroorganismen wie Mikroalgen wie Phaeodactylum tricornutum, Porphiridium-Arten, Thraustochytrien-Arten, Schizochytrien-Arten oder Crypthecodinium-Arten, Ciliaten, wie Stylonychia oder Colpidium, Pilze, wie Mortierella, Entomophthora oder Mucor und/oder Moosen wie Physcomitrella, Ceratodon und Marchantia (R. Vazhappilly & F. Chen (1998) Botanica Marina 41: 553-558; K. Totani & K. Oba (1987) Lipids 22: 1060-1062; M. Akimoto et al. (1998) Appl. Biochemistry and Biotechnology 73: 269-278). Durch Stammselektion ist eine Anzahl von Mutantenstämmen der entsprechenden Mikroorganismen entwickelt worden, die eine Reihe wünschenswerter Verbindungen, einschließlich PUFAs, produzieren. Die Mutation und Selektion von Stämmen mit verbesserter Produktion eines bestimmten Moleküls wie den mehrfach ungesättigten Fettsäuren ist jedoch ein zeitraubendes und schwieriges Verfahren. Deshalb werden, wann immer möglich wie oben beschrieben gentechnologische Verfahren bevorzugt. Mit Hilfe der vorgenannten Mikroorganismen lassen sich jedoch nur begrenzte Mengen der gewünschten mehrfach ungesättigten Fettsäuren wie DPA, EPA oder ARA herstellen. Wobei diese in der Regel je nach verwendeten Mikroorganismus als Fettsäuregemische aus beispielsweise EPA, DPA und ARA anfallen.

Höhere Pflanzen enthalten mehrfach ungesättigte Fettsäuren wie Linolsäure (C18:2) und Linolensäure (C18:3). ARA, EPA und DHA kommen im Samenöl höherer Pflanzen gar nicht oder nur in Spuren vor (E. Ucciani: Nouveau Dictionnaire des Huiles Végétales. Technique & Documentation - Lavoisier, 1995. ISBN: 2-7430-0009-0). Es wäre jedoch vorteilhaft, in höheren Pflanzen, bevorzugt in Ölsaaten wie Raps, Lein, Sonnenblume und Soja, LCPUFAs herzustellen, da auf diese Weise große Mengen qualitativ hochwertiger LCPUFAs für die Lebensmittelindustrie, die Tierernährung und für pharmazeutische Zwecke kostengünstig gewonnen werden können. Hierzu werden vorteilhafterweise über gentechnische Methoden Gene, die für Enzyme der Biosynthese von LCPUFAs kodieren, in Ölsaaten eingeführt und exprimiert, vorteilhaft im Samen exprimiert. Dies sind Gene, die beispielsweise für Δ-6-Desaturasen, Δ-6-Elongasen, Δ-5-Desaturasen, Δ-5-Elongasen oder Δ-4-Desaturasen kodieren. Diese Gene können vorteilhaft aus Mikroorganismen und niederen Pflanzen isoliert werden, die LCPUFAs herstellen und in den Membranen oder Triacylglyceriden einbauen. So konnten bereits Δ-6-Desaturase-Gene aus dem Moos Physcomitrella patens und Δ-6-Elongase-Gene aus P. patens und dem Nematoden C. elegans isoliert werden.

Für die Synthese von Arachidonsäure, Eicosapentaensäure (EPA) und Docosahexaensäure (DHA) werden verschiedene Synthesewege diskutiert (Figur. 1). So erfolgt die Produktion von EPA bzw. DHA in marinen Bakterien wie Vibrio sp. oder Shewanella sp. nach dem Polyketid-Weg (Yu, R. et al. Lipids 35:1061-1064, 2000; Takeyama, H. et al. Microbiology 143:2725-2731, 1997).

Ein alternative Strategie verläuft über die wechselnde Aktivität von Desaturasen und Elongasen (Zank, T.K. et al. Plant Journal 31:255-268, 2002; Sakuradani, E. et al. Gene 238:445-453, 1999). Eine Modifikation des beschriebenen Weges über Δ6-Desaturase, Δ6-Elongase, Δ5-Desaturase, Δ5-Elongase, Δ4-Desaturase ist der Sprecher-Syntheseweg

(Sprecher 2000, Biochim. Biophys. Acta 1486:219-231) in Säugetieren. Anstelle der Δ4-Desaturierung erfolgt hier ein weiterer Elongationsschritt auf $C_{24}$, eine weitere Δ6-Desaturierung und abschliessend eine β-Oxidation auf die $C_{22}$-Kettenlänge. Für die Herstellung in Pflanzen und Mikroorganismen ist der sogenannte Sprecher-Syntheseweg (siehe Figur 1) allerdings nicht geeignet, da die Regulationsmechanismen nicht bekannt sind.

Die polyungesättigten Fettsäuren können entsprechend ihrem Desaturierungsmuster in zwei große Klassen, in ω-6- oder ω-3-Fettsäuren eingeteilt werden, die metabolisch und funktionell unterschiedlich Aktivitäten haben (Fig. 1).

Als Ausgangsprodukt für den ω-6-Stoffwechselweg fungiert die Fettsäure Linolsäure ($18:2^{\Delta 9,12}$), während der ω-3-Weg über Linolensäure ($18:3^{\Delta 9,12,15}$) abläuft. Linolensäure wird dabei durch Aktivität einer ω-3-Desaturase gebildet (Tocher et al. 1998, Prog. Lipid Res. 37, 73-117 ; Domergue et al. 2002, Eur. J. Biochem. 269, 4105-4113).

Säugetiere und damit auch der Mensch verfügen über keine entsprechende Desaturaseaktivität (Δ-12- und ω-3-Desaturase) und müssen diese Fettsäuren (essentielle Fettsäuren) über die Nahrung aufnehmen. Über die Abfolge von Desaturase- und Elongase-Reaktionen werden dann aus diesen Vorstufen die physiologisch wichtigen polyungesättigten Fettsäuren Arachidonsäure (= ARA, $20:4^{\Delta 5,8,11,14}$), eine ω-6-Fettsäure und die beiden ω-3-Fettsäuren Eicosapentaen- (= EPA, $20:5^{\Delta 5,8,11,14,17}$) und Docosahexaensäure (DHA, $22:6^{\Delta 4,7,10,13,17,19}$) synthetisiert. Die Applikation von ω-3-Fettsäuren zeigt dabei die wie oben beschrieben therapeutische Wirkung bei der Behandlung von Herz-Kreislaufkrankheiten (Shimikawa 2001, World Rev. Nutr. Diet. 88, 100-108), Entzündungen (Calder 2002, Proc. Nutr. Soc. 61, 345-358) und Arthridis (Cleland und James 2000, J. Rheumatol. 27, 2305-2307).

Aus ernährungsphysiologischer Sicht ist es deshalb günstig eine Verschiebung zwischen dem ω-6-Syntheseweg und dem ω-3-Syntheseweg (siehe Figur 1) zu erreichen, so dass mehr ω-3-Fettsäuren hergestellt werden. In der Literatur wurden die enzymatischen Aktivitäten verschiedener ω-3-Desaturasen beschrieben, die $C_{18:2}$-, C22:4- oder $C_{22:5}$-Fettsäuren desaturieren (siehe Figur 1). Keine der biochemisch beschriebenen Desaturasen setzt jedoch ein breites Substratspektrum des ω-6-Synthesewegs zu den entsprechenden Fettsäuren des ω-3-Syntheseweg um.

Die Verlängerung von Fettsäuren durch Elongasen um 2 bzw. 4 C-Atome ist für die Produktion von $C_{20}$- bzw. $C_{22}$-PUFAs von entscheidender Bedeutung. Dieser Prozess verläuft über 4 Stufen. Der erste Schritt stellt die Kondensation von Malonyl-CoA an das Fettsäure-Acyl-CoA durch die Ketoacyl-CoA-Synthase (KCS, im weiteren Text als Elongase bezeichnet). Es folgt dann ein Reduktionschritt (Ketoacyl-CoA-Reduktase, KCR), ein Dehydratationsschritt (Dehydratase) und ein abschliessender Reduktionsschritt (enoyl-CoA-Reduktase). Es wurde postuliert, dass die Aktivität der Elongase die Spezifität und Geschwindigkeit des gesamten Prozesses beeinflussen (Millar and Kunst, 1997 Plant Journal 12:121-131).

Zur Herstellung von DHA (C22:6 n-3) in Organismen, die diese Fettsäure natürlicherweise nicht produzieren, wurde bisher keine spezifische Elongase beschrieben. Bisher wurden nur Elongasen beschrieben, die $C_{20}$- bzw. $C_{24}$-Fettsäuren bereitstellen. Eine Δ-5-Elongase-Aktivität wurde bisher noch nicht beschrieben.

Erste transgene Pflanzen, die für Enzyme der LCPUFA-Biosynthese kodierende Gene enthalten und exprimieren und als Folge dessen LCPUFAs produzieren, wurden beispielsweise in DE 102 19 203 (Verfahren zur Herstellung mehrfach ungesättigter Fettsäuren in Pflanzen) oder WO 2004/071467 beschrieben. Diese Pflanzen produzieren allerdings LCPUFAs in Mengen, die für eine Aufarbeitung der in den Pflanzen enthaltenen Öle noch weiter optimiert werden müssen. So beträgt der Gehalt von ARA in den in DE-A-102 19 203 beschriebenen Pflanzen lediglich 0,4 bis 2% und der Gehalt von EPA lediglich 0,5 bis 1%, jeweils bezogen auf den Gesamtlipidgehalt der Pflanze. In WO 2004/071467 werden höhere Gehalte an mehrfach ungesättigten $C_{20}$- und $C_{22}$-Fettsäuren, wie ARA, EPA oder DHA offenbart. Jedoch weist das offenbarte Verfahren einige gravierende Nachteile auf. DHA lässt sich im offenbarten Verfahren offenbar überhaupt nicht im Samen nachweisen. Für eine Herstellung von PUFAs ist Soja aufgrund des geringen Ölgehalts von ca. nur 20 Gew.-% weniger geeignet. Soja ist eine vorteilhafte Proteinquelle und wird deshalb in großem Umfang angebaut. Der Ölgehalt von Soja ist jedoch eher gering. Weiterhin ist der im Herstellungsverfahren erzielte Gehalt an Dihomo-y-linolensäure (=DGHL oder HGLA) viel zu hoch. In Fischoder Algenölen oder mikrobiellen Ölen ist HGLA kaum nachweisbar. Ein weiterer Nachteil ist, dass die in WO 2004/071467 offenbarten Pflanzen durch Cotransformation erzeugt wurden, dies führt zur Aufspaltung der Eigenschaften in den folgenden Generationen und damit zu einem erhöhten Selektionsaufwand.

Um eine Anreicherung der Nahrung und/oder des Futters mit diesen mehrfach ungesättigten Fettsäuren zu ermöglichen, besteht daher nachwievor ein großer Bedarf an einem einfachen, kostengünstigen Verfahren zur Herstellung dieser mehrfach ungesättigten Fettsäuren in pflanzlichen Systemen speziell im Samen von transgenen Pflanzen.

Daher bestand die Aufgabe der Erfindung darin, ein Verfahren zur Herstellung großer Mengen von mehrfach ungesättigten Fettsäuren, speziell ARA, EPA und DHA, im Samen einer transgenen Pflanze zu entwickeln.

Diese Aufgabe wurde gelöst durch durch das erfindungsgemäße Zurverfügungstellen einer Öl-produzierende Pflanze der Gattung Brassica, wobei die Pflanze unter Kontrolle von Samen-spezifischen Promotoren oder Promotoren aktiv in Pflanzenembryo oder Endosperm

a) eine delta-6 Desaturase,
b) eine delta-6 Elongase,

c) eine delta-5 Desaturase,
d) eine delta-12 Desaturase, und
e) eine delta-5 Elongase

- Aktivität aufweist, wobei die Pflanze Öl mit DHA und/oder EPA in den Samen herstellt.

Hierin beschrieben ist auch ein Verfahren zur Herstellung von Verbindungen der allgemeinen Formel I

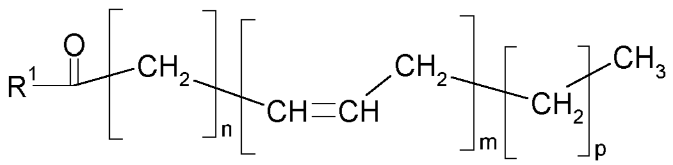

(I)

im Samen von transgenen Pflanzen mit einem Gehalt von mindestens 20 Gew.-% bezogen auf den Gesamtlipidgehalt, dass es folgende Verfahrensschritte umfasst:

a) Einbringen mindestens einer Nukleinsäuresequenz in den Organismus, welche für eine Δ-9-Elongase- und/oder eine Δ-6-Desaturase-Aktivität codiert, und

b) Einbringen mindestens einer Nukleinsäuresequenz in den Organismus, welche für eine Δ-8-Desaturase- und/oder eine Δ-6-Elongase-Aktivität codiert, und

c) Einbringen mindestens einer Nukleinsäuresequenz in den Organismus, welche für eine Δ-5-Desaturase-Aktivität codiert, und

d) Einbringen mindestens einer Nukleinsäuresequenz in den Organismus, welche für eine Δ-5-Elongase-Aktivität codiert, und

e) Einbringen mindestens einer Nukleinsäuresequenz in den Organismus, welche für eine Δ-4-Desaturase-Aktivität codiert, und

wobei die Variablen und Substituenten in der Formel I die folgende Bedeutung haben:

$R^1$ = Hydroxyl-, CoenzymA-(Thioester), Lyso-Phosphatidylcholin-, Lyso-Phosphatidylethanolamin-, Lyso-Phosphatidylglycerol-, Lyso-Diphosphatidylglycerol-, Lyso-Phosphatidylserin-, Lyso-Phosphatidylinositol-, Sphingobase-, oder einen Rest der allgemeinen Formel II

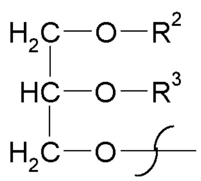

(II)

$R^2$ = Wasserstoff-, Lyso-Phosphatidylcholin-, Lyso-Phosphatidylethanolamin-, Lyso-Phosphatidylglycerol-, Lyso-Diphosphatidylglycerol-, Lyso-Phosphatidylserin-, Lyso-Phosphatidylinositol- oder gesättigtes oder ungesättigtes $C_2$-$C_{24}$-Alkylcarbonyl-,

$R^3$ = Wasserstoff-, gesättigtes oder ungesättigtes $C_2$-$C_{24}$-Alkylcarbonyl-, oder $R^2$ oder $R^3$ unabhängig voneinander einen Rest der allgemeinen Formel Ia:

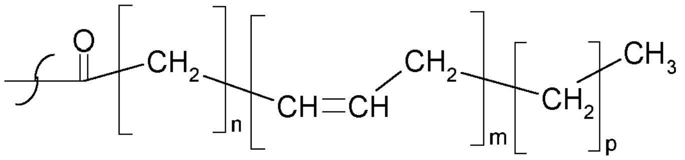

(Ia)

n = 2, 3, 4, 5, 6, 7 oder 9, m = 2, 3, 4, 5 oder 6 und p = 0 oder 3, gelöst. Vorteilhaft bedeuten die Variablen n, m und p in den vorgenannten Formel I und Ia folgendes: n = 2, 3 oder 5, m = 4, 5 oder 6 und p = 0 oder 3. In einer besonders vorteilhaften Ausführung des Verfahrens bedeuten die Variable n, m und p in den Formeln I und Ia das folgende: m = 4, n = 3, p = 3 und die Verbindungen der allgemeinen Formel I und Ia bedeuten damit Arachidonsäure und/oder m = 5, n = 3, p = 0 und die Verbindungen der allgemeinen Formel I und Ia bedeuten damit Eicosapentaensäure und/oder m = 5, n = 5, p = 0 und die Verbindungen der allgemeinen Formel I und Ia bedeuten damit Docosapentaensäure ist und/oder m = 6, n = 3, p = 0 und die Verbindungen der allgemeinen Formel I und Ia bedeuten damit Docosahexaensäure ist.

$R^1$ bedeutet in der allgemeinen Formel I Hydroxyl-, CoenzymA-(Thioester), Lyso-Phosphatidylcholin-, Lyso-Phosphatidylethanolamin-, Lyso-Phosphatidylglycerol-, Lyso-Diphosphatidylglycerol-, Lyso-Phosphatidylserin-, Lyso-Phosphatidylinositol-, Sphingobase-, oder einen Rest der allgemeinen Formel II

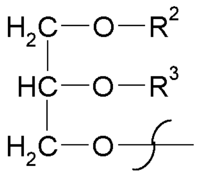

(II)

Die oben genannten Reste von $R^1$ sind immer in Form ihrer Thioester an die Verbindungen der allgemeinen Formel I gebunden.

$R^2$ bedeutet in der allgemeinen Formel II Wasserstoff-, Lyso-Phosphatidylcholin-, Lyso-Phosphatidylethanolamin-, Lyso-Phosphatidylglycerol-, Lyso-Diphosphatidylglycerol-, Lyso-Phosphatidylserin-, Lyso-Phosphatidylinositol-oder gesättigtes oder ungesättigtes C2-C24-Alkylcarbonyl-,

Als Alkylreste seien substituiert oder unsubstituiert, gesättigt oder ungesättigte $C_2$-$C_{24}$-Alkylcarbonyl-Ketten wie Ethylcarbonyl-, n-Propylcarbonyl-, n-Butylcarbonyl-, n-Pentylcarbonyl-, n-Hexylcarbonyl-,n-Heptylcarbonyl-, n-Octylcarbonyl-, n-Nonylcarbonyl-, n-Decylcarbonyl-, n-Undecylcarbonyl-, n-Dodecylcarbonyl-, n-Tridecylcarbonyl-, n-Tetradecylcarbonyl-, n-Pentadecylcarbonyl-, n-Hexadecylcarbonyl-, n-Heptadecylcarbonyl-, n-Octadecylcarbonyl-, n-Nonadecylcarbonyl-, n-Eicosylcarbonyl-, n-Docosanylcarbonyl- or n-Tetracosanylcarbonyl- genannt, die ein oder mehrere Doppelbindungen enthalten. Gesättigte oder ungesättigte $C_{10}$-$C_{22}$-Alkylcarbonylreste wie n-Decylcarbonyl-, n-Undecylcarbonyl-, n-Dodecylcarbonyl-, n-Tridecylcarbonyl-, n-Tetradecylcarbonyl-, n-Pentadecylcarbonyl-, n-Hexadecylcarbonyl-, n-Heptadecylcarbonyl-, n-Octadecylcarbonyl-, n-Nonadecylcarbonyl-, n-Eicosylcarbonyl-, n-Docosanylcarbonyl-oder n-Tetracosanylcarbonyl-., die ein oder mehrere Doppelbindungen enthalten, sind bevorzugt. Besonders bevorzugt sind gesättigte und/oder ungesättigte $C_{10}$-$C_{22}$-Alkylcarbonylreste wie $C_{10}$-Alkylcarbonyl-, $C_{11}$-Alkylcarbonyl-, $C_{12}$-Alkylcarbonyl-, $C_{13}$-Alkylcarbonyl-, $C_{14}$-Alkylcarbonyl-, $C_{16}$-Alkylcarbonyl-, $C_{18}$-Alkylcarbonyl-, $C_{20}$-Alkylcarbonyl- oder $C_{22}$-Alkylcarbonylreste, die ein oder mehrere Doppelbindungen enthalten. Ganz besonders bevorzugt sind gesättigte oder ungesättigte $C_{16}$-$C_{22}$-Alkylcarbonylreste wie $C_{16}$-Alkylcarbonyl-, $C_{18}$-Alkylcarbonyl-, $C_{20}$-Alkylcarbonyl- oder $C_{22}$-Alkylcarbonylreste, die ein oder mehrere Doppelbindungen enthalten. Diese vorteilhaften Reste können zwei, drei, vier, fünf oder sechs Doppelbindungen enthalten. Die besonders vorteilhaften Reste mit 20 oder 22 Kohlenstoffatomen in der Fettsäurekette enthalten bis zu sechs Doppelbindungen, vorteilhaft drei, vier, fünf oder sechs Doppelbindungen, besonders bevorzugt vier, fünf oder sechs Doppelbindungen, ganz besonders bevorzugt fünf oder sechs. Alle genannten Reste leiten sich von den entsprechenden Fettsäuren ab.

$R^3$ bedeutet in der allgemeinen Formel II Wasserstoff-, gesättigtes oder ungesättigtes C2-C24-Alkylcarbonyl.

Als Alkylreste seien substituiert oder unsubstituiert, gesättigt oder ungesättigte $C_2$-$C_{24}$-Alkylcarbonyl-Ketten wie Ethylcarbonyl-, n-Propylcarbonyl-, n-Butylcarbonyl-, n-Pentylcarbonyl-, n-Hexylcarbonyl-,n-Heptylcarbonyl-, n-Octylcarbonyl-, n-Nonylcarbonyl-, n-Decylcarbonyl-, n-Undecylcarbonyl-, n-Dodecylcarbonyl-, n-Tridecylcarbonyl-, n-Tetradecylcarbonyl-, n-Pentadecylcarbonyl-, n-Hexadecylcarbonyl-, n-Heptadecylcarbonyl-, n-Octadecylcarbonyl-, n-Nonadecylcarbonyl-, n-Eicosylcarbonyl-, n-Docosanylcarbonyl- or n-Tetracosanylcarbonyl- genannt, die ein oder mehrere Doppelbindungen enthalten. Gesättigte oder ungesättigte $C_{10}$-$C_{22}$-Alkylcarbonylreste wie n-Decylcarbonyl-, n-Undecylcarbonyl-, n-Dodecylcarbonyl-, n-Tridecylcarbonyl-, n-Tetradecylcarbonyl-, n-Pentadecylcarbonyl-, n-Hexadecylcarbonyl-, n-Heptadecylcarbonyl-, n-Octadecylcarbonyl-, n-Nonadecylcarbonyl-, n-Eicosylcarbonyl-, n-Docosanylcarbonyl-oder n-Tetracosanylcarbonyl-, die ein oder mehrere Doppelbindungen enthalten, sind bevorzugt. Besonders bevorzugt

sind gesättigte und/oder ungesättigte $C_{10}$-$C_{22}$-Alkylcarbonylreste wie $C_{10}$-Alkylcarbonyl-, $C_{11}$-Alkylcarbonyl-, $C_{12}$-Alkylcarbonyl-, $C_{13}$-Alkylcarbonyl-, $C_{14}$-Alkylcarbonyl-, $C_{16}$-Alkylcarbonyl-, $C_{18}$-Alkylcarbonyl-, $C_{20}$-Alkylcarbonyl- oder $C_{22}$-Alkylcarbonylreste, die ein oder mehrere Doppelbindungen enthalten. Ganz besonders bevorzugt sind gesättigte oder unge sättigte $C_{16}$-$C_{22}$-Alkylcarbonylreste wie $C_{16}$-Alkylcarbonyl-, $C_{18}$-Alkylcarbonyl-, $C_{20}$-Alkylcarbonyl- oder $C_{22}$-Alkylcarbonylreste, die ein oder mehrere Doppelbindungen enthalten. Diese vorteilhaften Reste können zwei, drei, vier, fünf oder sechs Doppelbindungen enthalten. Die besonders vorteilhaften Reste mit 20 oder 22 Kohlenstoffatomen in der Fettsäurekette enthalten bis zu sechs Doppelbindungen, vorteilhaft drei, vier, fünf oder sechs Doppelbindungen, besonders bevorzugt vier, fünf oder sechs Doppelbindungen, ganz besonders bevorzugt fünf oder sechs.. Alle genannten Reste leiten sich von den entsprechenden Fettsäuren ab.

Die oben genannten Reste von $R^1$, $R^2$ and $R^3$ können mit Hydroxyl- und/oder Epoxygruppen substituierte sein und/oder können Dreifachbindungen enthalten.

Die im erfindungsgemäßen Verfahren hergestellten mehrfach ungesättigten Fettsäuren mindestens zwei vorteilhaft drei, vier, fünf oder sechs Doppelbindungen. Besonders vorteilhaft enthalten die Fettsäuren vier fünf oder sechs Doppelbindungen. Im Verfahren hergestellte Fettsäuren haben vorteilhaft 18-, 20- oder 22-C-Atome in der Fettsäurekette, bevorzugt enthalten die Fettsäuren 20 oder 22 Kohlenstoffatome in der Fettsäurekette. Vorteilhaft werden gesättigte Fettsäuren mit den im Verfahren verwendeten Nukleinsäuren wenig oder gar nicht umgesetzt. Unter wenig ist zu verstehen, das im Vergleich zu mehrfach ungesättigten Fettsäuren die gesättigten Fettsäuren mit weniger als 5 % der Aktivität, vorteilhaft weniger als 3 %, besonders vorteilhaft mit weniger als 2 %, ganz besonders bevorzugt mit weniger als 1; 0,5; 0,25 oder 0,125 % umgesetzt werden. Diese hergestellten Fettsäuren können als einziges Produkt im Verfahren hergestellt werden oder in einem Fettsäuregemisch vorliegen.

Bei den im erfindungsgemäßen Verfahren verwendeten Nukleinsäuresequenzen handelt es sich um isolierte Nukleinsäuresequenzen, die für Polypeptide mit Δ-9-Elongase-, Δ-6-Desaturase-, Δ-8-Desaturase-, Δ-6-Elongase-, Δ-5-Desaturase-, Δ-5-Elongase- und/oder Δ-4-Desaturaseaktivität codieren.

Vorteilhaft werden im erfindungsgemäßen Verfahren Nukleinsäuresequenzen, die für Polypeptide mit Δ-9-Elongase-, Δ-6-Desaturase-, Δ-8-Desaturase-, Δ-6-Elongase-, Δ-5-Desaturase-, Δ-5-Elongase- oder Δ-4-Desaturaseaktivität codieren, verwendet ausgewählt aus der Gruppe bestehend aus:

a) einer Nukleinsäuresequenz mit der in SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137, SEQ ID NO: 183, SEQ ID NO: 193, SEQ ID NO: 197, SEQ ID NO: 199 oder SEQ ID NO: 201 dargestellten Sequenz, oder

b) Nukleinsäuresequenzen, die sich als Ergebnis des degenerierten genetischen Codes von den in SEQ ID NO: 2, SEQ ID NO: 4, SEQ ID NO:6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 60, SEQ ID NO: 62, SEQ ID NO: 64, SEQ ID NO: 66, SEQ ID NO: 68, SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 86, SEQ ID NO: 88, SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 112, SEQ ID NO: 114, SEQ ID NO: 118, SEQ ID NO: 120, SEQ ID NO: 132, SEQ ID NO: 134, SEQ ID NO: 136, SEQ ID NO: 138, SEQ ID NO: 184, SEQ ID NO: 194, SEQ ID NO: 198, SEQ ID NO: 200 oder SEQ ID NO: 202 dargestellten Aminosäuresequenzen ableiten lassen, oder

c) Derivate der in SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 111, SEQ ID NO: 113, SEQ ID NO: 117, SEQ ID NO: 119,

SEQ ID NO: 131, SEQ ID NO: 133, SEQ ID NO: 135, SEQ ID NO: 137, SEQ ID NO: 183, SEQ ID NO: 193, SEQ ID NO: 197, SEQ ID NO: 199 oder SEQ ID NO: 201 dargestellten Nukleinsäuresequenz, die für Polypeptide mit mindestens 40 % Identität auf Aminosäureebene mit SEQ ID NO: 2, SEQ ID NO: 4, SEQ ID NO:6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 60, SEQ ID NO: 62, SEQ ID NO: 64, SEQ ID NO: 66, SEQ ID NO: 68, SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 86, SEQ ID NO: 88, SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 112, SEQ ID NO: 114, SEQ ID NO: 118, SEQ ID NO: 120, SEQ ID NO: 132, SEQ ID NO: 134, SEQ ID NO: 136, SEQ ID NO: 138, SEQ ID NO: 184, SEQ ID NO: 194, SEQ ID NO: 198, SEQ ID NO: 200 oder SEQ ID NO: 202 codieren und eine Δ-9-Elongase-, Δ-6-Desaturase-, Δ-8-Desaturase-, Δ-6-Elongase-, Δ-5-Desaturase-, Δ-5-Elongase- oder Δ-4-Desaturaseaktivität aufweisen.

Vorteilhaft bedeuten die Substituenten $R^2$ oder $R^3$ in den allgemeinen Formeln I und II unabhängig voneinander gesättigtes oder ungesättigtes $C_{18}$-$C_{22}$-Alkylcarbonyl-, besonders vorteilhaft bedeuten sie unabhängig voneinander ungesättigtes $C_{18}$-, $C_{20}$-oder $C_{22}$-Alkylcarbonyl- mit mindestens zwei Doppelbindungen, vorteilhaft mit mindestens drei, vier, fünf oder sechs Doppelbindungen, besonders vorteilhaft mit mindestens vier, fünf oder sechs Doppelbindungen.

Eine bevorzugte Ausführungsform des Verfahrens ist dadurch gekennzeichnet, dass eine Nukleinsäuresequenz zusätzlich in die transgene Pflanze eingebracht wird, die für Polypeptide mit ω-3-Desaturase-Aktivität codiert, ausgewählt aus der Gruppe bestehend aus:

a) einer Nukleinsäuresequenz mit der in SEQ ID NO: 87 oder SEQ ID NO: 105 dargestellten Sequenz, oder

b) Nukleinsäuresequenzen, die sich als Ergebnis des degenerierten genetischen Codes von der in SEQ ID NO: 88 oder SEQ ID NO: 106 dargestellten Aminosäuresequenz ableiten lassen, oder

c) Derivate der in SEQ ID NO: 87 oder SEQ ID NO: 105 dargestellten Nukleinsäuresequenz, die für Polypeptide mit mindestens 60 % Identität auf Aminosäureebene mit SEQ ID NO: 88 oder SEQ ID NO: 106 codieren und eine ω3-Desaturaseaktivität aufweisen.

In einer weiteren bevorzugten Ausführungsform ist das Verfahren dadurch gekennzeichnet, dass eine Nukleinsäuresequenz zusätzlich in die transgene Pflanze eingebracht wird, die für Polypeptide mit Δ-12-Desaturaseaktivität codiert, ausgewählt aus der Gruppe bestehend aus:

a) einer Nukleinsäuresequenz mit der in SEQ ID NO: 107, SEQ ID NO: 109 oder SEQ ID NO: 195 dargestellten Sequenz, oder

b) Nukleinsäuresequenzen, die sich als Ergebnis des degenerierten genetischen Codes von der in SEQ ID NO: 108, SEQ ID NO: 110 oder SEQ ID NO: 196 dargestellten Aminosäuresequenz ableiten lassen, oder

c) Derivate der in SEQ ID NO: 107, SEQ ID NO: 109 oder SEQ ID NO: 195 dargestellten Nukleinsäuresequenz, die für Polypeptide mit mindestens 60 % Identität auf Aminosäureebene mit SEQ ID NO: 108, SEQ ID NO: 110 oder SEQ ID NO: 196 codieren und eine Δ-12-Desaturaseaktivität aufweisen.

Diese vorgenannten Δ-12-Desaturasesequenzen können allein oder in Kombination mit den ω3-Desaturasesequenzen mit den im Verfahren verwendeten Nukleinsäuresequenzen, die für Δ-9-Elongasen, Δ-6-Desaturasen, Δ-8-Desaturasen, Δ-6-Elongasen, Δ-5-Desaturasen, Δ-5-Elongasen und/oder Δ-4-Desaturasen codieren verwendet werden.

Tabelle 1 gibt die Nukleinsäuresequenzen, den Herkunftsorganismus und die Sequenz-I D-Nummer wieder.

| Nr. | Organismus | Aktivität | Sequenznummer |
|---|---|---|---|
| 1. | Euglena gracilis | Δ-8-Desaturase | SEQ ID NO: 1 |
| 2. | Isochrysis galbana | Δ-9-Elongase | SEQ ID NO: 3 |
| 3. | Phaeodactylum tricornutum | Δ-5-Desaturase | SEQ ID NO: 5 |

(fortgesetzt)

| Nr. | Organismus | Aktivität | Sequenznummer |
|---|---|---|---|
| 4. | Ceratodon purpureus | Δ-5-Desaturase | SEQ ID NO: 7 |
| 5. | Physcomitrella patens | Δ-5-Desaturase | SEQ ID NO: 9 |
| 6. | Thraustrochytrium sp. | Δ-5-Desaturase | SEQ ID NO: 11 |
| 7. | Mortierella alpina | Δ-5-Desaturase | SEQ ID NO: 13 |
| 8. | Caenorhabditis elegans | Δ-5-Desaturase | SEQ ID NO: 15 |
| 9. | Borago officinalis | Δ-6-Desaturase | SEQ ID NO: 17 |
| 10. | Ceratodon purpureus | Δ-6-Desaturase | SEQ ID NO: 19 |
| 11. | Phaeodactylum tricornutum | Δ-6-Desaturase | SEQ ID NO: 21 |
| 12. | Physcomitrella patens | Δ-6-Desaturase | SEQ ID NO: 23 |
| 13. | Caenorhabditis elegans | Δ-6-Desaturase | SEQ ID NO: 25 |
| 14. | Physcomitrella patens | Δ-6-Elongase | SEQ ID NO: 27 |
| 15. | Thraustrochytrium sp. | Δ-6-Elongase | SEQ ID NO: 29 |
| 16. | Phytophtora infestans | Δ-6-Elongase | SEQ ID NO: 31 |
| 17. | Mortierella alpina | Δ-6-Elongase | SEQ ID NO: 33 |
| 18. | Mortierella alpina | Δ-6-Elongase | SEQ ID NO: 35 |
| 19. | Caenorhabditis elegans | Δ-6-Elongase | SEQ ID NO: 37 |
| 20. | Euglena gracilis | Δ-4-Desaturase | SEQ ID NO: 39 |
| 21. | Thraustrochytrium sp. | Δ-4-Desaturase | SEQ ID NO: 41 |
| 22. | Thalassiosira pseudonana | Δ-5-Elongase | SEQ ID NO: 43 |
| 23. | Thalassiosira pseudonana | Δ-6-Elongase | SEQ ID NO: 45 |
| 24. | Crypthecodinium cohnii | Δ-5-Elongase | SEQ ID NO: 47 |
| 25. | Crypthecodinium cohnii | Δ-5-Elongase | SEQ ID NO: 49 |
| 26. | Oncorhynchus mykiss | Δ-5-Elongase | SEQ ID NO: 51 |
| 27. | Oncorhynchus mykiss | Δ-5-Elongase | SEQ ID NO: 53 |
| 28. | Thalassiosira pseudonana | Δ-5-Elongase | SEQ ID NO: 59 |
| 29. | Thalassiosira pseudonana | Δ-5-Elongase | SEQ ID NO: 61 |
| 30. | Thalassiosira pseudonana | Δ-5-Elongase | SEQ ID NO: 63 |
| 31. | Thraustrochytrium aureum | Δ-5-Elongase | SEQ ID NO: 65 |
| 32. | Ostreococcus tauri | Δ-5-Elongase | SEQ ID NO: 67 |
| 33. | Ostreococcus tauri | Δ-6-Elongase | SEQ ID NO: 69 |
| 34. | Primula farinosa | Δ-6-Desaturase | SEQ ID NO: 71 |
| 35. | Primula vialii | Δ-6-Desaturase | SEQ ID NO: 73 |
| 36. | Ostreococcus tauri | Δ-5-Elongase | SEQ ID NO: 75 |
| 37. | Ostreococcus tauri | Δ-5-Elongase | SEQ ID NO: 77 |
| 38. | Ostreococcus tauri | Δ-5-Elongase | SEQ ID NO: 79 |
| 39. | Ostreococcus tauri | Δ-6-Elongase | SEQ ID NO: 81 |
| 40. | Thraustrochytrium sp. | Δ-5-Elongase | SEQ ID NO: 83 |
| 41. | Thalassiosira pseudonana | Δ-5-Elongase | SEQ ID NO: 85 |

(fortgesetzt)

| Nr. | Organismus | Aktivität | Sequenznummer |
|---|---|---|---|
| 42. | Phytophtora infestans | ω-3-Desaturase | SEQ ID NO: 87 |
| 43. | Ostreococcus tauri | Δ-6-Desaturase | SEQ ID NO: 89 |
| 44. | Ostreococcus tauri | Δ-5-Desaturase | SEQ ID NO: 91 |
| 45. | Ostreococcus tauri | Δ-5-Desaturase | SEQ ID NO: 93 |
| 46. | Ostreococcus tauri | Δ-4-Desaturase | SEQ ID NO: 95 |
| 47. | Thalassiosira pseudonana | Δ-6-Desaturase | SEQ ID NO: 97 |
| 48. | Thalassiosira pseudonana | Δ-5-Desaturase | SEQ ID NO: 99 |
| 49. | Thalassiosira pseudonana | Δ-5-Desaturase | SEQ ID NO: 101 |
| 50. | Thalassiosira pseudonana | Δ-4-Desaturase | SEQ ID NO: 103 |
| 51. | Thalassiosira pseudonana | ω-3-Desaturase | SEQ ID NO: 105 |
| 52. | Ostreococcus tauri | Δ-12-Desaturase | SEQ ID NO: 107 |
| 53. | Thalassiosira pseudonana | Δ-12-Desaturase | SEQ ID NO: 109 |
| 54. | Ostreococcus tauri | Δ-6-Elongase | SEQ ID NO: 111 |
| 55. | Ostreococcus tauri | Δ-5-Elongase | SEQ ID NO: 113 |
| 56. | Xenopus laevis (BC044967) | Δ-5-Elongase | SEQ ID NO: 117 |
| 57. | Ciona intestinalis (AK112719) | Δ-5-Elongase | SEQ ID NO: 119 |
| 58. | Euglena gracilis | Δ-5-Elongase | SEQ ID NO: 131 |
| 59. | Euglena gracilis | Δ-5-Elongase | SEQ ID NO: 133 |
| 60. | Arabidopsis thaliana | Δ-5-Elongase | SEQ ID NO: 135 |
| 61. | Arabidopsis thaliana | Δ-5-Elongase | SEQ ID NO: 137 |
| 62. | Phaeodactylum tricornutum | Δ-6-Elongase | SEQ ID NO: 183 |
| 63. | Phytium irregulare | Δ-6-Desaturase | SEQ ID NO: 193 |
| 64. | Calendula officinalis | Δ-12-Desaturase | SEQ ID NO: 195 |
| 65. | Ostreococcus tauri | Δ-5-Elongase | SEQ ID NO: 197 |
| 66. | Ostreococcus tauri | Δ-6-Elongase | SEQ ID NO: 199 |
| 67. | Ostreococcus tauri | Δ-6-Desaturase | SEQ ID NO: 201 |

Beschrieben wird auch ein Verfahren zur Herstellung großer Mengen von mehrfach ungesättigten Fettsäuren, speziell ARA und EPA, in einer transgenen Pflanze zu entwickeln. Dieses Verfahren ist ebenfalls zur Herstellung von DHA geeignet. So lassen sich im Verfahren ARA, EPA, DHA oder deren Mischungen hergestellen.

Ebenfalls beschrieben wird ein Verfahren zur Herstellung von Verbindungen der allgemeinen Formel I

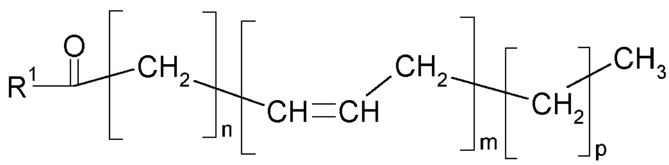

(I)

in transgenen Pflanzen gelöst, wobei das Verfahren umfasst:

a) Einbringen mindestens einer Nukleinsäuresequenz in eine Pflanze, welche für ein Polypeptid mit der Aktivität einer Δ-6-Desaturase-Aktivität kodiert, und ausgewählt ist aus der Gruppe bestehend aus:

i) einer Nukleinsäuresequenz mit der in SEQ ID NO: 193 oder SEQ ID NO: 201 dargestellten Sequenz,
ii) Nukleinsäuresequenzen, die für die in SEQ ID NO: 194 oder SEQ ID NO: 202 angegebene Aminosäuresequenz kodieren,
iii) Nukleinsäuresequenzen, die mit dem komplementären Strang der in SEQ ID NO: 193 oder SEQ ID NO: 201 angegebenen Nukleinsäuresequenz unter stringenten Bedingungen hybridisieren, und
iv) Nukleinsäuresequenzen, die zu der in SEQ ID NO: 193 oder SEQ ID NO: 201 angegebenen Sequenz zu mindestens 60% identisch sind, und

b) Einbringen mindestens einer Nukleinsäuresequenz in eine Pflanze, welche für ein Polypeptid mit einer Δ-6-Elongase-Aktivität kodiert, und ausgewählt ist aus der Gruppe bestehend aus:

i) einer Nukleinsäuresequenz mit der in SEQ ID NO: 27 oder SEQ ID NO: 199 dargestellten Sequenz,
ii) Nukleinsäuresequenzen, die für die in SEQ ID NO: 28 oder SEQ ID NO: 200 angegebene Aminosäuresequenz kodieren,
iii) Nukleinsäuresequenzen, die mit dem komplementären Strang der in SEQ ID NO: 27 oder SEQ ID NO: 199 angegebenen Nukleinsäuresequenz unter stringenten Bedingungen hybridisieren, und
iv) Nukleinsäuresequenzen, die zu der in SEQ ID NO: 27 oder SEQ ID NO: 199 angegebenen Sequenz zu mindestens 60% identisch sind,

c) Einbringen mindestens einer Nukleinsäuresequenz in eine Pflanze, welche für ein Polypeptid mit einer Δ-5-Desaturase-Aktivität kodiert, und ausgewählt ist aus der Gruppe bestehend aus:

i) einer Nukleinsäuresequenz mit der in SEQ ID NO: 11 dargestellten Sequenz,
ii) Nukleinsäuresequenzen, die für die in SEQ ID NO: 12 angegebene Aminosäuresequenz kodieren,
iii) Nukleinsäuresequenzen, die mit dem komplementären Strang der in SEQ ID NO: 11 angegebenen Nukleinsäuresequenz unter stringenten Bedingungen hybridisieren, und
iv) Nukleinsäuresequenzen, die zu der in SEQ ID NO: 11 angegebenen Sequenz zu mindestens 60% identisch sind,

wobei die Variablen und Substituenten in der Formel I die oben genannte Bedeutung haben.

Die im erfindungsgemäßen Verfahren verwendbaren Nukleinsäuresequenzen sind beschrieben in WO 02/26946 (Δ-5-Desaturase aus Thraustochytrium ssp., SEQ ID NO: 11 und Δ-6-Desaturase aus Phytium irregulare, SEQ ID NO: 193) sowie in WO 01/59128 (Δ-6-Elongase aus Physcomitrella patens, SEQ ID NO: 27), auf die hier ausdrücklich Bezug genommen wird. Allerdings wurde in diesen Fällen die Bildung von ARA und EPA entweder nicht in transgenen Pflanzen, sondern lediglich in Mikroorganismen untersucht, oder es konnte keine Steigerung der ARA- und EPA-Synthese in den transgenen Pflanzen nachgewiesen werden. Darüber hinaus wurden in diesen Anmeldungen die erfindungsgemäßen Nukleinsäuren nicht mit Nukleinsäuren, die für andere Enzyme des Fettsäuresynthesewegs kodieren, kombiniert.

Es wurde nun überraschend gefunden, dass die Co-Expression der Nukleinsäuren mit den in SEQ ID NO: 11, 27, 193, 199 und 201 angegebenen Sequenzen in transgenen Pflanzen zu einer starken Erhöhung des ARA-Gehalts auf bis zu mehr als 8%, vorteilhaft bis zu mehr als 10%, 11%, 12%, 13%, 14%, 15%, 16%, 17%, 18%, 19% oder 20%, besonders vorteilhaft auf mehr als 21 %, 22%, 23%, 24% oder 25%, bezogen auf den gesamten Lipidgehalt der Pflanze, führt (vgl. Tabelle 2, Tabelle 3, Tabelle 4 und Figur 31). Bei den vorgenannten Prozentwerten handelt es sich um Gewichtsprozentangaben.

Zur weiteren Steigerung der Ausbeute im beschriebenen Verfahren zur Herstellung von Ölen und/oder Triglyceriden mit einem vorteilhaft gegenüber Ölen und/oder Triglyceriden aus Wildtyp-Pflanzen erhöhten Gehalt an mehrfach ungesättigten Fettsäuren, vor allem von ARA, EPA oder DHA oder deren Mischungen, kann es vorteilhaft sein, die Menge des Ausgangsstoffs für die Fettsäuresynthese zu steigern. Dies kann beispielsweise durch das Einbringen einer Nukleinsäure, die für ein Polypeptid mit der Aktivität einer Δ-12-Desaturase kodiert, und deren Co-Expression in dem Organismus erreicht werden.

Dies ist besonders vorteilhaft in Öl-produzierenden Organismen wie der Familie der Brassicaceae wie der Gattung Brassica, z.B. Raps, Rübsen oder Sareptasenf; der Familie der Elaeagnaceae wie die Gattung Elaeagnus z.B. die Gattung und Art Olea *europaea* oder der Familie Fabaceae wie der Gattung Glycine z.B. die Gattung und Art *Glycine max,* die einen hohen Ölsäuregehalt, aber nur einen geringen Gehalt an Linolsäure aufweisen (Mikoklajczak et al., Journal of the American Oil Chemical Society, 38, 1961, 678 - 681).

Daher wird in einer bevorzugten Ausführungsform der vorliegenden Erfindung zusätzlich eine Nukleinsäuresequenz in die transgene Pflanze eingebracht, die für ein Polypeptid mit Δ-12-Desaturaseaktivität kodiert.

Besonders bevorzugt ist diese Nukleinsäuresequenz ausgewählt aus der Gruppe bestehend aus:

a) einer Nukleinsäuresequenz mit der in SEQ ID NO: 195 dargestellten Sequenz,

b) Nukleinsäuresequenzen, die für die in SEQ ID NO: 196 dargestellte Aminosäuresequenz kodieren,

c) Nukleinsäuresequenzen, die mit dem komplementären Strang der in SEQ ID NO: 195 angegebenen Nukleinsäuresequenz unter stringenten Bedingungen hybridisieren, und

d) Nukleinsäuresequenzen, die zu der in SEQ ID NO: 195 angegebenen Sequenz zu mindestens 60% identisch sind.

Die Nukleinsäuresequenz mit der SEQ ID NO: 195 stammt aus Calendula officinalis und ist beschrieben in WO 01/85968, deren Offenbarung hier ebenfalls durch Bezugnahme in die vorliegende Anmeldung mit aufgenommen ist.

Vorteilhaft setzen die im erfingungsgemäßen Verfahren verwendeten Δ-12-Desaturasen Ölsaure (C18:$1^{\Delta 9}$) zu Linolsäure (C18:$2^{\Delta 9,12}$) oder C18:$2^{\Delta 6,9}$ zu C18:$3^{\Delta 6,9,12}$ (Gammalinolensäure = GLA), den Ausgangssubstanzen für die Synthese von ARA, EPA und DHA um. Vorteilhaft setzen die verwendeten Δ-12-Desaturasen Fettsäuren gebunden an Phospholipide oder CoA-Fettsäureester, vorteilhaft gebunden an CoA-Fettsäureester, um. Dies führt, wenn vorher ein Elongationsschritt stattgefunden hat, vorteilhaft zu höheren Ausbeuten an Syntheseprodukten, da die Elongation in der Regel an CoA-Fettsäureestern erfolgt, während die Desaturierung überwiegend an den Phospholipiden oder an den Triglyceriden erfolgt. Ein Ausstausch, der eine weitere möglicherweise limitierende Enzymreaktion erfoderlich machen würde, zwischen den CoA-Fettsäureestern und den Phospholipiden oder Triglyceriden ist somit nicht erforderlich.

Die zusätzliche Expression der Δ-12-Desaturase in den transgenen Pflanzen führt zu einer weiteren Steigerung des ARA-Gehalts auf bis zu mehr als 10%, 11%, 12%, 13%, 14%, 15%, 16%, 17%, 18%, 19% oder 20%, besonders vorteilhaft auf mehr als 21 %, 22%, 23%, 24% oder 25%, bezogen auf den gesamten Lipidgehalt der Pflanze (vgl. Tabelle 3 und 4 und Figur 32). Bei den vorgenannten Prozentwerten handelt es sich um Gewichtsprozentangaben.

Vorteilhaft können im erfindungsgemäßen Verfahren weitere Nukleinsäuresequenzen in die Pflanzen eingebracht werden, die für ein Polypeptid mit einer Δ-5-Elongase-Aktivität kodieren.

Bevorzugt werden derartige Nukleinsäuresequenzen, die für Δ-5-Elongaseaktivität kodieren, ausgewählt ist aus der Gruppe bestehend aus:

a) einer Nukleinsäuresequenz mit der in SEQ ID NO: 43, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 75, SEQ ID NO: 77, SEQ ID NO: 79, SEQ ID NO: 83, SEQ ID NO: 85, SEQ ID NO: 113, SEQ ID NO: 117, SEQ ID NO: 119, SEQ ID NO: 131, SEQ ID NO: 133, SEQ ID NO: 135, SEQ ID NO: 137 oder SEQ ID NO: 197 dargestellten Sequenz,

b) Nukleinsäuresequenzen, die für die in SEQ ID NO: 44, SEQ ID NO: 48, SEQ ID NO: 50, SEQ ID NO: 52, SEQ ID NO: 54, SEQ ID NO: 60, SEQ ID NO: 62, SEQ ID NO: 64, SEQ ID NO: 66, SEQ ID NO: 68, SEQ ID NO: 76, SEQ ID NO: 78, SEQ ID NO: 80, SEQ ID NO: 81, SEQ ID NO: 86, SEQ ID NO: 114, SEQ ID NO: 118, SEQ ID NO: 120, SEQ ID NO: 132, SEQ ID NO: 134, SEQ ID NO: 136, SEQ ID NO: 138 oder SEQ ID NO: 198 angegebene Aminosäuresequenz kodieren,

c) Nukleinsäuresequenzen, die mit dem komplementären Strang der in SEQ ID NO: 43, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 75, SEQ ID NO: 77, SEQ ID NO: 79, SEQ ID NO: 83, SEQ ID NO: 85, SEQ ID NO: 113, SEQ ID NO: 117, SEQ ID NO: 119, SEQ ID NO: 131, SEQ ID NO: 133, SEQ ID NO: 135, SEQ ID NO: 137 oder SEQ ID NO: 197 angegebenen Nukleinsäuresequenz unter stringenten Bedingungen hybridisieren, und

d) Nukleinsäuresequenzen, die zu der in SEQ ID NO: 43, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 75, SEQ ID NO: 77, SEQ ID NO: 79, SEQ ID NO: 83, SEQ ID NO: 85, SEQ ID NO: 113, SEQ ID NO: 117, SEQ ID NO: 119, SEQ ID NO: 131, SEQ ID NO: 133, SEQ ID NO: 135, SEQ ID NO: 137 oder SEQ ID NO: 197 angegebenen Sequenz zu mindestens 60% identisch sind.

In einer bevorzugten Ausführungsform des Verfahrens werden die Δ-5-Elongase-Gene unter der Kontrolle eines samenspezifischen Promotors exprimiert.

In einer weiteren vorteilhaften Ausführungsform des Verfahrens werden alle Nukleinsäuresequenzen auf einem gemeinsamen rekombinanten Nukleinsäuremolekül in die Pflanzen eingebracht werden, wobei jede Nukleinsäuresequenz unter Kontrolle eines eigenen Promotors steht kann und es sich bei diesem eigenen Promotor um einen samenspezifischen Promotor handelt kann.

Die Erfindung kann aber nicht nur mit den im Sequenzprotokoll angegebenen Nukleinsäuren erfolgreich umgesetzt werden, vielmehr können auch von diesen Sequenzen bis zu einem gewissen Grad abweichende Sequenzen, die für Proteine mit der im Wesentlichen gleichen enzymatischen Aktivität kodieren, eingesetzt werden. Hierbei handelt

es sich um Nukleinsäuren, die zu den im Sequenzprotokoll spezifizierten Sequenzen einen bestimmten Identitäts- oder Homologiegrad aufweisen. Unter im wesentlichen gleiche enzymatische Aktivität sind Proteine zu verstehen, die mindestens 20%, 30%, 40%, 50% oder 60%, vorteilhaft mindestens 70%, 80%, 90% oder 95%, besonders vorteilhaft mindestens 96%, 97%, 98% oder 99% der enzymatischen Aktivität der Wildtyp-Enzyme aufweisen.

Zur Bestimmung der prozentualen Homologie (= Identität) von zwei Aminosäuresequenzen oder von zwei Nukleinsäuren werden die Sequenzen untereinander geschrieben (z.B. können Lücken in die Sequenz eines Proteins oder einer Nukleinsäure eingefügt werden, um ein optimales Alignment mit dem anderen Protein oder der anderen Nukleinsäure zu erzeugen). Die Aminosäurereste oder Nukleotide an den entsprechenden Aminosäurepositionen oder Nukleotidpositionen werden dann verglichen. Wenn eine Position in einer Sequenz durch den gleichen Aminosäurerest oder das gleiche Nukleotid wie die entsprechende Stelle in der anderen Sequenz belegt wird, dann sind die Moleküle an dieser Position homolog (d.h. Aminosäure- oder Nukleinsäure-"Homologie", wie hier verwendet, entspricht Aminosäure- oder Nukleinsäure-"Identität"). Die prozentuale Homologie zwischen den beiden Sequenzen ist eine Funktion der Anzahl an Positionen, die den Sequenzen gemeinsam sind (d.h. % Homologie = Anzahl der identischen Positionen/Gesamtanzahl der Positionen x 100). Die Begriffe Homologie und Identität sind damit als synonym anzusehen.

Die Homologie wurde über den gesamten Aminosäure- bzw. Nukleinsäuresequenzbereich berechnet. Für den Vergleich verschiedener Sequenzen stehen dem Fachmann eine Reihe von Programmen, die auf verschiedenen Algorithmen beruhen, zur Verfügung. Dabei liefern die Algorithmen von Needleman und Wunsch oder Smith und Waterman besonders zuverlässige Ergebnisse. Für die Sequenzvergleiche wurde das Programm PileUp verwendet (J. Mol. Evolution., 25, 351-360, 1987, Higgins et al., CABIOS, 5 1989: 151-153) oder die Programme Gap und BestFit [Needleman and Wunsch (J. Mol. Biol. 48; 443-453 (1970) und Smith and Waterman (Adv. Appl. Math. 2; 482-489 (1981)], die im GCG Software-Packet [Genetics Computer Group, 575 Science Drive, Madison, Wisconsin, USA 53711 (1991)] enthalten sind. Die oben in Prozent angegebenen Sequenzhomologiewerte wurden mit dem Programm GAP über den gesamten Sequenzbereich mit folgenden Einstellungen ermittelt: Gap Weight: 50, Length Weight: 3, Average Match: 10.000 und Average Mismatch: 0.000. Diese Einstellungen wurden, falls nicht anders angegeben, immer als Standardeinstellungen für Sequenzvergleiche verwendet.

Der Fachmann erkennt, dass innerhalb einer Population DNA-Sequenzpolymorphismen, die zu Änderungen der Aminosäuresequenz der SEQ ID NO: 12, 28, 194, 196, 198, 200 und/oder 202 führen, auftreten können. Diese natürlichen Varianten bewirken üblicherweise eine Varianz von 1 bis 5 % in der Nukleotidsequenz des Δ-12-Desaturase-, Δ-6-Desaturase-, Δ-5-Desaturase-, Δ-5-Elongase- und/oder Δ-6-Elongase-Gens. Sämtliche und alle dieser Nukleotidvariationen und daraus resultierende Aminosäurepolymorphismen in der Δ-12-Desaturase, Δ-6-Desaturase, Δ-5-Desaturase, Δ-5-Elongase und/oder Δ-6-Elongase, die das Ergebnis natürlicher Variation sind und die die enzymatische Aktivität nicht wesentlich verändern, sollen im Umfang der Erfindung enthalten sein.

Unter wesentlicher enzymatischer Aktivität der im erfindungsgemäßen Verfahren verwendeten Δ-12-Desaturase-, Δ-6-Desaturase, Δ-5-Elongase, Δ-6-Elongase oder Δ-5-Desaturase ist zu verstehen, dass sie gegenüber den durch die Sequenz und deren Derivate kodierten Proteinen/Enzymen im Vergleich noch eine enzymatische Aktivität von mindestens 10 %, bevorzugt von mindestens 20 %, besonders bevorzugt von mindestens 30 %, 40 %, 50 % oder mind. 60 % und am meisten bevorzugt von mindestens 70 %, 80 %, 90 %, 95 %, 96 %, 97 %, 98 % oder 99 % aufweisen und damit am Stoffwechsel von Verbindungen, die zum Aufbau von Fettsäuren, Fettsäureestern wie Diacylglyceriden und/oder Triacylglyceriden in einer Pflanze oder Pflanzenzelle benötigt werden oder am Transport von Molekülen über Membranen teilnehmen können, wobei $C_{18}$-, $C_{20}$- oder $C_{22}$-Kohlenstoffketten im Fettsäuremolekül mit Doppelbindungen an mindestens zwei, vorteilhaft drei, vier oder fünf Stellen gemeint sind.

Ebenfalls im Umfang der Erfindung enthalten sind Nukleinsäuremoleküle, die unter stringenten Bedingungen mit dem komplementären Strang der hier verwendeten Δ-12-Desaturase-, Δ-6-Desaturase-, Δ-5-Desaturase-, Δ-5-Elongase- und/oder Δ-6-Elongase-Nukleinsäuren hybridisieren. Der Begriff "hybridisiert unter stringenten Bedingungen", wie hier verwendet, soll Hybridisierungs- und Waschbedingungen beschreiben, unter denen Nukleotidsequenzen, die mindestens 60 % homolog zueinander sind, gewöhnlich aneinander hybridisiert bleiben. Die Bedingungen sind vorzugsweise derart, dass Sequenzen, die mindestens etwa 65 %, 70 %, 80 % oder 90 %, bevorzugt mindestens etwa 91 %, 92 %, 93 %, 94 % oder 95 % und besonders bevorzugt mindestens etwa 96 %, 97 %, 98 %, 99 % oder stärker zueinander homolog sind, gewöhnlich aneinander hybridisiert bleiben. Diese stringenten Bedingungen sind dem Fachmann bekannt und z.B. in Current Protocols in Molecular Biology, John Wiley & Sons, N. Y. (1989), 6.3.1-6.3.6, beschrieben.

Ein bevorzugtes, nicht einschränkendes Beispiel für stringente Hybridisierungsbedingungen sind Hybridisierungen in 6 x Natriumchlorid/Natriumcitrat (sodium chloride/sodium citrate = SSC) bei etwa 45°C, gefolgt von einem oder mehreren Waschschritten in 0,2 x SSC, 0,1 % SDS bei 50 bis 65°C. Dem Fachmann ist bekannt, dass sich diese Hybridisierungsbedingungen je nach dem Typ der Nukleinsäure und, wenn beispielsweise organische Lösungsmittel vorliegen, hinsichtlich der Temperatur und der Konzentration des Puffers unterscheiden. Die Hybridisierungstemperatur liegt beispielsweise unter "Standard-Hybridisierungsbedingungen" je nach dem Typ der Nukleinsäure zwischen 42°C und 58°C in wässrigem Puffer mit einer Konzentration von 0,1 bis 5 x SSC (pH 7,2). Falls organisches Lösungsmittel, zum Beispiel 50 % Formamid, im obengenannten Puffer vorliegt, beträgt die Temperatur unter Standardbedingungen

etwa 42°C. Vorzugsweise sind die Hybridisierungsbedingungen für DNA:DNA-Hybride zum Beispiel 0,1 x SSC und 20°C bis 45°C, vorzugsweise 30°C bis 45°C. Vorzugsweise sind die Hybridisierungsbedingungen für DNA:RNA-Hybride zum Beispiel 0,1 x SSC und 30°C bis 55°C, vorzugsweise 45°C bis 55°C. Die vorstehend genannten Hybridisierungstemperaturen sind für eine Nukleinsäure mit etwa 100 bp (= Basenpaare) Länge und einem G + C-Gehalt von 50 % in Abwesenheit von Formamid bestimmt. Der Fachmann weiß, wie die für eine bestimmte Nukleinsäure erforderlichen Hybridisierungsbedingungen anhand von Lehrbüchern, wie etwa Sambrook et al., "Molecular Cloning", Cold Spring Harbor Laboratory, 1989; Hames und Higgins (Hrsgb.) 1985, "Nucleic Acids Hybridization: A Practical Approach", IRL Press at Oxford University Press, Oxford; Brown (Hrsgb.) 1991, "Essential Molecular Biology: A Practical Approach", IRL Press at Oxford University Press, Oxford, bestimmt werden können.

Durch Einbringen einer oder mehrerer Nukleotidsubstitutionen, -additionen oder - deletionen in eine Nukleotidsequenz kann ein isoliertes Nukleinsäuremolekül erzeugt werden, das für eine Δ-12-Desaturase, Δ-6-Desaturase, Δ-5-Desaturase, Δ-5-Elongase und/oder Δ-6-Elongase mit einer oder mehreren Aminosäuresubstitutionen, -additionen oder -deletionen kodiert. Mutationen können in eine der Sequenzen durch Standardtechniken, wie stellenspezifische Mutagenese und PCR-vermittelte Mutagenese, eingebracht werden. Vorzugsweise werden konservative Aminosäuresubstitutionen an einem oder mehreren der vorhergesagten nicht-essentiellen Aminosäurereste hergestellt. Bei einer "konservativen Aminosäuresubstitution" wird der Aminosäurerest gegen einen Aminosäurerest mit einer ähnlichen Seitenkette ausgetauscht. Im Fachgebiet sind Familien von Aminosäureresten mit ähnlichen Seitenketten definiert worden. Diese Familien umfassen Aminosäuren mit basischen Seitenketten (z.B. Lysin, Arginin, Histidin), sauren Seitenketten (z.B. Asparaginsäure, Glutaminsäure), ungeladenen polaren Seitenketten (z.B. Glycin, Asparagin, Glutamin, Serin, Threonin, Tyrosin, Cystein), unpolaren Seitenketten, (z.B. Alanin, Valin, Leucin, Isoleucin, Prolin, Phenylalanin, Methionin, Tryptophan), beta-verzweigten Seitenketten (z.B. Threonin, Valin, Isoleucin) und aromatischen Seitenketten (z.B. Tyrosin, Phenylalanin, Tryptophan, Histidin). Ein vorhergesagter nicht-essentieller Aminosäurerest in einer Δ-12-Desaturase, Δ-6-Desaturase, Δ-5-Desaturase, Δ-5-Elongase oder Δ-6-Elongase wird somit vorzugsweise durch einen anderen Aminosäurerest aus der gleichen Seitenkettenfamilie ausgetauscht. Alternativ können bei einer anderen Ausführungsform die Mutationen zufallsgemäß über die gesamte oder einen Teil der für die Δ-12-Desaturase, Δ-6-Desaturase, Δ-5-Desaturase, Δ-5-Elongase oder Δ-6-Elongase kodierenden Sequenz eingebracht werden, z.B. durch Sättigungsmutagenese, und die resultierenden Mutanten können durch rekombinante Expression nach der hier beschriebenen Δ-12-Desaturase-, Δ-6-Desaturase-, Δ-5-Desaturase-, Δ-5-Elongase-oder Δ-6-Elongase-Aktivität durchmustert werden, um Mutanten zu identifizieren, die die Δ-12-Desaturase-, Δ-6-Desaturase-, Δ-5-Desaturase-, Δ-5-Elongase- oder Δ-6-Elongase-Aktivität beibehalten haben.

In einer Ausfürungsform betrifft die Erfindung auch ein Verfahren zu Herstellung einer erfindungsgemäßen Pflanze, z.B, einer Öl-produzierende Pflanze der Gattung Brassica, wobei die Pflanze unter Kontrolle von Samen-spezifischen Promotoren oder Promotoren aktiv in Pflanzenembryo oder Endosperm

a) eine delta-6 Desaturase,
b) eine delta-6 Elongase,
c) eine delta-5 Desaturase,
d) eine delta-12 Desaturase, und
e) eine delta-5 Elongase

- Aktivität aufweist, wobei die Pflanze Öl mit DHA und/oder EPA in den Samen herstellt, umfassend das Einbringen der genannten Enzyme in eine Öl-produzierende Pflanze.

Vorteilhaft enthalten die im erfindungsgemäßen Verfahren hergestellten mehrfach ungesättigten Fettsäuren mindestens zwei, bevorzugt drei, vier, fünf oder sechs Doppelbindungen. Besonders bevorzugt enthalten die Fettsäuren vier, fünf oder sechs Doppelbindungen. Im Verfahren hergestellte Fettsäuren weisen bevorzugt eine Länge von 20C- oder 22C-Atomen auf.

Vorteilhaft werden gesättigte Fettsäuren mit den im Verfahren verwendeten Nukleinsäuren wenig oder gar nicht umgesetzt. Unter wenig ist zu verstehen, dass im Vergleich zu mehrfach ungesättigten Fettsäuren die gesättigten Fettsäuren mit weniger als 5 %, bevorzugt mit weniger als 3 %, besonders bevorzugt mit weniger als 2 %, am meisten bevorzugt mit weniger als 1; 0,5; 0,25 oder 0,125 % der Aktivität umgesetzt werden. Die hergestellten Fettsäuren können das einzige Produkt des Verfahrens darstellen oder in einem Fettsäuregemisch vorliegen.

Die im Verfahren hergestellten mehrfach ungesättigten Fettsäuren sind vorteilhaft in Membranlipiden und/oder Triacylglyceriden gebunden, können aber auch als freie Fettsäuren oder aber gebunden in Form anderer Fettsäureester in den Organismen vorkommen. Dabei können sie als "Reinprodukte" oder aber vorteilhaft in Form von Mischungen verschiedener Fettsäuren oder Mischungen unterschiedlicher Glyceride vorliegen. Die in den Triacylglyceriden gebundenen verschieden Fettsäuren lassen sich dabei von kurzkettigen Fettsäuren mit 4 bis 6 C-Atomen, mittelkettigen Fettsäuren mit 8 bis 12 C-Atomen oder langkettigen Fettsäuren mit 14 bis 24 C-Atomen ableiten, bevorzugt sind die

langkettigen Fettsäuren besonders bevorzugt sind die langkettigen Fettsäuren LCPUFAs von $C_{18}$-, $C_{20}$- und/oder $C_{22}$-Fettsäuren, ganz besonders bevorzugt sind die langkettigen Fettsäuren LCPUFAs von $C_{20}$- und/oder $C_{22}$-Fettsäuren wie ARA, EPA, DHA oder deren Kombination.

Im erfindungsgemäßen Verfahren werden vorteilhaft Fettsäureester mit mehrfach ungesättigten $C_{18}$-, $C_{20}$- und/oder $C_{22}$-Fettsäuremolekülen mit mindestens zwei Doppelbindungen im Fettsäureester, vorteilhaft mit mindestens drei, vier, fünf oder sechs Doppelbindungen im Fettsäureester, besonders vorteilhaft von mindestens vier, fünf oder sechs Doppelbindungen im Fettsäureester, ganz besonders vorteilhaft von mindestens fünf oder sechs Doppelbindungen im Fettsäureester hergestellt. Dies führt vorteilhaft zur Synthese von Linolsäure (=LA, C18:$2^{\Delta 9,12}$), y-Linolensäure (= GLA, C18:$3^{\Delta 6,9,12}$), Stearidonsäure (= SDA, C18:$4^{\Delta 6,9,12,15}$), Dihomo-y-Linolensäure (= DGLA, 20:$3^{\Delta 8,11,14}$), ω-3-Eicosatetraensäure (= ETA, C20:$4^{\Delta 5,8,11,14}$), Arachidonsäure (ARA, C20:$4^{\Delta 5,8,11,14}$), Eicosapentaensäure (EPA, C20:$5^{\Delta 5,8,11,14,17}$), oder deren Mischungen synthetisiert, bevorzugt werden ω-3-Eicosatetraensäure (= ETA, C20:$4^{\Delta 5,8,11,14}$), Arachidonsäure (ARA, C20:$4^{\Delta 5,8,11,14}$) , Eicosapentaensäure (EPA, C20:$5^{\Delta 5,8,11,14,17}$), ω-6-Docosapentaensäure (C22:$5^{\Delta 4,7,10,13,16}$), ω-6-Docosatetraensäure (C22:$4^{\Delta 7,10,13,16}$), ω-3-Docosapentaensäure (= DPA, C22:$5^{\Delta 7,10,13,16,19}$), Docosahexaensäure (= DHA, C22:$6^{\Delta 4,7,10,13,16,19}$) oder deren Mischungen, ganz besonders bevorzugt ARA, EPA und/oder DHA hergestellt. Vorteilhaft werden ω-3-Fettsäuren wie EPA und/oder DHA, bevorzugt DHA hergestellt.

Die Fettsäureester mit mehrfach ungesättigten $C_{18}$-, $C_{20}$- und/oder $C_{22}$-Fettsäure-molekülen vorteilhaft mit mehrfach ungesättigten $C_{20}$- und/oder $C_{22}$-Fettsäuremolekülen können aus den Pflanzen, die für die Herstellung der Fettsäureester verwendet wurden, in Form eines Öls oder Lipids beispielsweise in Form von Verbindungen wie Sphingolipide, Phosphoglyceride, Lipide, Glycolipide wie Glycosphingolipide, Phospholipide wie Phosphatidylethanolamin, Phosphatidylcholin, Phosphatidylserin, Phosphatidylglycerol, Phosphatidylinositol oder Diphosphatidylglycerol, Monoacylglyceride, Diacylglyceride, Triacylglyceride oder sonstige Fettsäureester wie die AcetylCoenzymA-Ester, die die mehrfach ungesättigten Fettsäuren mit mindestens zwei, drei, vier, fünf oder sechs, bevorzugt vier, fünf oder sechs, besonders bevorzugt fünf oder sechs Doppelbindungen enthalten, isoliert werden. Vorteilhaft werden sie in der Form ihrer Diacylglyceride, Triacylglyceride und/oder in Form des Phosphatidylcholin isoliert, besonders bevorzugt in der Form der Triacylglyceride isoliert. Neben diesen Estern sind die mehrfach ungesättigten Fettsäuren auch als freie Fettsäuren oder gebunden an andere Verbindungen in den Pflanzen enthalten. In der Regel liegen die verschiedenen vorgenannten Verbindungen (Fettsäureester und frei Fettsäuren) in den Organismen in einer ungefähren Verteilung von 80 bis 90 Gew.-% Triglyceride, 2 bis 5 Gew.-% Diglyceride, 5 bis 10 Gew.-% Monoglyceride, 1 bis 5 Gew.-% freie Fettsäuren, 2 bis 8 Gew.-% Phospholipide vor, wobei sich die Summe der verschiedenen Verbindungen zu 100 Gew.-% ergänzt.

Im erfindungsgemäßen Verfahren bzw. in den erfindungsgemäßen Verfahren (der singular soll im Sinne der Erfindung und der hier dargestellten Offenbarung den plural umfassen und umgekehrt) werden die hergestellten LCPUFAs mit einem Gehalt von mindestens 3, 5, 6, 7 oder 8 Gew.-%, vorteilhaft von mindestens 9, 10, 11, 12, 13, 14 oder 15 Gew.-%, bevorzugt von mindestens 16, 17, 18, 19 oder 20 Gew.-%, besonders bevorzugt von mindestens 21, 22, 23, 24 oder 25 Gew.-%, ganz besonders bevorzugt von mindestens 26, 27, 28, 29 oder 30 Gew.-% bezogen auf die gesamten Fettsäuren in den transgenen Organismen vorteilhaft im Samen der transgenen Pflanzen hergestellt. Dabei werden vorteilhaft $C_{18}$- und/oder $C_{20}$-Fettsäuren, die in den Wirtsorganismen vorhanden sind, zu mindestens 10 %, vorteilhaft zu mindestens 20 %, besonders vorteilhaft zu mindestens 30 %, ganz besonders vorteilhaft zu mindestens 40 % in die entsprechenden Produkte wie ARA, EPA, DPA oder DHA, um nur einige beispielhaft zu nennen, umgesetzt. Vorteilhaft werden die Fettsäuren in gebundener Form hergestellt.

Vorteilhaft werden dabei im Verfahren mehrfach ungesättigte $C_{20}$-Fettsäuren mit vier oder fünf Doppelbindungen im Molekül mit einem Gehalt von zusammen allen derartigen Fettsäuren von mindestens 15, 16, 17, 18, 19 oder 20 Gew.-%, vorteilhaft zu mindestens 21, 22, 23, 24 oder 25 Gew.-%, besonders vorteilhaft von mindestens 26, 27, 28, 29 oder 30 Gew.-% bezogen auf die gesamten Fettsäuren in den Samen der transgenen Pflanzen hergestellt.

Vorteilhaft werden dabei im Verfahren mehrfach ungesättigte $C_{20}$- und/oder $C_{22}$-Fettsäuren mit vier, fünf oder sechs Doppelbindungen im Molekül mit einem Gehalt von zusammen allen derartigen Fettsäuren von mindestens 15, 16, 17, 18, 19 oder 20 Gew.-%, vorteilhaft zu mindestens 21, 22, 23, 24 oder 25 Gew.-%, besonders vorteilhaft von mindestens 26, 27, 28, 29 oder 30 Gew.-%, ganz besonders vorteilhaft von mindestens 31, 32, 33, 34 oder 35 Gew.-% bezogen auf die gesamten Fettsäuren in den Samen der transgenen Pflanze hergestellt.

Im erfindungsgemäßen Verfahren wird ARA mit einem Gehalt von mindestens 3, 5, 6, 7, 8, 9 oder 10 Gew.-%, vorteilhaft von mindestens 11, 12, 13, 14 oder 15 Gew.-%, bevorzugt von mindestens 16, 17, 18, 19 oder 20 Gew.-%, besonders bevorzugt von mindestens 21, 22, 23, 24 oder 25 Gew.-%, am meisten bevorzugt von mindestens 26 Gew.-%, bezogen auf den gesamten Lipidgehalt in den Samen der transgenen Pflanzen, hergestellt.

EPA wird im erfindungsgemäßen Verfahren mit einem Gehalt von mindestens 0,2; 0,3; 0,4; 0,5; 0,6; 0,7; 0,8; 0,9 oder 1 Gew.-%, vorteilhaft von mindestens 2, 3, 4 oder 5 Gew.-%, bevorzugt von mindestens 6, 7, 8, 9 oder 10 Gew.-%, besonders bevorzugt von mindestens 11, 12, 13, 14 oder 15 Gew.-% und am meisten bevorzugt von mindestens 16 Gew.-%, bezogen auf den gesamten Lipidgehalt in den Samen der transgenen Pflanzen, hergestellt.

DHA wird im erfindungsgemäßen Verfahren mit einem Gehalt von mindestens 0,01 oder 0,02 Gew.-%, vorteilhaft von mindestens 0,03 oder 0,05 Gew.-%, bevorzugt von mindestens 0,09 oder 0,1 Gew.-%, besonders bevorzugt von mindestens 0,2 oder 0,3 Gew.-% und am meisten bevorzugt von mindestens 0,35 Gew.-%, bezogen auf den gesamten Lipidgehalt in den Samen der transgenen Pflanzen, hergestellt.

Mit Hilfe der im erfindungsgemäßen Verfahren verwendeten Nukleinsäuren lassen sich diese ungesättigten Fettsäuren an sn1-, sn2- und/oder sn3-Position der vorteilhaft hergestellten Triglyceride bringen. Da im erfindungsgemäßen Verfahren von den Ausgangsverbindungen Linolsäure (C18:2) bzw. Linolensäure (C18:3) mehrere Reaktionsschritte durchlaufen werden, fallen die Endprodukte des Verfahrens wie beispielsweise Arachidonsäure (ARA), Eicosapentaensäure (EPA), ω-6-Docosapentaensäure oder DHA nicht als absolute Reinprodukte an, es sind immer auch geringe Spuren der Vorstufen im Endprodukt enthalten. Sind in dem Ausgangsorganismus bzw. in der Ausgangspflanze beispielsweise sowohl Linolsäure als auch Linolensäure vorhanden, so liegen die Endprodukte wie ARA, EPA oder DHA als Mischungen vor. Vorteilhaft sollten in den Endprodukten ARA oder DHA nur geringe Mengen, der jeweils anderen Endprodukte vorhanden sein. In einem DHA haltigen Lipid und/oder Öl sollten deshalb weniger als 15, 14, 13, 12 oder 11 Gew.-%, vorteilhaft weniger als10, 9, 8, 7, 6 oder 5 Gew.-%, besonders vorteilhaft weniger als 4, 3, 2 oder 1 Gew.-% EPA und/oder ARA enthalten sein. In einem EPA haltigen Lipid und/oder Öl sollten deshalb weniger als 15, 14, 13, 12 oder 11 Gew.-%, vorteilhaft weniger als10, 9, 8, 7, 6 oder 5 Gew.-%, besonders vorteilhaft weniger als 4, 3, 2 oder 1 Gew.-% ARA enthalten sein. Auch in einem ARA haltigen Lipid und/oder Öl sollten deshalb weniger als 15, 14, 13, 12 oder 11 Gew.-%, vorteilhaft weniger als10, 9, 8, 7, 6 oder 5 Gew.-%, besonders vorteilhaft weniger als 4, 3, 2 oder 1 Gew.-% EPA und/oder DHA enthalten sein.

Es können aber auch Mischungen von verschiedenen mehrfach ungesättigten $C_{20}$-und/oder $C_{22}$-Fettsäuren in einem Produkt wünschenswert sein. In solchen Fällen können DHA haltige Lipide und/oder Öle mindestens 1, 2, 3, 4 oder 5 Gew.-% ARA und/oder EPA, vorteilhaft mindestens 6, 7 oder 8 Gew.-%, besonders vorteilhaft mindestens 9, 10, 11, 12, 13, 14 oder 15 Gew.-%, ganz besonders vorteilhaft mindestens 16, 17, 18, 19, 20, 21, 22, 23, 24 oder 25 Gew.-% bezogen auf den gesamten Lipidgehalt in den Samen der transgenen Pflanzen enthalten.

Die Vorstufen sollten vorteilhaft nicht mehr als 20 Gew.-%, bevorzugt nicht mehr als 15 Gew.-%, besonders bevorzugt nicht als 10 Gew.-%, ganz besonders bevorzugt nicht mehr als 5 Gew.-% bezogen auf die Menge des jeweilige Endprodukts betragen. Vorteilhaft werden in einer transgenen Pflanze als Endprodukte nur ARA, EPA oder nur DHA im erfindungsgemäßen Verfahren gebunden oder als freie Säuren hergestellt. Werden die Verbindungen ARA, EPA und DHA gleichzeitig hergestellt, werden sie vorteilhaft in einem Verhältnis von mindesten 1:1:2 (EPA:ARA:DHA), vorteilhaft von mindestens 1:1:3, bevorzugt von 1:1:4, besonders bevorzugt von 1:1:5 hergestellt. Werden die Verbindungen ARA und EPA gleichzeitig hergestellt, werden sie vorteilhaft in einem Verhältnis von mindestens 1:6 (EPA:ARA), vorteilhaft von mindestens 1:8, bevorzugt von mindestens 1:10, besonders bevorzugt von mindestens 1:12 in der Pflanze hergestellt.

Fettsäureester bzw. Fettsäuregemische, die nach dem erfindungsgemäßen Verfahren hergestellt wurden, enthalten vorteilhaft 6 bis 15 % Palmitinsäure, 1 bis 6 % Stearinsäure; 7 - 85 % Ölsäure; 0,5 bis 8 % Vaccensäure, 0,1 bis 1 % Arachinsäure, 7 bis 25 % gesättigte Fettsäuren, 8 bis 85 % einfach ungesättigte Fettsäuren und 60 bis 85 % mehrfach ungesättigte Fettsäuren jeweils bezogen auf 100 % und auf den Gesamtfettsäuregehalt der Organismen.

Weiterhin enthalten die Fettsäureester bzw. Fettsäuregemische, die nach dem erfindungsgemäßen Verfahren hergestellt wurden, vorteilhaft Fettsäuren ausgewählt aus der Gruppe der Fettsäuren Erucasäure (13-Docosaensäure), Sterculinsäure (9,10-Methylene octadec-9-enonsäure), Malvalinsäure (8,9-Methylen Heptadec-8-enonsäure), Chaulmoogrinsäure (Cyclopenten-dodecansäure), Furan-Fettsäure (9,12-Epoxy-octadeca-9,11-dienonsäure), Vernonsäure (9,10-Epoxyoctadec-12-enonsäure), Tarinsäure (6-Octadecynonsäure),6-Nonadecynonsäure, Santalbinsäure (t11-Octadecen-9-ynoic acid), 6,9-Octadecenynonsäure, Pyrulinsäure (t10-Heptadecen-8-ynonsäure), Crepenyninsäure (9-Octadecen-12-ynonsäure), 13,14-Dihydrooropheinsäure, Octadecen-13-ene-9,11-diynonsäure, Petroselensäure (cis-6-Octadecenonsäure), 9c,12t-Octadecadiensäure, Calendulasäure (8t10t12c-Octadecatriensäure), Catalpinsäure (9t11t13c-Octadecatriensäure), Eleosterinsäure (9c11t13t-Octadecatriensäure), Jacarinsäure (8c10t12c-Octadecatriensäure), Punicinsäure (9c11t13c-Octadecatriensäure), Parinarinsäure (9c11t13t15c-Octadecatetraensäure), Pinolensäure (all-cis-5,9,12-Octadecatriensäure), Laballensäure (5,6-Octadecadienallensäure), Ricinolsäure (12-Hydroxyölsäure) und/oder Coriolinsäure (13-Hydroxy-9c,11t-Octadecadienonsäure). Die vorgenannten Fettsäuren kommen in den nach dem erfindungsgemäßen Verfahren hergestellten Fettsäureester bzw. Fettsäuregemischen in der Regel vorteilhaft nur in Spuren vor, das heißt sie kommen bezogen auf die Gesamtfettsäuren zu weniger als 30 %, bevorzugt zu weniger als 25 %, 24 %, 23 %, 22 % oder 21 %, besonders bevorzugt zu weniger als 20 %, 15 %, 10 %, 9 %, 8 %, 7%, 6 % oder 5%, ganz besonders bevorzugt zu weniger als 4 %, 3 %, 2 % oder 1 % vor. In einer weiteren bevorzugten Form der Erfindung kommen diese vorgenannten Fettsäuren bezogen auf die Gesamtfettsäuren zu weniger als 0,9%; 0,8%; 0,7%; 0,6%; oder 0,5%, besonders bevorzugt zu weniger als 0,4%; 0,3%; 0,2%; 0,1% vor. Vorteilhaft enthalten die nach dem erfindungsgemäßen Verfahren hergestellten Fettsäureester bzw. Fettsäuregemische weniger als 0,1 % bezogen auf die Gesamtfettsäuren und/oder keine Buttersäure, kein Cholesterin, keine Clupanodonsäure (= Docosapentaensäure, $C22:5^{\Delta 4,8,12,15,21}$) sowie keine Nisinsäure (Tetracosahexaensäure, $C23:6^{\Delta 3,8,12,15,18,21}$).

Durch die erfindungsgemäßen Nukleinsäuresequenzen bzw. im erfindungsgemäßen Verfahren verwendeten Nukleinsäuresequenzen kann eine Steigerung der Ausbeute an mehrfach ungesättigten Fettsäuren, vor allem an ARA und EPA aber auch DHA, von mindestens 50, 80 oder 100 %, vorteilhaft von mindestens 150, 200 oder 250 %, besonders vorteilhaft von mindestens 300, 400, 500, 600, 700, 800 oder 900 %, ganz besonders vorteilhaft von mindestens 1000, 1100, 1200, 1300, 1400 oder 1500 % gegenüber der nicht transgenen Ausgangspflanze beispielsweise einer Pflanze wie Brassica juncea, Brassica napus, Camelina sativa, Arabidopsis thanliana oder Linum usitatissimum beim Vergleich in der GC-Analyse siehe Beispiele erreicht werden.

Vorteilhaft werden, wie oben beschrieben, die im Verfahren hergestellten mehrfach ungesättigten $C_{20}$- und/oder $C_{22}$-Fettsäuren mit vier, fünf oder sechs Doppelbindungen im Molekül im Samen von Pflanzen, die keine oder nur sehr geringe Mengen an C12:0-bzw. C14:0-Fettsäuren enthalten. Auch noch kürzere gesättigte Fettsäuren wie die Fettsäuren C4:0, C6:0, C8:0 oder C10:0 sollten nicht oder nur in geringen Mengen im Lipid und/oder Öl vorhanden sein. Unter nur sehr geringen Mengen sind vorteilhaft Mengen zu verstehen, die in der GC-Analyse vorteilhaft unter 5, 4, 3, 2 oder 1 %, vorteilhaft unter 0,9; 0,8; 0,7; 0,6 oder 0,5 %, besonders vorteilhaft unter 0,4; 0,3; 0,2 ider 0,1 %, ganz besonders bevorzugt unter 0,09; 0,08; 0,07; 0,06; 0,05; 0,04; 0,03; 0,02 oder 0,01 Flächeneinheiten in der GC liegen. Die Fettsäure C16:0 sollte vorteilhaft in einem Bereich von 1 bis 28 % GC-Flächeneinheiten liegen. Vorteilhaft sollte die Fettsäure C16:0 in GC-Flächeneinheiten von weniger als 25%, 20%, 15% oder 10%, vorteilhaft von weniger als 9%, 8%, 7%, 6% oder 5%, besonders vorteilhaft von weniger als 4%, 3%, 2% oder 1% oder gar nicht in den Lipiden, Ölen und/oder freien Fettsäuren vorhanden sein. Die Fettsäure C16:1 sollte vorteilhaft weniger als 1; 0,5; 0,4; 0,3; 0,2 oder 0,1 %, besonders vorteilhaft 0,09; 0,08; 0,07; 0,06; 0,05; 0,04; 0,03; 0,02 oder 0,01 Flächeneinheiten in der GC betragen. Ganz besonders bevorzugt sollte die Fettsäure C16:1 nicht in den nach dem Verfahren hergestellten Ölen und/oder Lipiden vorhanden sein. Gleiches gilt für die Fettsäuren C15:0, C17:0, C16:1 $^{\Delta 3}$trans, C16:4$^{\Delta 4,7,10,13}$ und C18:5$^{\Delta 3,6,9,12,15}$. Neben Ölsäure (C18:1$^{\Delta 9}$) können auch die Isomere (C18:1$^{\Delta 7}$, C18:1$^{\Delta 11}$) in den Lipiden, Ölen oder freien Fettsäuren vorhanden sein. Vorteilhaft in Mengen, gemessen als GC-Flächeneinheiten, von weniger als 5%, 4%, 3%, 2% oder 1%. Die Fettsäuren C20:0, C20:1, C24:0 und C24:1 sollten jeweils in einem Bereich von 0 bis 1 %, 0 bis 3% bzw. 0 bis 5 % Flächeneinheiten in der GC liegen. Weiterhin sollte in der GC-Analyse wenig Dihomo-y-linolensäure (= DGLA) im Samenöl und/oder -lipid in GC-Flächeneinheiten detektierbar sein. Unter wenig sind weniger als 2; 1,9; 1,8; 1,7; 1,6 oder 1,5 %, vorteilhaft weniger als 1,4; 1,3; 1,2; 1,1 oder 1 %, besonders vorteilhaft weniger als 0,9; 0,8; 0,7; 0,6; 0,5 oder 0,4 % in GC-Flächeneinheiten zu verstehen.

In einer bevorzugten Ausführungsform des Verfahrens sollte DGLA und ARA in einem Verhältnis von 1:1 bis zu 1:100, vorteilhaft von 1:2 bis zu 1:80, besonders vorteilhaft von 1:3 bis zu 1:70, ganz besonders bevorzugt von 1:5 bis zu 1:60 entstehen.

In weiteren bevorzugten Ausführungsform des Verfahrens sollte DGLA und EPA in einem Verhältnis von 1:1 bis zu 1:100, vorteilhaft von 1:2 bis zu 1:80, besonders vorteilhaft von 1:3 bis zu 1:70, ganz besonders bevorzugt von 1:5 bis zu 1:60 entstehen.

Vorteilhaft sollten die im erfindungsgemäßen Verfahren hergestellten Lipide und/oder Öle einen hohen Anteil von ungesättigten Fettsäuren vorteilhaft von mehrfach ungesättigten Fettsäuren von mindestens 30, 40 oder 50 Gew.-%, vorteilhaft von mindestens 60, 70 oder 80 Gew.-% bezogen auf den Gesamtfettsäuregehalt in den Samen der transgenen Pflanzen betragen.

Alle gesättigten Fettsäuren zusammen sollten vorteilhaft in den für das erfindungsgemäße Verfahren bevorzugt verwendeten Pflanzen nur einen geringen Anteil ausmachen. Unter geringen Anteil ist in diesem Zusammenhang ein Anteil in GC-Flächeneinheiten von weniger als 15%, 14%, 13%, 12%, 11% oder 10%, bevorzugt von weniger als 9%, 8%, 7% oder 6% zu verstehen.

Weiterhin sollten die im Verfahren vorteilhaft als Wirtspflanzen, die die über verschiedene Methoden eingebrachten im Verfahren verwendeten Gene zur Synthese der mehrfach ungesättigten Fettsäuren enthalten, vorteilhaft einen höheren Ölanteil als Proteinanteil im Samen haben, vorteilhafte Pflanzen haben einen Öl-/Proteingehaltverhältnis von 5 zu 1, 4 zu 1, 3 zu 1, 2 zu 1 oder 1 zu 1. Dabei sollte der Ölgehalt bezogen auf das Gesamtgewicht des Samens in einem Bereich von 15 - 55%, vorteilhaft zwischen 25 - 50%, besonders vorteilhaft zwischen 35 - 50% liegen.

Vorteilhafte im Verfahren verwendete Wirtspflanzen sollten am Triglycerid in sn1-, sn2und sn3-Position eine Verteilung der ungesättigten Fettsäuren wie Ölsäure, Linolsäure und Linolensäure, die die Ausgangsverbindungen im erfindungsgemäßen Verfahren zur Synthese mehrfach ungesättigter Fettsäuren sind, wie in der folgenden Tabelle 5 dargestellt haben, wobei die Zeilen Nr. 1 - 7 verschiedene vorteilhafte Alternativen derartiger Verteilungen wiedergeben. Die Bezeichnung n.v. bedeutet nicht vorhanden.

Tabelle 5: Pflanzen mit vorteilhafter Fettsäureverteilung in sn1-, sn2- und sn3-Postion am Triglycerid

| Nr. | Ölsäure | | | Linolsäure | | | α-Linolensäure | | |
|---|---|---|---|---|---|---|---|---|---|
| | sn1 | sn2 | sn3 | sn1 | sn2 | sn3 | sn1 | sn2 | sn3 |
| 1. | 1 | 1 | 1 | 2 | 4 | 1 | n.v. | n.v. | n.v. |
| 2. | 1,4 | 2,2 | 1 | 2,8 | 9 | 1 | 2 | 6,7 | 1 |
| 3. | 0,8 | 0,8 | 1 | 1,1 | 1,6 | 1 | 1 | 0,8 | 1 |
| 4. | 0,9 | 0,9 | 1 | 1,2 | 1,6 | 1 | 0,9 | 1 | 1 |
| 5. | 0,9 | 0,9 | 1 | 1 | 1,3 | 1 | 1 | 1 | 1 |
| 6. | 1 | 1,1 | 1 | 2 | 2,8 | 1 | 1 | 1 | n.v. |
| 7. | 1,3 | 9,7 | 1 | 1 | 9 | Spuren | 1 | n.v. | n.v. |

Die Zeilen geben die Verhältnisse der folgenden Pflanzen wieder: Zeile 1 = Arachis hypogaea, Zeile 2 = Brassica napus, Zeile 3 = Glycine max, Zeile 4 = Linum usitatissimum, Zeile 5 = Zea mays, Zeile 6 = Olea europaea und Zeile 7 = Theobroma cacao.

Für das Verfahren vorteilhafte Wirtspflanzen sind solche, die einen hohen Anteil an Ölsäure, das heißt von mindestens 40, 50, 60 oder 70 Gew.-% bezogen auf den gesamten Fettsäuregehalt der Pflanze haben, im Vergleich zu Linolsäure und/oder Linolensäure in den Lipiden und/oder Ölen besonders im Triglycerid haben wie z.B. Anarcardium occidentale, Argania spinosa, Bombax malabaricum, Brassica napus, Butyrospermum parkii, hoch Ölsäure Distel (Carthamus tinctorius), Citrullus colocythis, Corylus avellana, Curcurbita foetidissima, Curcurbita pepo, Guizotia abyssinica, hoch Ölsäure Sonneblume (Helianthus annus), Macadamia intergrifolia, Nigella sativa, Olea europaea, Papaver somniferium, Passiflora edulis, Persea americana, Prunus amygdalis, Prunus armeniaca, Prunus dulcis, Prunus communis, Sesamum indicum, Simarouba glauca, Thea sasumgua, oder Theobroma cacao. Weitere vorteilhafte Pflanzen haben einen höheren Anteil der ungesättigten Fettsäuren Ölsäure, Linolsäure und α-Linolensäure in sn2-Position im Vergleich zu den anderen Positionen sn1 und sn3. Unter höheren Anteil sind Verhältnisse von (sn1:sn2:sn3) 1:1,1:1; 1:1,5:1 bis 1:3:1 zu verstehen. Vorteilhafte Pflanzen wie Actinidia chinensis, Aleurites moluccana, Arnebia griffithii, Brassica alba, Brassica hirta, Brassica nigra, Brassica juncea, Brassica carinata, Camelina sativa, Cannabis sativa, Echium rubrum, Echium vulgare, Humulus lupulus, Juglans regia, Linum usitatissimum, Ocimum spp., Perilla frutescens, Portulaca oleracea, Prunus cerasus, Salicornia bigelovii, Salvia hispanica sind auch solche die einen hohen Anteil an α-Linolensäure im Lipid und/oder Öl der Pflanze aufweisen, das heißt eine Anteil an α-Linolensäure von mindestens 10, 15 oder 20 Gew.-%, vorteilhaft von mindestens 25, 30, 35, 40, 45 oder 50 Gew.-% bezogen auf den gesamten Fettsäuregehalt der Pflanze aufweisen. Ganz besonders vorteilhafte Pflanzen zeigen für die im Verfahren hergestellte Arachidonsäure, Eicosapentaensäure oder Docosahexaensäure ebenfalls eine Präferenz für die sn2-Position im Triglycerid gegenüber den Positionen sn1 und sn3 von vorteilhaft 1:1,1:1; 1:1,5:1 bis 1:3:1.

Für das Verfahren verwendete Pflanzen sollten vorteilhaft einen Gehalt an Erucasäure von weniger als 2 Gew.-% bezogen auf den Gesamtfettsäuregehalt der Pflanze haben. Auch sollte der Gehalt an gesättigten Fettsäuren C16:0 und/oder C18:0 vorteilhaft geringer als 19, 18, 17, 16, 15, 14, 13, 12, 11, oder 10 Gew.-%, vorteilhaft weniger als 9, 8, 7, 6 oder 5 Gew.-% bezogen auf den gesamten Fettsäuregehalt der Pflanze sein. Vorteilhaft sollten auch längere Fettsäuren wie C20:0 oder C22:1 gar nicht oder in nur geringen Mengen vorteilhaft geringer als 4, 3, 2 oder 1 Gew.-%, vorteilhaft weniger als 0,9; 0,8; 0,7; 0,6; 0,5; 0,4; 0,3; 0,2 oder 0,1 Gew.-% bezogen auf den gesamten Fettsäuregehalt der Pflanze in den im Verfahren verwendeten Pflanzen vorhanden sein. Typischerweise ist in den für das erfindungsgemäße Verfahren verwendeten Pflanzen kein oder nur in geringen Mengen C16:1 als Fettsäure enthalten. Unter geringen Mengen sind vorteilhaft Gehalte an Fettsäuren zu verstehen, die geringer als 4, 3, 2 oder 1 Gew.-%, vorteilhaft weniger als 0,9; 0,8; 0,7; 0,6; 0,5; 0,4; 0,3; 0,2 oder 0,1 Gew.-% bezogen auf den gesamten Fettsäuregehalt der Pflanze sind.

Aus wirtschaftlichen Gründen, das heißt aufgrund der Anbaufläche und Ölerträge werden Pflanzen bevorzugt, die auf großen Flächen angebaut werden, wie Soja, Raps, Senf, Camelina, Lein, Sonnenblume, Ölpalme, Baumwolle, Sesam, Mais, Olive bevorzugt Raps, Camelina, Lein, Sonnenblume im Verfahren als Wirtspflanze gern genommen.

Auch chemisch reine mehrfach ungesättigte Fettsäuren oder Fettsäurezusammensetzungen sind nach den vorbeschriebenen Verfahren darstellbar. Dazu werden die Fettsäuren oder die Fettsäurezusammensetzungen aus den Pflanzen vorteilhaft den Pflanzensamen in bekannter Weise beispielsweise über aufbrechen der Samen wie Mahlen und anschließender Extraktion, Destillation, Kristallisation, Chromatographie oder Kombinationen dieser Methoden isoliert. Diese chemisch reinen Fettsäuren oder Fettsäurezusammensetzungen sind für Anwendungen im Bereich der Lebensmittelindustrie, der Kosmetikindustrie und besonders der Pharmaindustrie vorteilhaft.

Als Pflanzen für das erfindungsgemäße Verfahren kommen prinzipiell alle Pflanzen in Frage, die in der Lage sind Fettsäuren zu synthetisieren wie alle dicotylen oder monokotylen Pflanzen, Algen oder Moose. Vorteilhaft Pflanzen sind ausgewählt aus der Gruppe der Pflanzenfamilien Adelotheciaceae, Anacardiaceae, Asteraceae, Apiaceae, Betulaceae, Boraginaceae, Brassicaceae, Bromeliaceae, Caricaceae, Cannabaceae, Compositae, Convolvulaceae, Cruciferae, Cucurbitaceae, Elaeagnaceae, Ericaceae, Euphorbiaceae, Fabaceae, Geraniaceae, Gramineae, Juglandaceae, Lauraceae, Leguminosae, Linaceae, Malvaceae, Moringaceae, Marchantiaceae, Onagraceae, Olacaceae, Oleaceae, Papaveraceae, Piperaceae, Pedaliaceae, Poaceae, Rosaceae oder Solanaceae, vorteilhaft Anacardiaceae, Asteraceae, Boraginaceae, Brassicaceae, Cannabaceae, Compositae, Cruciferae, Cucurbitaceae, Elaeagnaceae, Euphorbiaceae, Fabaceae, Geraniaceae, Gramineae, Leguminosae, Linaceae, Malvaceae, Moringaceae, Marchantiaceae, Onagraceae, Olacaceae, Oleaceae, Papaveraceae, Piperaceae, Pedaliaceae, Poaceae oder Solanaceae. Aber auch Gemüsepflanzen oder Zierpflanzen wie Tagetes kommen für das Verfahren in Betracht.

Beispielhaft seien die folgenden Pflanzen genannt ausgewählt aus der Gruppe: Anacardiaceae wie die Gattungen Pistacia, Mangifera, Anacardium z.B. die Gattung und Arten *Pistacia vera* [Pistazie], *Mangifer indica* [Mango] oder *Anacardium occidentale* [Cashew], Asteraceae wie die Gattungen Artemisia, Calendula, Carthamus, Centaurea, Cichorium, Cynara, Helianthus, Lactuca, Locusta, Tagetes, Valeriana z.B. die Gattung und Arten Artemisia sphaerocephala, Calendula officinalis [Garten-Ringelblume], Carthamus tinctorius [Färberdistel, safflower], *Centaurea cyanus* [Kornblume], *Cichorium intybus* [Wegwarte], Cynara scolymus [Artichoke], *Helianthus annus* [Sonnenblume], *Lactuca sativa, Lactuca crispa, Lactuca esculenta, Lactuca scariola L. ssp. sativa, Lactuca scariola L. var. integrata, Lactuca scariola L. var. integrifolia, Lactuca sativa subsp. romana, Locusta communis, Valeriana locusta* [Salat], *Tagetes lucida, Tagetes erecta* oder *Tagetes tenuifolia* [Studentenblume], Apiaceae wie die Gattung Daucus z.B. die Gattung und Art *Daucus carota* [Karotte], Betulaceae wie die Gattung Corylus z.B. die Gattungen und Arten *Corylus avellana* oder *Corylus colurna* [Haselnuss], Boraginaceae wie die Gattung Adelocaryum, Alkanna, Anchusa, Borago, Brunnera, Cerinthe, Cynoglossum, Echium, Gastrocatyle, Lithospermum, Moltkia, Nonea, Onosma, Onosmodium, Paracaryum, Pectocarya, Symphytum z.B. die Gattung und Art Adelocaryum coelestinum, Alkanna orientalis, Anchusa anzurea, Anchusa capensis, Anchusa hybrida, *Borago officinalis* [Borretsch], Brunnera orientalis, Cerinthe minor, Cynoglossum amabile, Cynoglossum lanceolatum, Echium rubrum, Echium vulgare, Gastrocatyle hispida, Lithospermum arvense, Lithospermum purpureocaeruleum, Moltkia aurea, Moltkia coerules, Nonea macrosperma, Onosma sericeum, Onosmodium molle, Onosmodium occidentale, Paracaryum caelestinum, Pectocarya platycarpa, Symphytum officinale, Brassicaceae wie die Gattungen Brassica, Camelina, Melanosinapis, Sinapis, Arabadopsis z.B. die Gattungen und Arten *Brassica alba, Brassica carinata, Brassica hirta, Brassica napus, Brassica rapa* ssp. [Raps], *Sinapis arvensis Brassica juncea, Brassica juncea var. juncea, Brassica juncea var. crispifolia, Brassica juncea var. foliosa, Brassica nigra, Brassica sinapioides,* Camelina sativa, *Melanosinapis communis* [Senf], *Brassica oleracea* [Futterrübe] oder Arabidopsis thaliana, Bromeliaceae wie die Gattungen Anana, Bromelia (Ananas) z.B. die Gattungen und Arten *Anana comosus, Ananas ananas* oder *Bromelia comosa* [Ananas], Caricaceae wie die Gattung Carica wie die Gattung und Art *Carica papaya* [Papaya], Cannabaceae wie die Gattung Cannabis wie die Gattung und Art *Cannabis sative* [Hanf], Convolvulaceae wie die Gattungen Ipomea, Convolvulus z.B. die Gattungen und Arten *Ipomoea batatus, Ipomoea pandurata, Convolvulus batatas, Convolvulus tiliaceus, Ipomoea fastigiata, Ipomoea tiliacea, Ipomoea triloba* oder *Convolvulus panduratus* [Süßkartoffel, Batate], Chenopodiaceae wie die Gattung Beta wie die Gattungen und Arten *Beta vulgaris, Beta vulgaris var. altissima, Beta vulgaris var. Vulgaris, Beta maritima, Beta vulgaris var. perennis, Beta vulgaris var. conditiva* oder *Beta vulgaris var. esculenta* [Zuckerrübe], Crypthecodiniaceae wie die Gattung Crypthecodinium z.B. die Gattung und Art *Cryptecodinium cohnii,* Cucurbitaceae wie die Gattung Cucubita z.B. die Gattungen und Arten *Cucurbita maxima, Cucurbita mixta, Cucurbita pepo* oder *Cucurbita moscha*ta [Kürbis], Elaeagnaceae wie die Gattung Elaeagnus z.B. die Gattung und Art Olea *europaea* [Olive], Ericaceae wie die Gattung Kalmia z.B. die Gattungen und Arten *Kalmia latifolia, Kalmia angustifolia, Kalmia microphylla, Kalmia polifolia, Kalmia occidentalis, Cistus chamaerhodendros* oder *Kalmia lucida* [Berglorbeer], Euphorbiaceae wie die Gattungen Manihot, Janipha, Jatropha, Ricinus z.B. die Gattungen und Arten *Manihot utilissima, Janipha manihot" Jatropha manihot., Manihot aipil, Manihot dulcis, Manihot manihot, Manihot melanobasis, Manihot esculenta* [Manihot] oder *Ricinus communis* [Rizinus], Fabaceae wie die Gattungen Pisum, Albizia, Cathormion, Feuillea, Inga, Pithecolobium, Acacia, Mimosa, Medicago, Glycine, Dolichos, Phaseolus, Soja z.B. die Gattungen und Arten *Pisum sativum, Pisum arvense, Pisum humile* [Erbse], *Albizia berteriana, Albizia julibrissin, Albizia lebbeck, Acacia berteriana, Acacia littoralis, Albizia berteriana, Albizzia berteriana, Cathormion berteriana, Feuillea berteriana, Inga fragrans, Pithecellobium berterianum, Pithecellobium fragrans, Pithecolobium berterianum, Pseudalbizzia berteriana, Acacia julibrissin, Acacia nemu, Albizia nemu, Feuilleea julibrissin, Mimosa julibrissin, Mimosa speciosa, Sericanrda julibrissin, Acacia lebbeck, Acacia macrophylla, Albizia lebbek, Feuilleea lebbeck, Mimosa lebbeck, Mimosa speciosa* [Seidenbaum], *Medicago sativa, Medicago falcata, Medicago vana* [Alfalfa] *Glycine max, Dolichos soja, Glycine gracilis, Glycine hispida, Phaseolus max, Soja hispida* oder *Soja max* [Sojabohne], Geraniaceae wie die Gattungen Pelargonium, Cocos, Oleum z.B. die Gattungen und Arten *Cocos nucifera, Pelargonium grossularioides* oder *Oleum cocois* [Kokusnuss], Gramineae wie die Gattung Saccharum z.B. die Gattung und Art Saccharum officinarum, Juglandaceae wie die Gattungen Juglans, Wallia z.B. die Gattungen und Arten *Juglans regia, Juglans ailanthifolia,*

*Juglans sieboldiana, Juglans cinerea, Wallia cinerea, Juglans bixbyi, Juglans californica, Juglans hindsii, Juglans intermedia, Juglans jamaicensis, Juglans major, Juglans microcarpa, Juglans nigra* oder *Wallia nigra* [Walnuss], Lauraceae Wie die Gattungen Persea, Laurus z.B. die Gattungen und Arten *Laurus nobilis* [Lorbeer], *Persea americana, Persea gratissima* oder *Persea persea* [Avocado], Leguminosae wie die Gattung Arachis z.B. die Gattung und Art *Arachis hypogaea* [Erdnuss], Linaceae wie die Gattungen Linum, Adenolinum z.B. die Gattungen und Arten *Linum usitatissimum, Linum humile, Linum austriacum, Linum bienne, Linum angustifolium, Linum catharticum, Linum flavum, Linum grandiflorum, Adenolinum grandiflorum, Linum lewisii, Linum narbonense, Linum perenne, Linum perenne var. lewisii, Linum pratense* oder *Linum trigynum* [Lein], Lythrarieae wie die Gattung Punica z.B. die Gattung und Art *Punica granatum* [Granatapfel], Malvaceae wie die Gattung Gossypium z.B. die Gattungen und Arten *Gossypium hirsutum, Gossypium arboreum, Gossypium barbadense, Gossypium herbaceum* oder *Gossypium thurberi* [Baumwolle], Marchantiaceae wie die Gattung Marchantia z.B. die Gattungen und Arten *Marchantia berteroana, Marchantia foliacea, Marchantia macropora,* Musaceae wie die Gattung Musa z.B. die Gattungen und Arten *Musa nana, Musa acuminata, Musa paradisiaca, Musa* spp. [Banane], Onagraceae wie die Gattungen Camissonia, Oenothera z.B. die Gattungen und Arten *Oenothera biennis, Oenothera grandiflora* oder *Camissonia brevipes* [Nachtkerze], Palmae wie die Gattung Elacis z.B. die Gattung und Art *Elaeis guineensis* [Ölpalme], Papaveraceae wie die Gattung Papaver z.B. die Gattungen und Arten *Papaver orientale, Papaver rhoeas, Papaver dubium* [Mohn], Pedaliaceae wie die Gattung Sesamum z.B. die Gattung und Art *Sesamum indicum* [Sesam], Piperaceae wie die Gattungen Piper, Artanthe, Peperomia, Steffensia z.B. die Gattungen und Arten *Piper aduncum, Piper amalago, Piper angustifolium, Piper auritum, Piper betel, Piper cubeba, Piper longum, Piper nigrum, Piper retrofractum, Artanthe adunca, Artanthe elongata, Peperomia elongata, Piper elongatum, Steffensia elongata.* [Cayennepfeffer], Poaceae wie die Gattungen Hordeum, Secale, Avena, Sorghum, Andropogon, Holcus, Panicum, Oryza, Zea (Mais), Triticum z.B. die Gattungen und Arten *Hordeum vulgare, Hordeum jubatum, Hordeum murinum, Hordeum secalinum, Hordeum distichon Hordeum aegiceras, Hordeum hexastichon., Hordeum hexastichum, Hordeum irregulare, Hordeum sativum, Hordeum secalinum* [Gerste], *Secale cereale* [Roggen], Avena sativa, *Avena fatua, Avena byzantina, Avena fatua var. sativa, Avena hybrida* [Hafer], *Sorghum bicolor, Sorghum halepense, Sorghum saccharatum, Sorghum vulgare, Andropogon drummondii, Holcus bicolor, Holcus sorghum, Sorghum aethiopicum, Sorghum arundinaceum, Sorghum caffrorum, Sorghum cernuum, Sorghum dochna, Sorghum drummondii, Sorghum durra, Sorghum guineense, Sorghum lanceolatum, Sorghum nervosum, Sorghum saccharatum, Sorghum subglabrescens, Sorghum verticilliflorum, Sorghum vulgare, Holcus halepensis, Sorghum miliaceum, Panicum militaceum [Hirse], Oryza sativa, Oryza latifolia* [Reis], *Zea mays* [Mais] *Triticum aestivum, Triticum durum, Triticum turgidum, Triticum hybernum, Triticum macha, Triticum sativum* oder *Triticum vulgare* [Weizen], Porphyridiaceae wie die Gattungen Chroothece, Flintiella, Petrovanella, Porphyridium, Rhodella, Rhodosorus, Vanhoeffenia z.B. die Gattung und Art *Porphyridium cruentum,* Proteaceae wie die Gattung Macadamia z.B. die Gattung und Art *Macadamia intergrifolia* [Macadamia], Rosaceae wie die Gattung Prunus z.B. die Gattung und Art Prunus armeniaca, Prunus amygdalus, Prunus avilum, Rubiaceae wie die Gattung Coffea z.B. die Gattungen und Arten Cofea spp., *Coffea arabica, Coffea canephora* oder *Coffea liberica* [Kaffee], Scrophulariaceae wie die Gattung Scrophularia, Verbascum z.B. die Gattungen und Arten Scrophularia *marilandica, Verbascum blattaria, Verbascum chaixii, Verbascum densiflorum, Verbascum lagurus, Verbascum longifolium, Verbascum lychnitis, Verbascum nigrum, Verbascum olympicum, Verbascum phlomoides, Verbascum phoenicum, Verbascum pulverulentum* oder *Verbascum thapsus* [*Königskerze*], Solanaceae wie die Gattungen Capsicum, Nicotiana, Solanum, Lycopersicon z.B. die Gattungen und Arten *Capsicum annuum, Capsicum annuum var. glabriusculum, Capsicum frutescens* [Pfeffer], *Capsicum annuum* [Paprika], *Nicotiana tabacum, Nicotiana alata, Nicotiana attenuata, Nicotiana glauca, Nicotiana langsdorffii, Nicotiana obtusifolia, Nicotiana quadrivalvis, Nicotiana repanda, Nicotiana rustica, Nicotiana sylvestris* [Tabak], *Solanum tuberosum* [Kartoffel], *Solanum melongena* [Aubergine] *Lycopersicon esculentum, Lycopersicon lycopersicum., Lycopersicon pyriforme, Solanum integrifolium* oder *Solanum lycopersicum* [Tomate], Sterculiaceae wie die Gattung Theobroma z.B. die Gattung und Art *Theobroma cacao* [Kakao] oder Theaceae wie die Gattung Camellia z.B. die Gattung und Art *Camellia sinensis* [Tee]. Als weitere Pflanzen seien die Gattung und Art Argania spinosa, Arnebia griffithii, Adansonia digitata, Orbignya martiana, Carum carvi, Bertholletia excelsa, Aleurites moluccana, Hydnocarpus kurzii, Salvia hispanica, Vitis vinifera, Corvlus avellana, Humulus lupus, Hyptis spicigera und Shorea stenoptera genannt.

Vorteilhaft werden im erfindungsgemäßen Verfahren transgene Pflanzen wie zweikeimblättrige oder einkeimblättrige Pflanzen verwendet. Besonders vorteilhaft werden transgene Pflanzen im erfindungsgemäßen Verfahren verwendet, die zu den Öl-produzierenden Pflanzen gehören, das heißt die für die Herstellung von Ölen verwendet werden, wie bevorzugt Ölfruchtpflanzen, die große Mengen an Lipidverbindungen enthalten, wie Erdnuss, Raps, Canola, Sonnenblume, Saflor (Carthamus tinctoria), Mohn, Senf, Hanf, Rizinus, Olive, Sesam, Calendula, Punica, Nachtkerze, Königskerze, Distel, Wildrosen, Haselnuss, Mandel, Macadamia, Avocado, Lorbeer, Kürbis, Lein, Soja, Pistazien, Borretsch, Bäume (Ölpalme, Kokosnuss oder Walnuss) oder Feldfrüchte, wie Mais, Weizen, Roggen, Hafer, Triticale, Reis, Gerste, Baumwolle, Maniok, Pfeffer, Tagetes, Solanaceen-Pflanzen, wie Kartoffel, Tabak, Aubergine und Tomate, Vicia-Arten, Erbse, Alfalfa oder Buschpflanzen (Kaffee, Kakao, Tee), Salix-Arten sowie ausdauernde Gräser und Futterfeldfrüchte.

Bevorzugte erfindungsgemäße Pflanzen sind Ölsamen- oder Ölfruchtpflanzen, wie Erdnuss, Raps, Canola, Sonnenblume, Saflor, Mohn, Saeptasenf. Senf, Hanf, Rhizinus, Olive, Calendula, Punica, Nachtkerze, Kürbis, Lein, Soja, Borretsch, Bäume (Ölpalme, Kokosnuss). Besonders bevorzugt sind C18:2- und/oder C18:3-Fettsäure reiche Pflanzen wie Sonnenblume, Färberdistel, Tabak, Königskerze, Sesam, Baumwolle, Kürbis, Mohn, Nachtkerze, Walnuss, Lein, Hanf, Distel oder Färberdistel. Ganz besonders bevorzugt sind Pflanzen wie Färberdistel, Sonnenblume, Mohn, Nachtkerze, Walnuss, Lein, Sareptasenf, Camelina oder Hanf.

Für die erfindungsgemäßen beschriebenen Verfahren ist es vorteilhaft in die Pflanze zusätzlich zu den unter Verfahrensschritt (a) bis (e) bzw. (a) bis (c) eingebrachten Nukleinsäuren sowie den ggf. eingebrachten Nukleinsäuresequenzen, die für die ω-3-Desaturasen und/oder die für die Δ-12-Desaturasen codieren, zusätzlich weitere Nukleinsäuren einzubringen, die für Enzyme des Fettsäure- oder Lipidstoffwechsels codieren.

Im Prinzip können alle Gene des Fettsäure- oder Lipidstoffwechsels vorteilhaft in Kombination mit der(den) erfinderischen Δ-5-Elongase(n), Δ-6-Elongase(n) und/oder ω-3-Desaturase(n) [im Sinne dieser Anmeldung soll der Plural den Singular und umgekehrt beinhalten] im Verfahren zur Herstellung mehrfach ungesättigter Fettsäuren verwendet werden vorteilhaft werden Gene des Fettsäure- oder Lipidstoffwechsels ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferasen, Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen oder Fettsäure-Elongase(n) in Kombination mit der Δ-5-Elongase, Δ-6-Elongase und/oder ω-3-Desaturase verwendet. Besonders bevorzugt werden Gene ausgewählt aus der Gruppe der Δ-4-Desaturasen, Δ-5-Desaturasen, Δ-6-Desaturasen, Δ-8-Desatuasen, Δ-9-Desaturasen, Δ-12-Desaturasen, Δ-6-Elongasen oder Δ-9-Elongasen in Kombination mit den vorgenannten Genen für die Δ-5-Elongase, Δ-6-Elongase und/oder ω-3-Desaturase verwendet, wobei einzelne Gene oder mehrere Gene in Kombination verwendet werden können.Vorteilhaft werden die vorgenannten Geni in Kombination mit der erfindungsgemäß verwendeten Δ-6-Elongase, Δ-5-Elongase, Δ-5-Desaturase, Δ-6-Desaturase und/oder Δ-12-Desaturase verwendet

Besonders bevorzugt werden Gene ausgewählt aus der Gruppe der Δ-8-Desaturasen, Δ-9-Desaturasen, Δ-5-Elongase oder Δ-9-Elongasen in Kombination mit den vorgenannten Genen verwendet.

Durch die enzymatische Aktivität der im erfindungsgemäßen Verfahren verwendeten Nukleinsäuren, die für Polypeptide mit Δ-6-Elongase-, Δ-6-Desaturase, Δ-5-Desaturase- und/oder Δ-12-Desaturaseaktivität kodieren, vorteilhaft in Kombination mit Nukleinsäuresequenzen, die für Polypeptide des Fettsäure- oder Lipidstoffwechsels wie Polypeptide mit Δ-8-Desaturase- oder Δ-5- oder A-9-Elongaseaktivität kodieren, können im erfindungsgemäßen Verfahren unterschiedlichste mehrfach ungesättigte Fettsäuren hergestellt werden. Je nach Auswahl der für das erfindungsgemäße Verfahren verwendeten Pflanzen lassen sich Mischungen der verschiedenen mehrfach ungesättigten Fettsäuren oder einzelne mehrfach ungesättigte Fettsäuren wie EPA oder ARA in freier oder gebundener Form herstellen. Je nachdem welche Fettsäurezusammensetzung in der Ausgangspflanze vorherrscht (C18:2- oder C18:3-Fettsäuren) entstehen so Fettsäuren, die sich von C18:2-Fettsäuren ableiten, wie GLA, DGLA oder ARA, oder Fettsäuren, die sich von C18:3-Fettsäuren ableiten, wie SDA, ETA oder EPA. Liegt in der für das Verfahren verwendeten Pflanze als ungesättigte Fettsäure nur Linolsäure (= LA, C18:$2^{\Delta 9,12}$) vor, so können als Produkte des Verfahrens nur GLA, DGLA und ARA entstehen, die als freie Fettsäuren oder gebunden vorliegen können. Ist in der im Verfahren verwendeten Pflanze als ungesättigte Fettsäure nur α-Linolensäure (= ALA, C18:$3^{\Delta 9,12,15}$) vorhanden, wie beispielsweise in Lein, so können als Produkte des Verfahrens nur SDA, ETA oder EPA entstehen, die wie oben beschrieben als freie Fettsäuren oder gebunden vorliegen können.

Durch die Aktivität der Δ-6-Desaturase und Δ-6-Elongase entstehen beispielsweise GLA und DGLA bzw. SDA und ETA, je nach Ausgangspflanze und darin enthaltener ungesättigter Fettsäure. Bevorzugt entstehen DGLA bzw. ETA oder Mischungen daraus. Wird zusätzlich die Δ-5-Desaturase in die Pflanze eingebracht, so entstehen auch ARA und/oder EPA. Werden darüber hinaus noch Gene eingebracht, die für eine Δ-5-Elongase- und/oder Δ-4-Desaturaseaktivität codieren, so lassen sich die Fettsäuren DPA und/oder DHA im erfindungsgemäßen Verfahren herstellen. Vorteilhaft werden nur ARA, EPA und/oder DHA oder eine Mischung davon synthetisiert, abhängig von der in der Pflanze vorliegenden Fettsäure, die als Ausgangssubstanz für die Synthese dient. Da es sich um Biosyntheseketten handelt, liegen die jeweiligen Endprodukte nicht als Reinsubstanzen in den Organismen vor. Es sind immer auch geringe Mengen der Vorläuferverbindungen im Endprodukt enthalten. Diese geringen Mengen betragen weniger als 20 Gew.-%, bevorzugt weniger als 15 Gew.-%, besonders bevorzugt weniger als 10 Gew.-%, am meisten bevorzugt weniger als 5, 4, 3, 2 oder 1 Gew.-%, bezogen auf die Endprodukte DGLA, ETA oder deren Mischungen bzw. ARA, EPA oder deren Mischungen bzw. ARA, EPA, DHA oder deren Mischungen.

Neben der Produktion der Ausgangsfettsäuren für die erfindungsgemäß verwendeten Enzyme direkt in der Pflanze können die Fettsäuren auch von außen gefüttert werden. Aus Kostengründen ist die Produktion in der Pflanze bevorzugt. Bevorzugte Substrate für die Produktion von ARA sind die Linolsäure (C18:$2^{\Delta 9,12}$), die γ-Linolensäure (C18:$3^{\Delta 6,9,12}$) und die Dihomo-γ-linolensäure (C20:$3^{\Delta 8,11,14}$) . Bevorzugte Substrate für die Produktion von EPA sind die Linolensäure (C18:$3^{\Delta 9,12,15}$), die Stearidonsäure (C18:$4^{\Delta 6,9,12,15}$) und die Eicosatetraensäure (C20:$4^{\Delta 8,11,14,17}$).

Bevorzugte Substrate für die Produktion von DHA sind die Linolensäure ($C18:3^{\Delta 9,12,15}$), die Stearidonsäure ($C18:4^{\Delta 6,9,12,15}$), die Eicosatetraensäure ($C20:4^{\Delta 8,11,14,17}$), EPA und DPA.

Die erfindungsgemäßen Δ-5-Elongasen haben gegenüber den humanen Elongasen oder Elongasen aus nicht-humanen Tieren wie denen aus Oncorhynchus, Xenopus oder Ciona die vorteilhafte Eigenschaft, dass sie $C_{22}$-Fettsäuren nicht zu den entsprechenden $C_{24}$-Fettsäuren elongieren. Weiterhin setzen sie vorteilhaft keine Fettsäuren mit einer Doppelbindung in Δ-6-Position um, wie sie von den humanen Elongasen oder den Elongasen aus nicht-humanen Tieren umgesetzt werden. Besonders vorteilhafte Δ-5-Elongasen setzen bevorzugt nur ungesättigte $C_{20}$-Fettsäuren um. Diese vorteilhaften Δ-5-Elongasen weisen einige putative Transmembran-Helixes (5 - 7) auf. Vorteilhaft werden nur $C_{20}$-Fettsäuren mit einer Doppelbindung in Δ-5-Position umgesetzt, wobei ω-3-$C_{20}$ Fettsäuren bevorzugt werden (EPA). Weiterhin haben sie in einer bevorzugten Ausführungsform der Erfindung die Eigenschaft, dass sie neben der Δ-5-Elongaseaktivität vorteilhaft keine oder nur eine relativ geringe Δ-6-Elongaseaktivität aufweisen. Im Gegensatz dazu weisen die humanen Elongasen oder nicht-humanen Tier-Elongasen eine annäherend gleiche Aktivität gegenüber Fettsäuren mit einer Δ-6- oder Δ-5-Doppelbindung auf. Diese vorteilhaften Elongasen werden als sogenannte monofunktionelle Elongasen bezeichnet. Die humanen Elongasen oder die nicht-humanen Tierelongasen werden dem gegenüber als multifunktionelle Elongasen bezeichnet, die neben den vorgenannten Substraten auch monoungesättigte $C_{16}$- und $C_{18}$-Fettsäuren beispielsweise mit Δ-9- oder Δ-11-Doppelbindung umsetzen. Vorteilhaft setzen die monofunktionellen Elongasen in einem Hefefütterungstext, in dem als Substrat EPA den Hefen zugesetzt wurde, mindestens 15 Gew.-% des zugesetzten EPAs zu Docosapentaensäure (DPA, $C22:5^{\Delta 7,10,13,16,19}$), vorteilhaft mindestens 20 Gew.-%, besonders vorteilhaft mindestens 25 Gew.-% um. Wird als Substrat γ-Linolensäure (= GLA, $C18:3^{\Delta 6,9,12}$) gegeben, so wird diese vorteilhaft gar nicht elongiert.

Ebenfalls wird auch $C18:3^{\Delta 5,9,12}$ nicht elongiert. In einer anderen vorteilhaften Ausführungsform werden weniger als 60 Gew.-% des zugesetzten GLA zu Dihomo-γ-linolensäure (= $C20:3^{\Delta 8,11,14}$) umgesetzt, vorteilhaft weniger als 55 Gew.-%, bevorzugt weniger als 50 Gew.-%, besonders vorteilhaft weniger als 45 Gew.-%, ganz besonders vorteilhaft weniger als 40 Gew.-%. In einer weiteren ganz bevorzugten Ausführungsform der erfindungsgemäßen Δ-5-Elongaseaktivität wird GLA nicht umgesetzt.

Die Figuren 27 und 28 geben die gemessenen Substratspezifitäten der verschiedenen Elongasen wieder. In Figur 27 sind die Spezifitäten der multifunktnonellen Elongasen von Xenopus laevis (Fig. 27 A), Ciona intestinalis (Fig. 27 B) und Oncorhynchus mykiss (Fig. 27 C) wiedergegeben. Alle diese Elongasen setzen ein breites Spektrum an Substraten um. Dies kann im erfindungsgemäßen Verfahren zu Nebenprodukten führen, die durch weitere enzymatische Aktivitäten umgesetzt werden müssen. Diese Enzyme sind deshalb im erfindungsgemäßen Verfahren weniger bevorzugt. Die bevorzugten monofunktionellen Elongasen und ihre Substratspezifität werden in Figur 28 wiedergegeben. Figur 28 A zeigt die Spezifität der Ostreococcus tauri Δ-5-Elongase. Dies setzt nur Fettsäuren mit einer Doppelbindung in Δ-5-Position um. Vorteilhaft werden nur C20-Fettsäuren umgesetzt. Eine ähnlich hohe Substratspezifität weist die Δ-5-Elongase von Thalassiosira pseudonana (Fig. 28. C) auf. Sowohl die Δ-6-Elongase von Ostreococcus tauri (Fig. 28 B) als auch die von Thalassiosira pseudonana (Fig. 28 D) setzen vorteilhaft nur Fettsäuren mit einer Doppelbindung in Δ-6-Position um. Vorteilhaft werden nur C18-Fettsäuren umgesetzt. Auch die Δ-5-Elongasen aus Arabidopsis thaliana und Euglena gracilis zeichnen sich durch ihre Spezifität aus.

Vorteilhafte verwendete Δ-6-Elongasen zeichnen sich ebenfalls durch eine hohe Spezifität aus, das heißt bevorzugt werden $C_{18}$-Fettsäuren elongiert. Vorteilhaft setzen sie Fettsäuren mit einer Doppelbindung in Δ-6-Position um. Besonders vorteilhafte Δ-6-Elongasen setzen vorteilhaft $C_{18}$-Fettsäuren mit drei oder vier Doppelbindungen im Molekül um, wobei diese eine Doppelbindung in Δ-6-Position enthalten müssen. Weiterhin haben sie in einer bevorzugten Ausführungsform der Erfindung die Eigenschaft, dass sie neben der Δ-6-Elongaseaktivität vorteilhaft keine oder nur eine relativ geringe Δ-5-Elongaseaktivität aufweisen. Im Gegensatz dazu weisen die humanen Elongasen oder nicht-humanen Tier-Elongasen eine annäherend gleiche Aktivität gegenüber Fettsäuren mit einer Δ-6- oder Δ-5-Doppelbindung auf. Diese vorteilhaften Elongasen werden als sogenannte monofunktionelle Elongasen bezeichnet. Die humanen Elongasen oder die nicht-humanen Tierelongasen werden, wie oben beschrieben, dem gegenüber als multifunktionelle Elongasen bezeichnet, die neben den vorgenannten Substraten auch monoungesättigte $C_{16}$- und $C_{18}$-Fettsäuren beispielsweise mit Δ-9- oder Δ-11-Doppelbindung umsetzen. Vorteilhaft setzen die monofunktionellen Elongasen in einem Hefefütterungstext, in dem als Substrat EPA den Hefen zugesetzt wurde, mindestens 10 Gew.-% der zugesetzten α-Linolensäure (= ALA, $C18:3^{\Delta 9,12,15}$) bzw. mindestens 40 Gew.-% der zugesetzten γ-Linolensäure (= GLA, $C18:3^{\Delta 6,9,12}$), vorteilhaft mindestens 20 Gew.-% bzw. 50 Gew.-%, besonders vorteilhaft mindestens 25 Gew.-% bzw. 60 Gew.-% um. Besonders vorteilhaft wird auch $C18:4^{\Delta 6,9,12,15}$ (Stearidonsäure) elongiert. SDA wird dabei zu mindestens 40 Gew.-%, vorteilhaft zu mindestens 50 Gew.-%, besonders vorteilhaft zu mindestens 60 Gew.-%, ganz besonders vorteihaft zu mindestens 70 Gew.-% umgesetzt. Besonders vorteilhafte Δ-6-Elongasen zeigen keine oder nur eine sehr geringe Aktivität (weniger als 0,1 Gew-% Umsatz) gegenüber den folgenden Substraten: $C18:1^{\Delta 6}$, $C18:1^{\Delta 9}$, $C18:1^{\Delta 11}$, $C20:2^{\Delta 11,14}$, $C20:3^{\Delta 11,14,17}$, $C20:3^{\Delta 8,11,14}$, $C20:4^{\Delta 5,8,11,14}$, $C20:5^{\Delta 5,8,11,14,17}$ oder $C22:4^{\Delta 7,10,13,16}$.

Die Figuren 29 und 30 sowie die Tabelle 21 gibt die gemessenen Substratspezifitäten der verschiedenen Elongasen wieder.

Die im erfindungsgemäßen Verfahren verwendete ω-3-Desaturase hat gegenüber den bekannten ω-3-Desaturase die vorteilhafte Eigenschaft, dass sie ein breites Spektrum an ω-6-Fettsäuren desaturieren kann, bevorzugt werden $C_{20}$- und $C_{22}$-Fettsäuren wie $C_{20:2}$-, $C_{20:3}$-, $C_{20:4}$-, $C_{22:4}$- oder $C_{22:5}$-Fettsäuren desaturiert. Aber auch die kürzeren $C_{18}$-Fettsäuren wie $C_{18:2}$- oder $C_{18:3}$-Fettsäuren werden vorteilhaft desaturiert. Durch diese Eigenschaften der ω-3-Desaturase ist es vorteilhaft möglich das Fettsäurespektrum innerhalb eines Organismus vorteilhaft innerhalb einer Pflanze oder einem Pilz von den ω-6-Fettsäuren zu den ω-3-Fettsäuren hin zu verschieben. Bevorzugt werden von der erfindungsgemäßen ω-3-Desaturase $C_{20}$-Fettsäuren desaturiert. Innerhalb des Organismus werden diese Fettsäuren aus dem vorhandenen Fettsäurepool zu mindestens 10%, 15%, 20%, 25% oder 30% zu den entsprechenden ω-3-Fettsäuren umgesetzt. Gegenüber den $C_{18}$-Fettsäuren weist die ω-3-Desaturase eine um den Faktor 10 geringere Aktivität auf, das heißt es werden nur ca. 1,5 bis 3% der im Fettsäurepool vorhandenen Fettsäuren zu den entsprechenden ω-3-Fettsäuren umgesetzt. Bevorzugtes Substrat der erfindungsgemäßen ω-3-Desaturase sind die in Phospholipiden gebundenen ω-6-Fettsäuren. Figur 19 zeigt deutlich am Beispiel der Desaturierung von Dihomo-γ-linolensäure [$C_{20:4}^{\Delta 8,11,14}$], dass die ω-3-Desaturase bei der Desaturierung vorteilhaft nicht zwischen an sn1- oder sn2-Position gebundenen Fettsäuren unterscheidet. Sowohl an sn1- oder sn2-Position in den Phospholipide gebundene Fettsäuren werden desaturiert. Weiterhin ist vorteilhaft, dass die ω-3-Desaturase eine breite Palette von Phospholipiden wie Phosphatidylcholin (= PC), Phosphatidylinositol (= PIS) oder Phosphatidylethanolamin (= PE) umsetzt. Schließlich lassen sich auch Desaturierungsprodukte in den Neutrallipiden (= NL), das heißt in den Triglyceriden finden.

Die im erfingungsgemäßen Verfahren verwendeten Δ-4-Desaturasen, Δ-5-Desaturasen und Δ-6-Desaturasen haben gegenüber den bekannten Δ-4-Desaturasen, Δ-5-Desaturasen und Δ-6-Desaturasen den Vorteil, dass sie Fettsäuren gebunden an Phospholipide oder CoA-Fettsäureester, vorteilhaft CoA-Fettsäureester umsetzen können.

Vorteilhaft setzen die im erfingungsgemäßen Verfahren verwendeten Δ-12-Desaturasen Ölsäure ($C18:1^{\Delta 9}$) zu Linolsäure ($C18:2^{\Delta 9,12}$) oder $C18:2^{\Delta 6,9}$ zu $C18:3^{\Delta 6,9,12}$ (= GLA) um. Vorteilhaft setzen die verwendeten Δ-12-Desaturasen Fettsäuren gebunden an Phospholipide oder CoA-Fettsäureester, vorteilhaft gebunden an CoA-Fettsäureester um.

Durch die enzymatische Aktivität der im erfindungsgemäßen Verfahren verwendeten Nukleinsäuren, die für Polypeptide mit Δ-5-Elongase-, Δ-6-Elongase- und/oder ω-3-Desaturaseaktivität codieren, vorteilhaft in Kombination mit Nukleinsäuresequenzen, die für Polypeptide des Fettsäure- oder Lipidstoffwechsels wie weiteren Polypeptiden mit Δ-4-, Δ-5-, Δ-6-, Δ-8-, Δ-12-Desaturase- oder Δ-5-, Δ-6-oder Δ-9-Elongaseaktivität codieren, können unterschiedlichste mehrfach ungesättigte Fettsäuren im erfindungsgemäßen Verfahren hergestellt werden. Je nach Auswahl der für das erfindungsgemäße Verfahren verwendeten vorteilhaften Pflanze lassen sich Mischungen der verschiedenen mehrfach ungesättigten Fettsäuren oder einzelne mehrfach ungesättigte Fettsäuren wie EPA, ARA oder DHA in freier oder gebundener Form herstellen. Je nachdem welche Fettsäurezusammensetzung in der Ausgangspflanze vorherrscht (C18:2- oder C18:3-Fettsäuren) entstehen so Fettsäuren, die sich von C18:2-Fettsäuren ableiten, wie GLA, DGLA oder ARA oder, die sich von C18:3-Fettsäuren ableiten, wie SDA, ETA, EPA oder DHA. Liegt in der für das Verfahren verwendeten Pflanze als ungesättigte Fettsäure nur Linolsäure (= LA, $C18:2^{\Delta 9,12}$) vor, so können als Produkte des Verfahrens nur GLA, DGLA und ARA entstehen, die als freie Fettsäuren oder gebunden vorliegen können. Durch Expression der zusätzlichen ω-3-Desaturase in diesen Pflanzen kann das Fettsäurespektrum auch hin zu α-Linolensäure, DPA und DHA hin verschoben werden. Allerdings ist diese Verschiebung des Fettsäurespektrums nur relativ eingeschränkt möglich. Vorteilhafter ist eine solche Verschiebung in Pflanzen, die , wie im folgenden beschrieben, schon einen hohen Anteil an α-Linolensäure enthalten. Ist in der im Verfahren verwendeten Pflanze als ungesättigte Fettsäure nur α-Linolensäure (= ALA, $C18:3^{\Delta 9,12,15}$) beispielsweise wie in Lein, so können als Produkte des Verfahrens nur SDA, ETA, EPA und/oder DHA entstehen, die wie oben beschrieben als freie Fettsäuren oder gebunden vorliegen können. Durch Modifikation der Aktivität des an der Synthese beteiligten Enzyms Δ-5-Elongase vorteilhaft in Kombination mit der Δ-4-, Δ-5-, Δ-6-, Δ-12-Desaturase und/oder Δ-6-Elongase, oder der Δ-4-, Δ-5-, Δ-8-, Δ-12-Desaturase, und/oder Δ-9-Elongase lassen sich gezielt in den vorgenannten Pflanzen nur einzelne Produkte herstellten. Durch die Aktivität der Δ-6-Desaturase und Δ-6-Elongase entstehen beispielsweise GLA und DGLA bzw. SDA und ETA, je nach Ausgangspflanze und ungesättigter Fettsäure. Bevorzugt entstehen DGLA bzw. ETA oder deren Mischungen. Werden die Δ-5-Desaturase, die Δ-5-Elongase und die Δ-4-Desaturase zusätzlich in die Organismen vorteilhaft in die Pflanze eingebracht, so entstehen zusätzlich ARA, EPA und/oder DHA. Dies gilt auch für Organismen in die vorher die Δ-8-Desaturase und Δ-9-Elongase eingebracht wurde. Vorteilhaft werden nur ARA, EPA oder DHA oder deren Mischungen synthetisiert, abhängig von der in der Pflanze vorliegenden Fettsäure, die als Ausgangssubstanz für die Synthese dient. Da es sich um Biosyntheseketten handelt, liegen die jeweiligen Endprodukte nicht als Reinsubstanzen in den Organismen vor. Es sind immer auch geringe Mengen der Vorläuferverbindungen im Endprodukt enthalten. Diese geringen Mengen betragen weniger als 20 Gew.-%, vorteilhaft weniger als 15 Gew.-%, besonders vorteilhaft weniger als 10 Gew.-%, ganz besonders vorteilhaft weniger als 5, 4, 3, 2 oder 1 Gew.-% bezogen auf das Endprodukt DGLA, ETA oder deren Mischungen bzw. ARA, EPA, DHA oder deren Mischungen vorteilhaft EPA oder DHA oder deren Mischungen.

Die im erfindungsgemäßen Verfahren verwendbare aus Forelle stammende Nukleinsäure mit der SEQ ID NO: 53 kodiert für ein Protein, das eine hohe Spezifität für die beiden $C18:4^{\Delta 6,9,12,15}$- und $C20:5^{\Delta 5,8,11,14,17}$-Fettsäuren zeigt, diese sind Vorstufen zur Synthese von DHA (Vorstufen und Synthese von DHA siehe Figur 1). Aber auch andere

Fettsäuren werden durch das Enzym elongiert. Das von SEQ NO: 53 kodierte Protein hat damit eine Spezifität für Δ6- und Δ5-Fettsäuren mit zusätzlich einer ω3-Doppelbindung (Figur 2). Die Δ-5-Elongase hat eine keto-Acyl-CoA-Synthase-Aktivität, die vorteilhaft Fettsäurereste von Acyl-CoA-Estern um 2 Kohlenstoffatome verlängert.

Durch das Genprodukt des vorgenannten Fisch-Δ-5-Elongase-Gens und weiterer Δ-5-Elongasen, der Δ5-Desaturase aus Phaeodacylum sowie der Δ4-Desaturase aus Euglena konnte die Synthese von DHA in Hefe (Saccharomyces cerevisiae) nachgewiesen werden (Figur 3).

Neben der Produktion der Ausgangsfettsäuren für die im erfindungsgemäßen Verfahren vorteilhaft verwendeten Δ-5-Elongasen, Δ-6-Elongasen, Δ-9-Elongasen, Δ-4-Desaturasen, Δ-5-Desaturasen, Δ-6-Desaturasen, Δ-12-Desaturasen und/oder ω-3-Desaturasen direkt im transgenen Organismus vorteilhaft in der transgenen Pflanze können die Fettsäuren auch von außen gefüttert werden. Aus Kostengründen ist die Produktion im Organismus bevorzugt. Bevorzugt Substrate der ω-3-Desaturase sind die Linolsäure ($C18:2^{\Delta 9,12}$), die γ-Linolensäure ($C18:3^{\Delta 6,9,12}$), die Eicosadiensäure ($C20:2^{\Delta 11,14}$), die Dihomo-γ-linolensäure ($C20:3^{\Delta 8,11,14}$), die Arachidonsäure ($C20:4^{\Delta 5,8,11,14}$), die Docosatetraensäure ($C22:4^{\Delta 7,10,13,16}$) und die Docosapentaensäure ($C22:5^{\Delta 4,7,10,13,15}$).

Zur Steigerung der Ausbeute im beschriebenen Verfahren zur Herstellung von Ölen und/oder Triglyceriden mit einem vorteilhaft erhöhten Gehalt an mehrfach ungesättigten Fettsäuren ist es vorteilhaft die Menge an Ausgangsprodukt für die Fettsäuresynthese zu steigern, dies kann beispielsweise durch das Einbringen einer Nukleinsäure in den Organismus, die für ein Polypeptid mit Δ-12-Desaturase codiert, erreicht werden. Dies ist besonders vorteilhaft in Öl-produzierenden Organismen wie der Familie der Brassicaceae wie der Gattung Brassica z.B. Raps; der Familie der Elaeagnaceae wie die Gattung Elaeagnus z.B. die Gattung und Art *Olea europaea* oder der Familie Fabaceae wie der Gattung Glycine z.B. die Gattung und Art *Glycine max,* die einen hohen Ölsäuregehalt aufweisen. Da diese Organismen nur einen geringen Gehalt an Linolsäure aufweisen (Mikoklajczak et al., Journal of the American Oil Chemical Society, 38, 1961, 678 - 681) ist die Verwendung der genannten Δ-12-Desaturasen zur Herstellung des Ausgangsprodukts Linolsäure vorteilhaft.

Im erfindungsgemäßen Verfahren verwendeten Nukleinsäuren stammen vorteilhaft aus Pflanzen wie Algen beispielsweise Algen der Familie der Prasinophyceae wie aus den Gattungen Heteromastix, Mammella, Mantoniella, Micromonas, Nephroselmis, Ostreococcus, Prasinocladus, Prasinococcus, Pseudoscourfielda, Pycnococcus, Pyramimonas, Scherffelia oder Tetraselmis wie den Gattungen und Arten Heteromastix longifillis, Mamiella gilva, Mantoniella squamata, Micromonas pusilla, Nephroselmis olivacea, Nephroselmis pyriformis, Nephroselmis rotunda, Ostreococcus tauri, Ostreococcus sp. Prasinocladus ascus, Prasinocladus lubricus, Pycnococcus provasolii, Pyramimonas amylifera, Pyramimonas disomata, Pyramimonas obovata, Pyramimonas orientalis, Pyramimonas parkeae, Pyramimonas spinifera, Pyramimonas sp., Tetraselmis apiculata, Tetraselmis carteriaformis, Tetraselmis chui, Tetraselmis convolutae, Tetraselmis desikacharyi, Tetraselmis gracilis, Tetraselmis hazeni, Tetraselmis impellucida, Tetraselmis inconspicua, Tetraselmis levis, Tetraselmis maculata, Tetraselmis marina, Tetraselmis striata, Tetraselmis subcordiformis, Tetraselmis suecica, Tetraselmis tetrabrachia, Tetraselmis tetrathele, Tetraselmis verrucosa, Tetraselmis verrucosa fo. rubens oder Tetraselmis sp. oder aus Algen der Familie Euglenaceae wie aus den Gattungen Ascoglena, Astasia, Colacium, Cyclidiopsis, Euglena, Euglenopsis, Hyalophacus, Khawkinea, Lepocinclis, Phacus, Strombomonas oder Trachelomonas wie die Gattungen und Art Euglena acus, Euglena geniculata, Euglena gracilis, Euglena mixocylindracea, Euglena rostrifera, Euglena viridis, Colacium stentorium, Trachelomonas cylindrica oder Trachelomonas volvocina. Auch aus Algen wie der Alge Porphyridium cruentum, Isochrysis galbana oder Chlorella minutissima, Chlorella vulgaris, Thraustochytrium aureum oder Nannochloropsis oculata können vorteilhaft die im Verfahren verwendeten Nukleinsäuresequenzen stammen. Vorteilhaft stammen die verwendeten Nukleinsäuren aus Algen der Gattungen Euglena, Mantoniella oder Ostreococcus.

Weitere vorteilhafte Pflanzen als Quellen für die im erfindungsgemäße Verfahren verwendeten Nukleinsäuresequenzen sind Algen wie Isochrysis oder Crypthecodinium, Algen/Diatomeen wie Thalassiosira oder Phaeodactylum, Moose wie Physcomitrella oder Ceratodon oder höheren Pflanzen wie den Primulaceae wie Aleuritia, Calendula stellata, Osteospermum spinescens oder Osteospermum hyoseroides, Mikroorganismen wie Pilzen wie Aspergillus, Thraustochytrium, Phytophthora, Entomophthora, Mucor oder Mortierella, Bakterien wie Shewanella, Hefen oder Tieren wie Nematoden wie Caenorhabditis, Insekten, Fröschen, Seegurken oder Fischen. Vorteilhaft stammen die im erfindungsgemäßen Verfahren isolierten, verwendeten Nukleinsäuresequenzen aus einem Tier aus der Ordnung der Vertebraten. Bevorzugt stammen die Nukleinsäuresequenzen aus der Klasse der Vertebrata; Euteleostomi, Actinopterygii; Neopterygii; Teleostei; Euteleostei, Protacanthopterygii, Salmoniformes; Salmonidae bzw. Oncorhynchus oder Vertebrata, Amphibia, Anura, Pipidae, Xenopus oder Evertebrata wie Protochordata, Tunicata, Holothuroidea, Cionidae wie Amaroucium constellatum, Botryllus schlosseri, Ciona intestinalis, Molgula citrina, Molgula manhattensis, Perophora viridis oder Styela partita. Besonders vorteilhaft stammen die Nukleinsäuren aus Pilzen, Tieren oder aus Pflanzen wie Algen oder Moosen, bevorzugt aus der Ordnung der Salmoniformes wie der Familie der Salmonidae wie der Gattung Salmo beispielsweise aus den Gattungen und Arten Oncorhynchus mykiss, Trutta trutta oder Salmo trutta fario, aus Algen wie den Gattungen Mantoniella oder Ostreococcus oder aus den Diatomeen wie den Gattungen Thalassiosira oder Phaeodactylum oder aus Algen wie Crypthecodinium.

Auch aus Mikroorganismen wie Pilze wie der Gattung Mortierella, Phytium z.B. der Gattung und Art Mortierella alpina, Mortierella elongata, Phytium irregulare, Phytium ultimum oder Bakterien wie der Gattung Shewanella z.B. der Gattung und Art Shewanella hanedai können vorteilhafte im erfindungsgemäßen Verfahren verwendete Nukleinsäure stammen.

Vorteilhaft werden im erfindungsgemäßen Verfahren die vorgenannten Nukleinsäuresequenzen oder deren Derivat oder Homologe, die für Polypeptide codieren, die noch die enzymatische Aktivität der durch Nukleinsäuresequenzen codierten Proteine besitzen. Diese Sequenzen werden einzeln oder in Kombination mit den für die Δ-12-Desaturase, Δ-4-Desaturase, Δ-5-Desaturase, Δ-6-Desaturase, Δ-5-Elongase, Δ-6-Elongase und/oder ω-3-Desaturase codierenden Nukleinsäuresquenzen in Expressionskonstrukte cloniert und zum Einbringen und zur Expression in Organismen verwendet. Diese Expressionskonstrukte ermöglichen durch ihre Konstruktion eine vorteilhafte optimale Synthese der im erfindungsgemäßen Verfahren produzierten mehrfach ungesättigten Fettsäuren.

Bei einer bevorzugten Ausführungsform umfasst das Verfahren ferner den Schritt des Gewinnens einer transgenen Pflanze, die die im Verfahren verwendeten Nukleinsäuresequenzen enthält, wobei die Pflanze mit einer erfindungsgemäßen Nukleinsäuresequenz, die für die Δ-12-Desaturase, Δ-4-Desaturase, Δ-5-Desaturase, Δ-6-Desaturase, Δ-5-Elongase, Δ-6-Elongase und/oder ω-3-Desaturase codiert, einem Genkonstrukt oder einem Vektor wie nachfolgend beschrieben, allein oder in Kombination mit weiteren Nukleinsäuresequenzen, die für Proteine des Fettsäure- oder Lipidsstoffwechsels codieren, transformiert wird. Bei einer weiteren bevorzugten Ausführungsform umfasst dieses Verfahren ferner den Schritt des Gewinnens der Öle, Lipide oder freien Fettsäuren aus dem Samen der Pflanze wie aus dem Samen einer Ölfruchtpflanze wie beispielsweise Erdnuss, Raps, Canola, Lein, Hanf, Erdnuss, Soja, Safflower, Hanf, Sonnenblumen oder Borretsch.

Unter Anzucht ist beispielsweise die Kultivierung im Falle von Pflanzenzellen, -gewebe oder -organe auf oder in einem Nährmedium oder der ganzen Pflanze auf bzw. in einem Substrat beispielsweise in Hydrokultur, Blumentopferde oder auf einem Ackerboden zu verstehen.

Offenbart sind somit sind Genkonstrukte, die die erfindungsgemäßen Nukleinsäuresequenzen, die für eine Δ-5-Desaturase, Δ-6-Desaturase, Δ-5-Elongase oder Δ-6-Elongase codieren, enthalten, wobei die Nukleinsäure funktionsfähig mit einem oder mehreren Regulationssignalen verbunden ist. Zusätzlich können weitere Biosynthesegene des Fettsäure- oder Lipidstoffwechsels ausgewählt aus der Gruppe bestehend aus Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen oder Fettsäure-Elongase(n) im Genkonstrukt enthalten sein. Vorteilhaft sind zusätzlich Biosynthesegene des Fettsäure- oder Lipidstoffwechsels ausgewählt aus der Gruppe der Δ-8-Desaturase, Δ-9-Desaturase, Δ-9-Elongase oder ω-3-Desaturase enthalten.

Die im Verfahren verwendeten Nukleinsäuresequenzen, die für Proteine mit Δ-5-Desaturase-, Δ-6-Desaturase-, Δ-12-Desaturase-, Δ-5-Elongase- oder Δ-6-Elongase-Aktivität kodieren, werden vorteilhaft allein oder bevorzugt in Kombination in einer Expressionskassette (= Nukleinsäurekonstrukt), die die Expression der Nukleinsäuren in einer Pflanze ermöglicht, in die Pflanze eingebracht. Es kann im Nukleinsäurekonstrukt mehr als eine Nukleinsäuresequenz einer enzymatischen Aktivität wie z.B. einer Δ-12-Desaturase, Δ-5-Desaturase, Δ-6-Desaturase, Δ-5-Elongase und/oder Δ-6-Elongase enthalten sein.

Zum Einbringen der Nukleinsäuren in die Genkonstrukte werden die im Verfahren verwendeten Nukleinsäuren vorteilhaft einer Amplifikation und Ligation in bekannter Weise unterworfen. Vorzugsweise geht man in Anlehnung an das Protokoll der Pfu-DNA-Polymerase oder eines Pfu/Taq-DNA-Polymerasegemisches vor. Die Primer werden unter Berücksichtigung der zu amplifizierenden Sequenz ausgewählt. Zweckmäßigerweise sollten die Primer so gewählt werden, dass das Amplifikat die gesamte kodogene Sequenz vom Start- bis zum Stop-Kodon umfasst. Im Anschluss an die Amplifikation wird das Amplifikat zweckmäßigerweise analysiert. Beispielsweise kann nach gelelektrophoretischer Auftrennung eine quantitative und qualitative Analyse erfolgen. Im Anschluss kann das Amplifikat nach einem Standardprotokoll gereinigt werden (z.B. Qiagen). Ein Aliquot des gereinigten Amplifikats steht dann für die nachfolgende Klonierung zur Verfügung.

Geeignete Klonierungsvektoren sind dem Fachmann allgemein bekannt. Hierzu gehören insbesondere Vektoren, die in mikrobiellen Systemen replizierbar sind, also vor allem Vektoren, die eine effiziente Klonierung in Hefen oder Pilzen gewährleisten, und die die stabile Transformation von Pflanzen ermöglichen. Zu nennen sind insbesondere verschiedene für die T-DNA-vermittelte Transformation geeignete, binäre und co-integrierte Vektorsysteme. Derartige Vektorsysteme sind in der Regel dadurch gekennzeichnet, dass sie zumindest die für die Agrobakterium-vermittelte Transformation benötigten vir-Gene sowie die T-DNA begrenzenden Sequenzen (T-DNA-Border) beinhalten. Vorzugsweise umfassen diese Vektorsysteme auch weitere cisregulatorische Regionen wie Promotoren und Terminatorsequenzen und/oder Selektionsmarker, mit denen entsprechend transformierte Organismen identifiziert werden können. Während bei co-integrierten Vektorsystemen vir-Gene und T-DNA-Sequenzen auf demselben Vektor angeordnet sind, basieren binäre Systeme auf wenigstens zwei Vektoren, von denen einer vir-Gene, aber keine T-DNA und ein zweiter

T-DNA, jedoch kein vir-Gen trägt. Dadurch sind letztere Vektoren relativ klein, leicht zu manipulieren und sowohl in *E. coli* als auch in Agrobacterium zu replizieren. Zu diesen binären Vektoren gehören Vektoren der Serien pBIB-HYG, pPZP, pBecks, pGreen. Erfindungsgemäß werden bevorzugt Bin19, pBI101, pBinAR, pGPTV und pCAMBIA verwendet. Eine Übersicht über binäre Vektoren und ihre Verwendung gibt Hellens et al, Trends in Plant Science (2000) 5, 446-451.

Für die Vektorpräparation können die Vektoren zunächst mit Restriktionsendonuklease(n) linearisiert und dann in geeigneter Weise enzymatisch modifiziert werden. Im Anschluss wird der Vektor gereinigt und ein Aliquot für die Klonierung eingesetzt. Bei der Klonierung wird das enzymatisch geschnittene und erforderlichenfalls gereinigte Amplifikat mit ähnlich präparierten Vektorfragmenten unter Einsatz von Ligase verbunden. Dabei kann ein bestimmtes Nukleinsäurekonstrukt bzw. Vektor- oder Plasmidkonstrukt einen oder auch mehrere kodogene Genabschnitte aufweisen. Vorzugsweise sind die kodogenen Genabschnitte in diesen Konstrukten mit regulatorischen Sequenzen funktional verknüpft. Zu den regulatorischen Sequenzen gehören insbesondere pflanzliche Sequenzen wie Promotoren und Terminatorsequenzen. Die Konstrukte lassen sich vorteilhafterweise in Mikroorganismen, insbesondere in *E. coli* und *Agrobacterium tumefaciens,* unter Selektionsbedingungen stabil propagieren und ermöglichen einen Transfer von heterologer DNA in Pflanzen oder Mikroorganismen.

Unter der vorteilhaften Verwendung von Klonierungsvektoren können die im Verfahren verwendeten Nukleinsäuren in Pflanzen eingebracht werden und damit bei der Transformation von Pflanzen verwendet werden, wie denjenigen, die veröffentlicht und dort zitiert sind: Plant Molecular Biology and Biotechnology (CRC Press, Boca Raton, Florida), Kapitel 6/7, S. 71-119 (1993); F.F. White, Vectors for Gene Transfer in Higher Plants; in: Transgenic Plants, Bd. 1, Engineering and Utilization, Hrsgb.: Kung und R. Wu, Academic Press, 1993, 15-38; B. Jenes et al., Techniques for Gene Transfer, in: Transgenic Plants, Bd. 1, Engineering and Utilization, Hrsgb.: Kung und R. Wu, Academic Press (1993), 128-143; Potrykus, Annu. Rev. Plant Physiol. Plant Molec. Biol. 42 (1991), 205-225. Die im Verfahren verwendeten Nukleinsäuren und/oder Vektoren lassen sich damit zur gentechnologischen Veränderung eines breiten Spektrums an Pflanzen verwenden, so dass diese bessere und/oder effizientere Produzenten von PUFAs werden.

Es gibt eine Reihe von Mechanismen, durch die eine Veränderung des Δ-12-Desaturase-, Δ-5-Elongase-, Δ-6-Elongase-, Δ-5-Desaturase- und/oder Δ-6-Desaturase-Proteins möglich ist, so dass die Ausbeute, Produktion und/oder Effizienz der Produktion der mehrfach ungesättigten Fettsäuren in einer Pflanze, bevorzugt in einer Ölsamen- oder Ölfruchtpflanze, aufgrund dieses veränderten Proteins direkt beeinflusst werden kann. Die Anzahl oder Aktivität der Δ-12-Desaturase-, Δ-6-Desaturase-, Δ-5-Elongase-, Δ-6-Elongase- oder Δ-5-Desaturase-Proteine oder -Gene kann erhöht werden, so dass größere Mengen der Genprodukte und damit letztlich größere Mengen der Verbindungen der allgemeinen Formel I hergestellt werden. Auch eine de novo Synthese in einer Pflanze, der die Aktivität und Fähigkeit zur Biosynthese der Verbindungen vor dem Einbringen des/der entsprechenden Gens/Gene fehlte, ist möglich. Entsprechendes gilt für die Kombination mit weiteren Desaturasen oder Elongasen oder weiteren Enzymen aus dem Fettsäure- und Lipidstoffwechsel. Auch die Verwendung verschiedener divergenter, d.h. auf DNA-Sequenzebene unterschiedlicher Sequenzen kann dabei vorteilhaft sein bzw. die Verwendung von Promotoren, die eine andere zeitliche Genexpression z.B. abhängig vom Reifegrad eines Samens oder Öl-speichernden Gewebes ermöglichen.

Durch das Einbringen einer Kombination von Δ-12-Desaturase-, Δ-6-Desaturase-, Δ-5-Elongase-, Δ-6-Elongase- und/oder Δ-5-Desaturase-Genen in die Pflanze allein oder in Kombination mit anderen Genen kann nicht nur der Biosynthesefluss zum Endprodukt erhöht, sondern auch die entsprechende Triacylglycerin-Zusammensetzung erhöht oder de novo geschaffen werden. Ebenso kann die Anzahl oder Aktivität anderer Gene, die am Import von Nährstoffen, die zur Biosynthese einer oder mehrerer Fettsäuren, Ölen, polaren und/oder neutralen Lipiden nötig sind, erhöht sein, so dass die Konzentration dieser Vorläufer, Cofaktoren oder Zwischenverbindungen innerhalb der Zellen oder innerhalb des Speicherkompartiments erhöht ist, wodurch die Fähigkeit der Zellen zur Produktion von PUFAs weiter gesteigert wird. Durch Optimierung der Aktivität oder Erhöhung der Anzahl eines oder mehrerer Δ-12-Desaturase-, Δ-6-Desaturase-, Δ-5-Elongase-, Δ-6-Elongase- oder Δ-5-Desaturase-Gene, die an der Biosynthese dieser Verbindungen beteiligt sind, oder durch Zerstören der Aktivität einer oder mehrerer Gene, die am Abbau dieser Verbindungen beteiligt sind, wird die Steigerung der Ausbeute, Produktion und/oder Effizienz der Produktion von Fettsäure-und Lipidmolekülen in Pflanzen ermöglicht.

Die im Verfahren verwendeten Nukleinsäuresequenzen werden vorteilhaft in einer Expressionskassette, die die Expression der Nukleinsäuren in Pflanzen ermöglicht, eingebracht.

Dabei werden die Nukleinsäuresequenzen, die für die Δ-12-Desaturase, Δ-6-Desaturase, Δ-5-Elongase, Δ-6-Elongase oder Δ-5-Desaturase kodieren, mit einem oder mehreren Regulationssignalen vorteilhafterweise zur Erhöhung der Genexpression funktionell verknüpft. Diese regulatorischen Sequenzen sollen die gezielte Expression der Gene und Proteine ermöglichen. Dies kann beispielsweise je nach Wirtsorganismus bedeuten, dass das Gen erst nach Induktion exprimiert und/oder überexprimiert wird, oder dass es sofort exprimiert und/oder überexprimiert wird. Beispielsweise handelt es sich bei diesen regulatorischen Sequenzen um Sequenzen, an die Induktoren oder Repressoren binden und dadurch die Expression der Nukleinsäure regulieren. Zusätzlich zu diesen neuen Regulationssequenzen oder anstelle dieser Sequenzen können die natürlichen Regulationselemente dieser Sequenzen vor den eigentlichen Strukturgenen noch vorhanden sein und gegebenenfalls genetisch so verändert worden sein, dass ihre natürliche Regulation

ausgeschaltet und die Expression der Gene erhöht wird. Diese veränderten Promotoren können in Form von Teilsequenzen (= Promotor mit Teilen der erfindungsgemäß verwendeten Nukleinsäuresequenzen) auch allein vor das natürliche Gen zur Steigerung der Aktivität gebracht werden. Das Genkonstrukt kann außerdem vorteilhafterweiser auch eine oder mehrere sogenannte "Enhancer-Sequenzen" funktionell verknüpft mit dem Promotor enthalten, die eine erhöhte Expression der Nukleinsäuresequenz ermöglichen. Auch am 3'-Ende der DNA-Sequenzen können zusätzliche vorteilhafte Sequenzen inseriert werden wie weitere regulatorische Elemente oder Terminatorsequenzen.

Die Δ-12-Desaturase-, Δ-5-Desaturase-, Δ-6-Desaturase-, Δ-5-Elongase- und/oder Δ-6-Elongase-Gene können in einer oder mehreren Kopien in der Expressionskassette (= Genkonstrukt) enthalten sein. Vorteilhaft liegt nur jeweils eine Kopie der Gene in der Expressionskassette vor. Dieses Genkonstrukt oder die Genkonstrukte können zusammen in der Wirtspflanze exprimiert werden. Dabei kann das Genkonstrukt oder die Genkonstrukte in einem oder mehreren Vektoren inseriert sein und frei in der Zelle vorliegen oder aber im Genom inseriert sein. Es ist vorteilhaft für die Insertion weiterer Gene im Wirtsgenom, wenn die zu exprimierenden Gene zusammen in einem Genkonstrukt vorliegen.

Die regulatorischen Sequenzen bzw. Faktoren können dabei wie oben beschrieben vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Offenbart sind somit ein oder mehrere Genkonstrukte, die eine oder mehrere Sequenzen enthalten, die durch SEQ ID NO: 11, SEQ ID NO: 27, SEQ ID NO: 193, SEQ ID NO: 195, SEQ ID NO: 197, SEQ ID NO: 199, SEQ ID NO: 201 oder deren Derivate definiert sind und für Polypeptide gemäß SEQ ID NO: 12, SEQ ID NO: 28, SEQ ID NO: 194, SEQ ID NO: 196, SEQ ID NO: 198, SEQ ID NO: 200, SEQ ID NO: 202 kodieren. Die genannten Δ-12-Desaturase-, Δ-6-Desaturase-, Δ-5-Elongase-, Δ-6-Elongase-oder Δ-5-Desaturase-Proteine führen dabei vorteilhaft zu einer Desaturierung oder Elongierung von Fettsäuren, wobei das Substrat vorteilhaft ein, zwei, drei oder vier Doppelbindungen und vorteilhaft 18, 20 oder 22 Kohlenstoffatome im Fettsäuremolekül aufweist. Gleiches gilt für ihre Homologen, Derivate oder Analoga, die funktionsfähig mit einem oder mehreren Regulationssignalen, vorteilhafterweise zur Steigerung der Genexpression, verbunden sind.

Es ist im Prinzip möglich, alle natürlichen Promotoren mit ihren Regulationssequenzen, wie die oben genannten, für das neue Verfahren zu verwenden. Es ist ebenfalls möglich und vorteilhaft, zusätzlich oder alleine synthetische Promotoren zu verwenden, besonders wenn sie eine Samen-spezifische Expression vermitteln, wie z.B. die in WO 99/16890 beschriebenen.

Um einen besonders hohen Gehalt an PUFAs vor allem in transgenen Pflanzen zu erzielen, sollten die PUFA-Biosynthesegene vorteilhaft samenspezifisch in Ölsaaten exprimiert werden. Hierzu können Samen-spezifische Promotoren verwendet werden, bzw. solche Promotoren, die im Embryo und/oder im Endosperm aktiv sind. Samen-spezifische Promotoren können prinzipiell sowohl aus dikotyledonen als auch aus monokotyledonen Pflanzen isoliert werden. Im folgenden sind bevorzugte Promotoren aufgeführt: USP (= unknown seed protein) und Vicilin (Vicia faba) [Bäumlein et al., Mol. Gen Genet., 1991, 225(3)], Napin (Raps) [US 5,608,152], Conlinin (Lein) [WO 02/102970], Acyl-Carrier Protein (Raps) [US 5,315,001 und WO 92/18634], Oleosin (Arabidopsis thaliana) [WO 98/45461 und WO 93/20216], Phaseolin (Phaseolus vulgaris) [US 5,504,200], Bce4 [WO 91/13980], Leguminosen B4 (LegB4-Promotor) [Bäumlein et al., Plant J., 2,2, 1992], Lpt2 und lpt1 (Gerste) [WO 95/15389 u. WO95/23230], Samen-spezifische Promotoren aus Reis, Mais u. Weizen [WO 99/16890], Amy32b, Amy 6-6 und Aleurain [US 5,677,474], Bce4 (Raps) [US 5,530,149], Glycinin (Soja) [EP 571 741], Phosphoenol-Pyruvatcarboxylase (Soja) [JP 06/62870], ADR12-2 (Soja) [WO 98/08962], Isocitratlyase (Raps) [US 5,689,040] oder α-Amylase (Gerste) [EP 781 849].

Die Pflanzengenexpression lässt sich auch über einen chemisch induzierbaren Promotor erleichtern (siehe eine Übersicht in Gatz 1997, Annu. Rev. Plant Physiol. Plant Mol. Biol., 48:89-108). Chemisch induzierbare Promotoren eignen sich besonders, wenn gewünscht wird, dass die Genexpression auf zeitspezifische Weise erfolgt. Beispiele für solche Promotoren sind ein Salicylsäure-induzierbarer Promotor (WO 95/19443), ein Tetracyclin-induzierbarer Promotor (Gatz et al. (1992) Plant J. 2, 397-404) und ein Ethanol-induzierbarer Promotor.

Um eine stabile Integration der Biosynthesegene in die transgene Pflanze über mehrere Generation sicherzustellen, sollte jede der im Verfahren verwendeten Nukleinsäuren, die für die Δ-12-Desaturase, Δ-6-Desaturase, Δ-5-Elongase, Δ-6-Elongase und/oder Δ-5-Desaturase kodieren, unter der Kontrolle eines eigenen, bevorzugt eines von den anderen Promotoren verschiedenen, Promotors exprimiert werden, da sich wiederholende Sequenzmotive zur Instabilität der T-DNA bzw. zu Rekombinationsereignissen führen können. Die Expressionskassette ist dabei vorteilhaft so aufgebaut, dass einem Promotor eine geeignete Schnittstelle, vorteilhaft in einem Polylinker, zur Insertion der zu exprimierenden Nukleinsäure folgt und gegebenenfalls eine Terminatorsequenz hinter dem Polylinker liegt. Diese Abfolge wiederholt sich mehrfach, bevorzugt drei-, vier-, fünf-, sechs- oder siebenmal, so dass bis zu sieben Gene in einem Konstrukt zusammengeführt werden und zur Expression in die transgene Pflanze eingebracht werden können. Vorteilhaft wiederholt sich die Abfolge bis zu viermal. Die Nukleinsäuresequenzen werden zur Expression über eine geeignete

Schnittstelle beispielsweise im Polylinker hinter den Promotor inseriert. Vorteilhaft hat jede Nukleinsäuresequenz ihren eigenen Promotor und gegebenenfalls ihre eigene Terminatorsequenz. Derartige vorteilhafte Konstrukte sind beispielsweise in DE 101 02 337 oder DE 101 02 338 offenbart. Es ist aber auch möglich, mehrere Nukleinsäuresequenzen hinter einem gemeinsamen Promotor und ggf. vor einer gemeinsamen Terminatorsequenz zu inserieren. Dabei ist die Insertionsstelle bzw. die Abfolge der inserierten Nukleinsäuren in der Expressionskassette nicht von entscheidender Bedeutung, das heißt eine Nukleinsäuresequenz kann an erster oder letzter Stelle in der Kassette inseriert sein, ohne dass dadurch ihre Expression wesentlich beeinflusst wird. Es können in der Expressionskassette vorteilhaft unterschiedliche Promotoren wie beispielsweise der USP-, LegB4 oder DC3-Promotor und unterschiedliche Terminatorsequenzen verwendet werden. Es ist aber auch möglich, nur einen Promotortyp in der Kassette zu verwenden, was jedoch zu unerwünschten Rekombinationsereignissen führen kann.

Wie oben beschrieben sollte die Transkription der eingebrachten Gene vorteilhaft durch geeignete Terminatorsequenzen am 3'-Ende der eingebrachten Biosynthesegene (hinter dem Stopcodon) abgebrochen werden. Verwendet werden kann hier z.B. die OCS1-Terminatorsequenz. Wie auch für die Promotoren, so sollten für jedes Gen unterschiedliche Terminatorsequenzen verwendet werden.

Das Genkonstrukt kann, wie oben beschrieben, auch weitere Gene umfassen, die in die Pflanzen eingebracht werden sollen. Es ist möglich und vorteilhaft, in die Wirtspflanzen Regulationsgene, wie Gene für Induktoren, Repressoren oder Enzyme, welche durch ihre Enzymaktivität in die Regulation eines oder mehrerer Gene eines Biosynthesewegs eingreifen, einzubringen und zu exprimieren. Diese Gene können heterologen oder homologen Ursprungs sein.

Weiterhin können vorteilhaft im Nukleinsäurekonstrukt bzw. Genkonstrukt weitere Biosynthesegene des Fettsäure- oder Lipidstoffwechsels enthalten sein, diese Gene können aber auch auf einem oder mehreren weiteren Nukleinsäurekonstrukten liegen. Vorteilhaft werden als Biosynthesegen des Fettsäure- oder Lipidstoffwechsels ein Gen ausgewählt aus der Gruppe bestehend aus Acyl-CoA-Dehydrogenase(n), Acyl-ACP[= acyl carrier protein]-Desaturase(n), Acyl-ACP-Thioesterase(n), Fettsäure-AcylTransferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenase(n), Lipoxygenase(n), Triacylglycerol-Lipase(n), Allenoxid-Synthase(n), Hydroperoxid-Lyase(n) oder Fettsäure-Elongase(n) oder Kombinationen davon verwendet.

Besonders vorteilhafte Nukleinsäuresequenzen sind Biosynthesegene des Fettsäure-oder Lipidstoffwechsels ausgewählt aus der Gruppe der Acyl-CoA:Lysophospholipid-Acyltransferase, ω-3-Desaturase, Δ-8-Desaturase, Δ-4-Desaturase, Δ-9-Desaturase, Δ-5-Elongase und/oder Δ-9-Elongase.

Dabei können die vorgenannten Nukleinsäuren bzw. Gene in Kombination mit anderen Elongasen und Desaturasen in Expressionskassetten, wie den vorgenannten, kloniert werden und zur Transformation von Pflanzen mit Hilfe von Agrobakterium eingesetzt werden.

Die regulatorischen Sequenzen bzw. Faktoren können dabei wie oben beschrieben vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird. Die Expressionskassetten können prinzipiell direkt zum Einbringen in die Pflanze verwendet werden oder aber in einen Vektor eingebracht werden.

Diese vorteilhaften Vektoren, vorzugsweise Expressionsvektoren, enthalten die im Verfahren verwendeten Nukleinsäuren, die für die Δ-12-Desaturasen, Δ-6-Desaturasen, Δ-5-Elongasen, Δ-6-Elongasen oder Δ-5-Desaturasen kodieren, oder ein Nukleinsäurekonstrukt, das die verwendete Nukleinsäure allein oder in Kombination mit weiteren Biosynthesegenen des Fettsäure- oder Lipidstoffwechsels wie den Acyl-CoA:Lysophospholipid-Acyltransferasen, ω-3-Desaturasen, Δ-8-Desaturasen, Δ-9-Desaturasen, ω3-Desaturasen, Δ-4-Desaturasen, Δ-5-Elongasen und/oder Δ-9-Elongasen enthält.

Wie hier verwendet, betrifft der Begriff "Vektor" ein Nukleinsäuremolekül, das eine andere Nukleinsäure transportieren kann, die an es gebunden ist. Ein Vektortyp ist ein "Plasmid", eine zirkuläre doppelsträngige DNA-Schleife, in die zusätzliche DNA-Segmente ligiert werden können. Ein weiterer Vektortyp ist ein viraler Vektor, wobei zusätzliche DNA-Segmente in das virale Genom ligiert werden können. Bestimmte Vektoren können in einer Wirtszelle, in die sie eingebracht worden sind, autonom replizieren (z.B. Bakterienvektoren mit bakteriellem Replikationsursprung). Andere Vektoren werden vorteilhaft beim Einbringen in die Wirtszelle in das Genom einer Wirtszelle integriert und dadurch zusammen mit dem Wirtsgenom repliziert. Zudem können bestimmte Vektoren die Expression von Genen, mit denen sie funktionsfähig verbunden sind, steuern. Diese Vektoren werden hier als "Expressionsvektoren" bezeichnet. Gewöhnlich haben Expressionsvektoren, die für DNA-Rekombinationstechniken geeignet sind, die Form von Plasmiden. In der vorliegenden Beschreibung können "Plasmid" und "Vektor" austauschbar verwendet werden, da das Plasmid die am häufigsten verwendete Vektorform ist. Die Erfindung soll jedoch auch andere Expressionsvektorformen, wie virale Vektoren, die ähnliche Funktionen ausüben, umfassen. Ferner soll der Begriff "Vektor" auch andere Vektoren, die dem Fachmann bekannt sind, wie Phagen, Viren, wie SV40, CMV, TMV, Transposons, IS-Elemente, Phasmide, Phagemide, Cosmide, lineare oder zirkuläre DNA, umfassen.

Die im Verfahren vorteilhaft verwendeten rekombinanten Expressionsvektoren umfassen die erfindungsgemäß verwendeten Nukleinsäuren oder das beschriebene Genkonstrukt in einer Form, die sich zur Expression der verwendeten Nukleinsäuren in einer Wirtszelle eignet, was bedeutet, dass die rekombinanten Expressionsvektoren eine oder mehrere Regulationssequenzen, ausgewählt auf der Basis der zur Expression verwendeten Wirtszellen, die mit der zu exprimierenden Nukleinsäuresequenz funktionsfähig verbunden ist, umfassen. In einem rekombinanten Expressionsvektor bedeutet "funktionsfähig verbunden", dass die Nukleotidsequenz von Interesse derart an die Regulationssequenz(en) gebunden ist, dass die Expression der Nukleotidsequenz möglich ist und sie aneinander gebunden sind, so dass beide Sequenzen die vorhergesagte, der Sequenz zugeschriebene Funktion erfüllen (z.B. in einem In-vitro-Transkriptions-/Translationssystem oder in einer Wirtszelle, wenn der Vektor in die Wirtszelle eingebracht wird).

Der Begriff "Regulationssequenz" soll Promotoren, Enhancer und andere Expressionskontrollelemente (z.B. Polyadenylierungssignale) umfassen. Diese Regulationssequenzen sind z.B. beschrieben in Goeddel: Gene Expression Technology: Methods in Enzymology 185, Academic Press, San Diego, CA (1990), oder siehe: Gruber und Crosby, in: Methods in Plant Molecular Biology and Biotechnolgy, CRC Press, Boca Raton, Florida, Hrsgb.: Glick und Thompson, Kapitel 7, 89-108, einschließlich der Literaturstellen darin. Regulationssequenzen umfassen solche, welche die konstitutive Expression einer Nukleotidsequenz in vielen Wirtszelltypen steuern, und solche, die die direkte Expression der Nukleotidsequenz nur in bestimmten Wirtszellen unter bestimmten Bedingungen steuern. Der Fachmann weiß, dass die Gestaltung des Expressionsvektors von Faktoren, wie der Auswahl der zu transformierenden Wirtszelle, der gewünschten Expressionsstärke des Proteins usw., abhängen kann.

Bei einer weiteren Ausführungsform des Verfahrens können die Δ-12-Desaturasen, Δ-6-Desaturasen, Δ-5-Elongasen, Δ-6-Elongasen und/oder Δ-5-Desaturasen in einzelligen Pflanzenzellen (wie Algen), siehe Falciatore et al., 1999, Marine Biotechnology 1 (3):239-251 und darin zitierte Literaturangaben, und Pflanzenzellen aus höheren Pflanzen (z.B. Spermatophyten, wie Feldfrüchten) exprimiert werden. Beispiele für Pflanzen-Expressionsvektoren umfassen solche, die eingehend beschrieben sind in: Becker, D., Kemper, E., Schell, J., und Masterson, R. (1992) "New plant binary vectors with selectable markers located proximal to the left border", Plant Mol. Biol. 20:1195-1197; und Bevan, M.W. (1984) "Binary Agrobacterium vectors for plant transformation", Nucl. Acids Res. 12:8711-8721; Vectors for Gene Transfer in Higher Plants; in: Transgenic Plants, Bd. 1, Engineering and Utilization, Hrsgb.: Kung und R. Wu, Academic Press, 1993, S. 15-38.

Eine Pflanzen-Expressionskassette enthält vorzugsweise Regulationssequenzen, welche die Genexpression in Pflanzenzellen steuern können und die funktionsfähig verbunden sind, so dass jede Sequenz ihre Funktion, wie Termination der Transkription, erfüllen kann, beispielsweise Polyadenylierungssignale. Bevorzugte Polyadenylierungssignale sind diejenigen, die aus *Agrobacterium tumefaciens-T-DNA* stammen, wie das als Octopinsynthase bekannte Gen 3 des Ti-Plasmids pTiACH5 (Gielen et al., EMBO J. 3 (1984) 835ff.) oder funktionelle Äquivalente davon, aber auch alle anderen in Pflanzen funktionell aktive Terminatorsequenzen sind geeignet.

Da die Regulation der Pflanzengenexpression sehr oft nicht auf Transkriptionsebene beschränkt ist, enthält eine Pflanzen-Expressionskassette vorzugsweise andere funktionsfähig verbundene Sequenzen, wie Translationsenhancer, beispielsweise die Overdrive-Sequenz, welche die 5'-untranslatierte Leader-Sequenz aus Tabakmosaikvirus, die das Protein/RNA-Verhältnis erhöht, enthält (Gallie et al., 1987, Nucl. Acids Research 15:8693-8711).

Das zu exprimierende Gen muss, wie oben beschrieben, funktionsfähig mit einem geeigneten Promotor verbunden sein, der die Genexpression auf rechtzeitige, zell-oder gewebespezifische Weise auslöst. Nutzbare Promotoren sind konstitutive Promotoren (Benfey et al., EMBO J. 8 (1989) 2195-2202), wie diejenigen, die von Pflanzenviren stammen, wie 35S CAMV (Franck et al., Cell 21 (1980) 285-294), 19S CaMV (siehe auch US 5352605 und WO 84/02913) oder konstitutive Pflanzenpromotoren, wie der in US 4,962,028 beschriebene der kleinen Untereinheit der Rubisco.

Die Pflanzengenexpression lässt sich auch wie oben beschrieben über einen chemisch induzierbaren Promotor erreichen (siehe eine Übersicht in Gatz 1997, Annu. Rev. Plant Physiol. Plant Mol. Biol., 48:89-108). Chemisch induzierbare Promotoren eignen sich besonders, wenn gewünscht wird, dass die Genexpression auf zeitspezifische Weise erfolgt. Beispiele für solche Promotoren sind ein Salicylsäure-induzierbarer Promotor (WO 95/19443), ein Tetracyclin-induzierbarer Promotor (Gatz et al. (1992) Plant J. 2, 397-404) und ein Ethanol-induzierbarer Promotor.

Auch Promotoren, die auf biotische oder abiotische Stressbedingungen reagieren, sind geeignet, beispielsweise der pathogeninduzierte PRP1-Gen-Promotor (Ward et al., Plant. Mol. Biol. 22 (1993) 361-366), der hitzeinduzierbare hsp80-Promotor aus Tomate (US 5,187,267), der kälteinduzierbare Alpha-Amylase-Promotor aus Kartoffel (WO 96/12814) oder der durch Wunden induzierbare pinII-Promotor (EP-A-0 375 091).

Es sind insbesondere solche Promotoren bevorzugt, welche die Genexpression in Geweben und Organen herbeiführen, in denen die Fettsäure-, Lipid- und Ölbiosynthese stattfindet, in Samenzellen, wie den Zellen des Endosperms und des sich entwickelnden Embryos. Geeignete Promotoren sind der Napin-Promotor aus Raps (US 5,608,152), der Conlinin-Promotor aus Lein (WO 02/102970), der USP-Promotor aus Vicia faba (Baeumlein et al., Mol Gen Genet, 1991, 225 (3):459-67), der Oleosin-Promotor aus Arabidopsis (WO 98/45461), der Phaseolin-Promotor aus Phaseolus vulgaris (US 5,504,200), der Bce4-Promotor aus Brassica (WO 91/13980) oder der Legumin-B4-Promotor (LeB4; Baeumlein et al., 1992, Plant Journal, 2 (2):233-9) sowie Promotoren, die die samenspezifische Expression in

monokotyledonen Pflanzen, wie Mais, Gerste, Weizen, Roggen, Reis usw. herbeiführen. Geeignete beachtenswerte Promotoren sind der Ipt2- oder Ipt1-Gen-Promotor aus Gerste (WO 95/15389 und WO 95/23230) oder die in WO 99/16890 beschriebenen Promotoren aus dem Gersten-Hordein-Gen, dem Reis-Glutelin-Gen, dem Reis-Oryzin-Gen, dem Reis-Prolamin-Gen, dem Weizen-Gliadin-Gen, Weizen-Glutelin-Gen, dem Mais-Zein-Gen, dem Hafer-Glutelin-Gen, dem Sorghum-Kasirin-Gen, dem Roggen-Secalin-Gen.

Ebenfalls besonders geeignet sind Promotoren, welche die plastidenspezifische Expression herbeiführen, da Plastiden das Kompartiment sind, in dem die Vorläufer sowie einige Endprodukte der Lipidbiosynthese synthetisiert werden. Geeignete Promotoren sind der virale RNA-Polymerase-Promotor, beschrieben in WO 95/16783 und WO 97/06250, und der clpP-Promotor aus Arabidopsis, beschrieben in WO 99/46394.

Insbesondere kann die multiparallele Expression der im Verfahren verwendeten Δ-12-Desaturasen, Δ-6-Desaturasen, Δ-5-Elongasen, Δ-6-Elongasen und/oder Δ-5-Desaturasen gewünscht sein. Die Einführung solcher Expressionskassetten kann über eine simultane Transformation mehrerer einzelner Expressionskonstrukte erfolgen oder bevorzugt durch Kombination mehrerer Expressionskassetten auf einem Konstrukt. Auch können mehrere Vektoren mit jeweils mehreren Expressionskassetten transformiert und auf die Wirtszelle übertragen werden.

Andere bevorzugte Sequenzen für die Verwendung zur funktionsfähigen Verbindung in Pflanzengenexpressions-Kassetten sind Targeting-Sequenzen, die zur Steuerung des Genproduktes in sein entsprechendes Zellkompartiment, beispielsweise in die Vakuole, den Zellkern, alle Arten von Plastiden, wie Amyloplasten, Chloroplasten, Chromoplasten, den extrazellulären Raum, die Mitochondrien, das Endoplasmatische Retikulum, Ölkörper, Peroxisomen und andere Kompartimente von Pflanzenzellen notwendig sind (siehe eine Übersicht in Kermode, Crit. Rev. Plant Sci. 15, 4 (1996) 285-423 und darin zitierte Literaturstellen).

Im erfindungsgemäßen Verfahren werden die Nukleinsäuresequenzen mit den SEQ ID NO: 11, SEQ ID NO: 27, SEQ ID NO: 193, SEQ ID NO: 195, SEQ ID NO:197, SEQ ID NO: 199, SEQ ID NO: 201 oder deren Derivate oder Homologe, die für Polypeptide kodieren, die noch die enzymatische Aktivität der durch Nukleinsäuresequenzen kodierten Proteine besitzen, verwendet. Diese Sequenzen werden einzeln oder in Kombination mit den Nukleinsäuresquenzen, die für die anderen verwendeten Enzyme kodieren, in Expressionskonstrukte kloniert und zur Transformation und Expression in Pflanzen verwendet. Diese Expressionskonstrukte ermöglichen durch ihre Konstruktion eine vorteilhafte optimale Synthese der im erfindungsgemäßen Verfahren produzierten mehrfach ungesättigten Fettsäuren.

Bei einer bevorzugten Ausführungsform umfasst das Verfahren ferner den Schritt des Gewinnens einer Zelle oder einer ganzen Pflanze, die die im Verfahren verwendeten Nukleinsäuresequenzen enthält, wobei die Zelle und/oder die Pflanze mit einer Nukleinsäuresequenz, die für ein Polypeptid mit einer Δ-12-Desaturase-, Δ-5-Desaturase-, Δ-6-Desaturase-, Δ-5-Elongase- und/oder Δ-6-Elongase-Aktivität kodiert, einem Genkonstrukt oder einem Vektor wie vorstehend beschrieben, allein oder in Kombination mit weiteren Nukleinsäuresequenzen, die für Proteine des Fettsäure- oder Lipidstoffwechsels kodieren, transformiert wird. Die so hergestellte Zelle ist vorteilhaft eine Zelle eines Öl-produzierenden Organismus wie einer Ölfruchtpflanze wie beispielsweise Erdnuss, Raps, Canola, Lein, Hanf, Erdnuss, Soja, Färbersaflor, Hanf, Senf, Sonnenblumen oder Borretsch.

"Transgen" bzw. "Rekombinant" im Sinne der Erfindung bedeutet bezüglich zum Beispiel einer Nukleinsäuresequenz, einer Expressionskassette (= Genkonstrukt) oder einem Vektor enthaltend die erfindungsgemäße Nukleinsäuresequenz oder einem Organismus transformiert mit den erfindungsgemäßen Nukleinsäuresequenzen, Expressionskassette oder Vektor alle solche durch gentechnische Methoden zustandegekommenen Konstruktionen, in denen sich entweder

a) die erfindungsgemäße Nukleinsäuresequenz, oder

b) eine mit der erfindungsgemäßen Nukleinsäuresequenz funktionell verknüpfte genetische Kontrollsequenz, zum Beispiel ein Promotor, oder

c) (a) und (b)

sich nicht in ihrer natürlichen, genetischen Umgebung befinden oder durch gentechnische Methoden modifiziert wurden, wobei die Modifikation beispielhaft eine Substitution, Addition, Deletion, Inversion oder Insertion eines oder mehrerer Nukleotidreste sein kann. Natürliche genetische Umgebung meint den natürlichen genomischen bzw. chromosomalen Locus in dem Herkunftsorganismus oder das Vorliegen in einer genomischen Bibliothek. Im Fall einer genomischen Bibliothek ist die natürliche, genetische Umgebung der Nukleinsäuresequenz bevorzugt zumindest noch teilweise erhalten. Die Umgebung flankiert die Nukleinsäuresequenz zumindest an einer Seite und hat eine Sequenzlänge von mindestens 50 bp, bevorzugt mindestens 500 bp, besonders bevorzugt mindestens 1000 bp, ganz besonders bevorzugt mindestens 5000 bp. Eine natürlich vorkommende Expressionskassette - beispielsweise die natürlich vorkommende Kombination des natürlichen Promotors der im erfindungsgemäßen Verfahren verwendeten Nukleinsäuresequenzen mit den entsprechenden Δ-12-Desaturase-, Δ-4-Desaturase-, Δ-5-Desaturase-, Δ-6-Desaturase-, Δ-8-Desaturase-, ω-3-Desaturase-, Δ-9-Elongase-, Δ-6-Elongase- und/oder Δ-5-Elongasegenen - wird zu einer transgenen Expressionskassette, wenn diese durch nicht-natürliche, synthetische ("künstliche") Verfahren wie beispielsweise einer Mutagenisierung geändert wird. Entsprechende Verfahren sind beispielsweise beschrieben in US 5,565,350 oder WO 00/15815.

Unter transgenen Pflanzen im Sinne der Erfindung ist daher zu verstehen, dass sich die im Verfahren verwendeten Nukleinsäuren nicht an ihrer natürlichen Stelle im Genom der Pflanze befinden, wobei die Nukleinsäuren homolog oder heterolog exprimiert werden können. Transgen bedeutet aber auch, dass die erfindungsgemäßen Nukleinsäuren an ihrem natürlichen Platz im Genom der Pflanze sind, dass jedoch die Sequenz gegenüber der natürlichen Sequenz verändert wurde und/oder das die Regulationssequenzen, der natürlichen Sequenz verändert wurden. Bevorzugt ist unter transgen die Expression der erfindungsgemäßen Nukleinsäuren oder der im erfindungsgemäßen Verfahren verwendeten Nukleinsäuresequenzen an nicht natürlicher Stelle im Genom zu verstehen, das heißt eine homologe oder bevorzugt heterologe Expression der Nukleinsäuren liegt vor. Bevorzugte transgene Pflanzen sind Ölsamen-oder Ölfruchtpflanzen.

Als Pflanzen zur Verwendung im erfindungsgemäßen Verfahren eignen sich prinzipiell vorteilhaft alle Pflanzen, die in der Lage sind Fettsäuren, speziell ungesättigte Fettsäuren wie ARA, EPA und/oder DHA, zu synthetisieren und die für die Expression rekombinanter Gene geeignet sind. Beispielhaft seien Pflanzen wie Arabidopsis, Asteraceae wie Calendula oder Kulturpflanzen wie Soja, Erdnuss, Rizinus, Sonnenblume, Mais, Baumwolle, Flachs, Raps, Kokosnuss, Ölpalme, FärberSaflor (Carthamus tinctorius) oder Kakaobohne genannt. Bevorzugt werden Pflanzen, die natürlicherweise Öle in größeren Mengen synthetisieren können wie Soja, Raps, Camelina, Sareptasenf, Kokosnuss, Ölpalme, Färbersaflor (Carthamus tinctorius), Flachs, Hanf, Rizinus, Calendula, Erdnuss, Kakaobohne oder Sonnenblume oder Hefen wie Saccharomyces cerevisiae, besonders bevorzugt werden Soja, Flachs, Raps, FärberSaflor, Sonnenblume, Camelina, Sareptasenf oder Calendula.

Weitere für die Klonierung der im erfindungsgemäßen Verfahren verwendeten Nukleinsäuresequenzen nutzbare Wirtszellen sind weiterhin genannt in: Goeddel, Gene Expression Technology: Methods in Enzymology 185, Academic Press, San Diego, CA (1990).

Verwendbare Expressionsstämme z.B. solche, die eine geringere Proteaseaktivität aufweisen sind beschrieben in: Gottesman, S., Gene Expression Technology: Methods in Enzymology 185, Academic Press, San Diego, California (1990) 119-128.

Hierzu gehören auch Pflanzenzellen und bestimmte Gewebe, Organe und Teile von Pflanzen in all ihren Erscheinungsformen, wie Antheren, Fasern, Wurzelhaare, Stängel, Embryos, Kalli, Kotelydonen, Petiolen, Erntematerial, pflanzliches Gewebe, reproduktives Gewebe und Zellkulturen, das von der eigentlichen transgenen Pflanze abgeleitet ist und/oder dazu verwendet werden kann, die transgene Pflanze hervorzubringen.

Transgene Pflanzen bzw. vorteilhaft deren Samen, die die im erfindungsgemäßen Verfahren synthetisierten mehrfach ungesättigten Fettsäuren, insbesondere ARA, EPA und/oder DHA enthalten, können vorteilhaft direkt vermarktet werden ohne dass die synthetisierten Öle, Lipide oder Fettsäuren isoliert werden müssen. Unter Pflanzen im erfindungsgemäßen Verfahren sind ganze Pflanzen sowie alle Pflanzenteile, Pflanzenorgane oder Pflanzenteile wie Blatt, Stiel, Samen, Wurzel, Knollen, Antheren, Fasern, Wurzelhaare, Stängel, Embryos, Kalli, Kotelydonen, Petiolen, Erntematerial, pflanzliches Gewebe, reproduktives Gewebe, Zellkulturen, die sich von der transgenen Pflanze abgeleiten und/oder dazu verwendet werden können, die transgene Pflanze hervorzubringen. Der Samen umfasst dabei alle Samenteile wie die Samenhüllen, Epidermis- und Samenzellen, Endosperm oder Embyrogewebe.

Grundsätzlich eignet sich das erfindungsgemäße Verfahren auch zur Herstellung mehrfach ungesättigter Fettsäuren, insbesondere von ARA, EPA und/oder DHA in pflanzlichen Zellkulturen und anschließender Gewinnung der Fettsäuren aus den Kulturen. Dabei kann es sich insbesondere um Suspensions- oder Kalluskulturen handeln.

Die im erfindungsgemäßen Verfahren hergestellten Verbindungen können aber auch aus den Pflanzen vorteilhaft aus den Pflanzensamen in Form ihrer Öle, Fett, Lipide und/oder freien Fettsäuren isoliert werden. Durch dieses Verfahren hergestellte mehrfach ungesättigten Fettsäuren, insbesondere ARA, EPA und/oder DHA, lassen sich durch Ernten der Pflanzen bzw. Pflanzensamen entweder aus der Kultur, in der sie wachsen, oder vom Feld ernten.

Bei einer weiteren bevorzugten Ausführungsform umfasst dieses Verfahren ferner den Schritt des Gewinnens der Öle, Lipide oder freien Fettsäuren aus der Pflanze oder aus der Kultur. Bei der Kultur kann es sich beispielsweise um eine Treibhaus- oder Feldkultur einer Pflanze handeln.

Das Isolieren der Öle, Lipide oder freien Fettsäuren kann über Pressen oder Extraktion der Pflanzenteile bevorzugt der Pflanzensamen, erfolgen. Dabei können die Öle, Fette, Lipide und/oder freien Fettsäuren durch sogenanntes kalt schlagen oder kalt pressen ohne Zuführung von Wärme durch Pressen gewonnen werden. Damit sich die Pflanzenteile speziell die Samen leichter aufschließen lassen, werden sie vorher zerkleinert, gedämpft oder geröstet. Die so vorbehandelten Samen können anschließend gepresst werden oder mit Lösungsmittel wie warmen Hexan extrahiert werden. Anschließend wird das Lösungsmittel wieder entfernt.

Danach werden die so erhaltenen Produkte, die die mehrfach ungesättigten Fettsäuren enthalten, weiter bearbeitet, das heißt raffiniert. Dabei werden zunächst beispielsweise die Pflanzenschleime und Trübstoffe entfernt. Die

sogenannte Entschleimung kann enzymatisch oder beispielsweise chemisch/physikalisch durch Zugabe von Säure wie Phosphorsäure erfolgen. Anschließend werden die freien Fettsäuren durch Behandlung mit einer Base beispielsweise Natronlauge entfernt. Das erhaltene Produkt wird zur Entfernung der im Produkt verbliebenen Lauge mit Wasser gründlich gewaschen und getrocknet. Um die noch im Produkt enthaltenen Farbstoffe zu entfernen werden die Produkte einer Bleichung mit beispielsweise Bleicherde oder Aktivkohle unterzogen. Zum Schluss wird das Produkt noch beispielsweise mit Wasserdampf desodoriert.

Vorzugsweise sind die durch dieses Verfahren produzierten PUFAs bzw. LCPUFAs $C_{18}$-, $C_{20}$- oder $C_{22}$-Fettsäuremoleküle vorteilhaft $C_{20}$- oder $C_{22}$-Fettsäuremoleküle mit mindestens zwei Doppelbindungen im Fettsäuremolekül, vorzugsweise drei, vier, fünf oder sechs Doppelbindungen, besonders bevorzugt mit vier, fünf oder sechs Doppelbindungen. Diese $C_{18}$-, $C_{20}$- oder $C_{22}$-Fettsäuremoleküle lassen sich aus der Pflanze in Form eines Öls, Lipids oder einer freien Fettsäure isolieren. Geeignete Pflanzen sind beispielsweise die vorstehend erwähnten. Bevorzugte Organismen sind transgene Pflanzen.

Eine Ausführungsform der Erfindung werden Öle, Lipide oder Fettsäuren oder Fraktionen davondurch das oben beschriebene Verfahren hergestellt, besonders bevorzugt Öl, Lipid oder eine Fettsäurezusammensetzung, die PUFAs umfassen und von transgenen Pflanzen herrühren.

Die im Verfahren gewonnenen Fettsäuren eignen sich auch als Ausgangsmaterial für die chemische Synthese von weiteren Wertprodukten. Sie können beispielsweise in Kombination miteinander oder allein zur Herstellung von Pharmaka, Nahrungsmittel, Tierfutter oder Kosmetika verwendet werden.

Diese Öle, Lipide oder Fettsäuren enthalten wie oben beschrieben vorteilhaft 6 bis 15 % Palmitinsäure, 1 bis 6 % Stearinsäure; 7 - 85 % Ölsäure; 0,5 bis 8 % Vaccensäure, 0,1 bis 1 % Arachinsäure, 7 bis 25 % gesättigte Fettsäuren, 8 bis 85 % einfach ungesättigte Fettsäuren und 60 bis 85 % mehrfach ungesättigte Fettsäuren jeweils bezogen auf 100 % und auf den Gesamtfettsäuregehalt der Organismen. Als vorteilhafte mehrfach ungesättigte Fettsäure sind in den Fettsäureester bzw. Fettsäuregemische bevorzugt mindestens 0,1; 0,2; 0,3; 0,4; 0,5; 0,6; 0,7; 0,8; 0,9 oder 1 % bezogen auf den Gesamtfettsäuregehalt an Arachidonsäure enthalten. Weiterhin enthalten die Fettsäureester bzw. Fettsäuregemische, die nach dem erfindungsgemäßen Verfahren hergestellt wurden, vorteilhaft Fettsäuren ausgewählt aus der Gruppe der Fettsäuren Erucasäure (13-Docosaensäure), Sterculinsäure (9,10-Methylene octadec-9-enonsäure), Malvalinsäure (8,9-Methylen Heptadec-8-enonsäure), Chaulmoogrinsäure (Cyclopenten-dodecansäure), Furan-Fettsäure (9,12-Epoxy-octadeca-9,11-dienonsäure), Vernonsäure (9,10-Epoxyoctadec-12-enonsäure), Tarinsäure (6-Octadecynonsäure),6-Nonadecynonsäure, Santalbinsäure (t11-Octadecen-9-ynoic acid), 6,9-Octadecenynonsäure, Pyrulinsäure (t10-Heptadecen-8-ynonsäure), Crepenyninsäure (9-Octadecen-12-ynonsäure), 13,14-Dihydrooropheinsäure, Octadecen-13-ene-9,11-diynonsäure, Petroselensäure (cis-6-Octadecenonsäure), 9c,12t-Octadecadiensäure, Calendulasäure (8t10t12c-Octadecatriensäure), Catalpinsäure (9t11t13c-Octadecatriensäure), Eleosterinsäure (9c11t13t-Octadecatriensäure), Jacarinsäure (8c10t12c-Octadecatriensäure), Punicinsäure (9c11t13c-Octadecatriensäure),Parinarinsäure (9c11t13t15c-Octadecatetraensäure), Pinolensäure (all-cis-5,9,12-Octadecatriensäure), Laballensäure (5,6-Octadecadienallensäure), Ricinolsäure (12-Hydroxyölsäure) und/oder Coriolinsäure (13-Hydroxy-9c,11t-Octadecadienonsäure). Die vorgenannten Fettsäuren kommen in den nach dem erfindungsgemäßen Verfahren hergestellten Fettsäureester bzw. Fettsäuregemischen in der Regel vorteilhaft nur in Spuren vor, das heißt sie kommen bezogen auf die Gesamtfettsäuren zu weniger als 30 %, bevorzugt zu weniger als 25 %, 24 %, 23 %, 22 % oder 21 %, besonders bevorzugt zu weniger als 20 %, 15 %, 10 %, 9 %, 8 %, 7%, 6 % oder 5%, ganz besonders bevorzugt zu weniger als 4 %, 3 %, 2 % oder 1 % vor. In einer weiteren bevorzugten Form der Erfindung kommen diese vorgenannten Fettsäuren bezogen auf die Gesamtfettsäuren zu weniger als 0,9%; 0,8%; 0,7%; 0,6%; oder 0,5%, besonders bevorzugt zu weniger als 0,4%; 0,3%; 0,2%; 0,1% vor. Vorteilhaft enthalten die nach dem erfindungsgemäßen Verfahren hergestellten Fettsäureester bzw. Fettsäuregemische weniger als 0,1 % bezogen auf die Gesamtfettsäuren und/oder keine Butterbuttersäure, kein Cholesterin, keine Clupanodonsäure (= Docosapentaensäure, $C22{:}5^{\Delta 4,8,12,15,21}$) sowie keine Nisinsäure (Tetracosahexaensäure, $C23{:}6^{\Delta 3,8,12,15,18,21}$).

Die vorgenannten Fettsäuren kommen in den nach dem erfindungsgemäßen Verfahren hergestellten Fettsäureester bzw. Fettsäuregemischen in der Regel vorteilhaft nur in Spuren vor, das heißt sie kommen bezogen auf die Gesamtfettsäuren zu weniger als 30 %, bevorzugt zu weniger als 25 %, 24 %, 23 %, 22 % oder 21 %, besonders bevorzugt zu weniger als 20 %, 15 %, 10 %, 9 %, 8 %, 7%, 6 % oder 5%, ganz besonders bevorzugt zu weniger als 4 %, 3 %, 2 % oder 1 % vor. In einer weiteren bevorzugten Form der Erfindung kommen diese vorgenannten Fettsäuren bezogen auf die Gesamtfettsäuren zu weniger als 0,9%; 0,8%; 0,7%; 0,6%; oder 0,5%, besonders bevorzugt zu weniger als 0,4%; 0,3%; 0,2%; 0,1% vor. Vorteilhaft enthalten die nach dem erfindungsgemäßen Verfahren hergestellten Fettsäureester bzw. Fettsäuregemische weniger als 0,1 % bezogen auf die Gesamtfettsäuren und/oder keine Buttersäure, kein Cholesterin, keine Clupanodonsäure (= Docosapentaensäure, $C22{:}5^{\Delta 4,8,12,15,21}$) sowie keine Nisinsäure (Tetracosahexaensäure, $C23{:}6^{\Delta 3,8,12,15,18,21}$).

Vorteilhaft enthalten die beschriebenen Öle, Lipide oder Fettsäuren mindestens 0,5%, 1%, 2%, 3%, 4%, 5%, 6%, 7%, 8%, 9% oder 10%, vorteilhaft mindestens 11%, 12%, 13%, 14%, 15%, 16% oder 17%, besonders vorteilhaft mindestens 18%, 19%, 20%, 21%, 22%, 23%, 24% oder 25% ARA oder mindestens 0,5%, 1%, 2%, 3%, 4%, 5% oder

6%, vorteilhaft mindestens 7%, 8%, 9%, 10% oder 11% besonders vorteilhaft mindestens 12%, 13%, 14%, 15%, 16%, 17'%, 18%, 19% oder 20% EPA oder mindestens 0,01%, 0,02%, 0,03%, 0,04% oder 0,05% oder 0,06%, vorteilhaft mindestens 0,07%, 0,08%, 0,09 oder 0,1%, besonders vorteilhaft mindestens 0,2%, 0,3% oder 0,4% DHA bezogen auf den Gesamtfettsäuregehalt des Produktionsorganismus vorteilhaft einer Pflanze, besonders vorteilhaft einer Ölfruchtpflanze wie Soja, Raps, Kokosnuss, Ölpalme, Färbersafflor, Flachs, Hanf, Rizinus, Calendula, Erdnuss, Kakaobohne, Sonnenblume oder den oben genannten weiteren ein- oder zweikeimblättrigen Ölfruchtpflanzen. Alle Prozentangaben beziehen sich auf Gewichtsprozente.

Durch die hierin beschriebenen Nukleinsäuresequenzen bzw. im erfindungsgemäßen Verfahren verwendeten Nukleinsäuresequenzen kann eine Steigerung der Ausbeute an mehrfach ungesättigten Fettsäuren, vor allem an ARA und EPA aber auch DHA, von mindestens 50, 80 oder 100 %, vorteilhaft von mindestens 150, 200 oder 250 %, besonders vorteilhaft von mindestens 300, 400, 500, 600, 700, 800 oder 900 %, ganz besonders vorteilhaft von mindestens 1000, 1100, 1200, 1300, 1400 oder 1500 % gegenüber der nicht transgenen Ausgangspflanze beispielsweise einer Pflanze wie Brassica juncea, Brassica napus, Camelina sativa, Arabidopsis thanliana oder Linum usitatissimum beim Vergleich in der GC-Analyse siehe Beispiele erreicht werden.

Die im erfindungsgemäßen Verfahren hergestellten Lipide und/oder Öle haben einen höheren Anteil der ungesättigten Fettsäuren Ölsäure, Linolsäure und $\alpha$-Linolensäure in sn2-Position im Vergleich zu den anderen Positionen sn1 und sn3. Unter höheren Anteil sind Verhältnisse von (sn1:sn2:sn3) 1:1,1:1; 1:1,5:1 bis 1:3:1 zu verstehen. Auch die im Verfahren hergestellte Arachidonsäure, Eicosapentaensäure oder Docosahexaensäure zeigen in den Lipiden und/oder Ölen ebenfalls eine Präferenz für die sn2-Position im Triglycerid gegenüber den Positionen sn1 und sn3 von vorteilhaft 1:1,1:1; 1:1,5:1 bis 1:3:1.

Vorteilhaft werden, wie oben beschrieben, die im Verfahren hergestellten mehrfach ungesättigten $C_{20}$- und/oder $C_{22}$-Fettsäuren mit vier, fünf oder sechs Doppelbindungen im Molekül im Samen von Pflanzen, die keine oder nur sehr geringe Mengen an C12:0-bzw. C14:0-Fettsäuren enthalten. Auch noch kürzere gesättigte Fettsäuren wie die Fettsäuren C4:0, C6:0, C8:0 oder C10:0 sollten nicht oder nur in geringen Mengen im Lipid und/oder Öl vorhanden sein. Unter nur sehr geringen Mengen sind vorteilhaft Mengen zu verstehen, die in der GC-Analyse vorteilhaft unter 5, 4, 3, 2 oder 1 %, vorteilhaft unter 0,9; 0,8; 0,7; 0,6 oder 0,5 %, besonders vorteilhaft unter 0,4; 0,3; 0,2 ider 0,1 %, ganz besonders bevorzugt unter 0,09; 0,08; 0,07; 0,06; 0,05; 0,04; 0,03; 0,02 oder 0,01 Flächeneinheiten in der GC liegen. Die Fettsäure C16:0 sollte vorteilhaft in einem Bereich von 1 bis 28 % GC-Flächeneinheiten liegen. Vorteilhaft sollte die Fettsäure C16:0 in GC-Flächeneinheiten von weniger als 25%, 20%, 15% oder 10%, vorteilhaft von weniger als 9%, 8%, 7%, 6% oder 5%, besonders vorteilhaft von weniger als 4%, 3%, 2% oder 1% oder gar nicht in den Lipiden, Ölen und/oder freien Fettsäuren vorhanden sein. Die Fettsäure C16:1 sollte vorteilhaft weniger als 1; 0,5; 0,4; 0,3; 0,2 oder 0,1 %, besonders vorteilhaft 0,09; 0,08; 0,07; 0,06; 0,05; 0,04; 0,03; 0,02 oder 0,01 Flächeneinheiten in der GC betragen. Ganz besonders bevorzugt sollte die Fettsäure C16:1 nicht in den nach dem Verfahren hergestellten Ölen und/oder Lipiden vorhanden sein. Gleiches gilt für die Fettsäuren C15:0, C17:0, C16:1 $^{\Delta 3}$trans, c16:4$^{\Delta 4,7,10,13}$ und C18:5$^{\Delta 3,6,9,12,15}$. Neben Ölsäure (C18:1$^{\Delta 9}$) können auch die Isomere (C18:1$^{\Delta 7}$, C18:1$^{\Delta 11}$) in den Lipiden, Ölen oder freien Fettsäuren vorhanden sein. Vorteilhaft in Mengen, gemessen als GC-Flächeneinheiten, von weniger als 5%, 4%, 3%, 2% oder 1%. Die Fettsäuren C20:0, C20:1, C24:0 und C24:1 sollten jeweils in einem Bereich von 0 bis 1 %, 0 bis 3% bzw. 0 bis 5 % Flächeneinheiten in der GC liegen. Weiterhin sollte in der GC-Analyse wenig Dihomo-$\gamma$-linolensäure (= DGLA) im Samenöl und/oder -lipid in GC-Flächeneinheiten detektierbar sein. Unter wenig sind weniger als 2; 1,9; 1,8; 1,7; 1,6 oder 1,5 %, vorteilhaft weniger als 1,4; 1,3; 1,2; 1,1 oder 1 %, besonders vorteilhaft weniger als 0,9; 0,8; 0,7; 0,6; 0,5 oder 0,4 % in GC-Flächeneinheiten zu verstehen.

In einer bevorzugten Ausführungsform des Verfahrens sollte DGLA und ARA in einem Verhältnis von 1:1 bis zu 1:100, vorteilhaft von 1:2 bis zu 1:80, besonders vorteilhaft von 1:3 bis zu 1:70, ganz besonders bevorzugt von 1:5 bis zu 1:60 entstehen.

In weiteren bevorzugten Ausführungsform des Verfahrens sollte DGLA und EPA in einem Verhältnis von 1:1 bis zu 1:100, vorteilhaft von 1:2 bis zu 1:80, besonders vorteilhaft von 1:3 bis zu 1:70, ganz besonders bevorzugt von 1:5 bis zu 1:60 entstehen.

Vorteilhaft sollten die im erfindungsgemäßen Verfahren hergestellten Lipide, Öle und/oder freien Fettsäuren einen hohen Anteil von ungesättigten Fettsäuren vorteilhaft von mehrfach ungesättigten Fettsäuren von mindestens 30, 40 oder 50 Gew.-%, vorteilhaft von mindestens 60, 70 oder 80 Gew.-% bezogen auf den Gesamtfettsäuregehalt in den Samen der transgenen Pflanzen betragen.

Alle gesättigten Fettsäuren zusammen sollten vorteilhaft in den Lipiden, Ölen und/oder freien Fettsäuren bevorzugt verwendeten Pflanzen nur einen geringen Anteil ausmachen. Unter geringen Anteil ist in diesem Zusammenhang ein Anteil in GC-Flächeneinheiten von weniger als 15%, 14%, 13%, 12%, 11% oder 10%, bevorzugt von weniger als 9%, 8%, 7% oder 6% zu verstehen.

Im Verfahren hergestellte Lipide, Öle und/oder freie Fettsäuren sollten vorteilhaft einen Gehalt an Erucasäure von weniger als 2 Gew.-% bezogen auf den Gesamtfettsäuregehalt der Pflanze haben. Vorteilhaft sollte keine Erucasäure in den Lipiden und/oder Ölen vorhanden sein. Auch sollte der Gehalt an gesättigten Fettsäuren C16:0 und/oder C18:0

vorteilhaft geringer als 19, 18, 17, 16, 15, 14, 13, 12, 11, oder 10 Gew.-%, vorteilhaft weniger als 9, 8, 7, 6 oder 5 Gew.-% bezogen auf den gesamten Fettsäuregehalt der Lipide und/oder Öle sein. Vorteilhaft sollten auch längere Fettsäuren wie C20:0 oder C22:1 gar nicht oder in nur geringen Mengen vorteilhaft geringer als 4, 3, 2 oder 1 Gew.-%, vorteilhaft weniger als 0,9; 0,8; 0,7; 0,6; 0,5; 0,4; 0,3; 0,2 oder 0,1 Gew.-% bezogen auf den gesamten Fettsäuregehalt der Lipde und/oder Öle sein. Typischerweise ist in den Lipden und/oder Ölen, die nach dem erfindungsgemäßen Verfahren hergestellt wurden, kein oder nur in geringen Mengen C16:1 als Fettsäure enthalten. Unter geringen Mengen sind vorteilhaft Gehalte an Fettsäuren zu verstehen, die geringer als 4, 3, 2 oder 1 Gew.-%, vorteilhaft weniger als 0,9; 0,8; 0,7; 0,6; 0,5; 0,4; 0,3; 0,2 oder 0,1 Gew.-% bezogen auf den gesamten Fettsäuregehalt der Lipide und/oder Öle.

Die nach dem Pressen erhaltenen hierin beschriebenen Öle, Lipide, Fettsäuren oder Fettsäuregemische werden als sogenannte Rohöle bezeichnet. Diese enthalten noch die gesamten Öl- und/oder Lipidkomponenten, sowie Verbindungen, die in diesen löslich sind. Derartige Verbindunge sind die verschiedenen Tocopherole wie $\alpha$-Tocopherol, $\beta$-Tocopherol, $\gamma$-Tocopherol und/oder $\delta$-Tocopherol oder Phytosterole wie Brassicasterol, Campesterol, Stigmasterol, $\beta$-Sitosterol, Sitostanol, $\Delta^5$-Avenasterol, $\Delta^5$,24-Stigmastadienol, $\Delta^7$-Stigmastenol oder $\Delta^7$-Avenasterol. Diese Verbindungen sind in einem Bereich von 1 bis 1000 mg/100 g vorteilhaft von 10 bis 800 mg/100 g Lipid oder Öl enthalten. Auch Triterpene wie Germaniol, Amyrin, Cycloartanol und andere können in diesen Lipiden und Ölen enthalten sein. Diese Lipide und/oder Öle enthalten die im Verfahren hergestellten mehrfach ungesättigten Fettsäuren wie ARA, EPA und/oder DHA gebunden in polaren und unpolaren Lipiden wie Phospholipiden z.B. Phosphatidylcholin, Phosphatidylethanolamin, Phosphatidylinositol, Phosphatidylserin, Phosphatidylglycerin, Galactolipiden, Monoglyceride, Diglyceride oder Triglyceride um nur einige zu nennen. Auch Lysophospholipide können in den Lipiden und/oder Ölen vorkommen. Diese Komponenten der Lipide und/oder Öle können durch geeignete Methoden voneinander getrennt werden. Nicht enthalten in diesen Rohölen ist Cholesterol.

Eine weitere erfindungsgemäße Ausführungsform ist die Verwendung des Öls, Lipids, der Fettsäuren und/oder der Fettsäurezusammensetzung in Futtermitteln, Nahrungsmitteln, Kosmetika oder Pharmazeutika. Die erfindungsgemäßen Öle, Lipide, Fettsäuren oder Fettsäuregemische können in der dem Fachmann bekannten Weise zur Abmischung mit anderen Ölen, Lipiden, Fettsäuren oder Fettsäuregemischen tierischen Ursprungs wie z.B. Fischölen verwendet werden. Typisch für derartige Fischöle kurzkettige Fettsäuren wie C12:0, C14:0, C14:1, verzweigtkettiges C15:0, C15:0, C16:0 oder C16:1. Auch mehrfach ungesättige C16-Fettsäuren wie C16:2, C16:3 oder C16:4, verzweigtkettiges C17:0, C17:1, verzweigtkettiges C18:0 und C19:0 sowie C19:0 und C19:1 kommen im Fischöl vor. Derartige Fettsäuren sind typisch für Fischöle und werden nur selten oder gar nicht in pflanzlichen Ölen gefunden. Wirtschaftlich relevante Fischöle sind z.B. Anchovissöl, Menhadneöl, Tunfischöl, Sardinenöl, Heringsöl, Markrelenöl, Walöl und Lachsöl. Diese Lipide und/oder Öle tierischen Ursprungs können zum Abmischen mit den erfindungsgemäßen Ölen in Form von Rohölen, das heißt in Form von Lipiden und/oder Ölen, die noch nicht aufgereinigt wurden, verwendet werden oder aber es können verschieden aufgereinigte Fraktionen zum Abmischen verwendet werden.

Eine weitere erfindungsgemäße Ausführungsform ist die Verwendung des Öls, Lipids, Fettsäuren und/oder der Fettsäurezusammensetzung in Futtermitteln, Nahrungsmitteln, Kosmetika oder Pharmazeutika.

Die hierin beschriebenen Öle, Lipide, Fettsäuren oder Fettsäuregemische können in der dem Fachmann bekannten Weise zur Abmischung mit anderen Ölen, Lipiden, Fettsäuren oder Fettsäuregemischen tierischen Ursprungs wie z.B. Fischölen verwendet werden. Auch diese Öle, Lipide, Fettsäuren oder Fettsäuregemische, die aus pflanzlichen und tierischen Bestandteilen bestehen, können zur Herstellung von Futtermitteln, Nahrungsmitteln, Kosmetika oder Pharmazeutika verwendet werden.

Unter dem Begriff "Öl", "Lipid" oder "Fett" wird ein Fettsäuregemisch verstanden, das ungesättigte, gesättigte, vorzugsweise veresterte Fettsäure(n) enthält. Bevorzugt ist, dass das Öl, Lipid oder Fett einen hohen Anteil an mehrfach ungesättigten freien oder vorteilhaft veresterten Fettsäure(n), insbesondere Linolsäure, $\gamma$-Linolensäure, Dihomo-$\gamma$-linolensäure, Arachidonsäure, $\alpha$-Linolensäure, Stearidonsäure, Eicosatetraensäure, Eicosapentaensäure, Docosapentaensäure oder Docosahexaensäure hat. Vorzugsweise ist der Anteil an ungesättigten veresterten Fettsäuren ungefähr 30 %, mehr bevorzugt ist ein Anteil von 50 %, noch mehr bevorzugt ist ein Anteil von 60 %, 70 %, 80 %, 85% oder mehr. Zur Bestimmung kann z.B. der Anteil an Fettsäure nach Überführung der Fettsäuren in die Methylestern durch Umesterung gaschromatographisch bestimmt werden. Das Öl, Lipid oder Fett kann verschiedene andere gesättigte oder ungesättigte Fettsäuren, z.B. Calendulasäure, Palmitin-, Palmitolein-, Stearin-, Ölsäure etc., enthalten. Insbesondere kann je nach Ausgangspflanze der Anteil der verschiedenen Fettsäuren in dem Öl oder Fett schwanken.

Bei den im Verfahren hergestellten mehrfach ungesättigte Fettsäuren mit vorteilhaft mindestens zwei Doppelbindungen enthalten, handelt es sich wie oben beschrieben beispielsweise um Sphingolipide, Phosphoglyceride, Lipide, Glycolipide, Phospholipide, Monoacylglycerin, Diacylglycerin, Triacylglycerin oder sonstige Fettsäureester.

Aus den so im erfindungsgemäßen Verfahren hergestellten mehrfach ungesättigte Fettsäuren mit vorteilhaft mindestens fünf oder sechs Doppelbindungen lassen sich die enthaltenden mehrfach ungesättigten Fettsäuren beispielsweise über eine Alkalibehandlung beispielsweise wäßrige KOH oder NaOH oder saure Hydrolyse vorteilhaft in Gegenwart eines Alkohols wie Methanol oder Ethanol oder über eine enzymatische Abspaltung freisetzen und isolieren über beispielsweise Phasentrennung und anschließender Ansäuerung über z.B. $H_2SO_4$. Die Freisetzung der Fettsäuren

kann auch direkt ohne die vorhergehend beschriebene Aufarbeitung erfolgen.

Moose und Algen sind die einzigen bekannten Pflanzensysteme, die erhebliche Mengen an mehrfach ungesättigten Fettsäuren, wie Arachidonsäure (ARA) und/oder Eicosapentaensäure (EPA) und/oder Docosahexaensäure (DHA) herstellen. Moose enthalten PUFAs in Membranlipiden während Algen, algenverwandte Organismen und einige Pilze auch nennenswerte Mengen an PUFAs in der Triacylglycerolfraktion akkumulieren. Daher eignen sich Nukleinsäuremoleküle, die aus solchen Stämmen isoliert werden, die PUFAs auch in der Triacylglycerolfraktion akkumulieren, besonders vorteilhaft für das erfindungsgemäße Verfahren und damit zur Modifikation des Lipid-und PUFA-Produktionssystems in einem Wirt, insbesondere Pflanzen, wie Ölfruchtpflanzen, beispielsweise Raps, Canola, Lein, Hanf, Soja, Sonnenblumen, Borretsch. Sie sind deshalb vorteilhaft im erfindungsgemäßen Verfahren verwendbar.

Die im Verfahren verwendeten Nukleinsäuren können nach Einbringung in eine Pflanzenzelle bzw. Pflanze entweder auf einem separaten Plasmid liegen oder vorteilhaft in das Genom der Wirtszelle integriert sein. Bei Integration in das Genom kann die Integration zufallsgemäß sein oder durch derartige Rekombination erfolgen, dass das native Gen durch die eingebrachte Kopie ersetzt wird, wodurch die Produktion der gewünschten Verbindung durch die Zelle moduliert wird, oder durch Verwendung eines Gens in trans, so dass das Gen mit einer funktionellen Expressionseinheit, welche mindestens eine die Expression eines Gens gewährleistende Sequenz und mindestens eine die Polyadenylierung eines funktionell transkribierten Gens gewährleistende Sequenz enthält, funktionell verbunden ist. Vorteilhaft werden die Nukleinsäuren über Multiexpressionskassetten oder Konstrukte zur multiparallelen Expression in die Organismen vorteilhaft zur multiparallelen samenspezifischen Expression von Genen in die Pflanzen gebracht.

Die Co-Expression mehrerer Gene kann natürlich nicht nur durch Einbringen der Gene auf einem gemeinsamen rekombinanten Nukleinsäurekonstrukt erfolgen. Vielmehr können einzelne Gene auch separat - gleichzeitig oder nacheinander - auf verschiedenen Konstrukten eingebracht werden. Hier wird z.B. durch die Verwendung verschiedener Selektionsmarker die gleichzeitige Anwesenheit in der alle Gene coexprimierenden Pflanze sichergestellt. Diese Pflanze kann das Produkt eines oder mehrerer Transformationsvorgänge sein, oder aber auch ein Kreuzungsprodukt von Pflanzen, die eines oder mehrere der Gene enthalten.

Als Substrate der im erfindungsgemäßen Verfahren verwendeten Nukleinsäuren, die für Polypeptide mit $\omega$-3-Desaturase-, $\Delta$-4-Desaturase-, $\Delta$-5-Desaturase-, $\Delta$-6-Desaturase-, $\Delta$-8-Desaturase-, $\Delta$-12-Desaturase-, $\Delta$-5-Elongase-, $\Delta$-6-Elongase-und/oder $\Delta$-9-Elongase-Aktivität kodieren, und/oder den weiteren verwendeten Nukleinsäuren wie den Nukleinsäuren, die für Polypeptide des Fettsäure- oder Lipidstoffwechsels ausgewählt aus der Gruppe Acyl-CoA-Dehydrogenase(n), Acyl-ACP[= acyl carrier protein]-Desaturase(n), Acyl-ACP-Thioesterase(n), Fettsäure-Acyl-Transferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenase(n), Lipoxygenase(n), Triacylglycerol-Lipase(n), Allenoxid-Synthase(n), Hydroperoxid-Lyase(n) oder Fettsäure-Elongase(n) kodieren, eignen sich vorteilhaft $C_{16}$-, $C_{18}$-, $C_{20}$-oder $C_{22}$-Fettsäuren. Bevorzugt werden die im Verfahren als Substrate umgesetzten Fettsäuren in Form ihrer Acyl-CoA-Ester und/oder ihrer Phospholipid-Ester umgesetzt. Vorteilhaft werden im Verfahren Desaturasen verwendet, die eine Spezifität für die Acyl-CoA-Ester haben. Dies hat den Vorteil, dass kein Ausstausch zwischen den Phospholipid-Estern, die in der Regel das Substrat der Desaturierung sind, und den Acyl-CoA-Estern stattfinden muss. Dadurch entfällt ein weiterer Enzymschritt, der, wie sich gezeigt hat, in einigen Fällen ein limitierender Schritt ist.

Zur Herstellung der hierin beschriebenen langkettigen PUFAs müssen die mehrfach ungesättigten $C_{16}$- oder $C_{18}$-Fettsäuren zunächst durch die enzymatische Aktivität einer Desaturase desaturiert und anschließend über eine Elongase um mindestens zwei Kohlenstoffatome verlängert werden. Nach einer Elongationsrunde führt diese Enzymaktivität zu $C_{18}$- oder $C_{20}$-Fettsäuren und nach zwei Elongationsrunden zu $C_{20}$- oder $C_{22}$-Fettsäuren. Die Aktivität der im erfindungsgemäßen Verfahren verwendeten Desaturasen und Elongasen führt vorzugsweise zu $C_{18}$-, $C_{20}$- und/oder $C_{22}$-Fettsäuren vorteilhaft mit mindestens zwei Doppelbindungen im Fettsäuremolekül, vorzugsweise mit drei, vier, fünf oder sechs Doppelbindungen, besonders bevorzugt zu $C_{20}$- oder $C_{22}$-Fettsäuren mit mindestens zwei Doppelbindungen im Fettsäuremolekül, vorzugsweise mit drei, vier, fünf oder sechs Doppelbindungen, am meisten bevorzugt mit vier, fünf oder sechs Doppelbindungen im Molekül. Besonders bevorzugte Produkte des erfindungsgemäßen Verfahrens sind Arachidonsäure, Eicosapentaensäure und/oder Docosahexaensäure. Die $C_{18}$-Fettsäuren mit mindestens zwei Doppelbindungen in der Fettsäure können durch die erfindungsgemäße enzymatische Aktivität in Form der freien Fettsäure oder in Form der Ester, wie Phospholipide, Glycolipide, Sphingolipide, Phosphoglyceride, Monoacylglycerin, Diacylglycerin oder Triacylglycerin, verlängert werden.

Der bevorzugte Biosyntheseort von Fettsäuren, Ölen, Lipiden oder Fette in den vorteilhaft verwendeten Pflanzen ist beispielsweise im allgemeinen der Samen oder Zellschichten des Samens, so dass eine samenspezifische Expression der im Verfahren verwendeten Nukleinsäuren sinnvoll ist. Es ist jedoch naheliegend, dass die Biosynthese von Fettsäuren, Ölen oder Lipiden nicht auf das Samengewebe beschränkt sein muss, sondern auch in allen übrigen Teilen der Pflanze - beispielsweise in Epidermiszellen oder in den Knollen - gewebespezifisch erfolgen kann.

Durch die Verwendung der erfindungsgemäßen Nukleinsäuren, die für eine $\Delta$-5-Elongase codieren, können im Verfahren die hergestellten mehrfach ungesättigten Fettsäuren mindestens um 5 % , bevorzugt mindestens um 10

%, besonders bevorzugt mindestens um 20 %, ganz besonders bevorzugt um mindestens 50 % gegenüber dem Wildtyp der Organismen, die die Nukleinsäuren nicht rekombinant enthalten, erhöht werden.

Durch das erfindungsgemäße Verfahren können die hergestellten mehrfach ungesättigten Fettsäuren in den im Verfahren verwendeten Pflanzen prinzipiell auf zwei Arten erhöht werden. Es kann entweder der Pool an freien mehrfach ungesättigten Fettsäuren und/oder der Anteil der über das Verfahren hergestellten veresterten mehrfach ungesättigten Fettsäuren erhöht werden. Vorteilhaft wird durch das erfindungsgemäße Verfahren der Pool an veresterten mehrfach ungesättigten Fettsäuren in den transgenen Organismen erhöht.

Hierin beschrieben sind auch isolierte Nukleinsäuresequenzen, die für Polypeptide mit Δ-5-Elongase codieren, wobei die durch die Nukleinsäuresequenzen codierten Δ-5-Elongasen $C_{20}$-Fettsäuren mit mindestens vier Doppelbindungen im Fettsäuremolekül umsetzen; die vorteilhaft letztlich in Diacylglyceride und/oder Triacylglyceride eingebaut werden.

Offenbart ist somit eine isolierte Nukleinsäuresequenz, die für Polypeptide mit Δ-5-Elongase codiert und die in SEQ ID NO: 197 dargestellte Sequenz hat.

Offenbart ist somit eine isolierte Nukleinsäuresequenz, die für Polypeptide mit Δ-6-Elongaseaktivität codiert und die in SEQ ID NO: 199 dargestellte Sequenz hat.

Offenbart ist somit eine isolierte Nukleinsäuresequenz, die für Polypeptide mit Δ-6-Desaturaseaktivität codiert und die in SEQ ID NO: 201 dargestellte Sequenz hat.

Offenbart ist somit ein rekombinantes Nukleinsäuremolekül, umfassend:

a) eine oder mehrere Kopien eines in Pflanzenzellen, bevorzugt in Samenzellen, aktiven Promotors,
b) mindestens eine Nukleinsäuresequenz mit der in SEQ ID NO: 193 oder SEQ ID NO: 201 dargestellten Sequenz, die für eine Δ-6-Desaturase-Aktivität kodiert,
c) mindestens eine Nukleinsäuresequenz mit der in SEQ ID NO: 11 dargestellten Sequenz, die für eine Δ-5-Desaturase-Aktivität kodiert,
d) mindestens eine Nukleinsäuresequenz mit der in SEQ ID NO: 27 oder SEQ ID NO: 199 dargestellten Sequenz, die für eine Δ-6-Elongase-Aktivität kodiert, und
e) eine oder mehrere Kopien einer Terminatorsequenz.

Vorteilhaft kann in dem rekombinanten vorgenannten Nukleinsäuremolekül noch zusätzlich eine Nukleinsäuresequenz mit der in SEQ ID NO: 195 dargestellten Sequenz, die für eine Δ-12-Desaturase kodiert, enthalten sein.

In einer weiteren vorteilhaften Ausführungsform kann in dem rekombinanten Nukleinsäuremolekül vorteilhaft noch zusätzlich eine Nukleinsäuresequenz mit der in SEQ ID NO: 197 dargestellten Sequenz, die für eine Δ-5-Elongase kodiert, enthalten sein.

Neben diesen genannten Sequenzen können in das rekombinanten Nukleinsäuremolekül noch weitere Biosynthesegene des Fettsäure- oder Lipidstoffwechsels ausgewählt aus der Gruppe bestehend aus Acyl-CoA-Dehydrogenase(n), Acyl-ACP[= acyl carrier protein]-Desaturase(n), Acyl-ACP-Thioesterase(n), Fettsäure-AcylTransferase(n), Acyl-CoA:Lysophospholipid-Acyltransferase(n), Fettsäure-Synthase(n), Fettsäure-Hydroxylase(n), Acetyl-Coenzym A-Carboxylase(n), Acyl-Coenzym A-Oxidase(n), Fettsäure-Desaturase(n), Fettsäure-Acetylenasen, Lipoxygenasen, Triacylglycerol-Lipasen, Allenoxid-Synthasen, Hydroperoxid-Lyasen und Fettsäure-Elongase(n) eingebracht werden.

Bevorzugt sind dies Gene des Fettsäure- oder Lipidstoffwechsels ausgewählt aus der Gruppe bestehend aus Δ-4-Desaturase-, Δ-8-Desaturase-, Δ-9-Desaturase- oder Δ-9-Elongase.

Offenbart sind somit Genkonstrukte, die die erfindungsgemäßen Nukleinsäuresequenzen SEQ ID NO: 11, SEQ ID NO: 27, SEQ ID NO: 193, SEQ ID NO: 195, SEQ ID NO: 197, SEQ ID NO: 199 oder SEQ ID NO: 201 enthalten, wobei die Nukleinsäure funktionsfähig mit einem oder mehreren Regulationssignalen verbunden ist.

Vorteilhaft stammen alle die im erfindungsgemäßen Verfahren verwendeten Nukleinsäuresequenzen aus einem eukaryontischen Organismus wie einer Pflanze, einem Mikroorganismus wie einer Alge oder einem Tier. Bevorzugt stammen die Nukleinsäuresequenzen aus der Ordnung Salmoniformes, Xenopus oder Ciona, Algen wie Mantoniella, Crypthecodinium, Euglena oder Ostreococcus, Pilzen wie der Gattung Phytophtora oder von Diatomeen wie den Gattungen Thalassiosira oder Phaeodactylum.

Die im Verfahren verwendeten Nukleinsäuresequenzen, die für Proteine mit ω-3-Desaturase-, Δ-4-Desaturase-, Δ-5-Desaturase-, Δ-6-Desaturase-, Δ-8-Desatuase-, Δ-9-Desaturase-, Δ-12-Desaturase-, Δ-5-Elongase-, Δ-6-Elongase- oder Δ-9-Elongase-Aktivität codieren, werden vorteilhaft allein oder bevorzugt in Kombination in einer Expressionskassette (= Nukleinsäurekonstrukt), die die Expression der Nukleinsäuren in einer Pflanze, eingebracht. Es kann im Nukleinsäurekonstrukt mehr als eine Nukleinsäuresequenz einer enzymatischen Aktivität wie z.B. einer Δ-12-Desaturase, Δ-4-Desaturase, Δ-5-Desaturase, Δ-6-Desaturase, Δ-5-Elongase, Δ-6-Elongase und/oder ω-3-Desaturase enthalten sein.

Zum Einbringen in die Pflanze werden die im Verfahren verwendeten Nukleinsäuren vorteilhaft einer Amplifikation und Ligation in bekannter Weise, wie oben beschrieben, unterworfen.

Es gibt eine Reihe von Mechanismen, durch die eine Veränderung des erfindungsgemäßen Δ-12-Desaturase-, Δ-5-Elongase-, Δ-6-Elongase, Δ-5-Desaturase-, Δ-4-Desaturase-, Δ-6-Desaturase- und/oder ω-3-Desaturase-Proteins sowie der weiteren im Verfahren verwendeten Proteine wie die Δ-12-Desaturase-, Δ-9-Elongase-, Δ-6-Desaturase-, Δ-8-Desaturase-, Δ-6-Elongase-, Δ-5-Desaturase- oder Δ-4-Desaturase-Proteine möglich ist, so dass die Ausbeute, Produktion und/oder Effizienz der Produktion der vorteilhaft mehrfach ungesättigten Fettsäuren in einer Pflanze bevorzugt in einer Ölfruchtpflanze aufgrund dieses veränderten Proteins direkt beeinflusst werden kann. Die Anzahl oder Aktivität der Δ-12-Desaturase-, ω-3-Desaturase-, Δ-9-Elongase-, Δ-6-Desaturase-, Δ-8-Desaturase-, Δ-6-Elongase-, Δ-5-Desaturase-, Δ-5-Elongase-oder Δ-4-Desaturase-Proteine oder -Gene kann erhöht werden, so dass größere Mengen der Genprodukte und damit letztlich größere Mengen der Verbindungen der allgemeinen Formel I hergestellt werden. Auch eine de novo Synthese in einer Pflanze, der die Aktivität und Fähigkeit zur Biosynthese der Verbindungen vor dem Einbringen des/der entsprechenden Gens/Gene fehlte, ist möglich. Entsprechendes gilt für die Kombination mit weiteren Desaturasen oder Elongasen oder weiteren Enzymen aus dem Fettsäure- und Lipidstoffwechsel. Auch die Verwendung verschiedener divergenter, d.h. auf DNA-Sequenzebene unterschiedlicher Sequenzen kann dabei vorteilhaft sein bzw. die Verwendung von Promotoren zur Genexpression, die eine andere zeitliche Genexpression z.B. abhängig vom Reifegrad eines Samens oder Öl-speichernden Gewebes ermöglicht.

Durch das Einbringen eines Δ-12-Desaturase-, ω-3-Desaturase-, Δ-9-Elongase-, Δ-6-Desaturase-, Δ-8-Desaturase-, Δ-6-Elongase-, Δ-5-Desaturase-, Δ-5-Elongase-und/oder Δ-4-Desaturase-Genes in eine Pflanze allein oder in Kombination mit anderen Genen in eine Zelle kann nicht nur den Biosynthesefluss zum Endprodukt erhöht, sondern auch die entsprechende Triacylglycerin-Zusammensetzung erhöht oder de novo geschaffen werden. Ebenso kann die Anzahl oder Aktivität anderer Gene, die am Import von Nährstoffen, die zur Biosynthese einer oder mehrerer Fettsäuren, Ölen, polaren und/oder neutralen Lipiden nötig sind, erhöht sein, so dass die Konzentration dieser Vorläufer, Cofaktoren oder Zwischenverbindungen innerhalb der Zellen oder innerhalb des Speicherkompartiments erhöht ist, wodurch die Fähigkeit der Zellen zur Produktion von PUFAs, wie im folgenden beschrieben, weiter gesteigert wird. Durch Optimierung der Aktivität oder Erhöhung der Anzahl einer oder mehrerer Δ-12-Desaturase-, ω-3-Desaturase-, Δ-9-Elongase-, Δ-6-Desaturase-, Δ-8-Desaturase-, Δ-6-Elongase-, Δ-5-Desaturase-, Δ-5-Elongase- oder Δ-4-Desaturase-Gene, die an der Biosynthese dieser Verbindungen beteiligt sind, oder durch Zerstören der Aktivität einer oder mehrerer Gene, die am Abbau dieser Verbindungen beteiligt sind, kann es möglich sein, die Ausbeute, Produktion und/oder Effizienz der Produktion von Fettsäure- und Lipidmolekülen aus Organismen und vorteilhaft aus Pflanzen zu steigern.

Die im erfindungsgemäßen Verfahren verwendeten isolierten Nukleinsäuremoleküle codieren für Proteine oder Teile von diesen, wobei die Proteine oder das einzelne Protein oder Teile davon eine Aminosäuresequenz enthält, die ausreichend homolog zu einer Aminosäuresequenz ist, die in den Sequenzen SEQ ID NO: 2, SEQ ID NO: 4, SEQ ID NO:6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 60, SEQ ID NO: 62, SEQ ID NO: 64, SEQ ID NO: 66, SEQ ID NO: 68, SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 86, SEQ ID NO: 88, SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 112, SEQ ID NO: 114, SEQ ID NO: 118, SEQ ID NO: 120, SEQ ID NO: 132, SEQ ID NO: 134, SEQ ID NO: 136, SEQ ID NO: 138, SEQ ID NO: 184, SEQ ID NO: 194, SEQ ID NO: 198, SEQ ID NO: 200 oder SEQ ID NO: 202 dargestellt ist, so dass die Proteine oder Teile davon noch eine Δ-12-Desaturase-, ω-3-Desaturase-, Δ-9-Elongase-, Δ-6-Desaturase-, Δ-8-Desaturase-, Δ-6-Elongase-, Δ-5-Desaturase-, Δ-5-Elongase- oder Δ-4-Desaturase-Aktivität aufweisen. Vorzugsweise haben die Proteine oder Teile davon, die von dem Nukleinsäuremolekül/den Nukleinsäuremolekülen kodiert wird/werden, noch seine wesentliche enzymatische Aktivität und die Fähigkeit, am Stoffwechsel von zum Aufbau von Zellmembranen oder Lipidkörperchen in Organismen vorteilhaft in Pflanzen notwendigen Verbindungen oder am Transport von Molekülen über diese Membranen teilzunehmen. Vorteilhaft sind die von den Nukleinsäuremolekülen kodierten Proteine zu mindestens etwa 50 %, vorzugsweise mindestens etwa 60 % und stärker bevorzugt mindestens etwa 70 %, 80 % oder 90 % und am stärksten bevorzugt mindestens etwa 85 %, 86 %, 87 %, 88 %, 89 %, 90 %, 91 %, 92 %, 93 %, 94 %, 95 %, 96 %, 97 %, 98 %, 99 % oder mehr identisch zu den in SEQ ID NO: 2, SEQ ID NO: 4, SEQ ID NO:6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 60, SEQ ID NO: 62, SEQ ID NO: 64, SEQ ID NO: 66, SEQ ID NO: 68, SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 86, SEQ ID NO: 88, SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 112, SEQ ID NO: 114, SEQ ID NO: 118, SEQ ID NO: 120, SEQ ID NO: 132, SEQ ID NO: 134, SEQ ID NO: 136, SEQ ID NO: 138, SEQ ID NO: 184, SEQ ID NO: 194, SEQ ID NO: 198, SEQ ID NO: 200 oder SEQ ID NO: 202 dargestellten Aminosäuresequenzen. Im Sinne der Erfindung ist unter Homologie oder

homolog, Identität oder identisch zu verstehen.

Die Homologie wurde über den gesamten Aminosäure- bzw. Nukleinsäuresequenzbereich berechnet. Für das Vergleichen verschiedener Sequenzen stehen dem Fachmann eine Reihe von Programmen, die auf verschiedenen Algorithmen beruhen zur Verfügung. Dabei liefern die Algorithmen von Needleman und Wunsch oder Smith und Waterman besonders zuverlässige Ergebnisse. Für die Sequenzvergleiche wurde das Programm PileUp verwendet (J. Mol. Evolution., 25, 351-360, 1987, Higgins et al., CABIOS, 5 1989: 151-153) oder die Programme Gap und BestFit [Needleman and Wunsch (J. Mol. Biol. 48; 443-453 (1970) und Smith and Waterman (Adv. Appl. Math. 2; 482-489 (1981)], die im GCG Software-Packet [Genetics Computer Group, 575 Science Drive, Madison, Wisconsin, USA 53711 (1991)] enthalten sind. Die oben in Prozent angegebenen Sequenzhomologiewerte wurden mit dem Programm GAP über den gesamten Sequenzbereich mit folgenden Einstellungen ermittelt: Gap Weight: 50, Length Weight: 3, Average Match: 10.000 und Average Mismatch: 0.000. Diese Einstellungen wurden, falls nicht anders angegeben, immer als Standardeinstellungen für Sequenzvergleiche verwendet wurden.

Unter wesentlicher enzymatischer Aktivität der im erfindungsgemäßen Verfahren verwendeten Δ-12-Desaturase-, ω-3-Desaturase-, Δ-9-Elongase, Δ-6-Desaturase, Δ-8-Desaturase, Δ-6-Elongase, Δ-5-Desaturase, Δ-5-Elongase oder Δ-4-Desaturase ist zu verstehen, dass sie gegenüber den durch die Sequenz mit SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137, SEQ ID NO: 183, SEQ ID NO: 193, SEQ ID NO: 197, SEQ ID NO: 199 oder SEQ ID NO: 201 und deren Derivate codierten Proteinen/Enzymen im Vergleich noch mindestens eine enzymatische Aktivität von mindestens 10 %, bevorzugt 20 %, besonders bevorzugt 30 % und ganz besonders 40 % aufweisen und damit am Stoffwechsel von zum Aufbau von Fettsäuren, Fettsäureester wie Diacylglyceride und/oder Triacylglyceride in einem Organismus vorteilhaft einer Pflanze oder Pflanzenzelle notwendigen Verbindungen oder am Transport von Molekülen über Membranen teilnehmen können, wobei $C_{18}$-, $C_{20}$- oder $C_{22}$-Kohlenstoffketten im Fettsäuremolekül mit Doppelbindungen an mindestens zwei, vorteilhaft drei, vier, fünf oder sechs Stellen gemeint sind.

Vorteilhaft im Verfahren verwendbare Nukleinsäuren stammen aus Bakterien, Pilzen, Diatomeen, Tieren wie Caenorhabditis oder Oncorhynchus oder Pflanzen wie Algen oder Moosen wie den Gattungen Shewanella, Physcomitrella, Thraustochytrium, Fusarium, Phytophthora, Ceratodon, Mantoniella, Ostreococcus, Isochrysis, Aleurita, Muscarioides, Mortierella, Borago, Phaeodactylum, Crypthecodinium, speziell aus den Gattungen und Arten Oncorhynchus mykiss, Xenopus laevis, Ciona intestinalis, Thalassiosira pseudonona, Mantoniella squamata, Ostreococcus sp., Ostreococcus tauri, Euglena gracilis, Physcomitrella patens, Phytophtora infestans, Fusarium graminaeum, Cryptocodinium cohnii, Ceratodon purpureus, Isochrysis galbana, Aleurita farinosa, Thraustochytrium sp., Muscarioides viallii, Mortierella alpina, Borago officinalis, Phaeodactylum tricornutum, Caenorhabditis elegans oder besonders vorteilhaft aus Oncorhynchus mykiss, Euglena gracilis, Thalassiosira pseudonana oder Crypthecodinium cohnii.

Alternativ können im erfindungsgemäßen Verfahren Nukleotidsequenzen verwendet werden, die für eine Δ-12-Desaturase, ω-3-Desaturase, Δ-9-Elongase, Δ-6-Desaturase, Δ-8-Desaturase, Δ-6-Elongase, Δ-5-Desaturase, Δ-5-Elongase oder Δ-4-Desaturase codieren und die an eine Nukleotidsequenz, wie in SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137, SEQ ID NO: 183, SEQ ID NO: 193, SEQ ID NO: 197, SEQ ID NO: 199 oder SEQ ID NO: 201 dargestellt, vorteilhaft unter stringenten Bedingungen hybridisieren.

Die im Verfahren verwendeten Nukleinsäuresequenzen werden vorteilhaft in einer Expressionskassette, die die Expression der Nukleinsäuren in Organismen wie Mikroorganismen oder Pflanzen ermöglicht, eingebracht.

Dabei werden die Nukleinsäuresequenzen, die für die Δ-12-Desaturase, ω-3-Desaturase, Δ-9-Elongase, Δ-6-Desaturase, Δ-8-Desaturase, Δ-6-Elongase, Δ-5-Desaturase, Δ-5-Elongase oder Δ-4-Desaturase codieren, mit einem oder mehreren Regulationssignalen vorteilhafterweise zur Erhöhung der Genexpression funktionell verknüpft. Diese regulatorischen Sequenzen sollen die gezielte Expression der Gene und der Proteinexpression ermöglichen. Dies kann

beispielsweise je nach Wirtspflanze bedeuten, dass das Gen erst nach Induktion exprimiert und/oder überexprimiert wird, oder dass es sofort exprimiert und/oder überexprimiert wird. Beispielsweise handelt es sich bei diesen regulatorischen Sequenzen um Sequenzen an die Induktoren oder Repressoren binden und so die Expression der Nukleinsäure regulieren. Zusätzlich zu diesen neuen Regulationssequenzen oder anstelle dieser Sequenzen kann die natürliche Regulation dieser Sequenzen vor den eigentlichen Strukturgenen noch vorhanden sein und gegebenenfalls genetisch verändert worden sein, so dass die natürliche Regulation ausgeschaltet und die Expression der Gene erhöht wurde. Die Expressionskassette (= Expressionskonstrukt = Genkonstrukt) kann aber auch einfacher aufgebaut sein, das heißt es wurden keine zusätzlichen Regulationssignale vor die Nukleinsäuresequenz oder dessen Derivate inseriert und der natürliche Promotor mit seiner Regulation wurde nicht entfernt. Stattdessen wurde die natürliche Regulationssequenz so mutiert, dass keine Regulation mehr erfolgt und/oder die Genexpression gesteigert wird. Diese veränderten Promotoren können in Form von Teilsequenzen (= Promotor mit Teilen der erfindungsgemäßen Nukleinsäuresequenzen) auch allein vor das natürliche Gen zur Steigerung der Aktivität gebracht werden. Das Genkonstrukt kann außerdem vorteilhafterweise auch eine oder mehrere sogenannte "enhancer Sequenzen" funktionell verknüpft mit dem Promotor enthalten, die eine erhöhte Expression der Nukleinsäuresequenz ermöglichen. Auch am 3'-Ende der DNA-Sequenzen können zusätzliche vorteilhafte Sequenzen inseriert werden wie weitere regulatorische Elemente oder Terminatoren. Die Δ-12-Desaturase-, ω-3-Desaturase-, Δ-4-Desaturase-, Δ5-Desaturase-, Δ-6-Desaturase-, Δ-8-Desaturase-, Δ-5-Elongase-, Δ-6-Elongase- und/oder Δ-9-Elongase-Gene können in einer oder mehreren Kopien in der Expressionskassette (= Genkonstrukt) enthalten sein. Vorteilhaft liegt nur jeweils eine Kopie der Gene in der Expressionskassette vor. Dieses Genkonstrukt oder die Genkonstrukte können zusammen im Wirtsorganismus exprimiert werden. Dabei kann das Genkonstrukt oder die Genkonstrukte in einem oder mehreren Vektoren inseriert sein und frei in der Zelle vorliegen oder aber im Genom inseriert sein. Es ist vorteilhaft für die Insertion weiterer Gene im Wirtsgenom, wenn die zu exprimierenden Gene zusammen in einem Genkonstrukt vorliegen.

Die regulatorischen Sequenzen bzw. Faktoren können dabei wie oben beschrieben vorzugsweise die Genexpression der eingeführten Gene positiv beeinflussen und dadurch erhöhen. So kann eine Verstärkung der regulatorischen Elemente vorteilhafterweise auf der Transkriptionsebene erfolgen, indem starke Transkriptionssignale wie Promotoren und/oder "Enhancer" verwendet werden. Daneben ist aber auch eine Verstärkung der Translation möglich, indem beispielsweise die Stabilität der mRNA verbessert wird.

Vorteilhafte Regulationssequenzen für das neue Verfahren liegen beispielsweise in Promotoren vor, wie den Pflanzenpromotoren CaMV/35S [Franck et al., Cell 21 (1980) 285-294], PRP1 [Ward et al., Plant. Mol. Biol. 22 (1993)], SSU, OCS, lib4, usp, STLS1, B33, nos oder im Ubiquitin- oder Phaseolin-Promotor vor. In diesem Zusammenhang vorteilhaft sind ebenfalls induzierbare Promotoren, wie die in EP-A-0 388 186 (Benzylsulfonamid-induzierbar), Plant J. 2, 1992:397-404 (Gatz et al., Tetracyclininduzierbar), EP-A-0 335 528 (Abzisinsäure-induzierbar) oder WO 93/21334 (Ethanol- oder Cyclohexenol-induzierbar) beschriebenen Promotoren. Weitere geeignete Pflanzenpromotoren sind der Promotor von cytosolischer FBPase oder der ST-LSI-Promotor der Kartoffel (Stockhaus et al., EMBO J. 8, 1989, 2445), der Phosphoribosylpyrophosphatamidotransferase-Promotor aus Glycine max (Genbank-Zugangsnr. U87999) oder der in EP-A-0 249 676 beschriebene nodienspezifische Promotor. Besonders vorteilhafte Promotoren sind Promotoren, welche die Expression in Geweben ermöglichen, die an der Fettsäurebiosynthese beteiligt sind. Ganz besonders vorteilhaft sind samenspezifische Promotoren, wie der ausführungsgemäße USP Promotor aber auch andere Promotoren wie der LeB4-, DC3, Phaseolin- oder Napin-Promotor. Weitere besonders vorteilhafte Promotoren sind samenspezifische Promotoren, die für monokotyle oder dikotyle Pflanzen verwendet werden können und in US 5,608,152 (Napin-Promotor aus Raps), WO 98/45461 (Oleosin-Promotor aus Arobidopsis), US 5,504,200 (Phaseolin-Promotor aus Phaseolus vulgaris), WO 91/13980 (Bce4-Promotor aus Brassica), von Baeumlein et al., Plant J., 2, 2, 1992:233-239 (LeB4-Promotor aus einer Leguminose) beschrieben sind, wobei sich diese Promotoren für Dikotyledonen eignen. Die folgenden Promotoren eignen sich beispielsweise für Monokotyledonen Ipt-2- oder Ipt-1-Promotor aus Gerste (WO 95/15389 und WO 95/23230), Hordein-Promotor aus Gerste und andere, in WO 99/16890 beschriebene geeignete Promotoren.

Es ist im Prinzip möglich, alle natürlichen Promotoren mit ihren Regulationssequenzen, wie die oben genannten, für das neue Verfahren zu verwenden. Es ist ebenfalls möglich und vorteilhaft, zusätzlich oder alleine synthetische Promotoren zu verwenden, besonders wenn sie eine Samen-spezifische Expression vermitteln, wie z.B. beschrieben in WO 99/16890.

Um einen besonders hohen Gehalt an PUFAs vor allem in transgenen Pflanzen zu erzielen, sollten die PUFA-Biosynthesegene vorteilhaft samenspezifisch in Ölsaaten exprimiert werden. Hierzu können Samen-spezifische Promotoren verwendet werden, bzw. solche Promotoren die im Embryo und/oder im Endosperm aktiv sind. Samen-spezifische Promotoren können prinzipiell sowohl aus dikotolydonen als auch aus monokotolydonen Pflanzen isoliert werden. Derartige vorteilhafte Promotoren sind weiter oben aufgeführt z.B. der USP-, Vicilin-, Napin-, Oleosin-, Phaseolin-, Bce4-, LegB4-, Lpt2-, Ipt1-, Amy32b-, Amy 6-6-, Aleurain- oder Bce4-Promotor.

Darüber hinaus sind auch chemisch induzierbaren Promotor vorteilhaft im erfindungsgemäßen Verfahren nutzbar.

Weitere vorteilhafte Promotoren, die vorteilhaft zur Expression in Soya geeignet sind, sind die Promotoren der

β-Conglycinin-α-Untereinheit, der β-Conglycinin-β-Untereinheit, des Kunitz-Trypsininhibitors, des Annexin, des Glysinin, des Albumin 2S, des Legumin A1, des Legumin A2 und der des BD30.

Besonders vorteilhafte Promotoren sind der USP-, LegB4-, Fad3-, SBP-, DC-3- oder Cruciferin820 Promotor.

Vorteilhafte Regulationssequenzen, die für die Expression der im erfindungsgemäßen Verfahren verwendeten Nukleinsäuresequenzen benutzt werden, sind Terminatoren für die Expression vorteilhaft in Soya sind der Leg2A3', Kti3', Phas3', BD30 3' oder der AIS3'.

Besonders vorteilhafte Terminatoren sind der A7T-, OCS-, LeB3T- oder cat-Terminator.

Um eine stabile Integration der Biosynthesegene in die transgene Pflanze über mehrere Generation sicherzustellen, sollte, wie oben beschrieben, jede der im Verfahren verwendeten Nukleinsäuren, die für die Δ-12-Desaturase, ω-3-Desaturase, Δ-9-Elongase, Δ-6-Desaturase, Δ-8-Desaturase, Δ-6-Elongase, Δ-5-Desaturase, Δ-5-Elongase und/oder Δ-4-Desaturase codieren, unter der Kontrolle eines eigenen bevorzugt eines unterschiedlichen Promotors exprimiert werden, da sich wiederholende Sequenzmotive zu Instabilität der T-DNA bzw. zu Rekombinationsereignissen führen können. Das Genkonstrukt kann, wie oben beschrieben, auch weitere Gene umfassen, die in die Pflanze eingebracht werden sollen.

Die zur Expression der im erfindungsgemäßen Verfahren verwendeten Nukleinsäuren vorteilhaft genutzten regulatorischen Sequenzen bzw. Faktoren können dabei wie oben beschrieben vorzugsweise die Genexpression der eingeführten Gene positiv beeinflussen und dadurch erhöhen.

Diese vorteilhaften Vektoren, vorzugsweise Expressionsvektoren, enthalten die im Verfahren verwendeten Nukleinsäuren, die für die Δ-12-Desaturasen, ω-3-Desaturasen, Δ-9-Elongasen, Δ-6-Desaturasen, Δ-8-Desaturasen, Δ-6-Elongasen, Δ-5-Desaturasen, Δ-5-Elongasen oder Δ-4-Desaturasen codieren, oder ein Nukleinsäurekonstrukt, die die verwendeten Nukleinsäure allein oder in Kombination mit weiteren Biosynthesegenen des Fettsäure- oder Lipidstoffwechsels wie den Acyl-CoA:Lysophospholipid-Acyltransferasen, ω-3-Desaturasen, Δ-4-Desaturasen, Δ-5-Desaturasen, Δ-6-Desaturasen, Δ-8-Desatuasen, Δ-9-Desaturasen, Δ-12-Desaturasen, ω3-Desaturasen, Δ-5-Elongasen, Δ-6-Elongasen und/oder Δ-9-Elongasen.

Wie hier verwendet und beschrieben, betrifft der Begriff "Vektor" ein Nukleinsäuremolekül, das eine andere Nukleinsäure transportieren kann, an welche es gebunden ist.

Die verwendeten rekombinanten Expressionsvektoren können zur Expression von Δ-12-Desaturasen, ω-3-Desaturasen, Δ-9-Elongasen, Δ-6-Desaturasen, Δ-8-Desaturasen, Δ-6-Elongasen, Δ-5-Desaturasen, Δ-5-Elongasen und/oder Δ-4-Desaturasen in prokaryotischen oder eukaryotischen Zellen gestaltet sein. Dies ist vorteilhaft, da häufig Zwischenschritte der Vektorkonstruktion der Einfachheithalber in Mikroorganismen durchgeführt werden. Beispielsweise können die Δ-12-Desaturase-, ω-3-Desaturase-, Δ-9-Elongase-, Δ-6-Desaturase-, Δ-8-Desaturase-, Δ-6-Elongase-, Δ-5-Desaturase-, Δ-5-Elongase- und/oder Δ-4-Desaturase-Gene in bakteriellen Zellen, Insektenzellen (unter Verwendung von Baculovirus-Expressionsvektoren), Hefe- und anderen Pilzzellen (siehe Romanos, M.A., et al. (1992) "Foreign gene expression in yeast: a review", Yeast 8:423-488; van den Hondel, C.A.M.J.J., et al. (1991) "Heterologous gene expression in filamentous fungi", in: More Gene Manipulations in Fungi, J.W. Bennet & L.L. Lasure, Hrsgb., S. 396-428: Academic Press: San Diego; und van den Hondel, C.A.M.J.J., & Punt, P.J. (1991) "Gene transfer systems and vector development for filamentous fungi, in: Applied Molecular Genetics of Fungi, Peberdy, J.F., et al., Hrsgb., S. 1-28, Cambridge University Press: Cambridge), Algen (Falciatore et al., 1999, Marine Biotechnology.1, 3:239-251), Ciliaten der Typen: Holotrichia, Peritrichia, Spirotrichia, Suctoria, Tetrahymena, Paramecium, Colpidium, Glaucoma, Platyophrya, Potomacus, Desaturaseudocohnilembus, Euplotes, Engelmaniella und Stylonychia, insbesondere der Gattung Stylonychia lemnae, mit Vektoren nach einem Transformationsverfahren, wie beschrieben in WO 98/01572, sowie bevorzugt in Zellen vielzelliger Pflanzen (siehe Schmidt, R. und Willmitzer, L. (1988) "High efficiency Agrobacterium tumefaciens-mediated transformation of Arabidopsis thaliana leaf and cotyledon explants" Plant Cell Rep.:583-586; Plant Molecular Biology and Biotechnology, C Press, Boca Raton, Florida, Kapitel 6/7, S.71-119 (1993); F.F. White, B. Jenes et al., Techniques for Gene Transfer, in: Transgenic Plants, Bd. 1, Engineering and Utilization, Hrsgb.: Kung und R. Wu, Academic Press (1993), 128-43; Potrykus, Annu. Rev. Plant Physiol. Plant Molec. Biol. 42 (1991), 205-225 (und darin zitierte Literaturstellen)) exprimiert werden. Geeignete Wirtszellen werden ferner erörtert in Goeddel, Gene Expression Technology: Methods in Enzymology 185, Academic Press, San Diego, CA (1990). Der rekombinante Expressionsvektor kann alternativ, zum Beispiel unter Verwendung von T7-Promotor-Regulationssequenzen und T7-Polymerase, in vitro transkribiert und translatiert werden.

Die Expression von Proteinen in Prokaryoten, vorteilhaft zu einfachen Detektion der enzymatischen Aktivität z.B. zum Nachweis der Desaturase- oder Elongaseaktivität, erfolgt meist mit Vektoren, die konstitutive oder induzierbare Promotoren enthalten, welche die Expression von Fusions- oder nicht-Fusionsproteinen steuern. Typische Fusions-Expressionsvektoren sind u.a. pGEX (Pharmacia Biotech Inc; Smith, D.B., und Johnson, K.S. (1988) Gene 67:31-40), pMAL (New England Biolabs, Beverly, MA) und pRIT5 (Pharmacia, Piscataway, NJ), bei denen Glutathion-S-Transferase (GST), Maltose E-bindendes Protein bzw. Protein A an das rekombinante Zielprotein fusioniert wird.

Beispiele für geeignete induzierbare nicht-Fusions-E. coli-Expressionsvektoren sind u.a. pTrc (Amann et al. (1988) Gene 69:301-315) und pET 11d (Studier et al., Gene Expression Technology: Methods in Enzymology 185,

Academic Press, San Diego, Kalifornien (1990) 60-89). Die Zielgenexpression vom pTrc-Vektor beruht auf der Transkription durch Wirts-RNA-Polymerase von einem Hybrid-trp-lac-Fusionspromotor. Die Zielgenexpression aus dem pET 11d-Vektor beruht auf der Transkription von einem T7-gn10-lac-Fusions-Promotor, die von einer coexprimierten viralen RNA-Polymerase (T7 gn1) vermittelt wird. Diese virale Polymerase wird von den Wirtsstämmen BL21 (DE3) oder HMS174 (DE3) von einem residenten λ-Prophagen bereitgestellt, der ein T7 gn1-Gen unter der Transkriptionskontrolle des lacUV 5-Promotors birgt.

Andere in prokaryotischen Organismen geeignete Vektoren sind dem Fachmann bekannt, diese Vektoren sind beispielsweise in E. coli pLG338, pACYC184, die pBR-Reihe, wie pBR322, die pUC-Reihe, wie pUC18 oder pUC19, die M113mp-Reihe, pKC30, pRep4, pHS1, pHS2, pPLc236, pMBL24, pLG200, pUR290, pIN-III113-B1, λgt11 or pBdCl, in Streptomyces pIJ101, pIJ364, pIJ702 oder pIJ361, in Bacillus pUB110, pC194 oder pBD214, in Corynebacterium pSA77 oder pAJ667.

Bei einer weiteren Ausführungsform ist der Expressionsvektor ein Hefe-Expressionsvektor. Beispiele für Vektoren zur Expression in der Hefe S. cerevisiae umfassen pYeDesaturasec1 (Baldari et al. (1987) Embo J. 6:229-234), pMFa (Kurjan und Herskowitz (1982) Cell 30:933-943), pJRY88 (Schultz et al. (1987) Gene 54:113-123) sowie pYES2 (Invitrogen Corporation, San Diego, CA). Vektoren und Verfahren zur Konstruktion von Vektoren, die sich zur Verwendung in anderen Pilzen, wie den filamentösen Pilzen, eignen, umfassen diejenigen, die eingehend beschrieben sind in: van den Hondel, C.A.M.J.J., & Punt, P.J. (1991) "Gene transfer systems and vector development for filamentous fungi, in: Applied Molecular Genetics of fungi, J.F. Peberdy et al., Hrsgb., S. 1-28, Cambridge University Press: Cambridge, oder in: More Gene Manipulations in Fungi [J.W. Bennet & L.L. Lasure, Hrsgb., S. 396-428: Academic Press: San Diego]. Weitere geeignete Hefevektoren sind beispielsweise pAG-1, YEp6, YEp13 oder pEMBLYe23.

Alternativ können die Δ-12-Desaturasen, ω-3-Desaturasen, Δ-9-Elongasen, Δ-6-Desaturasen, Δ-8-Desaturasen, Δ-6-Elongasen, Δ-5-Desaturasen, Δ-5-Elongasen und/oder Δ-4-Desaturasen in Insektenzellen unter Verwendung von Baculovirus-Expressionsvektoren exprimiert werden. Baculovirus-Vektoren, die zur Expression von Proteinen in gezüchteten Insektenzellen (z.B. Sf9-Zellen) verfügbar sind, umfassen die pAc-Reihe (Smith et al. (1983) Mol. Cell Biol.. 3:2156-2165) und die pVL-Reihe (Lucklow und Summers (1989) Virology 170:31-39).

Die oben genannten Vektoren bieten nur einen kleinen Überblick über mögliche geeignete Vektoren. Weitere Plasmide sind dem Fachmann bekannt und sind zum Beispiel beschrieben in: Cloning Vectors (Hrsgb. Pouwels, P.H., et al., Elsevier, Amsterdam-New York-Oxford, 1985, ISBN 0 444 904018). Weitere geeignete Expressionssysteme für prokaryotische und eukaryotische Zellen siehe in den Kapiteln 16 und 17 von Sambrook, J., Fritsch, E.F., und Maniatis, T., Molecular Cloning: A Laboratory Manual, 2. Auflage, Cold Spring Harbor Laboratory, Cold Spring Harbor Laboratory Press, Cold Spring Harbor, NY, 1989.

Zum Nachweis der Enzymaktivität können die Δ-12-Desaturasen, ω-3-Desaturasen, Δ-9-Elongasen, Δ-6-Desaturasen, Δ-8-Desaturasen, Δ-6-Elongasen, Δ-5-Desaturasen, Δ-5-Elongasen und/oder Δ-4-Desaturasen in einzelligen Pflanzenzellen (wie Algen), siehe Falciatore et al., 1999, Marine Biotechnology 1 (3):239-251 und darin zitierte Literaturangaben, und Pflanzenzellen aus höheren Pflanzen (z.B. Spermatophyten, wie Feldfrüchten) exprimiert werden. Beispiele für Pflanzen-Expressionsvektoren umfassen solche, die eingehend beschrieben sind in: Becker, D., Kemper, E., Schell, J., und Masterson, R. (1992) "New plant binary vectors with selectable markers located proximal to the left border", Plant Mol. Biol. 20:1195-1197; und Bevan, M.W. (1984) "Binary Agrobacterium vectors for plant transformation", Nucl. Acids Res. 12:8711-8721; Vectors for Gene Transfer in Higher Plants; in: Transgenic Plants, Bd. 1, Engineering and Utilization, Hrsgb.: Kung und R. Wu, Academic Press, 1993, S. 15-38.

Eine Pflanzen-Expressionskassette enthält vorzugsweise Regulationssequenzen, welche die Genexpression in Pflanzenzellen steuern können und funktionsfähig verbunden sind, so dass jede Sequenz ihre Funktion, wie Termination der Transkription, erfüllen kann, beispielsweise Polyadenylierungssignale. Bevorzugte Polyadenylierungssignale sind diejenigen, die aus Agrobacterium tumefaciens-T-DNA stammen, wie das als Octopinsynthase bekannte Gen 3 des Ti-Plasmids pTiACH5 (Gielen et al., EMBO J. 3 (1984) 835ff.) oder funktionelle Äquivalente davon, aber auch alle anderen in Pflanzen funktionell aktiven Terminatoren sind geeignet.

Da die Pflanzengenexpression sehr oft nicht auf Transkriptionsebenen beschränkt ist, enthält eine Pflanzen-Expressionskassette vorzugsweise andere funktionsfähig verbunden Sequenzen, wie Translationsenhancer, beispielsweise die Overdrive-Sequenz, welche die 5'-untranslatierte Leader-Sequenz aus Tabakmosaikvirus, die das Protein/RNA-Verhältnis erhöht, enthält (Gallie et al., 1987, Nucl. Acids Research 15:8693-8711).

Die Pflanzengenexpression muss wie oben beschrieben funktionsfähig mit einem geeigneten Promotor verbunden sein, der die Genexpression auf rechtzeitige, zell- oder gewebespezifische Weise durchführt. Nutzbare Promotoren sind konstitutive Promotoren (Benfey et al., EMBO J. 8 (1989) 2195-2202), wie diejenigen, die von Pflanzenviren stammen, wie 35S CAMV (Franck et al., Cell 21 (1980) 285-294), 19S CaMV (siehe auch US 5352605 und WO 84/02913) oder Pflanzenpromotoren, wie der in US 4,962,028 beschriebene der kleinen Untereinheit der Rubisco.

Andere bevorzugte Sequenzen für die Verwendung zur funktionsfähigen Verbindung in Pflanzengenexpressions-Kassetten sind Targeting-Sequenzen, die zur Steuerung des Genproduktes in sein entsprechendes Zellkompartiment notwendig sind (siehe eine Übersicht in Kermode, Crit. Rev. Plant Sci. 15, 4 (1996) 285-423 und darin zitierte

Literaturstellen), beispielsweise in die Vakuole, den Zellkern, alle Arten von Plastiden, wie Amyloplasten, Chloroplasten, Chromoplasten, den extrazellulären Raum, die Mitochondrien, das Endoplasmatische Retikulum, Ölkörper, Peroxisomen und andere Kompartimente von Pflanzenzellen.

Die Pflanzengenexpression lässt sich auch wie oben beschrieben über einen chemisch induzierbaren Promotor erleichtern (siehe eine Übersicht in Gatz 1997, Annu. Rev. Plant Physiol. Plant Mol. Biol., 48:89-108). Chemisch induzierbare Promotoren eignen sich besonders, wenn gewünscht wird, dass die Genexpression auf zeitspezifische Weise erfolgt. Beispiele für solche Promotoren sind ein Salicylsäure-induzierbarer Promotor (WO 95/19443), ein Tetracyclin-induzierbarer Promotor (Gatz et al. (1992) Plant J. 2, 397-404) und ein Ethanol-induzierbarer Promotor.

Auch Promotoren, die auf biotische oder abiotische Stressbedingungen reagieren, sind geeignete Promotoren, beispielsweise der pathogeninduzierte PRP1-Gen-Promotor (Ward et al., Plant. Mol. Biol. 22 (1993) 361-366), der hitzeinduzierbare hsp80-Promotor aus Tomate (US 5,187,267), der kälteinduzierbare Alpha-Amylase-Promotor aus Kartoffel (WO 96/12814) oder der durch Wunden induzierbare pinII-Promotor (EP-A-0 375 091).

Es sind insbesondere solche Promotoren bevorzugt, welche die Genexpression in Geweben und Organen herbeiführen, in denen die Fettsäure-, Lipid- und Ölbiosynthese stattfindet, in Samenzellen, wie den Zellen des Endosperms und des sich entwickelnden Embryos. Geeignete Promotoren sind der Napingen-Promotor aus Raps (US 5,608,152), der USP-Promotor aus Vicia faba (Baeumlein et al., Mol Gen Genet, 1991, 225 (3):459-67), der Oleosin-Promotor aus Arabidopsis (WO 98/45461), der Phaseolin-Promotor aus Phaseolus vulgaris (US 5,504,200), der Bce4-Promotor aus Brassica (WO 91/13980) oder der Legumin-B4-Promotor (LeB4; Baeumlein et al., 1992, Plant Journal, 2 (2):233-9) sowie Promotoren, welche die samenspezifische Expression in Monokotyledonen-Pflanzen, wie Mais, Gerste, Weizen, Roggen, Reis usw. herbeiführen. Geeignete beachtenswerte Promotoren sind der lpt2- oder lpt1-Gen-Promotor aus Gerste (WO 95/15389 und WO 95/23230) oder die in WO 99/16890 beschriebenen (Promotoren aus dem Gersten-Hordein-Gen, dem Reis-Glutelin-Gen, dem Reis-Oryzin-Gen, dem Reis-Prolamin-Gen, dem Weizen-Gliadin-Gen, Weizen-Glutelin-Gen, dem Mais-Zein-Gen, dem Hafer-Glutelin-Gen, dem Sorghum-Kasirin-Gen, dem Roggen-Secalin-Gen).

Insbesondere kann die multiparallele Expression der im Verfahren verwendeten Δ-12-Desaturasen, ω-3-Desaturasen, Δ-9-Elongasen, Δ-6-Desaturasen, Δ-8-Desaturasen, Δ-6-Elongasen, Δ-5-Desaturasen, Δ-5-Elongasen und/oder Δ-4-Desaturasen gewünscht sein. Die Einführung solcher Expressionskassetten kann über eine simultane Transformation mehrerer einzelner Expressionskonstrukte erfolgen oder bevorzugt durch Kombination mehrerer Expressionskassetten auf einem Konstrukt. Auch können mehrere Vektoren mit jeweils mehreren Expressionskassetten transformiert und auf die Wirtszelle übertragen werden.

Ebenfalls besonders geeignet sind Promotoren, welche die plastidenspezifische Expression herbeiführen, da Plastiden das Kompartiment sind, in dem die Vorläufer sowie einige Endprodukte der Lipidbiosynthese synthetisiert werden. Geeignete Promotoren, wie der virale RNA-Polymerase-Promotor, sind beschrieben in WO 95/16783 und WO 97/06250, und der clpP-Promotor aus Arabidopsis, beschrieben in WO 99/46394.

Vektor-DNA lässt sich in prokaryotische oder eukaryotische Zellen über herkömmliche Transformations- oder Transfektionstechniken einbringen. Die Begriffe "Transformation" und "Transfektion", Konjugation und Transduktion, wie hier verwendet, sollen eine Vielzahl von im Stand der Technik bekannten Verfahren zum Einbringen fremder Nukleinsäure (z.B. DNA) in eine Wirtszelle, einschließlich Calciumphosphat- oder Calciumchlorid-Copräzipitation, DEAE-Dextran-vermittelte Transfektion, Lipofektion, natürliche Kompetenz, chemisch vermittelter Transfer, Elektroporation oder Teilchenbeschuss, umfassen. Geeignete Verfahren zur Transformation oder Transfektion von Wirtszellen, einschließlich Pflanzenzellen, lassen sich finden in Sambrook et al. (Molecular Cloning: A Laboratory Manual., 2. Aufl., Cold Spring Harbor Laboratory, Cold Spring Harbor Laboratory Press, Cold Spring Harbor, NY, 1989) und anderen Labor-Handbüchern, wie Methods in Molecular Biology, 1995, Bd. 44, Agrobacterium protocols, Hrsgb: Gartland und Davey, Humana Press, Totowa, New Jersey.

Die vorteilhafterweise verwendeten Wirtsorganismen sind Pflanzenzellen vorzugsweise Pflanzen oder Teile davon. Besonders bevorzugt sind Pflanzen, wie Ölsamen- oder Ölfruchtpflanzen, die große Mengen an Lipidverbindungen enthalten, wie Raps, Nachtkerze, Hanf, Diestel, Erdnuss, Canola, Lein, Soja, Saflor, Sareptasenf, Sonnenblume, Borretsch, oder Pflanzen, wie Mais, Weizen, Roggen, Hafer, Triticale, Reis, Gerste, Baumwolle, Maniok, Pfeffer, Tagetes, Solanaceen-Pflanzen, wie Kartoffel, Tabak, Aubergine und Tomate, Vicia-Arten, Erbse, Alfalfa, Buschpflanzen (Kaffee, Kakao, Tee), Salix-Arten, Bäume (Ölplame, Kokosnuss) sowie ausdauernde Gräser und Futterfeldfrüchte. Besonders bevorzugte erfindungsgemäße Pflanzen sind Ölfruchtpflanzen, wie Soja, Erdnuss, Raps, Canola, Lein, Hanf, Nachtkerze, Sonnenblume, Saflor, Bäume (Ölpalme, Kokosnuss).

Offenbart ist somit, wie oben beschrieben, eine isolierte Nukleinsäuresequenz, die für Polypeptide mit Δ-5-Elongase-Aktivität codiert und die die in in SEQ ID NO: 197 dargestellte Sequenz hat, wobei die durch die Nukleinsäuresequenz codierte Elongase $C_{16}$- und $C_{18}$- Fettsäuren mit einer Doppelbindung nicht elongiert. Auch mehrfach ungesättigte $C_{18}$-Fettsäuren mit einer Δ6-Doppelbindung oder $C_{22}$-Fettsäuren werden nicht umgesetzt. Durch die enzymatische Aktivität werden vorteilhaft nur mehrfach ungesättigte $C_{20}$-Fettsäuren mit einer Δ5-Doppelbindung elongiert. Weitere Erfindungsgegenstände sind, wie oben beschrieben, eine Δ-6-Elongase, Δ-6-Desaturase und eine Δ-12-Desaturase.

Der Begriff "Nukleinsäure(molekül)", wie hier verwendet, umfasst in einer vorteilhaften Ausführungsform zudem die am 3'- und am 5'-Ende des kodierenden Genbereichs gelegene untranslatierte Sequenz: mindestens 500, bevorzugt 200, besonders bevorzugt 100 Nukleotide der Sequenz stromaufwärts des 5'-Endes des kodierenden Bereichs und mindestens 100, bevorzugt 50, besonders bevorzugt 20 Nukleotide der Sequenz stromabwärts des 3'-Endes des kodierenden Genbereichs. Ein "isoliertes" Nukleinsäuremolekül wird von anderen Nukleinsäuremolekülen abgetrennt, die in der natürlichen Quelle der Nukleinsäure vorliegen. Eine "isolierte" Nukleinsäure hat vorzugsweise keine Sequenzen, welche die Nukleinsäure in der genomischen DNA des Organismus, aus dem die Nukleinsäure stammt, natürlicherweise flankieren (z.B. Sequenzen, die sich an den 5'- und 3'-Enden der Nukleinsäure befinden). Bei verschiedenen Ausführungsformen kann das isolierte Δ-12-Desaturase-, ω-3-Desaturase-, Δ-9-Elongase-, Δ-6-Desaturase-, Δ-8-Desaturase-, Δ-6-Elongase-, Δ-5-Desaturase-, Δ-5-Elongase- oder Δ-4-Desaturasemolekül zum Beispiel weniger als etwa 5 kb, 4 kb, 3 kb, 2 kb, 1 kb, 0,5 kb oder 0,1 kb an Nukleotidsequenzen enthalten, die natürlicherweise das Nukleinsäuremolekül in der genomischen DNA der Zelle, aus der die Nukleinsäure stammt flankieren.

Die im Verfahren verwendeten Nukleinsäuremoleküle, z.B. ein Nukleinsäuremolekül mit einer Nukleotidsequenz der SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137, SEQ ID NO: 183, SEQ ID NO: 193, SEQ ID NO: 197, SEQ ID NO: 199 oder SEQ ID NO: 201 oder eines Teils davon, kann unter Verwendung molekularbiologischer Standardtechniken und der hier bereitgestellten Sequenzinformation isoliert werden. Auch kann Mithilfe von Vergleichsalgorithmen beispielsweise eine homologe Sequenz oder homologe, konservierte Sequenzbereiche auf DNA oder Aminosäureebene identifiziert werden. Diese können als Hybridisierungssonde sowie Standard-Hybridisierungstechniken (wie z.B. beschrieben in Sambrook et al., Molecular Cloning: A Laboratory Manual. 2. Aufl., Cold Spring Harbor Laboratory, Cold Spring Harbor Laboratory Press, Cold Spring Harbor, NY, 1989) zur Isolierung weiterer im Verfahren nützlicher Nukleinsäuresequenzen verwendet werden. Überdies lässt sich ein Nukleinsäuremolekül, umfassend eine vollständige Sequenz der SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137, SEQ ID NO: 183, SEQ ID NO: 193, SEQ ID NO: 197, SEQ ID NO: 199 oder SEQ ID NO: 201 oder einen Teil davon, durch Polymerasekettenreaktion isolieren, wobei Oligonukleotidprimer, die auf der Basis dieser Sequenz oder von Teilen davon, verwendet werden (z.B. kann ein Nukleinsäuremolekül, umfassend die vollständigen Sequenz oder einen Teil davon, durch Polymerasekettenreaktion unter Verwendung von Oligonukleotidprimern isoliert werden, die auf der Basis dieser gleichen Sequenz erstellt worden sind). Zum Beispiel lässt sich mRNA aus Zellen isolieren (z.B. durch das Guanidiniumthiocyanat-Extraktionsverfahren von Chirgwin et al. (1979) Biochemistry 18:5294-5299) und cDNA mittels Reverser Transkriptase (z.B. Moloney-MLV-Reverse-Transkriptase, erhältlich von Gibco/BRL, Bethesda, MD, oder AMV-Reverse-Transkriptase, erhältlich von Seikagaku America, Inc., St.Petersburg, FL) herstellen. Synthetische Oligonukleotidprimer zur Amplifizierung mittels Polymerasekettenreaktion lassen sich auf der Basis einer der in SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137, SEQ ID NO: 183, SEQ ID NO: 193, SEQ ID NO: 197, SEQ ID NO: 199 oder SEQ ID NO: 201 gezeigten Sequenzen oder Mithilfe der in SEQ ID NO: 2, SEQ ID NO: 4, SEQ ID NO:6, SEQ ID NO: 8, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ

ID NO: 42, SEQ ID NO: 44, SEQ ID NO: 46, SEQ ID NO: 48, SEQ ID NO: 50, SEQ ID NO: 52, SEQ ID NO: 54, SEQ ID NO: 60, SEQ ID NO: 62, SEQ ID NO: 64, SEQ ID NO: 66, SEQ ID NO: 68, SEQ ID NO: 70, SEQ ID NO: 72, SEQ ID NO: 74, SEQ ID NO: 76, SEQ ID NO: 78, SEQ ID NO: 80, SEQ ID NO: 82, SEQ ID NO: 84, SEQ ID NO: 86, SEQ ID NO: 88, SEQ ID NO: 92, SEQ ID NO: 94, SEQ ID NO: 96, SEQ ID NO: 98, SEQ ID NO: 100, SEQ ID NO: 102, SEQ ID NO: 104, SEQ ID NO: 112, SEQ ID NO: 114, SEQ ID NO: 118, SEQ ID NO: 120, SEQ ID NO: 132, SEQ ID NO: 134, SEQ ID NO: 136, SEQ ID NO: 138, SEQ ID NO: 184, SEQ ID NO: 194, SEQ ID NO: 198, SEQ ID NO: 200 oder SEQ ID NO: 202 dargestellten Aminosäuresequenzen erstellen. Eine der vorgenannten Nukleinsäuren kann unter Verwendung von cDNA oder alternativ von genomischer DNA als Matrize und geeigneten Oligonukleotidprimern gemäß Standard-PCR-Amplifikationstechniken amplifiziert werden. Die so amplifizierte Nukleinsäure kann in einen geeigneten Vektor kloniert werden und mittels DNA-Sequenzanalyse charakterisiert werden. Oligonukleotide, die einer Desaturase-Nukleotidsequenz entsprechen, können durch Standard-Syntheseverfahren, beispielsweise mit einem automatischen DNA-Synthesegerät, hergestellt werden.

Homologe der verwendeten Δ-12-Desaturase-, ω-3-Desaturase-, Δ-9-Elongase-, Δ-6-Desaturase-, Δ-8-Desaturase-, Δ-6-Elongase-, Δ-5-Desaturase-, Δ-5-Elongase- oder Δ-4-Desaturase-Nukleinsäuresequenzen mit der Sequenz SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137, SEQ ID NO: 183, SEQ ID NO: 193, SEQ ID NO: 197, SEQ ID NO: 199 oder SEQ ID NO: 201 bedeutet beispielsweise allelische Varianten mit mindestens etwa 50 oder 60 %, vorzugsweise mindestens etwa 60 oder 70 %, stärker bevorzugt mindestens etwa 70 oder 80 %, 90 % oder 95 % und noch stärker bevorzugt mindestens etwa 85 %, 86 %, 87 %, 88 %, 89 %, 90 %, 91 %, 92 %, 93 %, 94 %, 95 %, 96 %, 97 %, 98 %, 99 % oder mehr Identität bzw. Homologie zu einer in SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137, SEQ ID NO: 183, SEQ ID NO: 193, SEQ ID NO: 197, SEQ ID NO: 199 oder SEQ ID NO: 201 gezeigten Nukleotidsequenzen oder ihren Homologen, Derivaten oder Analoga oder Teilen davon. Weiterhin sind isolierte Nukleinsäuremoleküle einer Nukleotidsequenz, die an eine der in SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137, SEQ ID NO: 183, SEQ ID NO: 193, SEQ ID NO: 197, SEQ ID NO: 199 oder SEQ ID NO: 201 gezeigten Nukleotidsequenzen oder einen Teil davon hybridisieren, z.B. unter stringenten Bedingungen hybridisiert. Unter einem Teil gemäß der Erfindung ist dabei zu verstehen, dass mindestens 25 Basenpaare (= bp), 50 bp, 75 bp, 100 bp, 125 bp oder 150 bp, bevorzugt mindestens 175 bp, 200 bp, 225 bp, 250 bp, 275 bp oder 300 bp, besonders bevorzugt 350 bp, 400 bp, 450 bp, 500 bp oder mehr Basenpaare für die Hybridisierung verwendet werden. Es kann auch vorteilhaft die Gesamtsequenz verwendet werden. Allelische Varianten umfassen insbesondere funktionelle Varianten, die sich durch Deletion, Insertion oder Substitution von Nukleotiden aus/in der in SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 89, SEQ ID NO: 91, SEQ ID NO: 93, SEQ ID NO: 95, SEQ ID NO: 97, SEQ ID NO:

99, SEQ ID NO: 101, SEQ ID NO: 103, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137, SEQ ID NO: 183, SEQ ID NO: 193, SEQ ID NO: 197, SEQ ID NO: 199 oder SEQ ID NO: 201 dargestellten Sequenz erhalten lassen, wobei aber die Absicht ist, dass die Enzymaktivität der davon herrührenden synthetisierten Proteine für die Insertion eines oder mehrerer Gene vorteilhafterweise beibehalten wird. Proteine, die noch die enzymatische Aktivität der Δ-12-Desaturase, ω-3-Desaturase, Δ-9-Elongase, Δ-6-Desaturase, Δ-8-Desaturase, Δ-6-Elongase, Δ-5-Desaturase, Δ-5-Elongase oder Δ-4-Desaturase besitzen, das heißt deren Aktivität im wesentlichen nicht reduziert ist, bedeutet Proteine mit mindestens 10 %, vorzugsweise 20 %, besonders bevorzugt 30 %, ganz besonders bevorzugt 40 % der ursprünglichen Enzymaktivität, verglichen mit dem durch SEQ ID NO: 1, SEQ ID NO: 3, SEQ ID NO:5, SEQ ID NO: 7, SEQ ID NO: 9, SEQ ID NO: 11, SEQ ID NO: 13, SEQ ID NO: 15, SEQ ID NO: 17, SEQ ID NO: 19, SEQ ID NO: 21, SEQ ID NO: 23, SEQ ID NO: 25, SEQ ID NO: 27, SEQ ID NO: 29, SEQ ID NO: 31, SEQ ID NO: 33, SEQ ID NO: 35, SEQ ID NO: 37, SEQ ID NO: 39, SEQ ID NO: 41, SEQ ID NO: 43, SEQ ID NO: 45, SEQ ID NO: 47, SEQ ID NO: 49, SEQ ID NO: 51, SEQ ID NO: 53, SEQ ID NO: 59, SEQ ID NO: 61, SEQ ID NO: 63, SEQ ID NO: 65, SEQ ID NO: 67, SEQ ID NO: 69, SEQ ID NO: 71, SEQ ID NO: 73, SEQ ID NO: 75, SEQ ID NO: 77, SEQ ID NO: 79, SEQ ID NO: 81, SEQ ID NO: 83, SEQ ID NO: 85, SEQ ID NO: 89, SEQ ID NO: 91, SEQ ID NO: 93, SEQ ID NO: 95, SEQ ID NO: 97, SEQ ID NO: 99, SEQ ID NO: 101, SEQ ID NO: 103, SEQ ID NO: 111, SEQ ID NO: 113, SEQ ID NO: 117, SEQ ID NO: 119, SEQ ID NO: 131, SEQ ID NO: 133, SEQ ID NO: 135, SEQ ID NO: 137, SEQ ID NO: 183, SEQ ID NO: 193, SEQ ID NO: 197, SEQ ID NO: 199 oder SEQ ID NO: 201 kodierten Protein. Die Homologie wurde über den gesamten Aminosäure- bzw. Nukleinsäuresequenzbereich berechnet. Für das Vergleichen verschiedener Sequenzen stehen dem Fachmann eine Reihe von Programmen, die auf verschiedenen Algorithmen beruhen zur Verfügung. Dabei liefern die Algorithmen von Needleman und Wunsch oder Smith und Waterman besonders zuverlässige Ergebnisse. Für die Sequenzvergleiche wurde das Programm PileUp verwendet (J. Mol. Evolution., 25, 351-360, 1987, Higgins et al., CABIOS, 5 1989: 151-153) oder die Programme Gap und BestFit [Needleman and Wunsch (J. Mol. Biol. 48; 443-453 (1970) und Smith and Waterman (Adv. Appl. Math. 2; 482-489 (1981)], die im GCG Software-Packet [Genetics Computer Group, 575 Science Drive, Madison, Wisconsin, USA 53711 (1991)] enthalten sind. Die oben in Prozent angegebenen Sequenzhomologiewerte wurden mit dem Programm GAP über den gesamten Sequenzbereich mit folgenden Einstellungen ermittelt: Gap Weight: 50, Length Weight: 3, Average Match: 10.000 und Average Mismatch: 0.000. Die falls nicht anders angegeben als Standardeinstellungen immer für Sequenzvergleiche verwendet wurden.

Homologen der vorgenannten Nukleinsäuresequenzen bedeuten beispielsweise auch bakterielle, Pilz- und Pflanzenhomologen, verkürzte Sequenzen, einzelsträngige DNA oder RNA der kodierenden und nicht-kodierenden DNA-Sequenz oder auch Derivate, wie beispielsweise Promotorvarianten. Die Promotoren stromaufwärts der angegebenen Nukleotidsequenzen können durch einen oder mehrere Nukleotidaustausche, durch Insertion(en) und/oder Deletion(en) modifiziert werden, ohne dass jedoch die Funktionalität oder Aktivität der Promotoren gestört wird. Es ist weiterhin möglich, dass die Aktivität der Promotoren durch Modifikation ihrer Sequenz erhöht ist oder dass sie vollständig durch aktivere Promotoren, sogar aus heterologen Organismen, ersetzt werden.

Die vorgenannten Nukleinsäuren und Proteinmoleküle mit Δ-12-Desaturase-, ω-3-Desaturase-, Δ-9-Elongase-, Δ-6-Desaturase-, Δ-8-Desaturase-, Δ-6-Elongase-, Δ-5-Desaturase-, Δ-5-Elongase- und/oder Δ-4-Desaturase-Aktivität, die am Stoffwechsel von Lipiden und Fettsäuren, PUFA-Cofaktoren und Enzymen oder am Transport lipophiler Verbindungen über Membranen beteiligt sind, werden im erfindungsgemäßen Verfahren zur Modulation der Produktion von PUFAs in transgenen Pflanzen, wie Mais, Weizen, Roggen, Hafer, Triticale, Reis, Gerste, Sojabohne, Erdnuss, Baumwolle, Linum Arten wie Öl- oder Faserlein, Brassica-Arten, wie Raps, Canola, Sareptasenf und Rübsen, Pfeffer, Sonnenblume, Borretsch, Nachtkerze und Tagetes, Solanacaen-Pflanzen, wie Kartoffel, Tabak, Aubergine und Tomate, Vicia-Arten, Erbse, Maniok, Alfalfa, Buschpflanzen (Kaffee, Kakao, Tee), Salix-Arten, Bäume (Ölpalme, Kokosnuss) und ausdauernden Gräsern und Futterfeldfrüchten, entweder direkt (z.B. wenn die Überexpression oder Optimierung eines Fettsäurebiosynthese-Proteins einen direkten Einfluss auf die Ausbeute, Produktion und/oder Effizienz der Produktion der Fettsäure aus modifizierten Organismen hat) verwendet und/oder können eine indirekt Auswirkung haben, die dennoch zu einer Steigerung der Ausbeute, Produktion und/oder Effizienz der Produktion der PUFAs oder einer Abnahme unerwünschter Verbindungen führt (z.B. wenn die Modulation des Stoffwechsels von Lipiden und Fettsäuren, Cofaktoren und Enzymen zu Veränderungen der Ausbeute, Produktion und/oder Effizienz der Produktion oder der Zusammensetzung der gewünschten Verbindungen innerhalb der Zellen führt, was wiederum die Produktion einer oder mehrerer Fettsäuren beeinflussen kann).

Besonders zur Herstellung von PUFAs, bevorzugt von Arachidonsäure, Eicosapentaensäure oder Docosahexaensäure, eignen sich Brassicaceen, Boraginaceen, Primulaceen, oder Linaceen. Besonders geeignet zur Herstellung von PUFAs mit den erfindungsgemäßen Nukleinsäuresequenzen, vorteilhaft, wie beschrieben, in Kombination mit weiteren Desaturasen und Elongasen, sind Sareptasenf (*Brassica juncea*), Raps und Camelina sativa.

Die Kombination verschiedener Vorläufermoleküle und Biosyntheseenzyme führt zur Herstellung verschiedener Fettsäuremoleküle, was eine entscheidende Auswirkung auf die Zusammensetzung der Lipide hat. Da mehrfach ungesättigte Fettsäuren (= PUFAs) nicht nur einfach in Triacylglycerin sondern auch in Membranlipide eingebaut werden.

Besonders zur Herstellung von PUFAs, beispielsweise Stearidonsäure , Eicosapentaensäure und Docosahexaensäure eignen sich Brasicaceae, Boraginaceen, Primulaceen, oder Linaceen. Besonders vorteilhaft eignet sich Lein (Linum usitatissimum), Brassica juncea und Camelina sativa zur Herstellung von PUFAS mit dem erfindungsgemäßen Nukleinsäuresequenzen vorteilhaft, wie beschrieben, in Kombination mit weiteren Desaturasen und Elongasen.

Die Lipidsynthese lässt sich in zwei Abschnitte unterteilen: die Synthese von Fettsäuren und ihre Bindung an sn-Glycerin-3-Phosphat sowie die Addition oder Modifikation einer polaren Kopfgruppe. Übliche Lipide, die in Membranen verwendet werden, umfassen Phospholipide, Glycolipide, Sphingolipide und Phosphoglyceride. Die Fettsäuresynthese beginnt mit der Umwandlung von Acetyl-CoA in Malonyl-CoA durch die Acetyl-CoA-Carboxylase oder in Acetyl-ACP durch die Acetyltransacylase. Nach einer Kondensationsreaktion bilden diese beiden Produktmoleküle zusammen Acetoacetyl-ACP, das über eine Reihe von Kondensations-, Reduktions- und Dehydratisierungsreaktionen umgewandelt wird, so dass ein gesättigtes Fettsäuremolekül mit der gewünschten Kettenlänge erhalten wird. Die Produktion der ungesättigten Fettsäuren aus diesen Molekülen wird durch spezifische Desaturasen katalysiert, und zwar entweder aerob mittels molekularem Sauerstoff oder anaerob (bezüglich der Fettsäuresynthese in Mikroorganismen siehe F.C. Neidhardt et al. (1996) E. coli und Salmonella. ASM Press: Washington, D.C., S. 612-636 und darin enthaltene Literaturstellen; Lengeler et al. (Hrsgb.) (1999) Biology of Procaryotes. Thieme: Stuttgart, New York, und die enthaltene Literaturstellen, sowie Magnuson, K., et al. (1993) Microbiological Reviews 57:522-542 und die enthaltenen Literaturstellen). Die so hergestellten an Phospholipide gebundenen Fettsäuren müssen anschließend wieder für die weitere Elongationen aus den Phospholipiden in den FettsäureCoA-Ester-Pool überführt werden. Dies ermöglichen Acyl-CoA:Lysophospholipid-Acyltransferasen. Weiterhin können diese Enzyme die elongierten Fettsäuren wieder von den CoA-Estern auf die Phospholipide übertragen. Diese Reaktionsabfolge kann gegebenenfalls mehrfach durchlaufen werden.

Vorläufer für die PUFA-Biosynthese sind beispielsweise Ölsäure, Linol- und Linolensäure. Diese $C_{18}$-Kohlenstoff-Fettsäuren müssen auf $C_{20}$ und $C_{22}$ verlängert werden, damit Fettsäuren vom Eicosa- und Docosa-Kettentyp erhalten werden. Mithilfe der im Verfahren verwendeten Desaturasen wie der Δ-12-, ω3-, Δ-4-, Δ-5-, Δ-6- und Δ-8-Desaturasen und/oder der Δ-5-, Δ-6-, Δ-9-Elongasen können Arachidonsäure, Eicosapentaensäure, Docosapentaensäure oder Docosahexaensäure vorteilhaft Eicosapentaensäure und/oder Docosahexaensäure hergestellt werden und anschließend für verschiedene Zwecke bei Nahrungsmittel-, Futter-, Kosmetik- oder pharmazeutischen Anwendungen verwendet werden. Mit den genannten Enzymen können $C_{20}$- und/oder $C_{22}$-Fettsäuren mit mindestens zwei vorteilhaft mindestens drei, vier, fünf oder sechs Doppelbindungen im Fettsäuremolekül, vorzugsweise $C_{20}$- oder $C_{22}$-Fettsäuren mit vorteilhaft vier, fünf oder sechs Doppelbindungen im Fettsäuremolekül hergestellt werden. Die Desaturierung kann vor oder nach Elongation der entsprechenden Fettsäure erfolgen. Daher führen die Produkte der Desaturaseaktivitäten und der möglichen weiteren Desaturierung und Elongation zu bevorzugten PUFAs mit höherem Desaturierungsgrad, einschließlich einer weiteren Elongation von $C_{20}$ zu $C_{22}$-Fettsäuren,zu Fettsäuren wie γ-Linolensäure, Dihomo-γ-linolensäure, Arachidonsäure, Stearidonsäure, Eicosatetraensäure oder Eicosapentaensäure. Substrate der verwendeten Desaturasen und Elongasen im erfindungsgemäßen Verfahren sind $C_{16}$-, $C_{18}$- oder $C_{20}$-Fettsäuren wie zum Beispiel Linolsäure, γ-Linolensäure, α-Linolensäure, Dihomo-γ-linolensäure, Eicosatetraensäure oder Stearidonsäure. Bevorzugte Substrate sind Linolsäure, γ-Linolensäure und/oder α-Linolensäure, Dihomo-γ-linolensäure bzw. Arachidonsäure, Eicosatetraensäure oder Eicosapentaensäure. Die synthetisierten $C_{20}$- oder $C_{22}$-Fettsäuren mit mindestens zwei, drei, vier, fünf oder sechs, vorteilhaft mit mindestens vier, fünf oder sechs Doppelbindungen in der Fettsäure fallen im erfindungsgemäßen Verfahren in Form der freien Fettsäure oder in Form ihrer Ester beispielsweise in Form ihrer Glyceride an.

Unter dem Begriff "Glycerid" wird ein mit ein, zwei oder drei Carbonsäureresten verestertes Glycerin verstanden (Mono-, Di- oder Triglycerid). Unter "Glycerid" wird auch ein Gemisch an verschiedenen Glyceriden verstanden. Das Glycerid oder das Glyceridgemisch kann weitere Zusätze, z.B. freie Fettsäuren, Antioxidantien, Proteine, Kohlenhydrate, Vitamine und/oder andere Substanzen enthalten.

Unter einem "Glycerid" im Sinne des erfindungsgemäßen Verfahrens werden ferner vom Glycerin abgeleitete Derivate verstanden. Dazu zählen neben den oben beschriebenen Fettsäureglyceriden auch Glycerophospholipide und Glyceroglycolipide. Bevorzugt seien hier die Glycerophospholipide wie Lecithin (Phosphatidylcholin), Cardiolipin, Phosphatidylglycerin, Phosphatidylserin und Alkylacylglycerophospholipide beispielhaft genannt.

Ferner müssen Fettsäuren anschließend an verschiedene Modifikationsorte transportiert und in das Triacylglycerin-Speicherlipid eingebaut werden. Ein weiterer wichtiger Schritt bei der Lipidsynthese ist der Transfer von Fettsäuren auf die polaren Kopfgruppen, beispielsweise durch Glycerin-Fettsäure-Acyltransferase (siehe Frentzen, 1998, Lipid, 100(4-5):161-166).

Veröffentlichungen über die Pflanzen-Fettsäurebiosynthese, Desaturierung, den Lipidstoffwechsel und Membrantransport von fetthaltigen Verbindungen, die Betaoxidation, Fettsäuremodifikation und Cofaktoren, Triacylglycerin-Speicherung und -Assemblierung einschließlich der Literaturstellen darin siehe in den folgenden Artikeln: Kinney, 1997, Genetic Engeneering, Hrsgb.: JK Setlow, 19:149-166; Ohlrogge und Browse, 1995, Plant Cell 7:957-970; Shanklin und Cahoon, 1998, Annu. Rev. Plant Physiol. Plant Mol. Biol. 49:611-641; Voelker, 1996, Genetic Engeneering, Hrsgb.: JK Setlow, 18:111-13; Gerhardt, 1992, Prog. Lipid R. 31:397-417; Gühnemann-Schäfer & Kindl, 1995, Biochim. Biophys

Acta 1256:181-186; Kunau et al., 1995, Prog. Lipid Res. 34:267-342; Stymne et al., 1993, in: Biochemistry and Molecular Biology of Membrane and Storage Lipids of Plants, Hrsgb.: Murata und Somerville, Rockville, American Society of Plant Physiologists, 150-158, Murphy & Ross 1998, Plant Journal. 13(1):1-16.

Die im Verfahren hergestellten PUFAs, umfassen eine Gruppe von Molekülen, die höhere Tiere nicht mehr synthetisieren können und somit aufnehmen müssen oder die höhere Tiere nicht mehr ausreichend selbst herstellen können und somit zusätzlich aufnehmen müssen, obwohl sie leicht von anderen Organismen, wie Bakterien, synthetisiert werden, beispielsweise können Katzen Arachidonsäure nicht mehr synthetisieren.

Unter Phospholipiden im Sinne der Erfindung sind zu verstehen Phosphatidylcholin, Phosphatidylethanolamin, Phosphatidylserin, Phosphatidylglycerin und/oder Phosphatidylinositol vorteilhafterweise Phosphatidylcholin.

Die Begriffe "Produktion" oder "Produktivität" sind im Fachgebiet bekannt und beinhalten die Konzentration des Fermentationsproduktes (Verbindungen der Formel I), das in einer bestimmten Zeitspanne und einem bestimmten Fermentationsvolumen gebildet wird (z.B. kg Produkt pro Stunde pro Liter). Sie umfassen auch die Produktivität innerhalb einer Pflanzenzelle oder einer Pflanze, das heißt den Gehalt an den gewünschten im Verfahren hergestellten Fettsäuren bezogen auf den Gehalt an allen Fettsäuren in dieser Zelle oder Pflanze. Der Begriff Effizienz der Produktion umfasst die Zeit, die zur Erzielung einer bestimmten Produktionsmenge nötig ist (z.B. wie lange die Zelle zur Aufrichtung einer bestimmten Durchsatzrate einer Feinchemikalie benötigt). Der Begriff "Ausbeute" oder "Produkt/Kohlenstoff-Ausbeute" ist im Fachgebiet bekannt und umfasst die Effizienz der Umwandlung der Kohlenstoffquelle in das Produkt (d.h. die Feinchemikalie). Dies wird gewöhnlich beispielsweise ausgedrückt als kg Produkt pro kg Kohlenstoffquelle. Durch Erhöhen der Ausbeute oder Produktion der Verbindung wird die Menge der gewonnenen Moleküle oder der geeigneten gewonnenen Moleküle dieser Verbindung in einer bestimmten Kulturmenge über einen festgelegten Zeitraum erhöht.

Die Begriffe "Biosynthese" oder "Biosyntheseweg" sind im Fachgebiet bekannt und umfassen die Synthese einer Verbindung, vorzugsweise einer organischen Verbindung, durch eine Zelle aus Zwischenverbindungen, beispielsweise in einem Mehrschritt- und stark regulierten Prozess.

Die Begriffe "Abbau" oder "Abbauweg" sind im Fachgebiet bekannt und umfassen die Spaltung einer Verbindung, vorzugsweise einer organischen Verbindung, durch eine Zelle in Abbauprodukte (allgemeiner gesagt, kleinere oder weniger komplexe Moleküle) beispielsweise in einem Mehrschritt- und stark regulierten Prozess.

Der Begriff "Stoffwechsel" ist im Fachgebiet bekannt und umfasst die Gesamtheit der biochemischen Reaktionen, die in einem Organismus stattfinden. Der Stoffwechsel einer bestimmten Verbindung (z.B. der Stoffwechsel einer Fettsäure) umfasst dann die Gesamtheit der Biosynthese-, Modifikations- und Abbauwege dieser Verbindung in der Zelle, die diese Verbindung betreffen.

Beispiele

Beispiel 1: Allgemeine Klonierungsverfahren:

Die Klonierungsverfahren wie z.B. Restriktionsspaltungen, Agarose-Gelelektrophorese, Reinigung von DNA-Fragmenten, Transfer von Nukleinsäuren auf Nitrozellulose und Nylon Membranen, Verknüpfen von DNA-Fragmenten, Transformation von Escherichia coli Zellen, Anzucht von Bakterien und die Sequenzanalyse rekombinanter DNA wurden wie bei Sambrook et al. (1989) (Cold Spring Harbor Laboratory Press: ISBN 0-87969-309-6) beschrieben durchgeführt.

Beispiel 2: Sequenzanalyse rekombinanter DNA:

Die Sequenzierung rekombinanter DNA-Moleküle erfolgte mit einem Laserfluoreszenz-DNA-Sequenzierer der Firma ABI nach der Methode von Sanger (Sanger et al. (1977) Proc. Natl. Acad. Sci. USA74, 5463-5467). Fragmente resultierend aus einer Polymerase Kettenreaktion wurden zur Vermeidung von Polymerasefehlern in zu exprimierenden Konstrukten sequenziert und überprüft.

Beispiel 3: Klonierung von Genen aus Oncorhynchus mykiss

Durch Suche nach konservierten Bereichen in den Proteinsequenzen entsprechend der in der Anmeldung aufgeführten Elongase-Gene wurden zwei Sequenzen mit entsprechenden Motiven in der Sequenzdatenbank von Genbank identifiziert.

| Gen-Name | Genbank No | Aminosäuren |
|---|---|---|
| OmELO2 | CA385234, CA364848, CA366480 | 264 |
| OmELO3 | CA360014, CA350786 | 295 |

Gesamt-RNA von Oncoryhnchus mykiss wurde mit Hilfe des RNAeasy Kits der Firma Qiagen (Valencia, CA, US) isoliert. Aus der Gesamt-RNA wurde mit Hilfe von oligo-dT-Cellulose poly-A+ RNA (mRNA) isoliert (Sambrook et al., 1989). Die RNA wurde mit dem Reverse Transcription System Kit von Promega revers transcribiert und die synthetisierte cDNA in den lambda ZAP Vektor (lambda ZAP Gold, Stratagene) kloniert. Entsprechend Herstellerangaben wurde die cDNA zur Plasmid-DNA entpackt. Die cDNA-Plasmid-Bank wurde dann für die PCR zur Klonierung von Expressionsplasmiden verwendet.

Beispiel 4: Klonierung von Expressionsplasmiden zur heterologen Expression in Hefen

Für die Klonierung der zwei Sequenzen zur heterologen Expression in Hefen wurden folgende Oligonukleotide für die PCR-Reaktion verwendet:

| Primer | Nukleotidsequenz |
|---|---|
| 5' f* OmELO2 | 5' aagcttacataatggcttcaacatggcaa (SEQ ID NO: 179) |
| 3' r* OmELO2 | 5' ggatccttatgtcttcttgctcttcctgtt (SEQ ID NO: 180) |
| 5' f OmELO3 | 5' aagcttacataatggagacttttaat (SEQ ID NO: 181) |
| 3' r OmELO3 | 5' ggatccttcagtccccctcactttcc (SEQ ID NO: 182) |
| * f: forward, r: reverse | |

Zusammensetzung des PCR-Ansatzes (50 µL):

5,00 µL Template cDNA
5,00 µL 10x Puffer (Advantage-Polymerase)+ 25mM MgCl2
5,00 µL 2mM dNTP
1,25 µL je Primer (10 pmol/µL)
0,50 µL Advantage-Polymerase
Die Advantage-Polymerase von Clontech wurden eingesetzt.

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR Produkt wurde für 2 h bei 37 °C mit den Restriktionsenzymen HindIII und BamHI inkubiert. Der Hefe-Expressionsvektor pYES3 (Invitrogen) wurde in gleicherweise inkubiert. Anschliessend wurde das 812 bp bzw. 905 bp große PCR Produkt sowie der Vektor durch Agarose-Gelelektrophorese aufgetrennt und die entsprechenden DNA-Fragmente ausgeschnitten. Die Aufreinigung der DNA erfolge mittels Qiagen Gel purification Kit gemäss Herstellerangaben. Anschliessend wurden Vektor und Elongase cDNA ligiert. Dazu wurde das Rapid Ligation Kit von Roche verwendet.

Die entstandenen Plasmide pYES3-OmELO2 und pYES3-OmELO3 wurden durch Sequenzierung verifiziert und in den Saccharomyces Stamm INVSc1 (Invitrogen) durch Elektroporation (1500 V) transformiert. Zur Kontrolle wurde pYES3 parallel transformiert. Anschliessend wurden die Hefen auf Komplett-Minimalmedium ohne Tryptophan mit 2 % Glucose ausplattiert. Zellen, die auf ohne Tryptophan im Medium wachstumsfähig waren, enthalten damit die entsprechenden Plasmide pYES3, pYES3-OmELO2 (SEQ ID NO: 51) und pYES3-OmELO3 (SEQ ID NO: 53). Nach der Selektion wurden je zwei Transformaten zur weiteren funktionellen Expression ausgewählt.

Beispiel 5: Klonierung von Expressionsplasmiden zur Samen-spezifischen Expression in Pflanzen

Für die Transformation von Pflanzen wurde ein weiterer Transformationsvektor auf Basis von pSUN-USP erzeugt. Dazu wurde mit folgendem Primerpaar NotI-Schnittstellen am 5' und 3'-Ende des kodierenden Sequenz eingefügt:

PSUN-OmELO2

Forward: 5'-GCGGCCGCATAATGGCTTCAACATGGCAA (SEQ ID NO: 175)
Reverse: 3'-GCGGCCGCTTATGTCTTCTTGCTCTTCCTGTT (SEQ ID NO: 176)

PSUN-OMELO3

Forward: 5'-GCGGCCGCataatggagacttttaat (SEQ ID NO: 177)
Reverse: 3'-GCGGCCGCtcagtcccccctcactttcc (SEQ ID NO: 178)

Zusammensetzung des PCR-Ansatzes (50 μL):

5,00 μL Template cDNA
5,00 μL 10x Puffer (Advantage-Polymerase)+ 25mM MgCl2
5,00 μL 2mM dNTP
1,25 μL je Primer (10 pmol/μL)
0,50 μL Advantage-Polymerase
Die Advantage-Polymerase von Clontech wurden eingesetzt.

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Die PCR Produkte wurden für 16 h bei 37 °C mit dem Restriktionsenzym NotI inkubiert. Der Pflanzen-Expressionsvektor pSUN300-USP wurde in gleicherweise inkubiert. Anschliessend wurde die PCR Produkte sowie der 7624 bp große Vektor durch Agarose-Gelelektrophorese aufgetrennt und die entsprechenden DNA-Fragmente ausgeschnitten. Die Aufreinigung der DNA erfolge mittels Qiagen Gel purification Kit gemäss Herstellerangaben. Anschliessend wurden Vektor und PCR-Produkte ligiert. Dazu wurde das Rapid Ligation Kit von Roche verwendet. Die entstandenen Plasmide pSUN-OmELO2 und pSUN-OmELO3 wurde durch Sequenzierung verifiziert.
pSUN300 ist ein Derivat des Plasmides pPZP (Hajdukiewicz,P, Svab, Z, Maliga, P., (1994) The small versatile pPZP family of Agrobacterium binary vectors forplant transformation. Plant Mol Biol 25:989-994). pSUN-USP entstand aus pSUN300, indem in pSUN300 ein USP-Promotor als EcoRI- Fragment inseriert wurde. Das Polyadenylierungssignal ist das des Octopinsynthase-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence and transcript map of the Agrobacterium tumefaciens Ti plasmid-encoded octopine synthase gene J. Mol. Appl. Genet. 1 (6), 499-511 (1982) Der USP-Promotor entspricht den Nukleotiden 1-684 (Genbank Accession X56240), wobei ein Teil der nichtcodierenden Region des USP-Gens im Promotor enthalten ist. Das 684 Basenpaar große Promotorfragment wurde mittels käuflichen T7-Standard-primer (Stratagene) und mit Hilfe eines synthetisierten Primers über eine PCR-Reaktion nach Standardmethoden amplifiziert. (Primersequenz: 5'-GTCGACCCGCGGACTAGTGGGCCCTCTAGACCCGGGGGATCC GGATCTGCTGGCTATGAA-3', SEQ ID NO: 174). Das PCR-Fragment wurde mit EcoRI/SalI nachgeschnitten und in den Vektor pSUN300 mit OCS Terminator eingesetzt. Es entstand das Plasmid mit der Bezeichnung pSUN-USP.Das Konstrukt wurde zur Transformation von Arabidopsis thaliana, Raps, Tabak und Leinsamen verwendet.

Beispiel 6: Lipidextraktion aus Hefen und Samen:

Die Auswirkung der genetischen Modifikation in Pflanzen, Pilzen, Algen, Ciliaten oder auf die Produktion einer gewünschten Verbindung (wie einer Fettsäure) kann bestimmt werden, indem die modifizierten Mikroorganismen oder die modifizierte Pflanze unter geeigneten Bedingungen (wie den vorstehend beschriebenen) gezüchtet werden und das Medium und/oder die zellulären Komponenten auf die erhöhte Produktion des gewünschten Produktes (d.h. von Lipiden oder einer Fettsäure) untersucht wird. Diese Analysetechniken sind dem Fachmann bekannt und umfassen Spektroskopie, Dünnschichtchromatographie, Färbeverfahren verschiedener Art, enzymatische und mikrobiologische Verfahren sowie analytische Chromatographie, wie Hochleistungs-Flüssigkeitschromatographie (siehe beispielsweise Ullman, Encyclopedia of Industrial Chemistry, Bd. A2, S. 89-90 und S. 443-613, VCH: Weinheim (1985); Fallon, A., et al., (1987) "Applications of HPLC in Biochemistry" in: Laboratory Techniques in Biochemistry and Molecular Biology, Bd. 17; Rehm et al. (1993) Biotechnology, Bd. 3, Kapitel III: "Product recovery and purification", S. 469-714, VCH: Weinheim; Belter, P.A., et al. (1988) Bioseparations: downstream processing for Biotechnology, John Wiley and Sons; Kennedy, J.F., und Cabral, J.M.S. (1992) Recovery processes for biological Materials, John Wiley and Sons; Shaeiwitz, J.A., und Henry,

J.D. (1988) Biochemical Separations, in: Ullmann's Encyclopedia of Industrial Chemistry, Bd. B3; Kapitel 11, S. 1-27, VCH: Weinheim; und Dechow, F.J. (1989) Separation and purification techniques in biotechnology, Noyes Publications).

Neben den oben erwähnten Verfahren werden Pflanzenlipide aus Pflanzenmaterial wie von Cahoon et al. (1999) Proc. Natl. Acad. Sci. USA 96 (22):12935-12940, und Browse et al. (1986) Analytic Biochemistry 152:141-145, beschrieben extrahiert. Die qualitative und quantitative Lipid- oder Fettsäureanalyse ist beschrieben bei Christie, William W., Advances in Lipid Methodology, Ayr/Scotland: Oily Press (Oily Press Lipid Library; 2); Christie, William W., Gas Chromatography and Lipids. A Practical Guide - Ayr, Scotland: Oily Press, 1989, Repr. 1992, IX, 307 S. (Oily Press Lipid Library; 1); "Progress in Lipid Research, Oxford: Pergamon Press, 1 (1952) - 16 (1977) u.d.T.: Progress in the Chemistry of Fats and Other Lipids CODEN.

Zusätzlich zur Messung des Endproduktes der Fermentation ist es auch möglich, andere Komponenten der Stoffwechselwege zu analysieren, die zur Produktion der gewünschten Verbindung verwendet werden, wie Zwischen- und Nebenprodukte, um die Gesamteffizienz der Produktion der Verbindung zu bestimmen. Die Analyseverfahren umfassen Messungen der Nährstoffmengen im Medium (z.B. Zucker, Kohlenwasserstoffe, Stickstoffquellen, Phosphat und andere Ionen), Messungen der Biomassezusammensetzung und des Wachstums, Analyse der Produktion üblicher Metabolite von Biosynthesewegen und Messungen von Gasen, die während der Fermentation erzeugt werden. Standardverfahren für diese Messungen sind in Applied Microbial Physiology; A Practical Approach, P.M. Rhodes und P.F. Stanbury, Hrsgb., IRL Press, S. 103-129; 131-163 und 165-192 (ISBN: 0199635773) und darin angegebenen Literaturstellen beschrieben.

Ein Beispiel ist die Analyse von Fettsäuren (Abkürzungen: FAME, Fettsäuremethylester; GC-MS, Gas-Flüssigkeitschromatographie-Massenspektrometrie; TAG, Triacylglycerin; TLC, Dünnschichtchromatographie).

Der unzweideutige Nachweis für das Vorliegen von Fettsäureprodukten kann mittels Analyse rekombinanter Organismen nach Standard-Analyseverfahren erhalten werden: GC, GC-MS oder TLC, wie verschiedentlich beschrieben von Christie und den Literaturstellen darin (1997, in: Advances on Lipid Methodology, Vierte Aufl.: Christie, Oily Press, Dundee, 119-169; 1998, Gaschromatographie-Massenspektrometrie-Verfahren, Lipide 33:343-353).

Das zu analysierende Material kann durch Ultraschallbehandlung, Mahlen in der Glasmühle, flüssigen Stickstoff und Mahlen oder über andere anwendbare Verfahren aufgebrochen werden. Das Material muss nach dem Aufbrechen zentrifugiert werden. Das Sediment wird in Aqua dest. resuspendiert, 10 min bei 100°C erhitzt, auf Eis abgekühlt und erneut zentrifugiert, gefolgt von Extraktion in 0,5 M Schwefelsäure in Methanol mit 2 % Dimethoxypropan für 1 Std. bei 90°C, was zu hydrolysierten Öl- und Lipidverbindungen führt, die transmethylierte Lipide ergeben. Diese Fettsäuremethylester werden in Petrolether extrahiert und schließlich einer GC-Analyse unter Verwendung einer Kapillarsäule (Chrompack, WCOT Fused Silica, CP-Wax-52 CB, 25 mikrom, 0,32 mm) bei einem Temperaturgradienten zwischen 170°C und 240°C für 20 min und 5 min bei 240°C unterworfen. Die Identität der erhaltenen Fettsäuremethylester muss unter Verwendung von Standards, die aus kommerziellen Quellen erhältlich sind (d.h. Sigma), definiert werden.

Pflanzenmaterial wird zunächst mechanisch durch Mörsern homogenisiert, um es einer Extraktion zugänglicher zu machen.

Dann wird 10 min auf 100°C erhitzt und nach dem Abkühlen auf Eis erneut sedimentiert. Das Zellsediment wird mit 1 M methanolischer Schwefelsäure und 2 % Dimethoxypropan 1h bei 90°C hydrolysiert und die Lipide transmethyliert. Die resultierenden Fettsäuremethylester (FAME) werden in Petrolether extrahiert. Die extrahierten FAME werden durch Gasflüssigkeitschromatographie mit einer Kapillarsäule (Chrompack, WCOT Fused Silica, CP-Wax-52 CB, 25 m, 0,32 mm) und einem Temperaturgradienten von 170°C auf 240°C in 20 min und 5 min bei 240°C analysiert. Die Identität der Fettsäuremethylester wird durch Vergleich mit entsprechenden FAME-Standards (Sigma) bestätigt. Die Identität und die Position der Doppelbindung kann durch geeignete chemische Derivatisierung der FAME-Gemische z.B. zu 4,4-Dimethoxyoxazolin-Derivaten (Christie, 1998) mittels GC-MS weiter analysiert werden.

Hefen, die wie unter Beispiel 4 mit den Plasmiden pYES3, pYES3-OmELO2 und pYES3-OmELO3 transformiert wurden, wurden folgendermaßen analysiert:

Die Hefezellen aus den Hauptkulturen wurden durch Zentrifugation (100 x g, 10 min, 20°C) geerntet und mit 100 mM $NaHCO_3$, pH 8,0 gewaschen, um restliches Medium und Fettsäuren zu entfernen. Aus den Hefe-Zellsedimenten wurden Fettsäuremethylester (FAMEs) durch saure Methanolyse hergestellt. Hierzu wurden die Zellsedimente mit 2 ml 1N methonolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM $NaHCO_3$, pH 8,0 und 2 ml Aqua dest. gewaschen. Anschließend wurden die PE-Phasen mit $Na_2SO_4$ getrocknet, unter Argon eingedampft und in 100 $\mu$l PE aufgenommen. Die Proben wurden auf einer DB-23-Kapillarsäule (30 m, 0,25 mm, 0,25 $\mu$m, Agilent) in einem Hewlett-Packard 6850-Gaschromatographen mit Flammenionisationsdetektor getrennt. Die Bedingungen für die GLC-Analyse waren wie folgt: Die Ofentemperatur wurde von 50°C bis 250°C mit einer Rate von 5°C/min und schließlich 10 min bei 250°C(halten) programmiert.

Die Identifikation der Signale erfolgte durch Vergleiche der Retentionszeiten mit entsprechenden Fettsäurestandards (Sigma).

Die Methodik ist beschrieben zum Beispiel in Napier and Michaelson, 2001,Lipids. 36(8):761-766; Sayanova

et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

Beispiel 7: Funktionelle Charakterisierung von OmELO2 und OmELO3:

OmELO2 zeigt keine Elongase-Aktivität, während für OmELO3 eine deutliche Aktivität mit verschiedenen Substraten nachgewiesen werden konnte. Die Substratspezifität der OmElo3 konnte nach Expression und Fütterung verschiedener Fettsäuren ermittelt werden (Figur 2). Die gefütterten Substrate sind in großen Mengen in allen transgenen Hefen nachzuweisen. Alle transgene Hefen zeigen die Synthese neuer Fettsäuren, den Produkten der OmElo3-Reaktion. Dies bedeutet, dass das Gen OmElo3 funktional exprimiert werden konnte.

Figur 2 zeigt, dass die OmElo3 eine Substratspezifität aufweist, die mit hoher Spezifität zur Verlängerung von Δ5- und Δ6-Fettsäuren mit einer ω3-Doppelbindung führt. Es konnte in geringerer Spezifität des weiteren auch ω6-Fettsäuren (C18 und C20) elongiert werden. Stearidonsäure (C18:4 ω3) und Eicosapentaensäure (C20:5 ω3) stellen die besten Substrate für die OmElo3 dar (bis zu 66 % Elongation).

Beispiel 8: Rekonstitution der Synthese von DHA in Hefe

Die Rekonstitution der Biosynthese von DHA (22:6 ω3) wurde ausgehend von EPA (20:5 ω3) bzw. Stearidonsäure (18:4 ω3) durch die Coexpression der OmElo3 mit der Δ-4-Desaturase aus *Euglena gracilis* bzw. der Δ-5-Desaturase aus *Phaeodactylum tricornutum* und der Δ-4-Desaturase aus *Euglena gracilis* durchgeführt. Dazu wurden weiterhin die Expressionsvektoren pYes2-EgD4 und pESCLeu-PtD5 konstruiert. Der o.g. Hefestamm, der bereits mit dem pYes3-OmElo3 (SEQ ID NO: 55) transformiert ist, wurde weiter mit dem pYes2-EgD4 bzw. gleichzeitig mit pYes2-EgD4 und pESCLeu-PtD5 transformiert. Die Selektion der transformierten Hefen erfolgte auf Komplett-Minimalmedium-Agarplatten mit 2% Glucose, aber ohne Tryptophan und Uracil im Falle des pYes3-OmELO/pYes2-EgD4-Stammes und ohne Tryptophan, Uracil und Leucin im Falle des pYes3-OmELO/pYes2-EgD4+pESCLeu-PtD5-Stammes. Die Expression wurde wie oben angegeben durch die Zugabe von 2% (w/v) Galactose induziert. Die Kulturen wurden für weitere 120 h bei 15°C inkubiert.

Figur 3 zeigt die Fettsäureprofile von transgenen Hefen, die mit 20:5 ω3 gefüttert wurden. In der Kontroll-Hefe (A), die mit dem pYes3-OmElo3-Vektor und dem leeren Vektor pYes2 transformiert wurden, wurde 20:5 ω3 sehr effizient zu 22:5 ω3 elongiert (65% Elongation). Die zusätzliche Einführung der EgΔ-4-Desaturase führte zu der Umsetzung von 22:5 ω3 zu 22:6 ω3 (DHA). Die Fettsäure-Zusammensetzung der transgenen Hefen ist in Figur 5 wiedergegeben. Nach der Co-Expression von OmElo3 und EgD4 konnte bis zu 3% DHA in Hefen nachgewiesen werden.

In einem weiteren Co-Expressionsexperiment wurden OmElo3, EgD4 und eine□ Δ5-Desaturase aus P. *tricornutum* (PtD5) zusammen exprimiert. Die transgenen Hefen wurden mit Stearidonsäure (18:4 ω3) gefüttert und analysiert (Figur 4). Die Fettsäure-Zusammensetzung dieser Hefen ist in Figur 5 aufgeführt. Durch OmElo3 wurde die gefütterte Fettsäure 18:4 ω3 zu 20:4 ω3 elongiert (60% Elongation). Letztere wurde durch die PtD5 zu 20:5 ω3 desaturiert. Die Aktivität der PtD5 betrug 15%. 20:5 ω3 konnte weiterhin durch die OmElo3 zu 22:5 ω3 elongiert werden. Im Anschluß wurde die neu synthetisierte 22:5 ω3 zu 22:6 ω3 (DHA) desaturiert. In diesen Experimenten konnte bis zu 0,7% DHA erzielt werden.

Aus diesen Experimenten geht hervor, dass die in dieser Erfindung verwendeten Sequenzen OmElo3, EgD4 und PtD5 für die Produktion von DHA in eukaryotischen Zellen geeignet sind.

Beispiel 9: Erzeugung von transgenen Pflanzen

a) Erzeugung transgener Rapspflanzen (verändert nach Moloney et al., 1992, Plant Cell Reports, 8:238-242)

Zur Erzeugung transgener Rapspflanzen können binäre Vektoren in Agrobacterium tumefaciens C58C1:pGV2260 oder Escherichia coli genutzt (Deblaere et al, 1984, Nucl. Acids. Res. 13, 4777-4788). Zur Transformation von Rapspflanzen (Var. Drakkar, NPZ Nordeutsche Pflanzenzucht, Hohenlieth, Deutschland), wird eine 1:50 Verdünnung einer Übernachtkultur einer positiv transformierten Agrobakterienkolonie in Murashige-Skoog Medium (Murashige und Skoog 1962 Physiol. Plant. 15, 473) mit 3 % Saccharose (3MS-Medium) benutzt. Petiolen oder Hypokotyledonen frisch gekeimter steriler Rapspflanzen (zu je ca. 1 $cm^2$) werden in einer Petrischale mit einer 1:50 Agrobakterienverdünnung für 5-10 Minuten inkubiert. Es folgt eine 3-tägige Colnkubation in Dunkelheit bei 25°C auf 3MS-Medium mit 0,8 % Bacto-Agar. Die Kultivierung wird nach 3 Tagen mit 16 Stunden Licht / 8 Stunden Dunkelheit weitergeführt und in wöchentlichem Rhythmus auf MS-Medium mit 500 mg/l Claforan (Cefotaxime-Natrium), 50 mg/l Kanamycin, 20 mikroM Benzylaminopurin (BAP) und 1,6 g/l Glukose weitergeführt. Wachsende Sprosse werden auf MS-Medium mit 2 % Saccharose, 250 mg/l Claforan und 0,8 % Bacto-Agar überführt. Bilden sich nach drei Wochen keine Wurzeln, so wurde als Wachstumshormon 2-Indolbuttersäure zum Bewurzeln zum Medium gegeben.

Regenerierte Sprosse werden auf 2MS-Medium mit Kanamycin und Claforan erhalten, nach Bewurzelung in Erde überführt und nach Kultivierung für zwei Wochen in einer Klimakammer oder im Gewächshaus angezogen, zur Blüte gebracht, reife Samen geerntet und auf Elongase-Expression wie Δ-5-Elongase- oder Δ-6-Elongaseaktivität oder ω-3-Desaturaseaktivität mittels Lipidanalysen untersucht. Linien mit erhöhten Gehalten an C20- und C22 mehrfachungesättigten Fettsäuren können so identifiziert werden.

b) Herstellung von transgenen Leinpflanzen

Die Herstellung von transgenen Leinpflanzen können zum Beispiel nach der Methode von Bell et al., 1999, In Vitro Cell. Dev. Biol.-Plant. 35(6):456-465 mittels particle bombartment erzeugt werden. In der Regel wurde eine Agrobakterien-vermittelte Transformation zum Beispiel nach Mlynarova et al. (1994), Plant Cell Report 13: 282-285 zur Leintransformation verwendet.

Beispiel 10: Klonierung von Δ5-Elongase-Genen aus Thraustochytrium aureum ATCC34304 und Thraustochytrium ssp.

Durch Vergleiche der verschiedenen in dieser Anmeldung gefundenen Elongase-Proteinsequenzen konnten konservierte Nukleinsäurebereiche definiert werden (Histidin-Box: His-Val-X-His-His, Tyrosin-Box: Met-Tyr-X-Tyr-Tyr). Mit Hilfe dieser Sequenzen wurde eine EST-Datenbank von T. aureum ATCC34304 und Thraustochytrium ssp. nach weiteren Δ-5-Elongasen durchsucht. Folgende neue Sequenzen konnten gefunden werden:

| Gen-Name | Nukleotide | Aminosäuren |
|---|---|---|
| BioTaurELO1 | 828 bp | 275 |
| TL16y2 | 831 | 276 |

Gesamt-RNA von T. aureum ATCC34304 und Thraustochytrium ssp. wurde mit Hilfe des RNAeasy Kits der Firma Qiagen (Valencia, CA, US) isoliert. Aus der Gesamt-RNA wurde mit Hilfe des PolyATract Isolierungssystems (Promega) mRNA isoliert. Die mRNA wurde mit dem Marathon cDNA Amplification-Kit (BD Biosciences) reverse transkribiert und entsprechend der Herstellerangaben Adaptoren ligiert. Die cDNA-Bank wurde dann für die PCR zur Klonierung von Expressionsplasmiden mittels 5'- und 3'-RACE (rapid amplification of cDNA ends) verwendet.

Beispiel 11: Klonierung von Expressionsplasmiden zur heterologen Expression in Hefen

Für die Klonierung der Sequenz zur heterologen Expression in Hefen wurden folgende Oligonukleotide für die PCR-Reaktion verwendet:

| Primer | Nukleotidsequenz |
|---|---|
| 5' f* BioTaurELO1 | 5' gacataatgacgagcaacatgag (SEQ ID NO: 170) |
| 3' r* BioTaurELO1 | 5' cggcttaggccgacttggccttggg (SEQ ID NO: 171) |
| 5'f*TL16y2 | 5' agacataatggacgtcgtcgagcagcaatg (SEQ ID NO: 172) |
| 3'r*TL16y2 | 5' ttagatggtcttctgcttcttgggcgcc (SEQ ID NO: 173) |

* f: forward, r: reverse

Zusammensetzung des PCR-Ansatzes (50 μL):

5,00 μL Template cDNA
5,00 μL 10x Puffer (Advantage-Polymerase)+ 25mM MgCl2
5,00 μL 2mM dNTP
1,25 μL je Primer (10 pmol/μL)
0,50 μL pfu-Polymerase

Die Advantage-Polymerase von Clontech wurde eingesetzt.
Reaktionsbedingungen der PCR:

Anlagerungstemperatur: 1 min 55°C

Denaturierungstemperatur: 1 min 94°C
Elongationstemperatur: 2 min 72°C
Anzahl der Zyklen: 35

Die PCR Produkte BioTaurELO1 (siehe SEQ ID NO: 65) und TL16y2 (siehe SEQ ID NO: 83) wurde für 30 min bei 21 °C mit dem Hefe-Expressionsvektor pYES2.1-TOPO (Invitrogen) inkubiert gemäss Herstellerangaben. Das PCR-Produkt wird dabei durch einen T-Überhang und Aktivität einer Topoisomerase (Invitrogen) in den Vektor ligiert. Nach der Inkubation erfolgte dann die Transformation von E. coli DH5a Zellen. Entsprechende Klone wurden durch PCR identifiziert, die Plasmid-DNA mittels Qiagen DNAeasy-Kit isoliert und durch Sequenzierung verifiziert. Die korrekte Sequenz wurde dann in den Saccharomyces Stamm INVSc1 (Invitrogen) durch Elektroporation (1500 V) transformiert. Zur Kontrolle wurde der leere Vektor pYES2.1 parallel transformiert. Anschließend wurden die Hefen auf Komplett-Minimalmedium ohne Uracil mit 2 % Glucose ausplattiert. Zellen, die ohne Uracil im Medium wachstumsfähig waren, enthalten damit die entsprechenden Plasmide pYES2.1, pYES2.1-BioTaurELO1 und pYES2.1-TL16y2. Nach der Selektion wurden je zwei Transformaten zur weiteren funktionellen Expression ausgewählt.

Beispiel 12: Klonierung von Expressionsplasmiden zur Samen-spezifischen Expression in Pflanzen

Für die Transformation von Pflanzen wurde ein weiterer Transformationsvektor auf Basis von pSUN-USP erzeugt. Dazu wurde mit folgendem Primerpaar NotI-Schnittstellen am 5' und 3'-Ende des kodierenden Sequenz eingefügt:

PSUN-BioTaurEL01

Forward: 5'-GCGGCCGCATAATGACGAGCAACATGAGC (SEQ ID NO: 166)
Reverse: 3'-GCGGCCGCTTAGGCCGACTTGGCCTTGGG (SEQ ID NO: 167)

PSUN-TL16y2

Forward: 5'-GCGGCCGCACCATGGACGTCGTCGAGCAGCAATG (SEQ ID NO: 168)
Reverse: 5'-GCGGCCGCTTAGATGGTCTTCTGCTTCTTGGGCGCC (SEQ ID NO: 169)

Zusammensetzung des PCR-Ansatzes (50 µL):

5,00 µL Template cDNA
5,00 µL 10x Puffer (Advantage-Polymerase)+ 25mM MgCl2
5,00 µL 2mM dNTP
1,25 µL je Primer (10 pmol/µL)
0,50 µL Advantage-Polymerase
Die Advantage-Polymerase von Clontech wurden eingesetzt.

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Die PCR Produkte wurden für 16 h bei 37 °C mit dem Restriktionsenzym NotI inkubiert. Der Pflanzen-Expressionsvektor pSUN300-USP wurde in gleicherweise inkubiert. Anschliessend wurde die PCR Produkte sowie der 7624 bp große Vektor durch Agarose-Gelelektrophorese aufgetrennt und die entsprechenden DNA-Fragmente ausgeschnitten. Die Aufreinigung der DNA erfolge mittels Qiagen Gel Purification Kit gemäss Herstellerangaben. Anschließend wurden Vektor und PCR-Produkte ligiert. Dazu wurde das Rapid Ligation Kit von Roche verwendet. Die entstandenen Plasmide pSUN-BioTaurEL01 und pSUN-TL16y2 wurden durch Sequenzierung verifiziert.
pSUN300 ist ein Derivat des Plasmides pPZP (Hajdukiewicz,P, Svab, Z, Maliga, P., (1994) The small versatile pPZP family of Agrobacterium binary vectors forplant transformation. Plant Mol Biol 25:989-994). pSUN-USP entstand aus pSUN300, indem in pSUN300 ein USP-Promotor als EcoRI- Fragment inseriert wurde. Das Polyadenylierungssignal ist das des Octopinsynthase-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence and transcript map

of the Agrobacterium tumefaciens Ti plasmid-encoded octopine synthase gene J. Mol. Appl. Genet. 1 (6), 499-511 (1982) Der USP-Promotor entspricht den Nukleotiden 1-684 (Genbank Accession X56240), wobei ein Teil der nichtcodierenden Region des USP-Gens im Promotor enthalten ist. Das 684 Basenpaar große Promotorfragment wurde mittels käuflichen T7-Standard-primer (Stratagene) und mit Hilfe eines synthetisierten Primers über eine PCR-Reaktion nach Standardmethoden amplifiziert. (Primersequenz: 5'-GTCGACCCGCGGACTAGTGGGCCCTCTAGACCCGGGGGATCC GGATCTGCTGGCTATGAA-3', SEQ ID NO: 165). Das PCR-Fragment wurde mit EcoRI/SalI nachgeschnitten und in den Vektor pSUN300 mit OCS Terminator eingesetzt. Es entstand das Plasmid mit der Bezeichnung pSUN-USP.Das Konstrukt wurde zur Transformation von Arabidopsis thaliana, Raps, Tabak und Leinsamen verwendet.

Die Lipidextraktion aus Hefen und Samen erfolgte identisch zu Beispiel 6.

Beispiel 13: Funktionelle Charakterisierung von BioTaurELO1 und TL16y2:

Die Substratspezifität der BioTaurELO1 konnte nach Expression und Fütterung verschiedener Fettsäuren ermittelt werden (Figur 6). Figur 6 zeigt die Fütterungsexperimente zur Bestimmung der Funktionalität und Substratspezifität mit Hefestämmen, die entweder den Vektor pYes2.1 (Kontrolle = Control) oder den Vektor pYes2.1-BioTaurELO1 (= BioTaur) mit der Δ-5-Elongase enthalten. In beiden Ansätzen wurde 200 uM γ-Linolensäure und Eicosapentaensäure dem Hefeinkubationsmedium zugesetzt und 24 h inkubiert. Nach Extraktion der Fettsäuren aus den Hefen wurden diese transmethyliert und gaschromatographisch aufgetrennt. Die aus den beiden gefütterten Fettsäuren entstandenen Elongationsprodukte sind durch Pfeile markiert.

Die gefütterten Substrate sind in großen Mengen in allen transgenen Hefen nachzuweisen. Alle transgene Hefen zeigen die Synthese neuer Fettsäuren, den Produkten der BioTaurELO1-Reaktion. Dies bedeutet, dass das Gen BioTaurELO1 funktional exprimiert werden konnte.

Figur 6 zeigt, dass die BioTaurELO1 eine Substratspezifität aufweist, die mit hoher Spezifität zur Verlängerung von Δ5- und Δ6-Fettsäuren mit einer ω3-Doppelbindung führt. Des weiteren konnten auch ω6-Fettsäuren (C18 und C20) elongiert werden.

Es werden γ-Linolensäure (C18:3 ω6) mit 65,28 %, Stearidonsäure (C18:4 ω3) mit 65.66 % und Eicosapentaensäure (C20:5 ω3) mit 22,01 % Konversion umgesetzt.

Die Substratspezifitäten der verschiedenen Fütterungsexperimente sind in Tabelle 6 dargestellt (siehe am Ende der Beschreibung).

Die Konversionsrate von GLA bei Fütterung von GLA und EPA betrug 65,28 %. Die Konversionsrate von EPA bei gleicher Fütterung von GLA und EPA betrug 9,99 %. Wurde nur EPA gefüttert, so betrug die Konversionsrate von EPA 22,01 %. Auch Arachidonsäure (= ARA) wurde bei Fütterung umgesetzt. Die Konversionsrate betrug 14,47 %. Auch Stearidonsäure (= SDA) wurde umgesetzt. In diesem Fall betrug die Konversionsrate 65,66 %.

Die Funktionalität und Substratspezifität von TL16y2 konnte nach Expression und Fütterung verschiedener Fettsäuren ermittelt werden. Tabelle 7 zeigt die Fütterungsexperimente. Die Fütterungsversuche wurden in gleicherweise durchgeführt wie für BioTaurELO1 beschrieben. Die gefütterten Substrate sind in großen Mengen in allen transgenen Hefen nachzuweisen. Die transgenen Hefen zeigten die Synthese neuer Fettsäuren, den Produkten der TL16y2-Reaktion (Fig. 11). Dies bedeutet, dass das Gen TL16y2 funktional exprimiert werden konnte.

Tabelle 7: Expression von TL16y2 in Hefe.

| Flächen der gaschromatographischen Analyse in % | | | | | | | | | |
|---|---|---|---|---|---|---|---|---|---|
| Plasmid | Fettsäure | C18:3 (n-6) | C18:4 (n-3) | C20:3 (n-6) | C20:4 (n-6) | C20:4 (n-3) | C20:5 (n-3) | C22:4 (n-6) | C22:5 (n-3) |
| pYES | 250 uM EPA | | | | | | 13,79 | | |
| TL16y2 | 250 uM EPA | | | | | | 25,81 | | **2,25** |
| pYES | 50 uM EPA | | | | | | 5,07 | | |
| TL16y2 | 50 uM EPA | | | | | | 2,48 | | **1,73** |
| pYES | 250 uMGLA | 8,31 | | | | | | | |

(fortgesetzt)

| Flächen der gaschromatographischen Analyse in % | | | | | | | | | |
|---|---|---|---|---|---|---|---|---|---|
| Plasmid | Fettsäure | C18:3 (n-6) | C18:4 (n-3) | C20:3 (n-6) | C20:4 (n-6) | C20:4 (n-3) | C20:5 (n-3) | C22:4 (n-6) | C22:5 (n-3) |
| TL16y2 | 250 uM GLA | 3,59 | | **10,71** | | | | | |
| pYES | 250 uM ARA | | | | 16,03 | | | | |
| TL16y2 | 250 uM ARA | | | | 15,2 | | **3,87** | | |
| pYES | 250 uM SDA | | 26,79 | | | 0,35 | | | |
| TL16y2 | 250 uM SDA | | 7,74 | | | **29,17** | | | |

Die in Tabelle 7 wiedergegebenen Ergebnisse zeigen mit TL16y2 gegenüber der Kontrolle folgende prozentuale Umsätze: a) % Umsatz EPA (250 uM): 8 %, b) % Umsatz EPA (50 uM): 41 %, c) % Umsatz ARA: 20,3 %, d) % Umsatz SDA: 79, 4% und e) % Umsatz GLA: 74,9 %.

TL16y2 zeigt damit Δ5-, Δ6- und Δ8-Elongaseaktivität. Dabei ist die Aktivität für C18-Fettsäuren mit Δ6-Doppelbindung am höchsten. Abhängig von der Konzentration an gefütterten Fettsäuren werden dann C20-Fettsäuren mit einer Δ5- bzw. Δ8-Doppelbindung verlängert.

Beispiel 14: Klonierung von Genen aus Ostreococcus tauri

Durch Suche nach konservierten Bereichen in den Proteinsequenzen mit Hilfe der in der Anmeldung aufgeführten Elongase-Gene mit Δ-5-Elongaseaktivität oder Δ-6-Elongaseaktivität konnten zwei Sequenzen mit entsprechenden Motiven in einer Ostreococcus tauri Sequenzdatenbank (genomische Sequenzen) identifiziert werden.

Es handelt sich dabei um die folgenden Sequenzen:

| Gen-Name | SEQ ID | Aminosäuren |
|---|---|---|
| OtELO1, (Δ-5-Elongase) | SEQ ID NO: 67 | 300 |
| OtELO2, (Δ-6-Elongase) | SEQ ID NO: 69 | 292 |

OtElo1 weist die höchste Ähnlichkeit zu einer Elongase aus Danio rerio auf (GenBank AAN77156; ca. 26 % Identität), während OtElo2 die größte Ähnlichkeit zur Physcomitrella Elo (PSE) [ca. 36 % Identität] aufweist (Alignments wurden mit dem tBLASTn-Aalgorithmus (Altschul et al., J. Mol. Biol. 1990, 215: 403 - 410) durchgeführt.

Die Klonierung wurde wie folgt durchgeführt:

40 ml einer *Ostreococcus tauri* Kultur in der stationären Phase wurden abzentrifugiert und in 100 μl Aqua bidest resuspendiert und bei -20°C gelagert. Auf der Basis des PCR-Verfahren wurden die zugehörigen genomischen DNAs amplifiziert. Die entsprechenden Primerpaare wurden so ausgewählt, dass sie die Hefe-Konsensus-Sequenz für hocheffiziente Translation (Kozak, Cell 1986, 44:283-292) neben dem Startcodon trugen. Die Amplifizierung der OtElo-DNAs wurde jeweils mit 1 μl aufgetauten Zellen, 200 μM dNTPs, 2,5 U *Taq*-Polymerase und 100 pmol eines jeden Primers in einem Gesamtvolumen von 50 μl durchgeführt. Die Bedingungen für die PCR waren wie folgt: Erste Denaturierung bei 95°C für 5 Minuten, gefolgt von 30 Zyklen bei 94°C für 30 Sekunden, 55°C für 1 Minute und 72°C für 2 Minuten sowie ein letzter Verlängerungsschritt bei 72°C für 10 Minuten.

Beispiel 15: Klonierung von Expressionsplasmiden zur heterologen Expression in Hefen:

Zur Charakterisierung der Funktion der Elongasen aus *Ostreococcus tauri* wurden die offenen Leserahmen der jeweiligen DNAs stromabwärts des Galactose-induzierbaren GAL1-Promotors von pYES2.1/V5-His-TOPO (Invitrogen) kloniert, wobei pOTE1 und pOTE2 erhalten wurden.

Der *Saccharomyces cerevisiae*-Stamm 334 wurde durch Elektroporation (1500 V) mit dem Vektor pOTE1 bzw.

pOTE2 transformiert. Als Kontrolle wurde eine Hefe verwendet, die mit dem leeren Vektor pYES2 transformiert wurde. Die Selektion der transformierten Hefen erfolgte auf Komplett-Minimalmedium (CMdum)-Agarplatten mit 2% Glucose, aber ohne Uracil. Nach der Selektion wurden je drei Transformanten zur weiteren funktionellen Expression ausgewählt.

Für die Expresssion der Ot-Elongasen wurden zunächst Vorkulturen aus jeweils 5 ml CMdum-Flüssigmedium mit 2% (w/v) Raffinose aber ohne Uracil mit den ausgewählten Transformanten angeimpft und 2 Tage bei 30°C, 200 rpm inkubiert.

5 ml CMdum-Flüssigmedium (ohne Uracil) mit 2% Raffinose und 300 μM verschiedener Fettsäuren wurden dann mit den Vorkulturen auf eine $OD_{600}$ von 0,05 angeimpft. Die Expression wurde durch die Zugabe von 2% (w/v) Galactose induziert. Die Kulturen wurden für weitere 96 h bei 20°C inkubiert.

Beispiel 16: Klonierung von Expressionsplasmiden zur Samen-spezifischen Expression in Pflanzen

Für die Transformation von Pflanzen wurde ein weiterer Transformationsvektor auf Basis von pSUN-USP erzeugt. Dazu wurden mittels PCR NotI-Schnittstellen am 5' und 3'-Ende der kodierenden Sequenzen eingefügt. Die entsprechenden Primersequenzen wurden von den 5'- und 3-Bereich von OtElo1 und OtElo2 abgeleitet.

Zusammensetzung des PCR-Ansatzes (50 μL):

5,00 μL Template cDNA
5,00 μL 10x Puffer (Advantage-Polymerase)+ 25mM $MgCl_2$
5,00 μL 2mM dNTP
1,25 μL je Primer (10 pmol/μL)
0,50 μL Advantage-Polymerase

Die Advantage-Polymerase von Clontech wurden eingesetzt.

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Die PCR Produkte wurden für 16 h bei 37 °C mit dem Restriktionsenzym NotI inkubiert. Der Pflanzen-Expressionsvektor pSUN300-USP wurde in gleicherweise inkubiert. Anschliessend wurde die PCR Produkte sowie der Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäss Herstellerangaben. Anschliessend wurden Vektor und PCR-Produkte ligiert. Dazu wurde das Rapid Ligation Kit von Roche verwendet. Die entstandenen Plasmide pSUN-OtELO1 und pSUN-OtELO2 wurde durch Sequenzierung verifiziert.

pSUN300 ist ein Derivat des Plasmides pPZP (Hajdukiewicz, P, Svab, Z, Maliga, P., (1994) The small versatile pPZP family of Agrobacterium binary vectors for plant transformation. Plant Mol Biol 25:989-994). pSUN-USP entstand aus pSUN300, indem in pSUN300 ein USP-Promotor als EcoRI- Fragment inseriert wurde. Das Polyadenylierungssignal ist das des Ostreococcus-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence and transcript map of the Agrobacterium tumefaciens Ti plasmid-encoded octopine synthase gene J. Mol. Appl. Genet. 1 (6), 499-511 (1982). Der USP-Promotor entspricht den Nukleotiden 1 bis 684 (Genbank Accession X56240), wobei ein Teil der nichtcodierenden Region des USP-Gens im Promotor enthalten ist. Das 684 Basenpaar große Promotorfragment wurde mittels käuflichen T7-Standardprimer (Stratagene) und mit Hilfe eines synthetisierten Primers über eine PCR-Reaktion nach Standardmethoden amplifiziert.

(Primersequenz:
5'–GTCGACCCGCGGACTAGTGGGCCCTCTAGACCCGGGGGATCC
GGATCTGCTGGCTATGAA–3', SEQ ID NO: 164).

Das PCR-Fragment wurde mit EcoRI/SalI nachgeschnitten und in den Vektor pSUN300 mit OCS Terminator eingesetzt. Es entstand das Plasmid mit der Bezeichnung pSUN-USP.Das Konstrukt wurde zur Transformation von Arabidopsis thaliana, Raps, Tabak und Leinsamen verwendet.

Beispiel 17: Expression von OtELO1 und OtELO2 in Hefen

Hefen, die wie unter Beispiel 15 mit den Plasmiden pYES3, pYES3-OtELO1 und pYES3-OtELO2 transformiert wurden, wurden folgendermaßen analysiert:
Die Hefezellen aus den Hauptkulturen wurden durch Zentrifugation (100 x g, 5 min, 20°C) geerntet und mit 100 mM $NaHCO_3$, pH 8,0 gewaschen, um restliches Medium und Fettsäuren zu entfernen. Aus den Hefe-Zellsedimenten wurden Fettsäuremethylester (FAMEs) durch saure Methanolyse hergestellt. Hierzu wurden die Zellsedimente mit 2 ml 1 N methanolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM $NaHCO_3$, pH 8,0 und 2 ml Aqua dest. gewaschen. Anschließend wurden die PE-Phasen mit $Na_2SO_4$ getrocknet, unter Argon eingedampft und in 100 $\mu$l PE aufgenommen. Die Proben wurden auf einer DB-23-Kapillarsäule (30 m, 0,25 mm, 0,25 $\mu$m, Agilent) in einem Hewlett-Packard 6850-Gaschromatographen mit Flammenionisationsdetektor getrennt. Die Bedingungen für die GLC-Analyse waren wie folgt: Die Ofentemperatur wurde von 50°C bis 250°C mit einer Rate von 5°C/min und schließlich 10 min bei 250°C(halten) programmiert.

Die Identifikation der Signale erfolgte durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

Beispiel 18: Funktionelle Charakterisierung von OtELO1 und OtELO2:

Die Substratspezifität der OtElo1 konnte nach Expression und Fütterung verschiedener Fettsäuren ermittelt werden (Tab.8). Die gefütterten Substrate sind in großen Mengen in allen transgenen Hefen nachzuweisen. Die transgenen Hefen zeigten die Synthese neuer Fettsäuren, den Produkten der OtElo1-Reaktion. Dies bedeutet, dass das Gen OtElo1 funktional exprimiert werden konnte.

Tabelle 7 zeigt, dass die OtElo1 eine enge Substratspezifität aufweist. Die OtElo1 konnte nur die C20-Fettsäuren Eicosapentaensäure (Figur 7) und Arachidonsäure (Figur 8) elongieren, bevorzugte aber die $\omega$-3-desaturierte Eicosapentaensäure.

Tabelle 8:

| Fettsäuresubstrat | Umsatz (in %) |
|---|---|
| 16:0$^{\square}$ | - |
| 16:10$^{\square\Delta 9}$ | - |
| 18:0$^{\square}$ | - |
| 18:1$^{\square\Delta 9}$ | - |
| 18:1$^{\Delta 11}$ | - |
| 18:2$^{\Delta 9,12}$ | - |
| 18:3$^{\square\Delta 6,9,12}$ | - |
| 18:3$^{\square\Delta 5,9,12}$ | - |
| 20:3 $^{\Delta 8,11,14}$ | - |
| 20:4 $^{\Delta 5,\square 8,11,14}$ | **10,8 $\pm$ 0,6** |
| 20:5 $^{\Delta 5,\square 8,11,14,17}$ | **46,8 $\pm$ 3,6** |
| 22:4 $^{\Delta 7,\square 10,13,16}$ | - |
| 22:6$^{\square\Delta 4,7,10,13,16,19}$ | - |

Tabelle 8 zeigt die Substratspezifität der Elongase OtElo1 für C20 polyungesättigte Fettsäuren mit einer Doppelbindung in $\Delta$5 Position gegenüber verschiedenen Fettsäuren.

Die Hefen, die mit dem Vektor pOTE1 transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert. Jeder Wert gibt den Mittelwert (n=3) $\pm$ Standardabweichung wieder.

Die Substratspezifität der OtElo2 (SEQ ID NO: 81) konnte nach Expression und Fütterung verschiedener Fettsäuren ermittelt werden (Tab. 9). Die gefütterten Substrate sind in großen Mengen in allen transgenen Hefen nachzuweisen. Die transgenen Hefen zeigten die Synthese neuer Fettsäuren, den Produkten der OtElo2-Reaktion. Dies bedeutet, dass das Gen OtElo2 funktional exprimiert werden konnte.

Tabelle 9:

| Fettsäuresubstrat | Umsatz (in %) |
|---|---|
| 16:0 | - |
| 16:1$^{\square A9}$ | - |
| 16:3$^{\square \Delta 7,10,13}$ | - |
| 18:0$^{\square}$ | - |
| 18:1$^{\square A6}$ | - |
| 18:1$^{\square \Delta 9}$ | - |
| 18:1$^{\square \Delta 11}$ | - |
| 18:2$^{\square \Delta 9,12}$ | - |
| 18:3$^{\Delta 6,9,12}$ | **15,3** ± |
| 18:30$^{\square\ \Delta 5,9,12}$ | - |
| 18:40$^{\square\ \Delta 6,9,12,15}$ | **21,1** ± |
| 20:2 $^{\Delta 11,14}$ | - |
| 20:3 $^{\Delta 8,11,14}$ | - |
| 20:4 $^{\Delta 5,\square 8,11,14}$ | - |
| 20:5 $^{\Delta 5,\square 8,11,14,17}$ | - |
| 22:4 $^{\Delta 7,\square 10,13,16}$ | - |
| 22:5 $^{\Delta 7,\square 10,13,16,19}$ | - |
| 22:6$^{\square 4,7,10,13,16,19}$ | - |

Tabelle 9 zeigt die Substratspezifität der Elongase OtElo2 gegenüber verschiedenen Fettsäuren.

Die Hefen, die mit dem Vektor pOTE2 transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert. Jeder Wert gibt den Mittelwert (n=3) ± Standardabweichung wieder.

Die enzymatische Aktivität, die in Tabelle 9 wiedergegeben wird, zeigt klar, dass OTELO2 eine Δ-6-Elongase ist.

Beispiel 19: Klonierung von Genen aus Thalassiosira pseudonana

Durch Suche nach konservierten Bereichen in den Proteinsequenzen mit Hilfe der in der Anmeldung aufgeführten Elongase-Gene mit Δ-5-Elongaseaktivität oder Δ-6-Elongaseaktivität konnten zwei Sequenzen mit entsprechenden Motiven in einer Thalassiosira pseudonana Sequenzdatenbank (genomische Sequenzen) identifiziert werden. Es handelt sich dabei um die folgenden Sequenzen:

| Gen-Name | SEQ ID | Aminosäuren |
|---|---|---|
| TpELO1 (Δ5-Elongase) | 43 | 358 |
| TpELO2 (Δ5-Elongase) | 59 | 358 |
| TpELO3 (Δ6-Elongase) | 45 | 272 |

Eine 2 L Kultur von T. pseudonana wurde in f/2 Medium (Guillard, R.R.L. 1975. Culture of phytoplankton for feeding marine invertebrates. In Culture of Marine Invertebrate Animals (Eds. Smith, W.L. and Chanley, M.H.), Plenum

Press, New York, pp 29-60.) für 14 d (= Tage) bei einer Lichtstärke von 80 E/cm$^2$ angezogen. Nach Zentrifugation der Zellen wurde RNA mit Hilfe des RNAeasy Kits der Firma Quiagen (Valencia, CA, US) nach Herstellerangaben isoliert. Die mRNA wurde mit dem Marathon cDNA Amplification-Kit (BD Biosciences) reverse transkribiert und entsprechend den Herstellerangaben Adaptoren ligiert. Die cDNA-Bank wurde dann für die PCR zur Klonierung von Expressionsplasmiden mittels 5'- und 3'-RACE (rapid amplification of cDNA ends) verwendet.

Beispiel 20: Klonierung von Expressionsplasmiden zur heterologen Expression in - Hefen

Die entsprechenden Primerpaare wurden so ausgewählt, dass sie die Hefe-Konsensus-Sequenz für hocheffiziente Translation (Kozak, Cell 1986, 44:283-292) neben dem Startcodon trugen. Die Amplifizierung der TpElo-DNAs wurde jeweils mit 1 µL cDNA, 200 µM dNTPs, 2,5 U *Advantage*-Polymerase und 100 pmol eines jeden Primers in einem Gesamtvolumen von 50 µl durchgeführt. Die Bedingungen für die PCR waren wie folgt: Erste Denaturierung bei 95°C für 5 Minuten, gefolgt von 30 Zyklen bei 94°C für 30 Sekunden, 55°C für 1 Minute und 72°C für 2 Minuten sowie ein letzter Verlängerungsschritt bei 72°C für 10 Minuten.

Für die Klonierung der Sequenz zur heterologen Expression in Hefen wurden folgende Oligonukleotide für die PCR-Reaktion verwendet:

*F=forward primer, R=reverse primer

| Gen-Name und SEQ ID NO: | Primersequenz |
|---|---|
| TpELO1 (Δ5-Elongase), SEQ ID NO: 59 | F:5'-accatgtgctcaccaccgccgtc (SEQ ID NO: 158)<br>R:5'- ctacatggcaccagtaac (SEQ ID NO: 159) |
| TpELO2 (Δ5-Elongase), SEQ ID NO: 85 | F:5'-accatgtgctcatcaccgccgtc (SEQ ID NO: 160)<br>R:5'-ctacatggcaccagtaac (SEQ ID NO: 161) |
| TpELO3 (Δ6-Elongase), SEQ ID NO:45 | F:5'-accatggacgcctacaacgctgc (SEQ ID NO: 162)<br>R:5'- ctaagcactcttcttcttt (SEQ ID NO: 163) |

Die PCR Produkte wurde für 30 min bei 21 °C mit dem Hefe-Expressionsvektor - pYES2.1-TOPO (Invitrogen) gemäß Herstellerangaben inkubiert. Das PCR-Produkt wird dabei durch einen T-Überhang und Aktivität einer Topoisomerase (Invitrogen) in den Vektor ligiert. Nach der Inkubation erfolgte dann die Transformation von E. coli DH5a Zellen. Entsprechende Klone wurden durch PCR identifiziert, die Plasmid-DNA mittels Qiagen DNAeasy-Kit isoliert und durch Sequenzierung verifiziert. Die korrekte Sequenz wurde dann in den Saccharomyces Stamm INVSc1 (Invitrogen) durch Elektroporation (1500 V) transformiert. Zur Kontrolle wurde der leere Vektor pYES2.1 parallel transformiert. Anschließend wurden die Hefen auf Komplett-Minimalmedium ohne Uracil mit 2 % Glucose ausplattiert. Zellen, die ohne Uracil im Medium wachstumsfähig waren, enthalten damit die entsprechenden Plasmide pYES2.1, pYES2.1-TpELO1, pYES2.1-TpELO2 und pYES2.1-TpELO3. Nach der Selektion wurden je zwei Transformaten zur weiteren funktionellen Expression ausgewählt.

Beispiel 21: Klonierung von Expressionsplasmiden zur Samen-spezifischen Expression in Pflanzen

Für die Transformation von Pflanzen wird ein weiterer Transformationsvektor auf Basis von pSUN-USP erzeugt. Dazu wird mit folgendem Primerpaar NotI-Schnittstellen am 5' und 3'-Ende der kodierenden Sequenz eingefügt:.

PSUN-TPELO1

Forward: 5'-GCGGCCGCACCATGTGCTCACCACCGCCGTC (SEQ ID NO: 152)
Reverse: 3'-GCGGCCGCCTACATGGCACCAGTAAC (SEQ ID NO: 153)

PSU N-TPELO2

Forward: 5'-GCGGCCGCACCATGTGCTCATCACCGCCGTC (SEQ ID NO: 154)
Reverse: 3'-GCGGCCGCCTACATGGCACCAGTAAC (SEQ ID NO: 155)

PSU N-TPELO3

Forward: 5'-GCGGCCGCaccatggacgcctacaacgctgc (SEQ ID NO: 156)

Reverse: 3'-GCGGCCGCCTAAGCACTCTTCTTCTTT (SEQ ID NO: 157)

Zusammensetzung des PCR-Ansatzes (50 μL):

5,00 μL Template cDNA
5,00 μL 10x Puffer (Advantage-Polymerase)+ 25mM MgCl2
5,00 μL 2mM dNTP
1,25 μL je Primer (10 pmol/μL)
0,50 μL Advantage-Polymerase

Die Advantage-Polymerase von Clontech wurden eingesetzt.
Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Die PCR Produkte werden für 16 h bei 37 °C mit dem Restriktionsenzym NotI inkubiert. Der Pflanzen-Expressionsvektor pSUN300-USP wird in gleicherweise inkubiert. Anschliessend werden die PCR Produkte sowie der 7624 bp große Vektor durch Agarose-Gelelektrophorese aufgetrennt und die entsprechenden DNA-Fragmente ausgeschnitten. Die Aufreinigung der DNA erfolgt mittels Qiagen Gel Purification Kit gemäss Herstellerangaben. Anschließend werden Vektor und PCR-Produkte ligiert. Dazu wird das Rapid Ligation Kit von Roche verwendet. Die entstandenen Plasmide pSUN-TPEL01, pSUN-TPELO2 und pSUN-TPELO3 werden durch Sequenzierung verifiziert.
pSUN300 ist ein Derivat des Plasmides pPZP (Hajdukiewicz, P, Svab, Z, Maliga, P., (1994) The small versatile pPZP family of Agrobacterium binary vectors forplant transformation. Plant Mol Biol 25:989-994). pSUN-USP entstand aus pSUN300, indem in pSUN300 ein USP-Promotor als EcoRI- Fragment inseriert wurde. Das Polyadenylierungssignal ist das des Octopinsynthase-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence and transcript map of the Agrobacterium tumefaciens Ti plasmid-encoded octopine synthase gene J. Mol. Appl. Genet. 1 (6), 499-511 (1982) Der USP-Promotor entspricht den Nukleotiden 1-684 (Genbank Accession X56240), wobei ein Teil der nichtcodierenden Region des USP-Gens im Promotor enthalten ist. Das 684 Basenpaar große Promotorfragment wurde mittels käuflichen T7-Standardprimer (Stratagene) und mit Hilfe eines synthetisierten Primers über eine PCR-Reaktion nach Standardmethoden amplifiziert.

(Primersequenz: 5'–GTCGACCCGCGGACTAGTGGGCCCTCTAGACCCGGGGGATCCGGATCTGCTGGCTATGAA–3'; SEQ ID NO: 151).

Das PCR-Fragment wurde mit EcoRI/SalI nachgeschnitten und in den Vektor pSUN300 mit OCS Terminator eingesetzt. Es entstand das Plasmid mit der Bezeichnung pSUN-USP.Das Konstrukt wurde zur Transformation von Arabidopsis thaliana, Raps, Tabak und Leinsamen verwendet.
Die Lipidextraktion aus Hefen und Samen erfolgte identisch zu Beispiel 6.

Beispiel 22: Expression von TpELO1, TpELO2 und TpELO3 in Hefen

Hefen, die wie unter Beispiel 4 mit den Plasmiden pYES2, pYES2-TpELO1, pYES2-TpELO2 und pYES2-TpELO3 transformiert wurden, wurden folgendermaßen analysiert:
Die Hefezellen aus den Hauptkulturen wurden durch Zentrifugation (100 x g, 5 min, 20°C) geerntet und mit 100 mM $NaHCO_3$, pH 8,0 gewaschen, um restliches Medium und Fettsäuren zu entfernen. Aus den Hefe-Zellsedimenten wurden Fettsäuremethylester (FAMEs) durch saure Methanolyse hergestellt. Hierzu wurden die Zellsedimente mit 2 ml 1 N methanolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM $NaHCO_3$, pH 8,0 und 2 ml Aqua dest. gewaschen. Anschließend wurden die PE-Phasen mit $Na_2SO_4$ getrocknet, unter Argon eingedampft und in 100 μl PE aufgenommen. Die Proben wurden auf einer DB-23-Kapillarsäule (30 m, 0,25 mm, 0,25 μm, Agilent) in einem Hewlett-Packard 6850-Gaschromatographen

mit Flammenionisationsdetektor getrennt. Die Bedingungen für die GLC-Analyse waren wie folgt: Die Ofentemperatur wurde von 50°C bis 250°C mit einer Rate von 5°C/min und schließlich 10 min bei 250°C(halten) programmiert.

Die Identifikation der Signale erfolgte durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

Beispiel 23: Funktionelle Charakterisierung von TpELO1 und TpELO3:

Die Substratspezifität der TpElo1 konnte nach Expression und Fütterung verschiedener Fettsäuren ermittelt werden (Fig. 9). Die gefütterten Substrate sind in großen Mengen in allen transgenen Hefen nachzuweisen. Die transgenen Hefen zeigten die Synthese neuer Fettsäuren, den Produkten der TpElo1-Reaktion. Dies bedeutet, dass das Gen TpElo1 funktional exprimiert werden konnte.

Tabelle 10 zeigt, dass die TpElo1 eine enge Substratspezifität aufweist. Die TpElo1 konnte nur die C20-Fettsäuren Eicosapentaensäure und Arachidonsäure elongieren, bevorzugte aber die ω-3-desaturierte Eicosapentaensäure.

Die Hefen, die mit dem Vektor pYES2-TpEL01 transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert.

Tabelle 10: Expression von TpELO1 in Hefe. In den Spalten 1 und 3 sind die Kontrolreaktionen für die Spalten 2 (gefüttert 250 μM 20:4 Δ5.8.11,14) und 4 (gefüttert 250 μM 20:5 Δ5,8,11,14,17) wiedergegeben.

| | Expression | Expression | Expression | Expression |
|---|---|---|---|---|
| Fettsäuren | 1 | 2 | 3 | 4 |
| 16:0 | 18.8 | 17.8 | 25.4 | 25.2 |
| $16:1^{\Delta 9}$ | 28.0 | 29.8 | 36.6 | 36.6 |
| 18:0 | 5.2 | 5.0 | 6.8 | 6.9 |
| $18:1^{\Delta 9}$ | 25.5 | 23.6 | 24.6 | 23.9 |
| $20:4^{\Delta 5,8,11,14}$ | **22.5** | **23.4** | - | - |
| $22:4^{\Delta 7,10,13,16}$ | - | **0.4** | - | - |
| $20:5^{\Delta 5,8,11,14,17}$ | - | - | **6.6** | **6.5** |
| $22:5^{\Delta 7,10,13,16,19}$ | - | - | - | **0.9** |
| **% Umsatz** | **0** | **1.7** | **0** | **12.2** |

Die Substratspezifität der TpElo3 konnte nach Expression und Fütterung verschiedener Fettsäuren ermittelt werden (Fig. 10). Die gefütterten Substrate sind in großen Mengen in allen transgenen Hefen nachzuweisen. Die transgenen Hefen zeigten die Synthese neuer Fettsäuren, den Produkten der TpElo3-Reaktion. Dies bedeutet, dass das Gen TpElo3 funktional exprimiert werden konnte.

Tabelle 11 zeigt, dass die TpElo3 eine enge Substratspezifität aufweist. Die TpElo3 konnte nur die C18-Fettsäuren γ-Linolensäure und Stearidonsäure elongieren, bevorzugte aber die ω-3-desaturierte Stearidonsäure.

Die Hefen, die mit dem Vektor pYES2-TpELO3 transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert.

Tabelle 11: Expression von TpELO3 in Hefe. Spalte 1 zeigt das Fettsäureprofil von Hefe ohne Fütterung. Spalte 2 zeigt die Kontrollreaktion. In den Spalten 3 bis 6 wurden γ-Linolensäure, Stearidonsäure, Arachidonsäure und Eicosapentaensäure gefüttert (250 μM jeder Fettsäure).

| Fettsäuren | 1 | 2 | 3 | 4 | 5 | 6 |
|---|---|---|---|---|---|---|
| 16:0 | 17.9 | 20.6 | 17.8 | 16.7 | 18.8 | 18.8 |
| $16:1^{\Delta 9}$ | 41.7 | 18.7 | 27.0 | 33.2 | 24.0 | 31.3 |
| 18:0 | 7.0 | 7.7 | 6.4 | 6.6 | 5.2 | 6,0 |

(fortgesetzt)

| Fettsäuren | 1 | 2 | 3 | 4 | 5 | 6 |
|---|---|---|---|---|---|---|
| $18:1^{\Delta 9}$ | 33.3 | 16.8 | 24.2 | 31.8 | 25.5 | 26.4 |
| $18:2^{\Delta 9,12}$ | - | **36.1** | - | - | - | - |
| $18:3^{\Delta 6,9,12}$ | - | - | **6.1** | - | - | |
| $18:4^{\Delta 6,9,12,15}$ | - | - | - | **1.7** | - | |
| $20:2^{\Delta 11,14}$ | - | **0** | - | - | - | |
| $20:3^{\Delta 8,11,14}$ | - | - | **18.5** | - | - | |
| $20:4^{\Delta 8,11,14,17}$ | - | - | - | **10.0** | - | |
| $20:4^{\Delta 5,8,11,14}$ | - | - | - | - | **22.5** | |
| $22:4^{\Delta 7,10,13,16}$ | - | - | - | - | **0** | |
| $20:5^{\Delta 5,8,11,14,17}$ | - | - | - | - | - | **17.4** |
| $22:5^{\Delta 7,10,13,16,19}$ | - | - | - | - | - | **0** |
| **% Umsatz** | **0** | **0** | **75** | **85** | **0** | **0** |

Beispiel 24: Klonierung eines Expressionsplasmides zur heterologen Expression der Pi-omega3Des in Hefen

Der Pi-omega3Des Klon wurde für die heterologe Expression in Hefen über PCR mit entsprechenden Pi-omega3Des spezifischen Primern in den Hefe-Expressionsvektor pYES3 kloniert. Dabei wurde ausschließlich der für das Pi-omega3Des Protein kodierende offene Leseraster des Gens amplifiziert und mit zwei Schnittstellen für die Klonierung in den pYES3 Expressionsvektor versehen:

Forward Primer: 5'-TAAGCTTACATGGCGACGAAGGAGG (SEQ ID NO: 149)
Reverse Primer: 5'-TGGATCCACTTACGTGGACTTGGT (SEQ ID NO: 150)

Zusammensetzung des PCR-Ansatzes (50 μL):

5,00 μL Template cDNA
5,00 μL 10x Puffer (Advantage-Polymerase)+ 25mM $MgCl_2$
5,00 μL 2mM dNTP
1,25 μL je Primer (10 pmol/μL des 5'-ATG sowie des 3'-Stopp Primers)
0,50 μL Advantage-Polymerase

Die Advantage-Polymerase von Clontech wurden eingesetzt.
Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR Produkt wurde für 2 h bei 37 °C mit den Restriktionsenzymen HindIII und BamHI inkubiert. Der Hefe-Expressionsvektor pYES3 (Invitrogen) wurde in gleicherweise inkubiert. Anschließend wurde das 1104 bp große PCR Produkt sowie der Vektor durch Agarose-Gelelektrophorese aufgetrennt und die entsprechenden DNA-Fragmente ausgeschnitten. Die Aufreinigung der DNA erfolge mittels Qiagen Gel purification Kit gemäss Herstellerangaben. Anschließend wurden Vektor und Desaturase-cDNA ligiert. Dazu wurde das Rapid Ligation Kit von Roche verwendet. Das entstandene Plasmid pYES3-Pi-omega3Des wurde durch Sequenzierung überprüftt und in den Saccharomyces Stamm INVSc1 (Invitrogen) durch Elektroporation (1500 V) transformiert. Zur Kontrolle wurde pYES3 parallel transformiert. Anschliessend wurden die Hefen auf Komplett-Minimalmedium ohne Tryptophan mit 2 % Glucose ausplattiert. Zellen, die auf ohne Tryptophan im Medium wachstumsfähig waren, enthalten damit die entsprechenden Plasmide pYES3, pYES3-Pi-omega3Des. Nach der Selektion wurden je zwei Transformaten zur weiteren funktionellen Expression ausgewählt.

Beispiel 25: Klonierung von Expressionsplasmiden zur Samen-spezifischen Expression in Pflanzen

Für die Transformation von Pflanzen wurde ein weiterer Transformationsvektor auf Basis von pSUN-USP erzeugt. Dazu wurde mit folgendem Primerpaar NotI-Schnittstellen am 5' und 3'-Ende der kodierenden Sequenz eingefügt:.
PSUN-Pi-omega3Des

Reverse: 3'-GCGGCCGCTTACGTGGACTTGGTC (SEQ ID NO: 147)
Forward: 5'-GCGGCCGCatGGCGACGAAGGAGG (SEQ ID NO: 148)

Zusammensetzung des PCR-Ansatzes (50 μL):

5,00 μL Template cDNA
5,00 μL 10x Puffer (Advantage-Polymerase)+ 25mM $MgCl_2$
5,00 μL 2mM dNTP
1,25 μL je Primer (10 pmol/μL)
0,50 μL Advantage-Polymerase

Die Advantage-Polymerase von Clontech wurden eingesetzt.
Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Die PCR Produkte wurden für 4 h bei 37 °C mit dem Restriktionsenzym NotI inkubiert. Der Pflanzen-Expressionsvektor pSUN300-USP wurde in gleicherweise inkubiert. Anschließend wurde die PCR Produkte sowie der 7624 bp große Vektor durch Agarose-Gelelektrophorese aufgetrennt und die entsprechenden DNA-Fragmente ausgeschnitten. Die Aufreinigung der DNA erfolge mittels Qiagen Gel purification Kit gemäss Herstellerangaben. Anschliessend wurden Vektor und PCR-Produkte ligiert. Dazu wurde das Rapid Ligation Kit von Roche verwendet. Das entstandene Plasmid pSUN-Piomega3Des wurde durch Sequenzierung verifiziert.

Beispiel 26: Expression von Pi-omega3Des in Hefen

Hefen, die wie unter Beispiel 24 mit dem Plasmid pYES3 oder pYES3- Pi-omega3Des transformiert wurden, wurden folgendermaßen analysiert:
Die Hefezellen aus den Hauptkulturen wurden durch Zentrifugation (100 x g, 5 min, 20°C) geerntet und mit 100 mM $NaHCO_3$, pH 8,0 gewaschen, um restliches Medium und Fettsäuren zu entfernen. Aus den Hefe-Zellsedimenten wurden Fettsäuremethylester (FAMEs) durch saure Methanolyse hergestellt. Hierzu wurden die Zellsedimente mit 2 ml 1 N methanolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM $NaHCO_3$, pH 8,0 und 2 ml Aqua dest. gewaschen. Anschließend wurden die PE-Phasen mit $Na_2SO_4$ getrocknet, unter Argon eingedampft und in 100 μl PE aufgenommen. Die Proben wurden auf einer DB-23-Kapillarsäule (30 m, 0,25 mm, 0,25 μm, Agilent) in einem Hewlett-Packard 6850-Gaschromatographen mit Flammenionisationsdetektor getrennt. Die Bedingungen für die GLC-Analyse waren wie folgt: Die Ofentemperatur wurde von 50°C bis 250°C mit einer Rate von 5°C/min und schließlich 10 min bei 250°C(halten) programmiert.
Die Identifikation der Signale erfolgte durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

Beispiel 27: Funktionelle Charakterisierung von Pi-omega3Des:

Die Substratspezifität der Pi-omega3Des konnte nach Expression und Fütterung verschiedener Fettsäuren ermittelt werden (Figur 12 bis 18). Die gefütterten Substrate liegen in großen Mengen in allen transgenen Hefen vor,

wodurch die Aufnahme dieser Fettsäuren in die Hefen bewiesen ist. Die transgenen Hefen zeigen die Synthese neuer Fettsäuren, den Produkten der Pi-omega3Des-Reaktion. Dies bedeutet, dass das Gen Pi-omega3Des funktional exprimiert werden konnte.

Figur 12 gibt die Desaturierung von Linolsäure (18:2 ω-6-Fettsäure) zu α-Linolensäure (18:3 ω-3-Fettsäure) durch die Pi-omega3Des wieder. Die Synthese der Fettsäuremethylester erfolgte durch saure Methanolyse intakter Zellen, die mit dem Leervektor pYES2 (Figur 12 A) oder dem Vektor pYes3-Pi-omega3Des (Figur 12 B) transformiert worden waren. Die Hefen wurden in Minimalmedium in Gegenwart von C18:$2^{\Delta 19,12}$-Fettsäure (300 μM) kultiviert. Anschließend wurden die FAMEs über GLC analysiert.

In Figur 13 ist die Desaturierung von γ-Linolensäure (18:3 ω-6-Fettsäure) zu Stearidonsäure (18:4 ω-3-Fettsäure) durch Pi-omega3Des wiedergegeben.

Die Synthese der Fettsäuremethylester erfolgte durch saure Methanolyse intakter Zellen, die mit dem Leervektor pYES2 (Figur 13 A) oder dem Vektor pYes3-Pi-omega3Des (Figur 13 B) transformiert worden waren. Die Hefen wurden in Minimalmedium in Gegenwart von γ-C18:$3^{\Delta 6,9,12}$-Fettsäure (300 μM) kultiviert. Anschließend wurden die FAMEs über GLC analysiert.

Figur 14 gibt die Desaturierung von C20:2-ω-6-Fettsäure zu C20:3-ω-3-Fettsäure durch Pi-omega3Des wieder. Die Synthese der Fettsäuremethylester erfolgte durch saure Methanolyse intakter Zellen, die mit dem Leervektor pYES2 (Figur 14 A) oder dem Vektor pYes3-Pi-omega3Des (Figur 14 B) transformiert worden waren. Die Hefen wurden in Minimalmedium in Gegenwart von C20:$2^{\Delta 111,14}$-Fettsäure (300 μM) kultiviert. Anschließend wurden die FAMEs über GLC analysiert.

Figur 15 gibt die Desaturierung von C20:3-ω-6-Fettsäure zu C20:4-ω-3-Fettsäure durch Pi-omega3Des wieder. Die Synthese der Fettsäuremethylester erfolgte durch saure Methanolyse intakter Zellen, die mit dem Leervektor pYES2 (Figur 15 A) oder dem Vektor pYes3-Pi-omega3Des (Figur 15 B) transformiert worden waren. Die Hefen wurden in Minimalmedium in Gegenwart von C20:$3^{\Delta 8,11,14}$-Fettsäure (300 μM) kultiviert. Anschließend wurden die FAMEs über GLC analysiert.

In Figur 16 wird die Desaturierung von Arachidonsäure (C20:4-ω-6-Fettsäure) zu Eicosapentaensäure (C20:5-ω-3-Fettsäure) durch die Pi-omega3Des gezeigt.

Die Synthese der Fettsäuremethylester erfolgte durch saure Methanolyse intakter Zellen, die mit dem Leervektor pYES2 (Figur 16 A) oder dem Vektor pYes3-Pi-omega3Des (Figur 16 B) transformiert worden waren. Die Hefen wurden in Minimalmedium in Gegenwart von C20:$4^{\Delta 5,8,11,14}$-Fettsäure (300 μM) kultiviert. Anschließend wurden die FAMEs über GLC analysiert.

Figur 17 gibt die Desaturierung von Docosatetraensäure (C22:4-ω-6-Fettsäure) zu Docosapentaensäure (C22:5-ω-3-Fettsäure) durch Pi-omega3Des wieder. Die Synthese der Fettsäuremethylester erfolgte durch saure Methanolyse intakter Zellen, die mit dem Leervektor pYES2 (Figur 17 A) oder dem Vektor pYes3-Pi-omega3Des (Figur 17 B) transformiert worden waren. Die Hefen wurden in Minimalmedium in Gegenwart von C22:$4^{\Delta 7,10,13,16}$-Fettsäure (300 μM) kultiviert. Anschließend wurden die FAMEs über GLC analysiert.

Die Substratspezifität der Pi-omega3Des gegenüber verschiedenen Fettsäuren ist Figur 18 zu entnehmen. Die Hefen, die mit dem Vektor pYes3-Pi-omega3Des transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert. Jeder Wert gibt einen Mittelwert aus drei Messungen wieder. Die Umsetzungsraten (% Desaturation) wurden mit der Formel:

$$[Produkt]/[Produkt]+[Substrat]*100 \text{ errechnet.}$$

Wie unter Beispiel 9 beschrieben kann auch die Pi-omega3Des zur Erzeugung transgener Pflanzen verwendet werden. Aus den Samen dieser Pflanzen kann dann die Lipidextraktion wie unter Beispiel 6 beschrieben erfolgen.

Beispiel 28: Klonierung von Desaturasegenen aus Ostreococcus tauri

Durch Suche nach konservierten Bereichen in den Proteinsequenzen mit Hilfe von konservierten Motiven (His-Boxen, Domergue et al. 2002, Eur. J. Biochem. 269, 4105-4113) konnten fünf Sequenzen mit entsprechenden Motiven in einer Ostreococcus tauri Sequenzdatenbank (genomische Sequenzen) identifiziert werden. Es handelt sich dabei um die folgenden Sequenzen:

| Gen-Name | SEQ ID | Aminosäuren | Homologie |
|---|---|---|---|
| OtD4 | SEQ ID NO: 95 | 536 | Δ-4-Desaturase |

(fortgesetzt)

| Gen-Name | SEQ ID | Aminosäuren | Homologie |
|---|---|---|---|
| OtD5.1 | SEQ ID NO: 91 | 201 | Δ-5-Desaturase |
| OtD5.2 | SEQ ID NO: 93 | 237 | Δ-5-Desaturase |
| OtD6.1 | SEQ ID NO: 89 | 456 | Δ-6-Desaturase |
| OtFad2 | SEQ ID NO: 107 | 361 | Δ-12-Desaturase |

Die Alignments zur Auffindung von Homologien der einzelnen Gene wurden mit dem tBLASTn-Aalgorithmus (Altschul et al., J. Mol. Biol. 1990, 215: 403 - 410) durchgeführt.

Die Klonierung erfolgte wie folgt:
40 ml einer *Ostreococcus tauri* Kultur in der stationären Phase wurden abzentrifugiert und in 100 µl Aqua bidest resuspendiert und bei -20°C gelagert. Auf der Basis des PCR-Verfahren wurden die zugehörigen genomischen DNAs amplifiziert. Die entsprechenden Primerpaare wurden so ausgewählt, dass sie die Hefe-Konsensus-Sequenz für hocheffiziente Translation (Kozak, Cell 1986, 44:283-292) neben dem Startcodon trugen. Die Amplifizierung der OtDes-DNAs wurde jeweils mit 1 µl aufgetauten Zellen, 200 µM dNTPs, 2,5 U *Taq*-Polymerase und 100 pmol eines jeden Primers in einem Gesamtvolumen von 50 µl durchgeführt. Die Bedingungen für die PCR waren wie folgt: Erste Denaturierung bei 95°C für 5 Minuten, gefolgt von 30 Zyklen bei 94°C für 30 Sekunden, 55°C für 1 Minute und 72°C für 2 Minuten sowie ein letzter Verlängerungsschritt bei 72°C für 10 Minuten.

Folgende Primer wurden für die PCR eingesetzt:

OtDes6.1 Forward: 5'ggtaccacataatgtgcgtggagacggaaaataacg3' (SEQ ID NO: 145)
OtDes6.1 Reverse: 5'ctcgagttacgccgtctttccggagtgttggcc3' (SEQ ID NO: 146)

Beispiel: 29 Klonierung von Expressionsplasmiden zur heterologen Expression in Hefen:

Zur Charakterisierung der Funktion der Desaturase OtDes6.1 (= Δ-6-Desaturase) aus *Ostreococcus tauri* wurde der offenen Leserahmen der DNA stromabwärts des Galactose-induzierbaren GAL1-Promotors von pYES2.1/V5-His-TOPO (Invitrogen) kloniert, wobei der entsprechenden pYES2.1-OtDes6.1 Klon erhalten wurde. In entsprechender Art und Weise können weitere Desaturase-Gene aus Ostreococcus kloniert werden.

Der *Saccharomyces cerevisiae*-Stamm 334 wurde durch Elektroporation (1500 V) mit dem Vektor pYES2.1-OtDes6.1 transformiert. Als Kontrolle wurde eine Hefe verwendet, die mit dem leeren Vektor pYES2 transformiert wurde. Die Selektion der transformierten Hefen erfolgte auf Komplett-Minimalmedium (CMdum)-Agarplatten mit 2% Glucose, aber ohne Uracil. Nach der Selektion wurden je drei Transformanten zur weiteren funktionellen Expression ausgewählt.

Für die Expresssion der OtDes6.1 Desaturase wurden zunächst Vorkulturen aus jeweils 5 ml CMdum-Flüssigmedium mit 2% (w/v) Raffinose aber ohne Uracil mit den ausgewählten Transformanten angeimpft und 2 Tage bei 30°C, 200 rpm inkubiert. 5 ml CMdum-Flüssigmedium (ohne Uracil) mit 2% Raffinose und 300 µM verschiedener Fettsäuren wurden dann mit den Vorkulturen auf eine $OD_{600}$ von 0,05 angeimpft. Die Expression wurde durch die Zugabe von 2% (w/v) Galactose induziert. Die Kulturen wurden für weitere 96 h bei 20°C inkubiert.

Beispiel: 30 Klonierung von Expressionsplasmiden zur Samen-spezifischen Expression in Pflanzen

Für die Transformation von Pflanzen wird ein weiterer Transformationsvektor auf Basis von pSUN-USP erzeugt. Dazu werden mittels PCR NotI-Schnittstellen am 5' und 3'-Ende der kodierenden Sequenzen eingefügt. Die entsprechenden Primersequenzen werden von den 5'- und 3-Bereich der Desaturasen abgeleitet.

Zusammensetzung des PCR-Ansatzes (50 µL):

5,00 µL Template cDNA
5,00 µL 10x Puffer (Advantage-Polymerase)+ 25mM $MgCl_2$
5,00 µL 2mM dNTP
1,25 µL je Primer (10 pmol/µL)
0,50 µL Advantage-Polymerase

Die Advantage-Polymerase von Clontech wurden eingesetzt.

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Die PCR Produkte werden für 16 h bei 37 °C mit dem Restriktionsenzym NotI inkubiert. Der Pflanzen-Expressionsvektor pSUN300-USP wird in gleicherweise inkubiert. Anschliessend werden die PCR Produkte sowie der Vektor durch Agarose-Gelelektrophorese aufgetrennt und die entsprechenden DNA-Fragmente ausgeschnitten. Die Aufreinigung der DNA erfolgt mittels Qiagen Gel Purification Kit gemäss Herstellerangaben. Anschliessend werden Vektor und PCR-Produkte ligiert. Dazu wurde das Rapid Ligation Kit von Roche verwendet. Die entstandenen Plasmide werden durch Sequenzierung verifiziert.
pSUN300 ist ein Derivat des Plasmides pPZP (Hajdukiewicz,P, Svab, Z, Maliga, P., (1994) The small versatile pPZP family of Agrobacterium binary vectors for plant transformation. Plant Mol Biol 25:989-994). pSUN-USP entstand aus pSUN300, indem in pSUN300 ein USP-Promotor als EcoRI- Fragment inseriert wurde. Das Polyadenylierungssignal ist das des Ostreococcus-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence and transcript map of the Agrobacterium tumefaciens Ti plasmid-encoded octopine synthase gene J. Mol. Appl. Genet. 1 (6), 499-511 (1982). Der USP-Promotor entspricht den Nukleotiden 1 bis 684 (Genbank Accession X56240), wobei ein Teil der nichtcodierenden Region des USP-Gens im Promotor enthalten ist. Das 684 Basenpaar große Promotorfragment wurde mittels käuflichen T7-Standardprimer (Stratagene) und mit Hilfe eines synthetisierten Primers über eine PCR-Reaktion nach Standardmethoden amplifiziert.

(Primersequenz: 5'–
GTCGACCCGCGGACTAGTGGGCCCTCTAGACCCGGGGGATCC
GGATCTGCTGGCTATGAA–3', SEQ ID NO: 144).

Das PCR-Fragment wurde mit EcoRI/SalI nachgeschnitten und in den Vektor pSUN300 mit OCS Terminator eingesetzt. Es entstand das Plasmid mit der Bezeichnung pSUN-USP.Das Konstrukt wurde zur Transformation von Arabidopsis thaliana, Raps, Tabak und Leinsamen verwendet.

Beispiel: 31 Expression von OtDes6.1 in Hefen

Hefen, die wie unter Beispiel 4 mit den Plasmiden pYES2 und pYES2-OtDes6.2 transformiert wurden, wurden folgendermaßen analysiert:
Die Hefezellen aus den Hauptkulturen wurden durch Zentrifugation (100 x g, 5 min, 20°C) geerntet und mit 100 mM $NaHCO_3$, pH 8,0 gewaschen, um restliches Medium und Fettsäuren zu entfernen. Aus den Hefe-Zellsedimenten wurden Fettsäuremethylester (FAMEs) durch saure Methanolyse hergestellt. Hierzu wurden die Zellsedimente mit 2 ml 1 N methanolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM $NaHCO_3$, pH 8,0 und 2 ml Aqua dest. gewaschen. Anschließend wurden die PE-Phasen mit $Na_2SO_4$ getrocknet, unter Argon eingedampft und in 100 μl PE aufgenommen. Die Proben wurden auf einer DB-23-Kapillarsäule (30 m, 0,25 mm, 0,25 μm, Agilent) in einem Hewlett-Packard 6850-Gaschromatographen mit Flammenionisationsdetektor getrennt. Die Bedingungen für die GLC-Analyse waren wie folgt: Die Ofentemperatur wurde von 50°C bis 250°C mit einer Rate von 5°C/min und schließlich 10 min bei 250°C(halten) programmiert.
Die Identifikation der Signale erfolgte durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

Beispiel: 32 Funktionelle Charakterisierung von Desaturasen aus Ostreococcus:

Die Substratspezifität von Desaturasen kann nach Expression in Hefe (siehe Beispiele Klonierung von Desaturase-Genen, Hefeexpression) durch die Fütterung mittels verschiedener Hefen ermittelt werden. Beschreibungen für die Bestimmung der einzelnen Aktivitäten finden sich in WO 93/11245 für Δ15-Desaturasen, WO 94/11516 für Δ12-Desaturasen, WO 93/06712, US 5,614,393, US5614393, WO 96/21022, WO0021557 und WO 99/27111 für Δ6-Desaturasen, Qiu et al. 2001, J. Biol. Chem. 276, 31561-31566 für Δ4-Desaturasen, Hong et al. 2002, Lipids 37,863-868 für Δ5-Desaturasen.

Tabelle 12 gibt die Substratspezifität der Desaturase OtDes6.1 gegenüber verschiedenen Fettsäuren wieder. Die Substratspezifität der OtDes6.1 konnte nach Expression und Fütterung verschiedener Fettsäuren ermittelt werden. Die gefütterten Substrate sind in großen Mengen in allen transgenen Hefen nachzuweisen. Die transgenen Hefen zeigten die Synthese neuer Fettsäuren, den Produkten der OtDes6.2-Reaktion (Fig. 20). Dies bedeutet, dass das Gen OtDes6.1 funktional exprimiert werden konnte.

Die Hefen, die mit dem Vektor pYES2-OtDes6.1 transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert. Jeder Wert gibt den Mittelwert (n=3) ± Standardabweichung wieder. Die Aktivität entspricht der Konversionsrate errechnet nach [Substrat/(Substrat+Produkt)*100].

Tabelle 12 zeigt, dass die OtDes6.1 eine Substratspezifität für Linol- und Linolensäure (18:2 und 18:3) aufweist, da mit diesen Fettsäuren die höchsten Aktivitäten erreicht werden. Die Aktivität für Ölsäure (18:1) und Palmitoleinsäure (16:1) ist dagegen deutlich geringer. Die bevorzugte Umsetzung von Linol- und Linolensäure zeigt die Eignung dieser Desaturase für die Herstellung von polyungesättigten Fettsäuren.

| Substrate | Aktivität in % |
|---|---|
| $16:1^{\Delta 9}$ | 5,6 |
| $18:1^{\Delta 9}$ | 13,1 |
| $18:2^{\Delta 9,12}$ | 68,7 |
| $18:3^{\Delta 9,12,15}$ | 64,6 |

Figur 20 zeigt die Umsetzung von Linolsäure durch OtDes6.1. Die Analyse der FAMEs erfolgte über Gaschromatographie. Das gefütterte Substrat (C18:2) wird zu γ-C18:3 umgesetzt. Sowohl Edukt als auch das entstandene Produkt sind durch Pfeile markiert.

In Figur 21 wird die Umsetzung von Linolsäure (= LA) und α-Linolensäure (= ALA) in Gegenwart von OtDes6.1 zu γ-Linolensäure (= GLA) bzw. Stearidonsäure (= STA) wiedergegeben (Figur 21 A und C). Weiterhin zeigt Figur 21 die Umsetzung von Linolsäure (= LA) und α-Linolensäure (= ALA) in Gegenwart der Δ-6-Desaturase OtDes6.1 zusammen mit der Δ-6-Elongase PSE1 aus Physcomitrella patens (Zank et al. 2002, Plant J. 31:255-268) und der Δ-5-Desaturase PtD5 aus Phaeodactylum tricornutum (Domergue et al. 2002, Eur. J. Biochem. 269, 4105-4113) zu Dihomo-γ-linolensäure (= DHGLA) und Arachidonsäure (= ARA, Figur 21 B) bzw. zu Dihomostearidonsäure (= DHSTA) bzw. Eicosapentaensäure (= EPA, Figur 21 D). Figur 21 zeigt deutlich, dass die Reaktionsprodukte GLA und STA der Δ-6-Desaturase OtDes6.1 in Gegenwart der Δ-6-Elongase PSE1 fast quantitativ zu DHGLA bzw. DHSTA elongiert wird. Die nachfolgende Desaturierung durch die Δ-5-Desaturase PtD5 erfolgt ebenfalls reibungslos zu ARA bzw. EPA. Es werden ca. 25 - 30% des Elongaseprodukts desaturiert (Figur 21 B und D).

Die folgenden Tabelle 13 gibt eine Übersiche über die klonierten Ostreococcus Desaturasen wieder:

| Ostreococcus tauri Desaturasen | | | | | | | |
|---|---|---|---|---|---|---|---|
| | | | | | | | |
| **Name** | **bp** | **aa** | **Homologie** | **Cyt. B5** | **His-Box1** | **His-Box2** | **His-Box3** |
| | | | | | | | |
| OtD4 | 1611 | 536 | Δ-4- Desaturase | HPGG | HCANH | WRYHHQVSHH | QVEHHLFP |
| OtD5.1 | 606 | 201 | Δ-5-Desaturase | - | - | - | QVVHHLFP |
| OtD5.2 | 714 | 237 | Δ-5-Desaturase | - | - | WRYHHMVSHH | QIEHHLPF |
| OtD6.1 | 1443 | 480 | Δ-6-Desaturase | HPGG | HEGGH | WNSMHNKHH | QVIHHLFP |
| OtFAD2 | 1086 | 361 | Δ-12-Desaturase | - | HECGH | WQRSHAVHH | HVAHH |

Beispiel: 33 Klonierung von Desaturasegenen aus Thalassiosira pseudonana

Durch Suche nach konservierten Bereichen in den Proteinsequenzen mit Hilfe von konservierten Motiven (His-Boxen, siehe Motive) konnten sechs Sequenzen mit entsprechenden Motiven in einer Thalassiosira pseudonana Sequenzdatenbank (genomische Sequenzen) identifiziert werden. Es handelt sich dabei um die folgenden Sequenzen:

| Gen-Name | SEQ ID | Aminosäuren | Homologie |
|---|---|---|---|
| TpD4 | SEQ ID NO: 103 | 503 | Δ-4-Desaturase |
| TpD5-1 | SEQ ID NO: 99 | 476 | Δ-5-Desaturase |
| TpD5-2 | SEQ ID NO: 101 | 482 | Δ-5-Desaturase |
| TpD6 | SEQ ID NO: 97 | 484 | Δ-6-Desaturase |
| TpFAD2 | SEQ ID NO: 109 | 434 | Δ-12-Desaturase |
| TpO3 | SEQ ID NO: 105 | 418 | ω-3-Desaturase |

Die Klonierung erfolgte wie folgt:
40 ml einer *Thalassiosira pseudonana* Kultur in der stationären Phase wurden abzentrifugiert und in 100 µl Aqua bidest resuspendiert und bei -20°C gelagert. Auf der Basis des PCR-Verfahren wurden die zugehörigen genomischen DNAs amplifiziert. Die entsprechenden Primerpaare wurden so ausgewählt, dass sie die Hefe-Konsensus-Sequenz für hocheffiziente Translation (Kozak, Cell 1986, 44:283-292) neben dem Startcodon trugen. Die Amplifizierung der TpDes-DNAs wurde jeweils mit 1 µl aufgetauten Zellen, 200 µM dNTPs, 2,5 U *Taq*-Polymerase und 100 pmol eines jeden Primers in einem Gesamtvolumen von 50 µl durchgeführt. Die Bedingungen für die PCR waren wie folgt: Erste Denaturierung bei 95°C für 5 Minuten, gefolgt von 30 Zyklen bei 94°C für 30 Sekunden, 55°C für 1 Minute und 72°C für 2 Minuten sowie ein letzter Verlängerungsschritt bei 72°C für 10 Minuten.

Beispiel: 34 Klonierung von Expressionsplasmiden zur heterologen Expression in Hefen:

Zur Charakterisierung der Funktion der Desaturasen aus *Thalassiosira pseudonana* wird der offenen Leserahmen der jeweiligen DNA stromabwärts des Galactose-induzierbaren GAL1-Promotors von pYES2.1/V5-His-TOPO (Invitrogen) kloniert, wobei der entsprechenden pYES2.1-Klone erhalten werden.
Der *Saccharomyces cerevisiae*-Stamm 334 wird durch Elektroporation (1500 V) mit den Vektoren pYES2.1-TpDesaturasen transformiert. Als Kontrolle wird eine Hefe verwendet, die mit dem leeren Vektor pYES2 transformiert wird. Die Selektion der transformierten Hefen erfolgt auf Komplett-Minimalmedium (CMdum)-Agarplatten mit 2% Glucose, aber ohne Uracil. Nach der Selektion werden je drei Transformanten zur weiteren funktionellen Expression ausgewählt.
Für die Expresssion der Tp-Desaturasen werden zunächst Vorkulturen aus jeweils 5 ml CMdum-Flüssigmedium mit 2% (w/v) Raffinose aber ohne Uracil mit den ausgewählten Transformanten angeimpft und 2 Tage bei 30°C, 200 rpm inkubiert.
5 ml CMdum-Flüssigmedium (ohne Uracil) mit 2% Raffinose und 300 µM verschiedener Fettsäuren werden dann mit den Vorkulturen auf eine $OD_{600}$ von 0,05 angeimpft. Die Expression wird durch die Zugabe von 2% (w/v) Galactose induziert. Die Kulturen werden für weitere 96 h bei 20°C inkubiert.

Beispiel: 35 Klonierung von Expressionsplasmiden zur Samen-spezifischen Expression in Pflanzen

Für die Transformation von Pflanzen wird ein weiterer Transformationsvektor auf Basis von pSUN-USP erzeugt. Dazu werden mittels PCR NotI-Schnittstellen am 5' und 3'-Ende der kodierenden Sequenzen eingefügt. Die entsprechenden Primersequenzen werden von den 5'- und 3-Bereich der Desaturasen abgeleitet.
Zusammensetzung des PCR-Ansatzes (50 µL):

5,00 µL Template cDNA
5,00 µL 10x Puffer (Advantage-Polymerase)+ 25mM $MgCl_2$
5,00 µL 2mM dNTP
1,25 µL je Primer (10 pmol/µL)
0,50 µL Advantage-Polymerase

Die Advantage-Polymerase von Clontech wurden eingesetzt.
Reaktionsbedingungen der PCR:

Anlagerungstemperatur: 1 min 55°C
Denaturierungstemperatur: 1 min 94°C
Elongationstemperatur: 2 min 72°C

Anzahl der Zyklen: 35

Die PCR Produkte werden für 16 h bei 37 °C mit dem Restriktionsenzym NotI inkubiert. Der Pflanzen-Expressionsvektor pSUN300-USP wird in gleicherweise inkubiert. Anschliessend werden die PCR Produkte sowie der Vektor durch Agarose-Gelelektrophorese aufgetrennt und die entsprechenden DNA-Fragmente ausgeschnitten. Die Aufreinigung der DNA erfolgt mittels Qiagen Gel Purification Kit gemäss Herstellerangaben. Anschliessend werden Vektor und PCR-Produkte ligiert. Dazu wurde das Rapid Ligation Kit von Roche verwendet. Die entstandenen Plasmide werden durch Sequenzierung verifiziert.
pSUN300 ist ein Derivat des Plasmides pPZP (Hajdukiewicz,P, Svab, Z, Maliga, P., (1994) The small versatile pPZP family of Agrobacterium binary vectors for plant transformation. Plant Mol Biol 25:989-994). pSUN-USP entstand aus pSUN300, indem in pSUN300 ein USP-Promotor als EcoRI- Fragment inseriert wurde. Das Polyadenylierungssignal ist das des OCS-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence and transcript map of the Agrobacterium tumefaciens Ti plasmid-encoded octopine synthase gene J. Mol. Appl. Genet. 1 (6), 499-511 (1982). Der USP-Promotor entspricht den Nukleotiden 1 bis 684 (Genbank Accession X56240), wobei ein Teil der nichtcodierenden Region des USP-Gens im Promotor enthalten ist. Das 684 Basenpaar große Promotorfragment wurde mittels käuflichen T7-Standardprimer (Stratagene) und mit Hilfe eines synthetisierten Primers über eine PCR-Reaktion nach Standardmethoden amplifiziert.

Das PCR-Fragment wurde mit EcoRI/SalI nachgeschnitten und in den Vektor pSUN300 mit OCS Terminator eingesetzt. Es entstand das Plasmid mit der Bezeichnung pSUN-USP.Das Konstrukt wurde zur Transformation von Arabidopsis thaliana, Raps, Tabak und Leinsamen verwendet.

Beispiel: 36 Expression von Tp-Desaturasen in Hefen

Hefen, die wie unter Beispiel 4 mit den Plasmiden pYES2 und pYES2-TpDesaturasen transformiert werden, werden folgendermaßen analysiert:
Die Hefezellen aus den Hauptkulturen werden durch Zentrifugation (100 x g, 5 min, 20°C) geerntet und mit 100 mM $NaHCO_3$, pH 8,0 gewaschen, um restliches Medium und Fettsäuren zu entfernen. Aus den Hefe-Zellsedimenten werden Fettsäuremethylester (FAMEs) durch saure Methanolyse hergestellt. Hierzu werden die Zellsedimente mit 2 ml 1 N methanolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren werden die organischen Phasen je einmal mit 2 ml 100 mM $NaHCO_3$, pH 8,0 und 2 ml Aqua dest. gewaschen. Anschließend werden die PE-Phasen mit $Na_2SO_4$ getrocknet, unter Argon eingedampft und in 100 $\mu$l PE aufgenommen. Die Proben werden auf einer DB-23-Kapillarsäule (30 m, 0,25 mm, 0,25 $\mu$m, Agilent) in einem Hewlett-Packard 6850-Gaschromatographen mit Flammenionisationsdetektor getrennt. Die Bedingungen für die GLC-Analyse sind wie folgt: Die Ofentemperatur wird von 50°C bis 250°C mit einer Rate von 5°C/min und schließlich 10 min bei 250°C(halten) programmiert.

Die Identifikation der Signale erfolgt durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

Beispiel: 37 Funktionelle Charakterisierung von Desaturasen aus Thalassiosira pseudonana:

Die Substratspezifität von Desaturasen kann nach Expression in Hefe (siehe Beispiele Klonierung von Desaturase-Genen, Hefeexpression) durch die Fütterung mittels verschiedener Hefen ermittelt werden. Beschreibungen für die Bestimmung der einzelnen Aktivitäten finden sich in WO 93/11245 für Δ15-Desaturasen, WO 94/11516 für Δ12-Desaturasen, WO 93/06712, US 5,614,393, US5614393, WO 96/21022, WO0021557 und WO 99/27111 für Δ6-Desaturasen, Qiu et al. 2001, J. Biol. Chem. 276, 31561-31566 für Δ4-Desaturasen, Hong et al. 2002, Lipids 37,863-868 für Δ5-Desaturasen.

Die Aktivität der einzelnen Desaturasen wird aus der Konversionsrate errechnet nach der Formel [Substrat/(Substrat+Produkt)*100].

Die folgenden Tabellen 11 und 12 geben eine Übersicht über die clonierten Thalassiosira pseudonana Desaturasen wieder.

Tabelle 14: Länge und charakteristische Merkmale der clonierten Thalassiosira Desaturasen.

| Desaturase | cDNA (bp) | Protein (aa) | Cyt. B5 | His-Box1 | His-Box2 | His-Box3 |
|---|---|---|---|---|---|---|
| | | | | | | |

(fortgesetzt)

| Desaturase | cDNA (bp) | Protein (aa) | Cyt. B5 | His-Box1 | His-Box2 | His-Box3 |
|---|---|---|---|---|---|---|
| TpD4 | 1512 | 503 | HPGG | HDGNH | WELQHMLGHH | QIEHHLFP |
| TpD5-1 | 1431 | 476 | HPGG | HDANH | WMAQHWTHH | QVEHHLFP |
| TpD5-2 | 1443 | 482 | HPGG | HDANH | WLAQHWTHH | QVEHHLFP |
| TpD6 | 1449 | 484 | HPGG | HDFLH | WKNKHNGHH | QVDHHLFP |
| TpFAD2 (d12) | 1305 | 434 | - | HECGH | HAKHH | HVAHHLFH |
| TpO3 | 1257 | 419 | - | HDAGH | WLFMVTYLQH H | HVVHHLF |

Tabelle 15: Länge, Exons, Homolgie und Identitäten der clonierten Desaturasen.

| Des. | GDN A (bp) | Exon 1 | Exon 2 | First Blast Hit | Hom./Iden. |
|---|---|---|---|---|---|
| | | | | | |
| TpD4 | 2633 | 496-1314 | 1571-2260 | Thrautochitrium D4-des | 56% / 43% |
| TpD5-1 | 2630 | 490-800 | 900-2019 | Phaeodactylum D5-des | 74% / 62% |
| TpD5-2 | 2643 | 532-765 | 854-2068 | Phaeodactylum D5-des | 72%/61% |
| TpD6 | 2371 | 379-480 | 630-1982 | Phaeodactylum D6-des | 83% / 69% |
| TpFAD2 | 2667 | 728-2032 | - | Phaeodactylum FAD2 | 76% / 61 % |
| TpO3 | 2402 | 403-988 | 1073-1743 | Chaenorhabdidis Fad2 | 49% / 28% |

Analog zu den vorgenannten Beispielen lassen sich auch die Δ-12-Desaturasegene aus Ostreococcus und Thalassiosira clonieren.

Beispiel 38 Klonierung von Elongase Genen aus Xenopus laevis und Ciona intestinalis

Durch Suche nach konservierten Bereichen (siehe Konsensus-Sequenzen, SEQ ID NO: 115 und SEQ ID NO: 116) in den Proteinsequenzen in Gendatenbanken (Genbank) mit Hilfe der in der Anmeldung aufgeführten Elongase-Gene mit Δ-5-Elongaseaktivität oder Δ-6-Elongaseaktivität konnten weitere Elongasesequenzen aus anderen Organismen identifiziert und isoliert werden. Aus X. laevis bzw. aus C. intestinalis konnten mit entsprechenden Motiven jeweils weitere Sequenzen identifiziert werden. Es handelt sich dabei um die folgenden Sequenzen:

| Gen-Name | Organismus | Genbank-Nr. | SEQ ID NO: | Aminosäuren |
|---|---|---|---|---|
| ELO(XI) | Xenopus laevis | BC044967 | 117 | 303 |
| ELO(Ci) | Ciona intestinalis | AK112719 | 119 | 290 |

Der cDNA Klon von X. laevis wurde vom NIH (National Institut of Health) bezogen [Genetic and genomic tools for Xenopus research: The NIH Xenopus initiative, Dev. Dyn. 225 (4), 384-391 (2002)].

Der cDNA Klon von C. inetstinalis wurde von der Universität von Kyto bezogen [Satou,Y., Yamada,L., Mochizuki,Y., Takatori,N., Kawashima,T., Sasaki,A., Hamaguchi,M., Awazu,S., Yagi,K., Sasakura,Y., Nakayama,A., Ishikawa,H., Inaba,K. and Satoh,N. "A cDNA resource from the basal chordate Ciona intestinalis" JOURNAL Genesis 33 (4), 153-154 (2002)].

Beispiel 39: Klonierung von Expressionsplasmiden zur heterologen Expression in - Hefen

Die Amplifizierung der Elongase-DNAs wurde jeweils mit 1 μL cDNA, 200 μM dNTPs, 2,5 U Advantage-Polymerase und 100 pmol eines jeden Primers in einem Gesamtvolumen von 50 μl durchgeführt. Die Bedingungen für die PCR waren wie folgt: Erste Denaturierung bei 95°C für 5 Minuten, gefolgt von 30 Zyklen bei 94°C für 30 Sekunden, 55°C für 1 Minute und 72°C für 2 Minuten sowie ein letzter Verlängerungsschritt bei 72°C für 10 Minuten.

Für die Klonierung der Sequenz zur heterologen Expression in Hefen wurden folgende Oligonukleotide für die PCR-Reaktion verwendet:

*F=forward primer, R=reverse primer

| Gen-Name und SEQ ID NO: | Primersequenz |
|---|---|
| ELO(XI) SEQ ID NO: 121<br>SEQ ID NO: 122 | F:5'- AGGATCCATGGCCTTCAAGGAGCTCACATC<br>R:5'- CCTCGAGTCAATGGTTTTTGCTTTTCAAT-GCACCG |
| ELO(Ci), SEQ ID NO: 123<br>SEQ ID NO: 124 | F:5'- TAAGCTTATGGACGTACTTCATCGT<br>R:5'- TCAGATCTTTAATCGGTTTTACCATT |

Die PCR Produkte wurde für 30 min bei 21 °C mit dem Hefe-Expressionsvektor - pYES2.1-TOPO (Invitrogen) gemäß Herstellerangaben inkubiert. Das PCR-Produkt wird dabei durch einen T-Überhang und Aktivität einer Topoisomerase (Invitrogen) nach Herstellerangaben in den Vektor ligiert. Nach der Inkubation erfolgte dann die Transformation von E. coli DH5a Zellen. Entsprechende Klone wurden durch PCR - identifiziert, die Plasmid-DNA mittels Qiagen DNAeasy-Kit isoliert und durch Sequenzierung verifiziert. Die korrekte Sequenz wurde dann in den Saccharomyces Stamm INVSc1 (Invitrogen) durch Elektroporation (1500 V) transformiert. Zur Kontrolle wurde der leere Vektor pYES2.1 parallel transformiert. Anschließend wurden die Hefen auf Komplett-Minimalmedium ohne Uracil mit 2 % Glucose ausplattiert. Zellen, die ohne Uracil im Medium wachstumsfähig waren, enthalten damit die entsprechenden Plasmide pYES2.1, pYES2.1-ELO(XI) und pYES2.1-ELO(Ci). Nach der Selektion wurden je zwei Transformaten zur weiteren funktionellen Expression ausgewählt.

Beispiel 40: Klonierung von Expressionsplasmiden zur Samen-spezifischen Expression in Pflanzen

Für die Transformation von Pflanzen wird ein weiterer Transformationsvektor auf Basis von pSUN-USP erzeugt. Dazu werden mit folgendem Primerpaar NotI-Schnittstellen am 5' und 3'-Ende der kodierenden Sequenz eingefügt:.

pSUN-ELO(XI)

Forward: 5'-GCGGCCGCACCATGGCCTTCAAGGAGCTCACATC (SEQ ID NO: 125)
Reverse: 3'-GCGGCCGCCTTCAATGGTTTTTGCTTTTCAATGCACCG (SEQ ID NO: 126)

pSUN-ELO(Ci)

Forward: 5'-GCGGCCGCACCATGGACGTACTTCATCGT (SEQ ID NO: 127)
Reverse: 3'-GCGGCCGCTTTAATCGGTTTTACCATT (SEQ ID NO: 128)

Zusammensetzung des PCR-Ansatzes (50 µL):

5,00 µL Template cDNA
5,00 µL 10x Puffer (Advantage-Polymerase)+ 25mM MgCl2
5,00 µL 2mM dNTP
1,25 µL je Primer (10 pmol/µL)
0,50 µL Advantage-Polymerase

Die Advantage-Polymerase von Clontech wurden eingesetzt.
Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Die PCR Produkte wurden für 16 h bei 37 °C mit dem Restriktionsenzym NotI inkubiert. Der Pflanzen-Expressionsvektor pSUN300-USP wurde in gleicherweise inkubiert. Anschliessend wurden die PCR Produkte sowie der 7624

bp große Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkte ligiert. Dazu wurde das Rapid Ligation Kit von Roche verwendet. Die entstandenen Plasmide pSUN-ELO(XI) und pSUN-ELO(Ci) wurden durch Sequenzierung verifiziert.
pSUN300 ist ein Derivat des Plasmides pPZP [Hajdukiewicz,P, Svab, Z, Maliga, P., (1994) The small versatile pPZP family of Agrobacterium binary vectors forplant transformation. Plant Mol Biol 25:989-994]. pSUN-USP entstand aus pSUN300, indem in pSUN300 ein USP-Promotor als EcoRI- Fragment inseriert wurde. Das Polyadenylierungssignal ist das des Octopinsynthase-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence and transcript map of the Agrobacterium tumefaciens Ti plasmid-encoded octopine synthase gene J. Mol. Appl. Genet. 1 (6), 499-511 (1982) Der USP-Promotor entspricht den Nukleotiden 1-684 (Genbank Accession X56240), wobei ein Teil der nichtcodierenden Region des USP-Gens im Promotor enthalten ist. Das 684 Basenpaar große Promotorfragment wurde mittels käuflichen T7-Standardprimer (Stratagene) und mit Hilfe eines synthetisierten Primers über eine PCR-Reaktion nach Standardmethoden amplifiziert.

Primersequenz: 5'–
GTCGACCCGCGGACTAGTGGGCCCTCTAGACCCGGGGGATCC
GGATCTGCTGGCTATGAA–3' (SEQ ID NO: 129).

Das PCR-Fragment wurde mit EcoRI/SalI nachgeschnitten und in den Vektor pSUN300 mit OCS Terminator eingesetzt. Es entstand das Plasmid mit der Bezeichnung pSUN-USP. Das Konstrukt wurde zur Transformation von Arabidopsis thaliana, Raps, Tabak und Leinsamen verwendet.
Die Lipidextraktion aus Hefen und Samen erfolgte identisch zu Beispiel 6.

Beispiel 41: Expression von ELO(XI) und ELO(Ci) in Hefen

Hefen, die wie unter Beispiel 4 mit den Plasmiden pYES2, pYES2-ELO(XI) und pYES2-ELO(Ci) transformiert wurden, wurden folgendermaßen analysiert:
Die Hefezellen aus den Hauptkulturen wurden durch Zentrifugation (100 x g, 5 min, 20°C) geerntet und mit 100 mM $NaHCO_3$, pH 8,0 gewaschen, um restliches Medium und Fettsäuren zu entfernen. Aus den Hefe-Zellsedimenten wurden Fettsäuremethylester (FAMEs) durch saure Methanolyse hergestellt. Hierzu wurden die Zellsedimente mit 2 ml 1 N methanolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM $NaHCO_3$, pH 8,0 und 2 ml Aqua dest. gewaschen. Anschließend wurden die PE-Phasen mit $Na_2SO_4$ getrocknet, unter Argon eingedampft und in 100 $\mu$l PE aufgenommen. Die Proben wurden auf einer DB-23-Kapillarsäule (30 m, 0,25 mm, 0,25 $\mu$m, Agilent) in einem Hewlett-Packard 6850-Gaschromatographen mit Flammenionisationsdetektor getrennt. Die Bedingungen für die GLC-Analyse waren wie folgt: Die Ofentemperatur wurde von 50°C bis 250°C mit einer Rate von 5°C/min und schließlich 10 min bei 250°C(halten) programmiert.
Die Identifikation der Signale erfolgte durch Vergleiche der Retentionszeiten mit entsprechenden Fettsäurestandards (Sigma). Die Methodik ist beschrieben zum Beispiel in Napier and Michaelson, 2001,Lipids. 36(8): 761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

Beispiel 42: Funktionelle Charakterisierung von ELO(XI) und ELO(Ci):

Die Substratspezifität der ELO(XI) konnte nach Expression und Fütterung verschiedener Fettsäuren ermittelt werden (Fig. 22). Die gefütterten Substrate sind in großen Mengen in allen transgenen Hefen nachzuweisen. Die transgenen Hefen zeigten die Synthese neuer Fettsäuren, den Produkten der ELO(XI)-Reaktion. Dies bedeutet, dass das Gen ELO(XI) funktional exprimiert werden konnte.
Tabelle 16 zeigt, dass die ELO(XI) eine breite Substratspezifität aufweist. Es werden sowohl C18 als auch C20 Fettsäuren verlängert, wobei ein Bevorzugung von $\Delta$5- und $\Delta$6-desaturierten Fettsäuren zu beobachten ist.
Die Hefen, die mit dem Vektor pYES2-ELO(XI) transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert.

Tabelle 16: Expression von ELO(XI) in Hefe. Beschrieben ist die Umsetzungsrate (Konversionsrate) verschiedener Edukte (gefüttert jeweils 250 μM).

| Edukte | Konversion der Edukte durch ELO(XI) in % |
|---|---|
| 16:0 | 3 |
| $16:1^{\Delta 9}$ | 0 |
| 18:0 | 2 |
| $18:1^{\Delta 9}$ | 0 |
| $18:2^{\Delta 9,12}$ | 3 |
| $18:3^{\Delta 6,9,12}$ | 12 |
| $18:3^{\Delta 5,9,12}$ | 13 |
| $18:3^{\Delta 9,12,15}$ | 3 |
| $18:4^{\Delta 6,9,12,15}$ | 20 |
| $20:3^{\Delta 8,11,14}$ | 5 |
| $20:3^{\Delta 11,14,17}$ | 13 |
| $20:4^{\Delta 5,8,11,14}$ | 15 |
| $20:5^{\Delta 5,8,11,14,17}$ | 10 |
| $22:4^{\Delta 7,10,13,16}$ | 0 |
| $22:6^{\Delta 4,7,10,13,16,19}$ | 0 |

Die Substratspezifität der ELO(Ci) konnte nach Expression und Fütterung verschiedener Fettsäuren ermittelt werden (Fig. 23). Die gefütterten Substrate sind in großen Mengen in allen transgenen Hefen nachzuweisen. Die transgenen Hefen zeigten die Synthese neuer Fettsäuren, den Produkten der ELO(Ci)-Reaktion. Dies bedeutet, dass das Gen ELO(Ci) funktional exprimiert werden konnte.

Tabelle 17: Expression von ELO(Ci) in Hefe. Beschrieben ist die Umsetzungsrate (Konversionsrate) verschiedener Edukte (gefüttert jeweils 250 μM).

| Edukte | Konversion der Edukte durch ELO(Ci) in % |
|---|---|
| 16:0 | 0 |
| $16:1^{\Delta 9}$ | 0 |
| 18:0 | 0 |
| $18:1^{\Delta 9}$ | 0 |
| $18^{2\Delta 9,12}$ | 23 |
| $18:3^{\Delta 6,9,12}$ | 10 |
| $18:3^{\Delta 5,9,12}$ | 38 |
| $18:3^{\Delta 9,12,15}$ | 25 |
| $18:4^{\Delta 6,9,12,15}$ | 3 |
| $20:3^{\Delta 8,11,14}$ | 10 |
| $20:3^{\Delta 11,14,17}$ | 8 |
| 20:4Δ5,8,11,14 | 10 |
| 20:5Δ5,8,11,14,17 | 15 |
| 22:4Δ7,10,13,16 | 0 |
| 22:6Δ4,7,10,13,16,19 | 0 |

Tabelle 17 zeigt, dass die ELO(Ci) eine breite Substratspezifität aufweist. Es werden sowohl C18 als auch C20 Fettsäuren verlängert, wobei ein Bevorzugung von Δ5- und Δ6-desaturierten Fettsäuren zu beobachten ist.

Die Hefen, die mit dem Vektor pYES2-ELO(Ci) transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert.

Beispiel 43: Klonierung von Genen aus Ostreococcus tauri

Durch Suche nach konservierten Bereichen in den Proteinsequenzen mit Hilfe der hierin beschriebenen Elongase-Gene mit Δ-5-Elongaseaktivität oder Δ-6-Elongaseaktivität konnten je zwei Sequenzen mit entsprechenden Motiven in einer Ostreococcus tauri Sequenzdatenbank (genomische Sequenzen) identifiziert werden. Es handelt sich dabei um die folgenden Sequenzen:

| Gen-Name | SEQ ID | Aminosäuren |
|---|---|---|
| OtELO1, (Δ-5-Elongase) | SEQ ID NO: 67 | 300 |
| OtELO1.2, (Δ-5-Elongase) | SEQ ID NO: 113 | 300 |
| OtELO2, (Δ-6-Elongase) | SEQ ID NO: 69 | 292 |
| OtELO2.1, (Δ-6-Elongase) | SEQ ID NO: 111 | 292 |

OtElo1 und OtElo1.2 weisen die höchste Ähnlichkeit zu einer Elongase aus Danio rerio auf (GenBank AAN77156; ca. 26 % Identität), während OtElo2 und OtElo2.1 die größte Ähnlichkeit zur Physcomitrella Elo (PSE) [ca. 36 % Identität] aufweisen (Alignments wurden mit dem tBLASTn-Aalgorithmus (Altschul et al., J. Mol. Biol. 1990, 215: 403 - 410) durchgeführt.

Die Klonierung der Elongasen wurde wie folgt durchgeführt:
40 ml einer *Ostreococcus tauri* Kultur in der stationären Phase wurden abzentrifugiert und in 100 μl Aqua bidest resuspendiert und bei -20°C gelagert. Auf der Basis des PCR-Verfahren wurden die zugehörigen genomischen DNAs amplifiziert. Die entsprechenden Primerpaare wurden so ausgewählt, dass sie die Hefe-Konsensus-Sequenz für hocheffiziente Translation (Kozak, Cell 1986, 44:283-292) neben dem Startcodon trugen. Die Amplifizierung der OtElo-DNAs wurde jeweils mit 1 μl aufgetauten Zellen, 200 μM dNTPs, 2,5 U *Taq*-Polymerase und 100 pmol eines jeden Primers in einem Gesamtvolumen von 50 μl durchgeführt. Die Bedingungen für die PCR waren wie folgt: Erste Denaturierung bei 95°C für 5 Minuten, gefolgt von 30 Zyklen bei 94°C für 30 Sekunden, 55°C für 1 Minute und 72°C für 2 Minuten sowie ein letzter Verlängerungsschritt bei 72°C für 10 Minuten.

Beispiel 44: Klonierung von Expressionsplasmiden zur heterologen Expression in Hefen:

Zur Charakterisierung der Funktion der Elongasen aus *Ostreococcus tauri* wurden die offenen Leserahmen der jeweiligen DNAs stromabwärts des Galactose-induzierbaren GAL1-Promotors von pYES2.1/V5-His-TOPO (Invitrogen) kloniert, wobei pOTE1, pOTE1.2, pOTE2 und pOTE2.1 erhalten wurden.

Der *Saccharomyces cerevisiae*-Stamm 334 wurde durch Elektroporation (1500 V) mit dem Vektor pOTE1, pOTE1.2, pOTE2 bzw. pOTE2.1 transformiert. Als Kontrolle wurde eine Hefe verwendet, die mit dem leeren Vektor pYES2 transformiert wurde. Die Selektion der transformierten Hefen erfolgte auf Komplett-Minimalmedium (CMdum)-Agarplatten mit 2% Glucose, aber ohne Uracil. Nach der Selektion wurden je drei Transformanten zur weiteren funktionellen Expression ausgewählt.

Für die Expresssion der Ot-Elongasen wurden zunächst Vorkulturen aus jeweils 5 ml CMdum-Flüssigmedium mit 2% (w/v) Raffinose aber ohne Uracil mit den ausgewählten Transformanten angeimpft und 2 Tage bei 30°C, 200 rpm inkubiert.

5 ml CMdum-Flüssigmedium (ohne Uracil) mit 2% Raffinose und 300 μM verschiedener Fettsäuren wurden dann mit den Vorkulturen auf eine $OD_{600}$ von 0,05 angeimpft. Die Expression wurde durch die Zugabe von 2% (w/v) Galactose induziert. Die Kulturen wurden für weitere 96 h bei 20°C inkubiert.

Beispiel 45: Klonierung von Expressionsplasmiden zur Samen-spezifischen Expression in Pflanzen

Für die Transformation von Pflanzen wurde ein weiterer Transformationsvektor auf Basis von pSUN-USP erzeugt. Dazu wurden mittels PCR NotI-Schnittstellen am 5' und 3'-Ende der kodierenden Sequenzen eingefügt. Die entsprechenden Primersequenzen wurden von den 5'- und 3-Bereich von OtElo1, OtElo1.2, OtElo2 und OtElo2.1 abgeleitet.

Zusammensetzung des PCR-Ansatzes (50 μL):

5,00 μL Template cDNA
5,00 μL 10x Puffer (Advantage-Polymerase)+ 25mM $MgCl_2$
5,00 μL 2mM dNTP
1,25 μL je Primer (10 pmol/μL)
0,50 μL Advantage-Polymerase

Die Advantage-Polymerase von Clontech wurden eingesetzt.

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Die PCR Produkte werden für 16 h bei 37 °C mit dem Restriktionsenzym NotI inkubiert. Der Pflanzen-Expressionsvektor pSUN300-USP wird in gleicherweise inkubiert. Anschließend wurden die PCR Produkte sowie der Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschliessend wurden Vektor und PCR-Produkte ligiert. Dazu wurde das Rapid Ligation Kit von Roche verwendet. Die entstandenen Plasmide pSUN-OtEL01, pSUN-OtELO1.2, pSUN-OtELO2 und pSUN-OtELO2.2 wurden durch Sequenzierung verifiziert.
pSUN300 ist ein Derivat des Plasmides pPZP [Hajdukiewicz, P, Svab, Z, Maliga, P., (1994) The small versatile pPZP family of Agrobacterium binary vectors for plant transformation. Plant Mol Biol 25:989-994]. pSUN-USP entstand aus pSUN300, indem in pSUN300 ein USP-Promotor als EcoRI- Fragment inseriert wurde. Das Polyadenylierungssignal ist das des Ostreococcus-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence and transcript map of the Agrobacterium tumefaciens Ti plasmid-encoded octopine synthase gene J. Mol. Appl. Genet. 1 (6), 499-511 (1982). Der USP-Promotor entspricht den Nukleotiden 1 bis 684 (Genbank Accession X56240), wobei ein Teil der nichtcodierenden Region des USP-Gens im Promotor enthalten ist. Das 684 Basenpaar große Promotorfragment wurde mittels käuflichen T7-Standardprimer (Stratagene) und mit Hilfe eines synthetisierten Primers über eine PCR-Reaktion nach Standardmethoden amplifiziert.

Primersequenz:

5'–GTCGACCCGCGGACTAGTGGGCCCTCTAGACCCGGGGGATCC
GGATCTGCTGGCTATGAA–3'). (SEQ ID NO: 130)

Das PCR-Fragment wurde mit EcoRI/SalI nachgeschnitten und in den Vektor pSUN300 mit OCS Terminator eingesetzt. Es entstand das Plasmid mit der Bezeichnung pSUN-USP. Das Konstrukt wurde zur Transformation von Arabidopsis thaliana, Raps, Tabak und Leinsamen verwendet.

Beispiel 46: Expression von OtElo1, OtElo1.2, OtElo2 und OtELO2.2 in Hefen

Hefen, die wie unter Beispiel 15 mit den Plasmiden pYES3, pYES3-OtELO1, pYES3-OtELO1.2, pYES3-OtELO2 und pYES3-OtELO2.2 transformiert wurden, wurden folgendermaßen analysiert:
Die Hefezellen aus den Hauptkulturen wurden durch Zentrifugation (100 x g, 5 min, 20°C) geerntet und mit 100 mM $NaHCO_3$, pH 8,0 gewaschen, um restliches Medium und Fettsäuren zu entfernen. Aus den Hefe-Zellsedimenten wurden Fettsäuremethylester (FAMEs) durch saure Methanolyse hergestellt. Hierzu wurden die Zellsedimente mit 2 ml 1 N methanolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM $NaHCO_3$, pH 8,0 und 2 ml Aqua dest. gewaschen. Anschließend wurden die PE-Phasen mit $Na_2SO_4$ getrocknet, unter Argon eingedampft und in 100 μl PE aufgenommen. Die Proben wurden auf einer DB-23-Kapillarsäule (30 m, 0,25 mm, 0,25 μm, Agilent) in einem Hewlett-Packard 6850-Gaschromatographen mit Flammenionisationsdetektor getrennt. Die Bedingungen für die GLC-Analyse waren wie folgt: Die Ofentemperatur wurde von 50°C bis 250°C mit einer Rate von 5°C/min und schließlich 10 min bei 250°C (halten) programmiert.

Die Identifikation der Signale erfolgte durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

Beispiel 47: Funktionelle Charakterisierung von OtElo1, OtElo1.2, OtElo2 und OtElo2.1 :

Die Substratspezifität der OtElo1 konnte nach Expression und Fütterung verschiedener Fettsäuren ermittelt werden (Tab. 18). Die gefütterten Substrate sind in großen Mengen in allen transgenen Hefen nachzuweisen. Die transgenen Hefen zeigten die Synthese neuer Fettsäuren, den Produkten der OtElo1-Reaktion. Dies bedeutet, dass das Gen OtElo1 funktional exprimiert werden konnte.

Tabelle 18 zeigt, dass OtElo1 bzw. OtElo1.2 eine enge Substratspezifität aufweist. OtElo1 bzw. OtElo1.2 konnte nur die C20-Fettsäuren Eicosapentaensäure (Figur 24A, 24B) und Arachidonsäure (Figur 25A, 25B) elongieren, bevorzugte aber die ω-3-desaturierte Eicosapentaensäure.

Tabelle 18 zeigt die Substratspezifität der Elongase OtElo1 und OtElo1.2 für C20 polyungesättigte Fettsäuren mit einer Doppelbindung in Δ5 Position gegenüber verschiedenen Fettsäuren.

Die Hefen, die mit dem Vektor pOTE1 bzw. pOTE1.2 transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert.

Die Substratspezifität der OtElo2 (SEQ ID NO: 81) OtElo2.1 (SEQ ID NO: 111) konnte nach Expression und Fütterung verschiedener Fettsäuren ermittelt werden (Tab. 19). Die gefütterten Substrate sind in großen Mengen in allen transgenen Hefen nachzuweisen. Die transgenen Hefen zeigten die Synthese neuer Fettsäuren, den Produkten der OtElo2-Reaktion. Dies bedeutet, dass die Gene OtElo2 und OtElo2.1 funktional exprimiert werden konnte.

Tabelle 18:

| Fettsäuresubstrat | Umsatz (in %) OtElo1 | Umsatz (in %) OtElo1.2 |
|---|---|---|
| 16:0$^{\square}$ | - | - |
| 16:1$^{\square\Delta 9}$ | - | - |
| 18:0$^{\square}$ | - | - |
| 18:1$^{\square\Delta 9}$ | - | - |
| 18:1$^{\Delta 11}$ | - | - |
| 18:2$^{\Delta 912}$ | - | - |
| 18:3$^{\square 6,9,12}$ | - | - |
| 18:3$^{\square\Delta 5,9,12}$ | - | - |
| 20:3 $^{\Delta 8,11,14}$ | - | - |
| 20:4 $^{\Delta 5,\square 8,11,14}$ | **10,8 ± 0,6** | **38,0** |
| 20:5 $^{\Delta 5,\square 8,11,14,17}$ | **46,8 ± 3,6** | **68,6** |
| 22:4 $^{\Delta 7,\square 10,13,16}$ | - | - |
| 22:6$^{\square 4,7,10,13,16,19}$ | - | - |

Tabelle 19 zeigt die Substratspezifität der Elongase OtElo2 und OtElo2.1 gegenüber verschiedenen Fettsäuren. OtElo2.1 zeigt eine deutlich höhere Aktivität.

Die Hefen, die mit dem Vektor pOTE2 bzw. pOTE2.1 transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert.

Die enzymatische Aktivität, die in Tabelle 19 wiedergegeben wird, zeigt klar, dass OtElo2 bzw. OtElo2.1 eine Δ-6-Elongase ist.

Tabelle 19:

| Fettsäuresubstrat | Umsatz (in %) OtElo2 | Umsatz (in %)OtELO2.2 |
|---|---|---|
| 16:0 | - | - |

(fortgesetzt)

| **Fettsäuresubstrat** | **Umsatz (in %) OtElo2** | **Umsatz (in %)OtELO2.2** |
|---|---|---|
| 16:1$^{\square\Delta 9}$ | - | - |
| 16:3$^{\square\Delta 7,10,13}$ | - | - |
| 18:0$^{\square}$ | - | - |
| 18:1$^{\square\Delta 6}$ | - | - |
| 18:1$^{\square\Delta 9}$ | - | - |
| 18:1$^{\square\Delta 11}$ | - | - |
| 18:2$^{\square\Delta 9,12}$ | - | - |
| 18:3$^{\Delta 6,9,12}$ | **15,3** | **55,7** |
| 18:3$^{\square\ \Delta 5,9,12}$ | - | - |
| 18:4$^{\square\ \Delta 6,9,12,15}$ | **21,1** | **70,4** |
| 20:2 $^{\Delta 11,14}$ | - | - |
| 20:3 $^{\Delta 8,11,14}$ | - | - |
| 20:4 $^{\Delta 5,\square 8,11,14}$ | - | - |
| 20:5 $^{\Delta 5,\square 8,11,14,17}$ | - | - |
| 22:4 $^{\Delta 7,\square 10,13,16}$ | - | - |
| 22:5 $^{\Delta 7,\square 10,13,16,19}$ | - | - |
| 22:6$^{\square 4,7,10,13,16,19}$ | - | - |

Figur 24 A - D zeigt die Elongation von Eicosapentaensäure durch OtElo1 (B) bzw.

OtElo1.2 (D). Die Kontrollen (A, C) zeigen nicht das Produkt der Elongation (22:5ω3).

Figur 25 A - D zeigt die Elongation von Arachidonsäure durch OtElo1 (B) bzw.

OtElo1.2 (D). Die Kontrollen (A, C) zeigen nicht das Produkt der Elongation (22:4ω6).

Beispiel 48: Klonierung von Elongase-Genen aus Euglena gracilis und Arabidopsis thaliana

Durch Suche nach konservierten Bereichen in den Proteinsequenzen mit Hilfe der in der Anmeldung aufgeführten Elongase-Gene mit Δ-5-Elongaseaktivität oder Δ-6-Elongaseaktivität konnten Sequenzen aus Arabidopsis thaliana bzw. Euglena gracilis mit entsprechenden Motiven in Sequenzdatenbanken (Genbank, Euglena EST Bank) identifiziert werden. Es handelt sich dabei um die folgenden Sequenzen:

| Gen-Name | SEQ ID | Aminosäuren |
|---|---|---|
| EGY1019 (E. gracilis) | SEQ ID NO: 131 | 262 |
| EGY2019 (E. gracilis) | SEQ ID NO: 133 | 262 |
| At3g06460 (A. thaliana) | SEQ ID NO: 135 | 298 |
| At3g06470 (A. thaliana) | SEQ ID NO: 137 | 278 |

Die Klonierung der Elongasen aus Euglena gracilis wurden wie folgt durchgeführt:
Der Euglena gracilis Stamm 1224-5/25 wurde erhalten von der Sammlung für Algenkulturen Göttingen (SAG). Zur Isolierung wurde der Stamm in Medium II (Calvayrac R and Douce R, FEBS Letters 7:259-262, 1970) für 4 Tage bei 23 °C unter einem Licht-/ Dunkelintervall von 8 h / 16 h (35 mol s-1 m-2 Lichtstärke) angezogen.

Gesamt-RNA von einer viertägigen Euglena Kultur wurde mit Hilfe des RNAeasy Kits der Firma Qiagen (Valencia, CA, US) isoliert. Aus der Gesamt-RNA wurde mit Hilfe von oligo-dT-Cellulose poly-A+ RNA (mRNA) isoliert (Sambrook et al., 1989). Die RNA wurde mit dem Reverse Transcription System Kit von Promega revers transcribiert und die synthetisierte cDNA in den lambda ZAP Vektor (lambda ZAP Gold, Stratagene) kloniert. Entsprechend der

Herstellerangaben wurde die cDNA zur Plasmid-DNA entpackt und Klone wurden zur Zufallssequenzierung ansequenziert. Aus der Gesamt-RNA wurde mit Hilfe des PolyATract Isolierungssystems (Promega) mRNA isoliert. Die mRNA wurde mit dem Marathon cDNA Amplification-Kit (BD Biosciences) reverse transkribiert und entsprechend der Herstellerangaben wurden die Adaptoren ligiert. Die cDNA-Bank wurde dann für die PCR zur Klonierung von Expressionsplasmiden mittels 5'- und 3'-RACE (rapid amplification of cDNA ends) verwendet.

Die Klonierung der Elongasen aus Arabidopsis thaliana wurde wie folgt durchgeführt:
Ausgehend von der genomischen DNA wurden für die beiden Gene Primer entsprechend am 5'- und 3'-Ende des offenen Leserahmens abgeleitet.

Zur Isolierung von Gesamt-RNA aus *A. thaliana* wurde nach Chrigwin *et al.,* (1979) verfahren. Blätter von 21 Tage alten Pflanzen wurden in flüssigem Stickstoff zermörsert, mit Aufschlusspuffer versetzt und für 15 min bei 37 ∘C inkubiert. Nach Zentrifugation (10 min, 4 oC, 12000xg) wurde die RNA im Überstand mit 0,02 Volumen 3 M Natriumacetat pH 5,0 und 0,75 Volumen Ethanol bei -20 oC für 5 h präzipitiert. Die RNA wurde dann nach einem weiteren Zentrifugationsschritt in 1 mL TES pro g Ausgangsmaterial aufgenommen, einmal mit einem Volumen Phenol-Chloroform und einmal mit einem Volumen Chloroform extrahiert und die RNA mit 2,5 M LiCl gefällt. Nach anschliessendem Zentrifugieren und Waschen mit 80 %igem Ethanol wurde die RNA in Wasser resuspendiert. Entsprechend Sambrook et al. 1989 wurde die cDNA synthetisiert und RT-PCR mit den abgeleiteten Primer durchgeführt. Die PCR-Produkte wurden nach Herstellerangaben in den Vektor pYES2.1-TOPO (Invitrogen) kloniert.

Beispiel 49: Klonierung von Expressionsplasmiden zur heterologen Expression in Hefen:

Zur Charakterisierung der Funktion der Elongasen aus *A. thalina* wurden die offenen Leserahmen der jeweiligen DNAs stromabwärts des Galactose-induzierbaren GAL1-Promotors von pYES2.1/V5-His-TOPO (Invitrogen) kloniert, wobei pAt60 und pAt70 erhalten wurden.

Der *Saccharomyces cerevisiae*-Stamm 334 wurde durch Elektroporation (1500 V) mit dem Vektor pAt60 bzw. pAt70 transformiert. Als Kontrolle wurde eine Hefe verwendet, die mit dem leeren Vektor pYES2.1 transformiert wurde. Die Selektion der transformierten Hefen erfolgte auf Komplett-Minimalmedium (CMdum)-Agarplatten mit 2% Glucose, aber ohne Uracil. Nach der Selektion wurden je drei Transformanten zur weiteren funktionellen Expression ausgewählt.

Für die Expresssion der At-Elongasen wurden zunächst Vorkulturen aus jeweils 5 ml CMdum-Flüssigmedium mit 2% (w/v) Raffinose aber ohne Uracil mit den ausgewählten Transformanten angeimpft und 2 Tage bei 30°C, 200 rpm inkubiert.

5 ml CMdum-Flüssigmedium (ohne Uracil) mit 2% Raffinose und 300 μM verschiedener Fettsäuren wurden dann mit den Vorkulturen auf eine $OD_{600}$ von 0,05 angeimpft. Die Expression wurde durch die Zugabe von 2% (w/v) Galactose induziert. Die Kulturen wurden für weitere 96 h bei 20°C inkubiert.

Beispiel 50: Expression von pAt60 und pAt70 in Hefen

Hefen, die wie unter Beispiel 5 mit den Plasmiden pYES2.1, pAt60 bzw. pAt70 transformiert wurden, wurden folgendermaßen analysiert:
Die Hefezellen aus den Hauptkulturen wurden durch Zentrifugation (100 x g, 5 min, 20°C) geerntet und mit 100 mM $NaHCO_3$, pH 8,0 gewaschen, um restliches Medium und Fettsäuren zu entfernen. Aus den Hefe-Zellsedimenten wurden Fettsäuremethylester (FAMEs) durch saure Methanolyse hergestellt. Hierzu wurden die Zellsedimente mit 2 ml 1 N methanolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM $NaHCO_3$, pH 8,0 und 2 ml Aqua dest. gewaschen. Anschließend wurden die PE-Phasen mit $Na_2SO_4$ getrocknet, unter Argon eingedampft und in 100 μl PE aufgenommen. Die Proben wurden auf einer DB-23-Kapillarsäule (30 m, 0,25 mm, 0,25 μm, Agilent) in einem Hewlett-Packard 6850-Gaschromatographen mit Flammenionisationsdetektor getrennt. Die Bedingungen für die GLC-Analyse waren wie folgt: Die Ofentemperatur wurde von 50°C bis 250°C mit einer Rate von 5°C/min und schließlich 10 min bei 250°C(halten) programmiert.

Die Identifikation der Signale erfolgte durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

Beispiel 51: Funktionelle Charakterisierung von pAt60 und pAt70

Die Substratspezifität der Elongasen At3g06460 bzw. At3g06470 konnte nach Expression und Fütterung verschiedener Fettsäuren ermittelt werden (Tab. 20, Fig. 26). Die gefütterten Substrate sind in allen transgenen Hefen nachzuweisen. Die transgenen Hefen zeigten die Synthese neuer Fettsäuren, den Produkten der Gene At3g06460 bzw.

At3g06470. Dies bedeutet, dass diese Gene funktional exprimiert werden konnte.

Tabelle 20: Elongation von EPA durch die Elongasen At3g06460 bzw. At3g06470. Messung der Hefeextrakte nach Fütterung mit 250 uM EPA.

| Gen | Gefütterte Fettsäure | | Gehalt anC20:5n-3 | Gehalt an C22: 5n-3 |
|---|---|---|---|---|
| At3g06460 | EPA (C20:5n-3) | | 20.8 | 0,6 |
| At3g06460 | EPA (C20:5n-3) | | 25,4 | 1,1 |
| Konversionsrate von EPA | | At3g06460: 3,0 % | At3g06470: 4,1 % | |

Figur 26 gibt die Elongation von 20:5n-3 durch die Elongasen At3g06470 wieder.

Beispiel 52: Klonierung einer Elongase aus Phaeodactylum tricornutum

Ausgehend von konservierten Bereichen in den Proteinsequenzen mit Hilfe der in der Anmeldung aufgeführten Elongase-Gene mit Δ-6-Elongaseaktivität wurden degenerierte Primer hergestellt und mit diesen eine *Phaeodactylum* cDNA Bank mittels PCR durchsucht. Folgende Primer-Sequenzen wurden eingesetzt:

| Primer-Name | Sequenz 5'-3' Orientierung | Korrespondierende Aminosäuren |
|---|---|---|
| Phaelo forward1 | AA(C/T)CTUCTUTGGCTUTT(C/T)TA (SEQ ID NO. 185) | NLLWLFY |
| Phaelo reverse1 | GA(C/T)TGUAC(A/G)AA(A/G)AA(C/T)TGUG C(A/G)AA (SEQ ID NO. 186) | FAQFFVQS |

Nukleotidbasen in Klammern bedeuten, dass eine Mischung von Oligonukleotiden mit jeweils der einen oder anderen Nukleotidbase vorliegen.

Herstellung der *Phaeodactylum* cDNA Bank:

Eine 2 L Kultur von P. tricornutum UTEX 646 wurde in f/2 Medium (Guillard, R.R.L. 1975. Culture of phytoplankton for feeding marine invertebrates. In Culture of Marine Invertebrate Animals (Eds. Smith, W.L. and Chanley, M.H.), Plenum Press, New York, pp 29-60.) für 14 d (= Tage) bei einer Lichtstärke von 35 E/cm$^2$ angezogen. Gefrorene Zellen wurden nach Zentrifugation in der Gegenwart von flüssigem Stickstoff zu einem feinen Pulver gemahlen und mit 2 mL Homogenisierungspuffer (0,33 M Sorbitol, 0,3 M NaCl, 10 mM EDTA, 10 mM EGTA, 2% SDS, 2% Mercaptoethanol in 0,2 M Tris-Cl ph 8,5) resuspendiert. Nach Zugabe von 4 mL Phenol und 2 mL Chloroform wurde 15 min kräftig bei 40-50 °C geschüttelt. Anschliessend wurde zentrifugiert (10 min x 10000g) und die wässerige Phase schrittweise mit Chloroform extrahiert. Nukleinsäuren wurden dann durch Zugabe von 1/20 Volumen 4 M Natriumhydrogencarbonatlösung gefällt und zentrifugiert. Das Pellet wurde in 80 mM Tris-borat pH 7,0 und 1 mM EDTA aufgenommen und die RNA mit 8 M Lithiumclorid gefällt. Nach Zentrifugation und Waschen mit 70%igem Ethanol wurde das RNA-Pellet mit Rnase-freiem Wasser aufgenommen. Poly(A)-RNA wurde mit Dynabeads (Dynal, Oslo, Norwegen) nach Herstellerangaben isoliert und die Erst-Strang-cDNA-Synthese mit MLV-Rtase von Roche (Mannheim) durchgeführt. Die Zweit-Strang-Synthese erfolgte dann mittels DNA Polymerase I und Klenow Fragment, gefolgt von einem RnaseH Verdau. Die cDNA wurde mit T4 DNA Polymerase behandelt und anschliessend EcoRI/XhoI Adaptoren (Pharmacia, Freiburg) mittels T4 Ligase angehängt. Nach XhoI Verdau, Phosphorylierung und Geltrennung wurden Fragmente grösser als 300 bp entsprechend der Herstellerangaben in den lambda ZAP Express Phagen ligiert (Stratagene, Amsterdam, Niederlande). Nach Massenexcision der cDNA-Bank und Plasmid-Rückgewinnung wurde die Plasmid-Bank in E. coli DH10B Zellen transformiert und zur PCR-Sichtung eingesetzt.

Mittels den oben genannten degenerierten Primern konnte das PCR-Fragment mit der Sequenznummer SEQ ID NO: 187 generiert werden.

Dieses Fragment wurde mit Digoxigenin markiert (Roche, Mannheim) und als Sonde für die Sichtung der Phagen-Bank verwendet.

Mit Hilfe der Sequenz SEQ ID NO: 187 konnte die Gensequenz SEQ ID NO: 183 erhalten werden, die das Volllängen-RNA-Molekül der Δ6-Elongase von Phaeodactylum darstellt:

Beispiel 53: Klonierung von Expressionsplasmiden zur heterologen Expression in - Hefen

Die entsprechenden Primerpaare wurden so ausgewählt, dass sie die Hefe-Konsensus-Sequenz für hocheffiziente Translation (Kozak, Cell 1986, 44:283-292) neben dem Startcodon trugen. Die Amplifizierung der PtELO6-DNA wurde jeweils mit 1 μL cDNA, 200 μM dNTPs, 2,5 U Advantage-Polymerase und 100 pmol eines jeden Primers in einem Gesamtvolumen von 50 μl durchgeführt. Die Bedingungen für die PCR waren wie folgt: Erste Denaturierung bei 95°C für 5 Minuten, gefolgt von 30 Zyklen bei 94°C für 30 Sekunden, 55°C für 1 Minute und 72°C für 2 Minuten sowie ein letzter Verlängerungsschritt bei 72°C für 10 Minuten.

Für die Klonierung der Sequenz zur heterologen Expression in Hefen wurden folgende Oligonukleotide für die PCR-Reaktion verwendet:

| Gen-Name und SEQ ID NO: | Primersequenz |
|---|---|
| PtELO6 (SEQ ID NO: 183) | F:5'-GCGGCCGCACA T AAT GATGGT ACCTTCAAG (SEQ ID NO: 188)<br>R:3'- GAAGACAGCTTAATAGACTAGT (SEQ ID NO: 189) |
| *F=forward primer, R=reverse primer | |

Die PCR Produkte wurden für 30 min bei 21 °C mit dem Hefe-Expressionsvektor - pYES2.1-TOPO (Invitrogen) gemäß Herstellerangaben inkubiert. Das PCR-Produkt (siehe SEQ ID NO: 192) wurde dabei durch einen T-Überhang und Aktivität einer Topoisomerase (Invitrogen) in den Vektor ligiert. Nach der Inkubation erfolgte dann die Transformation von E. coli DH5a Zellen. Entsprechende Klone wurden durch PCR - identifiziert, die Plasmid-DNA mittels Qiagen DNAeasy-Kit isoliert und durch Sequenzierung verifiziert. Die korrekte Sequenz wurde dann in den Saccharomyces Stamm INVSc1 (Invitrogen) durch Elektroporation (1500 V) transformiert. Zur Kontrolle wurde der leere Vektor pYES2.1 parallel transformiert. Anschließend wurden die Hefen auf Komplett-Minimalmedium ohne Uracil mit 2 % Glucose ausplattiert. Zellen, die ohne Uracil im Medium wachstumsfähig waren, enthalten damit die entsprechenden Plasmide pYES2.1 und pYES2.1-PtELO6. Nach der Selektion wurden je zwei Transformaten zur weiteren funktionellen Expression ausgewählt.

Beispiel 54: Klonierung von Expressionsplasmiden zur Samen-spezifischen Expression in Pflanzen

Für die Transformation von Pflanzen wird ein weiterer Transformationsvektor auf Basis von pSUN-USP erzeugt. Dazu wird mit folgendem Primerpaar NotI-Schnittstellen am 5' und 3'-Ende der kodierenden Sequenz eingefügt:. PSUN-PtELO6

Forward: 5'-GCGGCCGCACCATGATGGTACCTTCAAGTTA (SEQ ID NO: 190)
Reverse: 3'-GAAGACAGCTTAATAGGCGGCCGC (SEQ ID NO: 191)

Zusammensetzung des PCR-Ansatzes (50 μL):

5,00 μL Template cDNA
5,00 μL 10x Puffer (Advantage-Polymerase)+ 25mM $MgCl_2$
5,00 μL 2mM dNTP
1,25 μL je Primer (10 pmol/μL)
0,50 μL Advantage-Polymerase

Die Advantage-Polymerase von Clontech wurden eingesetzt.

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Die PCR Produkte werden für 16 h bei 37 °C mit dem Restriktionsenzym NotI inkubiert. Der Pflanzen-Expressionsvektor pSUN300-USP wird in gleicherweise inkubiert. Anschliessend werden die PCR Produkte sowie der 7624 bp große Vektor durch Agarose-Gelelektrophorese aufgetrennt und die entsprechenden DNA-Fragmente ausgeschnitten. Die Aufreinigung der DNA erfolgt mittels Qiagen Gel Purification Kit gemäss Herstellerangaben. Anschließend werden Vektor und PCR-Produkte ligiert. Dazu wird das Rapid Ligation Kit von Roche verwendet. Das entstandene

Plasmide pSUN-PtELO wird durch Sequenzierung verifiziert.
pSUN300 ist ein Derivat des Plasmides pPZP (Hajdukiewicz,P, Svab, Z, Maliga, P., (1994) The small versatile pPZP family of Agrobacterium binary vectors forplant transformation. Plant Mol Biol 25:989-994). pSUN-USP entstand aus pSUN300, indem in pSUN300 ein USP-Promotor als EcoRI- Fragment inseriert wurde. Das Polyadenylierungssignal ist das des Octopinsynthase-Gens aus dem A. tumefaciens Ti-Plasmid (ocs-Terminator, Genbank Accession V00088) (De Greve,H., Dhaese,P., Seurinck,J., Lemmers,M., Van Montagu,M. and Schell,J. Nucleotide sequence and transcript map of the Agrobacterium tumefaciens Ti plasmid-encoded octopine synthase gene J. Mol. Appl. Genet. 1 (6), 499-511 (1982). Der USP-Promotor entspricht den Nukleotiden 1-684 (Genbank Accession X56240), wobei ein Teil der nichtcodierenden Region des USP-Gens im Promotor enthalten ist. Das 684 Basenpaar große Promotorfragment wurde mittels käuflichen T7-Standardprimer (Stratagene) und mit Hilfe eines synthetisierten Primers über eine PCR-Reaktion nach Standardmethoden amplifiziert.

(Primersequenz: 5'–GTCGACCCGCGGACTAGTGGGCCCTCTAGACCCGGGGGATCC GGATCTGCTGGCTATGAA–3'; SEQ ID NO: 151).

Das PCR-Fragment wurde mit EcoRI/SalI nachgeschnitten und in den Vektor pSUN300 mit OCS Terminator eingesetzt. Es entstand das Plasmid mit der Bezeichnung pSUN-USP.Das Konstrukt wurde zur Transformation von Arabidopsis thaliana, Raps, Tabak und Leinsamen verwendet.

Die Lipidextraktion aus Hefen und Samen erfolgte identisch zu Beispiel 6.

Beispiel 55: Expression von PtElo in Hefen

Hefen, die wie unter Beispiel 4 mit den Plasmiden pYES2 und pYES2-PtELO6 transformiert wurden, wurden folgendermaßen analysiert:
Die Hefezellen aus den Hauptkulturen wurden durch Zentrifugation (100 x g, 5 min, 20°C) geerntet und mit 100 mM $NaHCO_3$, pH 8,0 gewaschen, um restliches Medium und Fettsäuren zu entfernen. Aus den Hefe-Zellsedimenten wurden Fettsäuremethylester (FAMEs) durch saure Methanolyse hergestellt. Hierzu wurden die Zellsedimente mit 2 ml 1 N methanolischer Schwefelsäure und 2% (v/v) Dimethoxypropan für 1 h bei 80°C inkubiert. Die Extraktion der FAMES erfolgte durch zweimalige Extraktion mit Petrolether (PE). Zur Entfernung nicht derivatisierter Fettsäuren wurden die organischen Phasen je einmal mit 2 ml 100 mM $NaHCO_3$, pH 8,0 und 2 ml Aqua dest. gewaschen. Anschließend wurden die PE-Phasen mit $Na_2SO_4$ getrocknet, unter Argon eingedampft und in 100 μl PE aufgenommen. Die Proben wurden auf einer DB-23-Kapillarsäule (30 m, 0,25 mm, 0,25 μm, Agilent) in einem Hewlett-Packard 6850-Gaschromatographen mit Flammenionisationsdetektor getrennt. Die Bedingungen für die GLC-Analyse waren wie folgt: Die Ofentemperatur wurde von 50°C bis 250°C mit einer Rate von 5°C/min und schließlich 10 min bei 250°C(halten) programmiert.

Die Identifikation der Signale erfolgte durch Vergleiche der Retentionszeiten mit entsprechenden Fettsäurestandards (Sigma). Die Methodik ist beschrieben zum Beispiel in Napier and Michaelson, 2001,Lipids. 36(8):761-766; Sayanova et al., 2001, Journal of Experimental Botany. 52(360):1581-1585, Sperling et al., 2001, Arch. Biochem. Biophys. 388(2):293-298 und Michaelson et al., 1998, FEBS Letters. 439(3):215-218.

Beispiel 56: Funktionelle Charakterisierung von PtELO6:

In Figur 29 ist die Umsetzung von $C18{:}3^{\Delta 6,9,12}$ und $C18{:}4^{\Delta 6,9,12,15}$ wiedergegeben. Die Substrate werden um je zwei Kohlenstoffatome elongiert es entstehen jeweils die Fettsäuren $C20{:}3^{\Delta 8,11,14}$ bzw. $C20{:}4^{\Delta 8,11,14,17}$. Die Substratspezifität von PtELO6 konnte nach Expression und Fütterung verschiedener Fettsäuren ermittelt werden (Fig. 30). Die gefütterten Substrate sind in großen Mengen in allen transgenen Hefen nachzuweisen. Die transgenen Hefen zeigten die Synthese neuer Fettsäuren, den Produkten der PtElo6-Reaktion. Dies bedeutet, dass das Gen PtELO6 funktional exprimiert werden konnte.

Tabelle 21 zeigt, dass die PtElo6 eine enge Substratspezifität aufweist. PtELO6 konnte nur die C18-Fettsäuren Linolsäure, Linolensäure, γ-Linolensäure und Stearidonsäure elongieren, bevorzugte aber die ω-3-desaturierte Stearidonsäure (siehe auch Figur 30). Fütterungsexperiment: Fettsäuren (fett) wurden jeweils mit 250 μM zugegeben. Die unterstrichenen Fettsäuren wurden neu gebildet.

Tabelle 21: Substratspezifität der PtElo6

| gefütterte Fettsäure: | | + 18:2 | + 18:3 | + 18:3 | + 18:4 |
|---|---|---|---|---|---|
| 16:0 | 16,2 | 18,2 | 15,2 | 20 | 04:48 |
| 16:1 | 50,6 | 20,5 | 22,8 | 33,5 | 34,2 |
| 18:0 | 5,4 | 6,3 | 6,2 | 5,2 | 12,4 |
| 18:1 | 27,7 | 14,6 | 19,6 | 19,3 | 16,7 |
| 18:2 | | 40 | | | |
| 18:3 | | | 32,9 | | |
| 18:3 | | | | 12,3 | |
| 18:4 | | | | | 4,5 |
| 20:2 | | 0,4 | | | |
| 20:3 | | | 3,4 | | |
| 20:3 | | | | 9,7 | |
| 20:4 | | | | | 14,5 |
| **% Elongation** | **0,0** | **0,99** | **9,37** | **44,09** | **76,32** |

Folgende Fettsäuren wurden gefüttert, aber nicht umgesetzt:

- $18{:}1^{\Delta 6}$, $18{:}1^{\Delta 9}$, $18{:}1^{\Delta 11}$
- $20{:}2^{\Delta 11,14}$, $20{:}3^{\Delta 11,14,17}$, $20{:}3^{\Delta 8,11,14}$, $20{:}4^{\Delta 5,8,11,14}$, $20{:}5^{\Delta 5,8,11,14,17}$
- $22{:}4^{\Delta 7,10,13,16}$

Die Hefen, die mit dem Vektor pYES2-PtELO6 transformiert worden waren, wurden in Minimalmedium in Gegenwart der angegebenen Fettsäuren kultiviert. Die Synthese der Fettsäuremethylester erfolgte durch saure Methanolyse intakter Zellen. Anschließend wurden die FAMEs über GLC analysiert. So wurden die Ergebnisse, die in den Figuren 29 und 30 sowie in der Tabelle 19 dargestellt wurden, ermittelt.

Beispiel 57: Klonierung von Expressionsplasmiden zur Samen-spezifischen Expression in Pflanzen

Die folgenden beschriebenen allgemeinen Bedingungen gelten für alle nachfolgenden Versuche, wenn nicht anders beschrieben.

Erfindungsgemäß bevorzugt verwendet werden für die folgenden Beispiele Bin19, pB1101, pBinAR, pGPTV und pCAMBIA. Eine Übersicht über binäre Vektoren und ihre Verwendung gibt Hellens et al, Trends in Plant Science (2000) 5, 446-451. Verwendet wurde ein pGPTV-Derivat wie in DE10205607 beschrieben. Dieser Vektor unterscheidet sich von pGPTV durch eine zusätzlich eingefügte Ascl-Restriktionsschnittstelle.

Ausgangspunkt der Klonierung war der Klonierungsvektor pUC19 (Maniatis et al.). Im ersten Schritt wurde das Conlinin-Promotor-Fragment mit folgenden Primern amplifiziert:

Cnl1 C 5': gaattcggcgcgccgagctcctcgagcaacggttccggcggtatagagttgggtaattcga
Cnl1 C 3': cccgggatcgatgccggcagatctccaccatttttggtggtgat

Zusammensetzung des PCR-Ansatzes (50 µl):

5,00 µl Template cDNA
5,00 µl 10x Puffer (Advantage-Polymerase) + 25mM $MgCl_2$
5,00 µl 2mM dNTP
1,25 µl je Primer (10 pmol/µl)
0,50 µl Advantage-Polymerase (Clontech)

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde zuerst für 2 h bei 37°C mit dem Restriktionsenzym EcoRI und dann für 12 h bei 25°C mit dem Restriktionsenzym *Sma*I inkubiert. Der Klonierungsvektor pUC19 wurde in gleicher Weise inkubiert. Anschließend wurden das PCR-Produkt und der 2668 bp große, geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-Cnl1-C wurde durch Sequenzierung verifiziert.

Im nächsten Schritt wurde der OCS-Terminator (Genbank Accession V00088; De Greve, H., Dhaese, P., Seurinck, J., Lemmers, M., Van Montagu, M. and Schell, J. Nucleotide sequence and transcript map of the Agrobacterium tumefaciens Ti plasmid-encoded octopine synthase gene J. Mol. Appl. Genet. 1 (6), 499-511 (1982)) aus dem Vektor pGPVT-USP/OCS

(DE 102 05 607) mit den folgenden Primern amplifiziert:

OCS_C 5': aggcctccatggcctgctttaatgagatatgcgagacgcc
OCS_C 3': cccgggccggacaatcagtaaattgaacggag

Zusammensetzung des PCR-Ansatzes (50 μl):

5,00 μl Template cDNA
5,00 μl 10x Puffer (Advantage-Polymerase) + 25mM $MgCl_2$
5,00 μl 2mM dNTP
1,25 μl je Primer (10 pmol/μl)
0,50 μl Advantage-Polymerase (Clontech)

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde zuerst für 2 h bei 37°C mit dem Restriktionsenzym *Stu*I und dann für 12 h bei 25°C mit dem Restriktionsenzym *Sma*I inkubiert. Der Vektor pUC19-Cnl1-C wurde 12 h bei 25°C mit dem Restriktionsenzym *Sma*I inkubiert. Anschließend wurden das PCR-Produkt und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-Cnl1C_OCS wurde durch Sequenzierung verifiziert.

Im nächsten Schritt wurde der Cnl1-B Promotor durch PCR mittels folgender Primer amplifiziert:

Cnl1-B 5': aggcctcaacggttccggcggtatag
Cnl1-B 3': cccggggttaacgctagcgggcccgatatcggatcccatttttggtggtgattggttct

Zusammensetzung des PCR-Ansatzes (50 μl):

5,00 μl Template cDNA
5,00 μl 10x Puffer (Advantage-Polymerase) + 25mM $MgCl_2$
5,00 μl 2mM dNTP
1,25 μl je Primer (10 pmol/μl)
0,50 μl Advantage-Polymerase (Clontech)

Reaktionsbedingungen der PCR:

Anlagerungstemperatur: 1 min 55°C

Denaturierungstemperatur: 1 min 94°C
Elongationstemperatur: 2 min 72°C
Anzahl der Zyklen: 35

Das PCR-Produkt wurde zuerst für 2 h bei 37°C mit dem Restriktionsenzym *Stu*I und dann für 12 h bei 25°C mit dem Restriktionsenzym SmaI inkubiert. Der Vektor pUC19-CnI1-C wurde 12 h bei 25°C mit dem Restriktionsenzym SmaI inkubiert. Anschließend wurden das PCR-Produkt und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-CnI1C_CnI1B_OCS wurde durch Sequenzierung verifiziert.

In einem weiteren Schritt wurde der OCS-Terminator für CnI1B eingefügt. Dazu wurde die PCR mit folgenden Primer durchgeführt:

OCS2 5': aggcctcctgctttaatgagatatgcgagac
OCS2 3': cccgggcggacaatcagtaaattgaacggag

Zusammensetzung des PCR-Ansatzes (50 µl):

5,00 µl Template cDNA
5,00 µl 10x Puffer (Advantage-Polymerase) + 25mM $MgCl_2$
5,00 µl 2mM dNTP
1,25 µl je Primer (10 pmol/µl)
0,50 µl Advantage-Polymerase (Clontech)

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde zuerst für 2 h bei 37°C mit dem Restriktionsenzym *Stu*I und dann für 12 h bei 25°C mit dem Restriktionsenzym SmaI inkubiert. Der Vektor pUC19-CnI1C_CnI1B_OCS wurde für 12 h bei 25°C mit dem Restriktionsenzym SmaI inkubiert. Anschließend wurden das PCR-Produkt und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-CnI1C_CnI1B_OCS2 wurde durch Sequenzierung verifiziert.

Im nächsten Schritt wurde der CnI1-A Promotor durch PCR mittels folgender Primer amplifiziert:

CnI1-B 5': aggcctcaacggttccggcggtatagag
CnI1-B 3': aggccttctagactgcaggcggccgcccgcattttttggtggtgattggt

Zusammensetzung des PCR-Ansatzes (50 µl):

5,00 µl Template cDNA
5,00 µl 10x Puffer (Advantage-Polymerase) + 25mM $MgCl_2$
5,00 µl 2mM dNTP
1,25 µl je Primer (10 pmol/µl)
0,50 µl Advantage-Polymerase (Clontech)

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde für 2 h bei 37°C mit dem Restriktionsenzym *Stu*I inkubiert. Der Vektor pUC19-Cnl1-C wurde für 12 h bei 25°C mit dem Restriktionsenzym *Sma*I inkubiert. Anschließend wurden das PCR-Produkt und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-Cnl1C_Cnl1B_Cnl1A_OCS2 wurde durch Sequenzierung verifiziert.

In einem weiteren Schritt wurde der OCS-Terminator für Cnl1A eingefügt. Dazu wurde die PCR mit folgenden Primer durchgeführt:

OCS2 5': ggcctcctgctttaatgagatatgcga
OCS2 3': aagcttggcgcgccgagctcgtcgacggacaatcagtaaattgaacggaga

Zusammensetzung des PCR-Ansatzes (50 µl):

5,00 µl Template cDNA
5,00 µl 10x Puffer (Advantage-Polymerase) + 25mM $MgCl_2$
5,00 µl 2mM dNTP
1,25 µl je Primer (10 pmol/µl)
0,50 µl Advantage-Polymerase (Clontech)

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde zuerst für 2 h bei 37°C mit dem Restriktionsenzym *Stu*I und dann für 2 h bei 37°C mit dem Restriktionsenzym *Hin*dIII inkubiert. Der Vektor pUC19-Cnl1C_Cnl1B_Cnl1A_OCS2 wurde für 2 h bei 37°C mit dem Restriktionsenzym *Stu*I und für 2 h bei 37°C mit dem Restriktionsenzym *Hin*dIII inkubiert. Anschließend wurden das PCR-Produkt und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-Cnl1C_Cnl1B_Cnl1A_OCS3 wurde durch Sequenzierung verifiziert.

Das Plasmid pUC19-Cnl1C_Cnl1B_Cnl1A_OCS3 wurde im nächsten Schritt zur Klonierung der Δ6-, Δ5-Desaturase und Δ6-Elongase verwendet. Dazu wurde die Δ6-Desaturase aus Phytium irregulare (WO02/26946) mit folgenden PCR-Primern amplifiziert:

D6Des(Pir) 5': agatctatggtggacctcaagcctggagtg
D6Des(Pir) 3': ccatggcccgggttacatcgctgggaactcggtgat

Zusammensetzung des PCR-Ansatzes (50 µl):

5,00 µl Template cDNA
5,00 µl 10x Puffer (Advantage-Polymerase) + 25mM $MgCl_2$
5,00 µl 2mM dNTP
1,25 µl je Primer (10 pmol/µl)
0,50 µl Advantage-Polymerase (Clontech)

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde zuerst für 2 h bei 37°C mit dem Restriktionsenzym *Bgl*II und dann für 2 h bei 37°C mit dem Restriktionsenzym *Nco*I inkubiert. Der Vektor pUC19-Cnl1C_Cnl1B_Cnl1A_OCS3 wurde für 2 h bei 37°C mit

dem Restriktionsenzym *Bgl*II und für 2 h bei 37°C mit dem Restriktionsenzym *Nco*I inkubiert. Anschließend wurden das PCR-Produkt und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-Cnl1_d6Des(Pir) wurde durch Sequenzierung verifiziert.

Das Plasmid pUC19-Cnl1_d6Des(Pir) wurde im nächsten Schritt zur Klonierung der Δ5-Desaturase aus Thraustochytrium ssp. (WO02/26946) verwendet. Dazu wurde die Δ5-Desaturase aus Thraustochytrium ssp. mit folgenden PCR-Primern amplifiziert:

D5Des(Tc) 5': gggatccatgggcaagggcagcgagggccg
D5Des(Tc) 3': ggcgccgacaccaagaagcaggactgagatatc

Zusammensetzung des PCR-Ansatzes (50 μl):

5,00 μl Template cDNA
5,00 μl 10x Puffer (Advantage-Polymerase) + 25mM $MgCl_2$
5,00 μl 2mM dNTP
1,25 μl je Primer (10 pmol/μl)
0,50 μl Advantage-Polymerase (Clontech)

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde zuerst für 2 h bei 37°C mit dem Restriktionsenzym *Bam*HI und dann für 2 h bei 37°C mit dem Restriktionsenzym EcoRV inkubiert. Der Vektor pUC19-Cnl1_d6Des(Pir) wurde für 2 h bei 37°C mit dem Restriktionsenzym *Bam*HI und für 2 h bei 37°C mit dem Restriktionsenzym EcoRV inkubiert. Anschließend wurden das PCR-Produkt und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-Cnl1_d6Des(Pir)_d5Des(Tc) wurde durch Sequenzierung verifiziert.

Das Plasmid pUC19-Cnl1_d6Des(Pir)_d5Des(Tc) wurde im nächsten Schritt zur Klonierung der Δ6-Elongase aus Physcomitrella patens (WO01/59128) verwendet, wozu diese mit folgenden PCR-Primern amplifiziert wurde:

D6Elo(Pp) 5': gcggccgcatggaggtcgtggagagattctacggtg
D6Elo(Pp) 3': gcaaaagggagctaaaactgagtgatctaga

Zusammensetzung des PCR-Ansatzes (50 μl):

5,00 μl Template cDNA
5,00 μl 10x Puffer (Advantage-Polymerase) + 25mM $MgCl_2$
5,00 μl 2mM dNTP
1,25 μl je Primer (10 pmol/μl)
0,50 μl Advantage-Polymerase (Clontech)

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde zuerst für 2 h bei 37°C mit dem Restriktionsenzym *Not*I und dann für 2 h bei 37°C mit dem Restriktionsenzym *Xba*I inkubiert. Der Vektor pUC19-Cnl1_d6Des(Pir)_d5Des(Tc) wurde für 2 h bei 37°C mit dem Restriktionsenzym *Not*I und für 2 h bei 37°C mit dem Restriktionsenzym *Xba*I inkubiert. Anschließend wurden das PCR-Produkt und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-Cnl1_d6Des(Pir)_d5Des(Tc)_D6Elo(Pp) wurde durch Sequenzierung verifiziert.

Ausgehend von pUC19-Cnl1_d6Des(Pir)_d5Des(Tc)_D6Elo(Pp) wurde der binäre Vektor für die Pflanzentransformation hergestellt. Dazu wurde pUC19-Cnl1_d6Des(Pir)_d5Des(Tc)_D6Elo(Pp) für 2 h bei 37°C mit dem Restriktionsenzym *Asc*I inkubiert. Der Vektor pGPTV wurde in gleicher Weise behandelt. Anschließend wurden das Fragment aus pUC19-Cnl1_d6Des(Pir)_d5Des(Tc)_D6Elo(Pp) und der geschnittene pGPTV-Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pGPTV-Cnl1_d6Des(Pir)_d5Des(Tc)_D6Elo(Pp) wurde durch Sequenzierung verifiziert.

Ein weiteres Konstrukt, pGPTV- Cnl1_d6Des(Pir)_d5Des(Tc)_D6Elo(Pp)_D12Des(Co), fand Verwendung. Dazu wurde ausgehend von pUC19-Cnl1C_OCS mit folgenden Primern amplifiziert:

Cnl1_OCS 5': gtcgatcaacggttccggcggtatagagttg
Cnl1_OCS 3': gtcgatcggacaatcagtaaattgaacggaga

Zusammensetzung des PCR-Ansatzes (50 µl):

5,00 µl Template cDNA
5,00 µl 10x Puffer (Advantage-Polymerase) + 25mM $MgCl_2$
5,00 µl 2mM dNTP
1,25 µl je Primer (10 pmol/µl)
0,50 µl Advantage-Polymerase (Clontech)

Reaktionsbedingungen der PCR:

Anlagerungstemperatur: 1 min 55°C
Denaturierungstemperatur: 1 min 94°C
Elongationstemperatur: 2 min 72°C
Anzahl der Zyklen: 35

Das PCR-Produkt wurde für 2 h bei 37°C mit dem Restriktionsenzym *Sal*I inkubiert. Der Vektor pUC19 wurde für 2 h bei 37°C mit dem Restriktionsenzym *Sal*I inkubiert. Anschließend wurden das PCR-Produkt und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-Cnl1_OCS wurde durch Sequenzierung verifiziert.

In einem weiteren Schritt wurde das Δ12-Desaturase-Gen aus Calendula officinalis (WO01/85968) in pUC19-Cnl1_OCS kloniert. Dazu wurde d12Des(Co) mit folgenden Primern amplifiziert:

D12Des(Co) 5': agatctatgggtgcaggcggtcgaatgc
D12Des(Co) 3': ccatggttaaatcttattacgatacc

Zusammensetzung des PCR-Ansatzes (50 µl):

5,00 µl Template cDNA
5,00 µl 10x Puffer (Advantage-Polymerase) + 25mM $MgCl_2$
5,00 µl 2mM dNTP
1,25 µl je Primer (10 pmol/µl)
0,50 µl Advantage-Polymerase (Clontech)

Reaktionsbedingungen der PCR:

Anlagerungstemperatur: 1 min 55°C
Denaturierungstemperatur: 1 min 94°C
Elongationstemperatur: 2 min 72°C

Anzahl der Zyklen: 35

Das PCR-Produkt wurde für 2 h bei 37°C mit dem Restriktionsenzym *Bgl*II und anschließend für 2 h bei gleicher Temperatur mit *Nco*I inkubiert. Der Vektor pUC19-CnI1_OCS wurde in gleicher Weise inkubiert. Anschließend wurden das PCR-Fragment und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-CnI1_D12Des(Co) wurde durch Sequenzierung verifiziert.

Das Plasmid pUC19-CnI1_D12Des(Co), sowie das Plasmid pUC19-CnI1_d6Des(Pir)_d5Des(Tc)_D6Elo(Pp) wurden für 2 h bei 37°C mit dem Restriktionsenzym *Sal*I inkubiert. Anschließend wurde das Vektor-Fragment sowie der Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und Vektor-Fragment ligiert. Dazu wurde das Rapid Ligation Kit von Roche verwendet. Das entstandene Plasmid pUC19-CnI1_ d6Des(Pir)_d5Des(Tc)_D6Elo(Pp)_D12Des(Co) wurde durch Sequenzierung verifiziert.

Ausgehend von pUC19-CnI1_d6Des(Pir)_d5Des(Tc)_D6Elo(Pp)_D12Des(Co) wurde der binäre Vektor für die Pflanzentransformation hergestellt. Dazu wurde pUC19-CnI1_d6Des(Pir)_d5Des(Tc)_D6Elo(Pp)_D12Des(Co) für 2 h bei 37°C mit dem Restriktionsenzym *Asc*I inkubiert. Der Vektor pGPTV wurde in gleicher Weise behandelt. Anschließend wurden das Fragment aus pUC19-CnI1_d6Des(Pir)_d5Des(Tc)_D6Elo(Pp)_D12Des(Co) und der geschnittene pGPTV-Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pGPTV- CnI1_d6Des(Pir)_d5Des(Tc)_D6Elo(Pp)_D12Des(Co) wurde durch Sequenzierung verifiziert.

Ein weiterer für die Pflanzentransformation geeigneter Vektor ist pSUN2. Um die Zahl der im Vektor enthaltenen Expressionskassetten auf mehr als vier zu erhöhen wurde dieser Vektor in Kombination mit dem Gateway-System (Invitrogen, Karlsruhe) verwendet. Dazu wurde in den Vektor pSUN2 gemäss Herstellerangaben die Gateway-Kassette A wie folgendermassen beschrieben, eingefügt:

Der pSUN2 Vektor (1 μg) wurde 1 h mit dem Restriktionsenzym EcoRV bei 37° inkubiert. Anschliessend wurde die Gateway-Kassette A (Invitrogen, Karlsruhe) in den geschnittene Vektor ligiert mittels des Rapid Ligation Kits von Roche, Mannheim. Das entstandene Plasmid wurde in E. coli DB3.1 Zellen (Invitrogen) transformiert. Das insolierte Plasmid pSUN-GW wurde anschliessend durch Sequenzierung verifiziert.

Im zweiten Schritt wurde die Expressionskassette aus pUC19-CnI1_ d6Des(Pir)_d5Des(Tc)_D6Elo(Pp)_D12Des(Co) mittels AscI ausgeschnitten und in den in gleicherweise behandelten Vektor pSUN-GW ligiert. Das so entstandene Plasmid pSUN-4G wurde für weitere Genkonstrukte verwendet.

Dazu wurde zuerst gemäss Herstellerangaben (Invitrogen) ein pENTR-Klon modifiziert. Das Plasmid pENTR1A (Invitrogen) wurde 1 h bei 37° mit dem Restriktionsenzym EcorI inkubiert, anschliessend für 30 min mit Klenow-Enzym, sowie einem 1 μM dNTP-Mix behandelt und dann der AscI-Adapter (5'-ggcgcgcc; am 5'-Ende phosphoryliert, doppelsträngig) in den pENTR1A-Vektor liegiert. In diesen modifierzerten wurde wie oben beschrieben schrittweise Gene in die CnI-Kassette eingefügt und über AscI in den pENTR-Vektor übertragen.

In dieser beschriebenen Art und Weise wurde das Gen TL16y2 aus Thraustochytrium ssp. (SEQ ID No. 83) in den pSUN-4G Vektor übertragen:

Das Plasmid pUC19-CnI1C_CnI1B_CnI1A_OCS3 wurde im nächsten Schritt zur Klonierung der Δ5-Elongase TL16y2 verwendet. Dazu wurde die Δ5-Elongase aus Thraustochytrium ssp. mit folgenden PCR-Primern amplifiziert:

TL16y2 5': agatct atggacgtcgtcgagcagca
TL16y2 3': ccatggcccggg agaagcagaagaccatctaa

Zusammensetzung des PCR-Ansatzes (50 μl):

5,00 μl Template cDNA
5,00 μl 10x Puffer (Advantage-Polymerase) + 25mM $MgCl_2$
5,00 μl 2mM dNTP
1,25 μl je Primer (10 pmol/μl)
0,50 μl Advantage-Polymerase (Clontech)

Reaktionsbedingungen der PCR:

Anlagerungstemperatur: 1 min 55°C
Denaturierungstemperatur: 1 min 94°C

Elongationstemperatur: 2 min 72°C
Anzahl der Zyklen: 35

Das PCR-Produkt wurde zuerst für 2 h bei 37°C mit dem Restriktionsenzym *Bgl*II und dann für 2 h bei 37°C mit dem Restriktionsenzym *Nco*I inkubiert. Der Vektor pUC19-Cnl1C_Cnl1B_Cnl1A_OCS3 wurde für 2 h bei 37°C mit dem Restriktionsenzym *Bgl*II und für 2 h bei 37°C mit dem Restriktionsenzym *Nco*I inkubiert. Anschließend wurden das PCR-Produkt und der geschnittene Vektor durch Agarose-Gelelektrophorese aufgetrennt und die entsprechenden DNA-Fragmente ausgeschnitten. Die Aufreinigung der DNA erfolgte mittels Qiagen Gel Purification Kit gemäß Herstellerangaben. Anschließend wurden Vektor und PCR-Produkt ligiert. Dazu wurde das Rapid Ligation Kit von Roche verwendet. Das entstandene Plasmid pUC19-Cnl1_TL16y2 wurde durch Sequenzierung verifiziert. Anschliessend wurde die Kassette mit AscI ausgeschnitten und in einen mit AscI vorbehandelten pENTR-Vektor ligiert. Das entstandene Plasmid pENTR-Cnl1_TL16y2 wurde dann gemäss Herstellerangaben (Invitrogen) in einer Rekombinationsreaktion mit dem Vektor pSUN-4G inkubiert. Das Produkt ergab den Vektor pSUN-5G, der für die Pflanzentransformation eingesetzt wurde.
In einem weiteren Schritt wurde das Konstrukt pSUN-8G mittels derselben beschriebenen Methodik erstellt. Dazu wurden 5'- und 3'-Primer für die Gene SEQ ID 41, 53, 87 und 113 mit den oben beschriebenen Restriktionsschnittstellen sowie den ersten und jeweils letzten 20 Nukleotiden des offenen Leserahmens erstellt und mit den Standardbedingungen (siehe oben) amplifiziert und in den pENTR-Cnl-Vektor ligiert.
Durch Rekombinationsreaktion mit dem Vektor pSUN-4G konnte so das Konstrukt pSUN-8G erstellt werden. Auch dieser Vektor wurde für die Pflanzentransformation eingesetzt.

Beispiel 58: Erzeugung von transgenen Pflanzen

a) Erzeugung transgener Sareptasenfpflanzen. Es wurde das Protokoll zur Transformation von Rapspflanzen verwendet (verändert nach Moloney et al., 1992, Plant Cell Reports, 8:238-242)

Zur Erzeugung transgener Pflanzen wurden die erzeugten binäre Vektoren pGPTV-Cnl1_d6Des(Pir)_d5Des(Tc)_D6Elo(Pp)_D12Des(Co), pSUN-5G und pSUN-8G in Agrobacterium tumefaciens C58C1:pGV2260 transformiert (Deblaere et al, 1984, Nucl. Acids. Res. 13, 4777-4788). Zur Transformation von Sareptasenfpflanzen wurde eine 1:50 Verdünnung einer Übernachtkultur einer positiv transformierten Agrobakterienkolonie in Murashige-Skoog Medium (Murashige und Skoog 1962 Physiol. Plant. 15, 473) mit 3 % Saccharose (3MS-Medium) verwendet. Petiolen oder Hypokotyledonen frisch gekeimter steriler Pflanzen (zu je ca. 1 $cm^2$) wurden in einer Petrischale mit einer 1:50 Agrobakterienverdünnung für 5-10 Minuten inkubiert. Es folgt eine 3-tägige Coinkubation in Dunkelheit bei 25°C auf 3MS-Medium mit 0,8 % Bacto-Agar. Die Kultivierung wurde anschließend mit 16 Stunden Licht / 8 Stunden Dunkelheit und in wöchentlichem Rhythmus auf MS-Medium mit 500 mg/l Claforan (Cefotaxime-Natrium), 50 mg/l Kanamycin, 20 mikroM Benzylaminopurin (BAP) und 1,6 g/l Glukose weitergeführt. Wachsende Sprosse wurden auf MS-Medium mit 2 % Saccharose, 250 mg/l Claforan und 0,8 % Bacto-Agar überführt. Bildeten sich nach drei Wochen keine Wurzeln, so wurde als Wachstumshormon 2-Indolbuttersäure zum Bewurzeln dem Medium zugegeben.
Regenerierte Sprosse wurden auf 2MS-Medium mit Kanamycin und Claforan erhalten, nach Bewurzelung in Erde überführt und nach Kultivierung für zwei Wochen in einer Klimakammer oder im Gewächshaus angezogen, zur Blüte gebracht, reife Samen geerntet und auf Elongase-Expression wie Δ-6-Elongaseaktivität oder Δ-5- oder Δ-6-Desaturaseaktivität mittels Lipidanalysen untersucht. Linien mit erhöhten Gehalten an C20- und C22 mehrfach ungesättigten Fettsäuren wurden so identifiziert.
Mit diesem Protokoll wurden auch transgene Rapspflanzen erfolgreich hergestellt.

b) Herstellung von transgenen Leinpflanzen

Die transgenen Leinpflanzen können zum Beispiel nach der Methode von Bell et al., 1999, In Vitro Cell. Dev. Biol.-Plant. 35(6):456-465 mittels particle bombartment erzeugt werden. Agrobakterien-vermittelte Transformationen können zum Beispiel nach Mlynarova et al. (1994), Plant Cell Report 13: 282-285 durchgeführt werden.

Beispiel 59: Lipidextraktion aus Samen:

Die Auswirkung der genetischen Modifikation in Pflanzen auf die Produktion einer gewünschten Verbindung (wie einer Fettsäure) kann bestimmt werden, indem die modifizierte Pflanze unter geeigneten Bedingungen (wie den vorstehend beschriebenen) gezüchtet wird und das Medium und/oder die zellulären Komponenten auf die erhöhte Produktion des gewünschten Produktes (d.h. der Lipide oder einer Fettsäure) untersucht werden. Diese Analysetechniken sind dem Fachmann bekannt und umfassen Spektroskopie, Dünnschichtchromatographie, Färbeverfahren verschiedener Art, enzymatische und mikrobiologische Verfahren sowie analytische Chromatographie, wie Hochleistungs-Flüssigkeitschromatographie (siehe beispielsweise Ullman, Encyclopedia of Industrial Chemistry, Bd. A2, S. 89-90 und S. 443-613, VCH: Weinheim (1985); Fallon, A., et al., (1987) "Applications of HPLC in Biochemistry" in: Laboratory Techniques in Biochemistry and Molecular Biology, Bd. 17; Rehm et al. (1993) Biotechnology, Bd. 3, Kapitel III: "Product recovery and purification", S. 469-714, VCH: Weinheim; Belter, P.A., et al. (1988) Bioseparations: downstream processing for Biotechnology, John Wiley and Sons; Kennedy, J.F., und Cabral, J.M.S. (1992) Recovery processes for biological Materials, John Wiley and Sons; Shaeiwitz, J.A., und Henry, J.D. (1988) Biochemical Separations, in: Ullmann's Encyclopedia of Industrial Chemistry, Bd. B3; Kapitel 11, S. 1-27, VCH: Weinheim; und Dechow, F.J. (1989) Separation and purification techniques in biotechnology, Noyes Publications).

Neben den oben erwähnten Verfahren werden Pflanzenlipide aus Pflanzenmaterial wie von Cahoon et al. (1999) Proc. Natl. Acad. Sci. USA 96 (22): 12935-12940, und Browse et al. (1986) Analytic Biochemistry 152:141-145 beschrieben extrahiert. Die qualitative und quantitative Lipid- oder Fettsäureanalyse ist beschrieben bei Christie, William W., Advances in Lipid Methodology, Ayr/Scotland: Oily Press (Oily Press Lipid Library; 2); Christie, William W., Gas Chromatography and Lipids. A Practical Guide - Ayr, Scotland: Oily Press, 1989, Repr. 1992, IX, 307 S. (Oily Press Lipid Library; 1); "Progress in Lipid Research, Oxford: Pergamon Press, 1 (1952) - 16 (1977) u.d.T.: Progress in the Chemistry of Fats and Other Lipids CODEN.

Zusätzlich zur Messung des Endproduktes der Fermentation ist es auch möglich, andere Komponenten der Stoffwechselwege zu analysieren, die zur Produktion der gewünschten Verbindung verwendet werden, wie Zwischen- und Nebenprodukte, um die Gesamteffizienz der Produktion der Verbindung zu bestimmen. Die Analyseverfahren umfassen Messungen der Nährstoffmengen im Medium (z.B. Zucker, Kohlenwasserstoffe, Stickstoffquellen, Phosphat und andere Ionen), Messungen der Biomassezusammensetzung und des Wachstums, Analyse der Produktion üblicher Metabolite von Biosynthesewegen und Messungen von Gasen, die während der Fermentation erzeugt werden. Standardverfahren für diese Messungen sind in Applied Microbial Physiology; A Practical Approach, P.M. Rhodes und P.F. Stanbury, Hrsgb., IRL Press, S. 103-129; 131-163 und 165-192 (ISBN: 0199635773) und darin angegebenen Literaturstellen beschrieben.

Ein Beispiel ist die Analyse von Fettsäuren (Abkürzungen: FAME, Fettsäuremethylester; GC-MS, Gas-Flüssigkeitschromatographie-Massenspektrometrie; TAG, Triacylglycerin; TLC, Dünnschichtchromatographie).

Der unzweideutige Nachweis für das Vorliegen von Fettsäureprodukten kann mittels Analyse rekombinanter Organismen nach Standard-Analyseverfahren erhalten werden: GC, GC-MS oder TLC, wie verschiedentlich beschrieben von Christie und den Literaturstellen darin (1997, in: Advances on Lipid Methodology, Vierte Aufl.: Christie, Oily Press, Dundee, 119-169; 1998, Gaschromatographie-Massenspektrometrie-Verfahren, Lipide 33:343-353).

Das zu analysierende Material kann durch Ultraschallbehandlung, Mahlen in der Glasmühle, flüssigen Stickstoff und Mahlen oder über andere anwendbare Verfahren aufgebrochen werden. Das Material muss nach dem Aufbrechen zentrifugiert werden. Das Sediment wird in Aqua dest. resuspendiert, 10 min bei 100°C erhitzt, auf Eis abgekühlt und erneut zentrifugiert, gefolgt von Extraktion in 0,5 M Schwefelsäure in Methanol mit 2 % Dimethoxypropan für 1 Std. bei 90°C, was zu hydrolysierten Öl- und Lipidverbindungen führt, die transmethylierte Lipide ergeben. Diese Fettsäuremethylester werden in Petrolether extrahiert und schließlich einer GC-Analyse unter Verwendung einer Kapillarsäule (Chrompack, WCOT Fused Silica, CP-Wax-52 CB, 25 mikrom, 0,32 mm) bei einem Temperaturgradienten zwischen 170°C und 240°C für 20 min und 5 min bei 240°C unterworfen. Die Identität der erhaltenen Fettsäuremethylester muss unter Verwendung von Standards, die aus kommerziellen Quellen erhältlich sind (d.h. Sigma), definiert werden.

Pflanzenmaterial wird zunächst mechanisch durch Mörsern homogenisiert, um es einer Extraktion zugänglicher zu machen.

Dann wird 10 min auf 100°C erhitzt und nach dem Abkühlen auf Eis erneut sedimentiert. Das Zellsediment wird mit 1 M methanolischer Schwefelsäure und 2 % Dimethoxypropan für 1h bei 90°C hydrolysiert und die Lipide transmethyliert. Die resultierenden Fettsäuremethylester (FAME) werden in Petrolether extrahiert. Die extrahierten FAME werden durch Gasflüssigkeitschromatographie mit einer Kapillarsäule (Chrompack, WCOT Fused Silica, CP-Wax-52 CB, 25 m, 0,32 mm) und einem Temperaturgradienten von 170°C auf 240°C in 20 min und 5 min bei 240°C analysiert. Die Identität der Fettsäuremethylester wird durch Vergleich mit entsprechenden FAME-Standards (Sigma) bestätigt. Die Identität und die Position der Doppelbindung kann durch geeignete chemische Derivatisierung der FAME-Gemische z.B. zu 4,4-Dimethoxyoxazolin-Derivaten (Christie, 1998) mittels GC-MS weiter analysiert werden.

Beispiel 60: Analyse der Samen von den erzeugten transgenen Pflanzen

Entsprechend Beispiel 59, wurden die Samen der Pflanzen, die mit den Konstrukten pGPTV-Cnl1_d6Des(Pir)_d5Des(Tc)_D6Elo(Pp)_D12Des(Co), pSUN-5G und pSUN-8G transformiert wurden, analysiert. FigurXX zeigt dabei das Fettsäurespektrum von Samen mit dem Konstrukt pGPTV-Cnl1_d6Des(Pir)_d5Des(Tc)_D6Elo(Pp)_D12Des(Co). Im Vergleich zu KontrollPflanzen, die nicht transformiert wurden (Wildtyp-Kontrolle, WT) konnte eine deutliche Veränderung im Fettsäurespektrum festgestellt werden. Damit konnte gezeigt werden, dass die transformierten Gene funktionell sind. Tabelle 22 fasst die Ergebnisse aus Figur 32 zusammen.

Tabelle 22:

| Linien | Fettsäuren | | | | | | | | |
|---|---|---|---|---|---|---|---|---|---|
| | 16:0 | 18:0 | 18:1 | 18:2 | GLA | 18:3 | SDA | ARA | EPA |
| WT Kontrolle | 5,6 | 6,5 | 31,7 | 41,7 | nd | 12,1 | nd | nd | nd |
| 1424_Ko82_4 | 6,6 | 1,5 | 8,9 | 10,5 | 42,2 | 3,1 | 2,8 | 17,2 | 0,2 |
| 1424_Ko82_5 | 6,1 | 1,5 | 11,0 | 9,0 | 40,6 | 2,9 | 4,0 | 15,0 | 1,5 |
| 1424_Ko82_6 | 5,7 | 1,6 | 15,5 | 10,6 | 37,1 | 3,0 | 3,2 | 14,6 | 0,2 |
| 1424_Ko82_7 | 5,4 | 2,0 | 20,4 | 10,7 | 32,6 | 3,5 | 3,2 | 12,1 | 1,0 |
| 1424_Ko82_8 | 5,4 | 1,4 | 15,1 | 12,5 | 39,9 | 2,6 | 2,4 | 12,2 | 0,7 |
| 1424_Ko82_9 | 6,0 | 1,8 | 25,0 | 9,9 | 29,7 | 2,2 | 2,5 | 10,2 | 0,8 |
| 1424_Ko82_10 | 5,7 | 1,3 | 10,1 | 10,3 | 42,5 | 2,6 | 3,5 | 13,9 | 1,1 |
| 1424_Ko82_11 | 5,4 | 1,4 | 15,7 | 11,3 | 38,2 | 2,6 | 2,8 | 14,1 | 1,0 |

Die Analyse der Samen mit dem Konstrukt pSUN-5G zeigt dabei Linien, die eine deutliche Erhöhung des Gehaltes an Arachidonsäure verglichen mit dem Konstrukt pGPTV-Cnl1_d6Des(Pir)_d5Des(Tc)_D6Elo(Pp)_D12Des(Co) haben. Dabei konnten Linien mit bis zu 25 % ARA erhalten werden. Die zusätzliche Elongase (TL16y2) muss für diesen Effekt vorantwortlich sein (Figur31, pSUN-5G). Die Ergebnisse dieser Linie sind in Tabelle 23 zusammengefasst.

Tab. 23: Fettsäureanalytik von transgenen Samen, die mit dem Konstrukt pSUN-5G transformiert wurden.

| Linien | Fettsäuren | | | | | | | | | |
|---|---|---|---|---|---|---|---|---|---|---|
| | 16:0 | 18:0 | 18:1 | 18:2 LA | 18:3 GLA | 18:3 ALA | 18:4 SDA | 20:3 HGLA | ARA | EPA |
| WT | 5,2 | 2,3 | 34,2 | 37,9 | 0,0 | 11,6 | 0,0 | 0,0 | 0,0 | 0,0 |
| 16-1-2 | 4,2 | 1,6 | 20,1 | 21,5 | 25,9 | 4,1 | 1,8 | 1,7 | 8,9 | 0,8 |
| 16-1-3 | 5,8 | 2,3 | 9,9 | 14,6 | 33,6 | 3,1 | 2,2 | 2,2 | 16,0 | 1,4 |
| 16-1-8 | 5,0 | 2,8 | 11,1 | 12,6 | 34,9 | 2,2 | 1,8 | 2,6 | 16,3 | 1,2 |
| 16-2-1 | 4,9 | 1,6 | 14,5 | 17,4 | 32,9 | 3,5 | 2,0 | 1,6 | 12,3 | 1,0 |
| 16-2-5 | 5,5 | 3,3 | 12,9 | 13,8 | 32,9 | 2,9 | 2,2 | 1,4 | 15,4 | 1,4 |
| 16-4-2 | 5,8 | 2,5 | 18,8 | 14,7 | 32,0 | 3,5 | 2,3 | 1,2 | 12,0 | 1,2 |
| 16-4-3 | 5,9 | 2,0 | 19,7 | 15,0 | 32,0 | 3,8 | 2,4 | 1,1 | 11,4 | 1,2 |
| 16-7-2 | 6,2 | 4,4 | 14,3 | 10,2 | 30,7 | 2,0 | 2,1 | 1,7 | 19,4 | 1,9 |
| 16-7-3 | 5,0 | 2,5 | 21,6 | 13,6 | 30,7 | 2,1 | 1,8 | 1,5 | 12,6 | 1,1 |
| 16-7-4 | 5,3 | 4,1 | 18,8 | 19,5 | 23,1 | 4,2 | 2,2 | 2,9 | 11,3 | 1,4 |
| 16-7-5 | 7,4 | 1,8 | 4,2 | 6,8 | 33,7 | 1,8 | 2,7 | 2,6 | 25,8 | 2,6 |

Beispiel 61: Nachweis von DHA in Samen von transgenen Sareptasenf-Pflanzen.

Samen von Pflanzen, die mit dem Konstrukt pSUN-8G wie unter Beispiel 58 beschrieben hergestellt wurden, wurden wie in Beispiel 59 beschrieben, analysiert. Neben den LCPUFA Arachidonsäure und Eicosapentaensäure konnte in diesen Samen auch Docosahexaensäure nachgewiesen werden, das Produkt nach Umsetzung durch die Δ4-Desaturase aus Thraustochytrium und den Δ5-Elongasen aus Onchorynchis mykiss und Ostreococcus tauri. Figur 32 zeigt das Chromatogramm mit dem geänderten Fettsäurespektrum im Vergleich zu einer nicht-transformierten Kontrollpflanze. In Tabelle 24 sind die Ergebnisse mehrerer Messungen zusammengefasst.

Tabelle. 24 gibt die Fettsäureanalytik von transgenen Samen, die mit dem Konstrukt pSUN-8G transformiert wurden.

Mit diesem Experiment konnte zum ersten Mal die Synthese von Docosahexaensäure in Samen demonstriert werden. Z.B. in WO 2004/071467 wird zwar die Synthese von DHA in höheren Pflanzen beschrieben, allerdings konnte die Synthese nicht für Samen gezeigt werden, nur für eine embryogene Zellkultur.

## Patentansprüche

1. Transgene Öl-produzierende Pflanze der Gattung Brassica, wobei die Pflanze unter Kontrolle von Samen-spezifischen Promotoren oder Promotoren aktiv in Pflanzenembryo oder Endosperm

   a) eine delta-6 Desaturase,
   b) eine delta-6 Elongase,
   c) eine delta-5 Desaturase,
   d) eine delta-12 Desaturase, und
   e) eine delta-5 Elongase
   - Aktivität aufweist, wobei die Pflanze Öl mit DHA und/oder EPA in den Samen herstellt.

2. Transgene Pflanze nach Anspruch 1, wobei eine oder mehr Desaturasen Acyl-CoA abhängige Desaturasen sind.

3. Transgene Pflanze nach Anspruch 1 oder 2, wobei das Öl weniger als 15% EPA enthält.

4. Transgene Pflanze nach einem der Ansprüche 1 bis 3, wobei das Öl einen Gehalt an Erucasäure von weniger als 2 Gew.-% bezogen auf den Gesamtfettsäuregehalt der Pflanzen hat.

5. Transgene Pflanze nach einem der Ansprüche 1 bis 4, wobei die Pflanze in ihrem Samen ungesättigte C18-, C20-, und/oder C22-Alkylcarbonylsäuren mit mindestens drei, vier, fünf oder sechs Doppelbindungen mit einem Gehalt von mindestens 15 Gew-% bezogen auf die gesamten Fettsäuren im Samen der Pflanze herstellt.

6. Transgene Pflanze nach einem der Ansprüche 1 bis 5, wobei die Pflanze unter Kontrolle von Samen-spezifischen Promotoren oder Promotoren aktiv in Pflanzenembryo oder Endosperm weiterhin delta-4-Desaturase; und/oder omega-3-Desaturase-Aktivität aufweist, .

7. Transgene Pflanze nach einem der Ansprüche 1 bis 6, exprimierend eine Nukleinsäuresequenz, die für Polypeptide mit omega-3-Desaturaseaktivität codiert, ausgewählt aus der Gruppe bestehend aus:

   a) einer Nukleinsäuresequenz mit der in SEQ ID NO: 87 oder SEQ ID NO: 105 dargestellten Sequenz,oder
   b) Nukleinsäuresequenzen, die sich als Ergebnis des degenerierten genetischen Codes von der in SEQ ID NO: 88 oder SEQ ID NO: 106 dargestellten Aminosäuresequenz ableiten lassen, oder
   c) Nukleinsäuresequenz, die für Polypeptide mit mindestens 60 % Identität auf Aminosäureebene mit SEQ ID NO: 88 oder SEQ ID NO: 106 codieren und eine omega-3-Desaturaseaktivität aufweisen.

8. Transgene Pflanze gemäß einem der Ansprüche 1 bis 7, ferner enthaltend eine Nukleinsäuresequenz, die für Polypeptide mit delta-12-Desaturaseaktivität codiert, ausgewählt aus der Gruppe bestehend aus:

   a) einer Nukleinsäuresequenz mit der in SEQ ID NO: 107, SEQ ID NO: 109 oder SEQ ID NO: 195 dargestellten Sequenz,oder
   b) Nukleinsäuresequenzen, die sich als Ergebnis des degenerierten genetischen Codes von der in SEQ ID NO: 108, SEQ ID NO: 110 oder SEQ ID NO: 196 dargestellten Aminosäuresequenz ableiten lassen, oder
   c) Nukleinsäuresequenzen, die für Polypeptide mit mindestens 60 % Identität auf Aminosäureebene mit SEQ ID NO: 108, SEQ ID NO: 110 oder SEQ ID NO: 196 codieren und eine delta-12-Desaturaseaktivität aufweisen.

9. Transgene Pflanze nach einem der Ansprüche 1 bis 8, ferner enthaltend eine Nukleinsäuresequenz, die für Polypeptide mit delta-6-Desaturaseaktivität codiert, ausgewählt aus der Gruppe bestehend aus

   a) Nukleinsäuresequenz codierend für eine Aminosäuresequenz gemäß SEQ ID NO 202 oder mindestens mit 60% Sequenzidentität zu der Aminosäuresequenz gemäß SEQ ID NO 202 und eine delta-6-Desaturaseaktivität aufweisend;
   b) Nukleinsäuresequenzen, die sich als Ergebnis des degenerierten genetischen Codes von der in SEQ ID NO:

202 dargestellten Aminosäuresequenz ableiten lassen und eine delta-6-Desaturaseaktivität aufweisend; und
c) einer Nukleinsäuresequenz mit der in SEQ ID NO: 201 dargestellten Sequenz oder mindestens 60% Sequenzidentität zu der Nukleinsäuresequenz gemäß SEQ ID NO 201.

**10.** Same einer transgenen Öl-produzierende Pflanze nach einem der Ansprüche 1 bis 9, ., wobei der Samen unter Kontrolle von Samen-spezifischen Promotoren oder Promotoren aktiv in Pflanzenembryo oder Endosperm

a) eine delta-6 Desaturase,
b) eine delta-6 Elongase,
c) eine delta-5 Desaturase,
d) eine delta-12 Desaturase, und
e) eine delta-5 Elongase
- Aktivität aufweist und wobei der Samen Öl mit einem Anteil von DHA von mindestens 0,2 Gew-% im enthält.

**11.** Transgene Pflanze nach einem der Ansprüche 1 bis 9 oder Same gemäß Anspruch 10, wobei der EPA Gehalt mindestens 5% und/oder der Docosahexaensäure-Gehalt mindestens 0,2 Gew-% bezogen auf den gesamten Lipidgehalt in den Samen der transgenen Pflanze beträgt.

**12.** Transgene Pflanze nach einem der Ansprüche 1 bis 11 oder Same gemäß Anspruch 10 oder 11, **dadurch gekennzeichnet, dass** im Samen der transgenen Pflanze der Gehalt an Docosahexaensäure mindestens 0,5 Gew.-% bezogen auf den Gesamtlipidgehalt beträgt.

**13.** Pflanze nach einem der Ansprüche 1 bis 12 oder Same gemäß einem der Ansprüche 10 bis 12, wobei der EPA Gehalt mindestens 5% und/oder der Docosahexaensäure-Gehalt mindestens 0,5 Gew-% bezogen auf den gesamten Lipidgehalt in den Samen der transgenen Pflanze beträgt.

**14.** Same der Pflanze nach einem der Ansprüche 1 bis 13. wobei der Samen unter Kontrolle von Samen-spezifischen Promotoren oder Promotoren aktiv in Pflanzenembryo oder Endosperm

a) ein delta-6 Desaturase,
b) ein delta-6 Elongase,
c) ein delta-5 Desaturase,
d) ein delta-12 Desaturase, und
e) ein delta-5 Elongase
- Gen aufweist, wobei die Pflanze Öl mit DHA und/oder EPA in dem Samen herstellt.

**15.** Verfahren zu Herstellung einer transgenen Pflanze nach einem der Ansprüche 1 bis 14, umfassend das Einbringen durch Transformation der genannten Enzyme in eine Öl-produzierende Pflanze.

**16.** Verwendung einer transgenen Pflanze nach einem der Ansprüche 1 bis 13 oder des Samens nach einem der Ansprüche 10 bis 14, zur Herstellung eines Öls, Lipids oder einer freien Fettsäure umfassend das Gewinnen der Öle, Lipide oder freien Fettsäuren aus der Pflanze oder Samen nach einem der vorgenannten Ansprüche.

**17.** Verwendung einer transgenen Pflanze nach einem der Ansprüche 1 bis 13 oder Samens nach einem der Ansprüche 10 bis 14 zu Herstellung von Ölen, Lipiden oder freien Fettsäure aus einer Öl-produzierende Pflanze nach einem der Ansprüche 1 bis 14 oder aus Samen nach einem der Ansprüche 10 bis 14, umfassend das Zerkleinern, Dämpfen oder Rösten von Samen der Öl-produzierende Pflanze nach einem der Ansprüche 1 bis 14 oder der Samen.

**18.** Verwendung einer transgenen Pflanze nach einem der Ansprüche 1 bis 14 oder Samens nach einem der Ansprüche 10 bis 14 zu Herstellung von Ölen, Lipiden oder freien Fettsäure aus einer Öl-produzierende Pflanze nach einem der Ansprüche 1 bis 16 oder aus Samen nach einem der Ansprüche 10 bis 14, umfassend das Pressen von Samen der Öl-produzierende Pflanze nach einem der Ansprüche 1 bis 16.

**19.** Verwendung nach einem der Ansprüche 16 bis 18, wobei das hergestellte Öl einem Anteil von DHA von mindestens 0,2 Gew-% und weniger als 15% EPA und/oder ARA enthält.

**20.** Verwendung nach einem der Ansprüche 16 bis 19, wobei Arachidonsäure, Eicosapentaensäure, Docosapentaensäure oder Docosahexaensäure, Eicosapentaensäure und/oder Docosahexaensäure hergestellt werden und anschließend für verschiedene Zwecke bei Nahrungsmittel-, Futter-, Kosmetik- oder pharmazeutischen Anwendungen verwendet werden.

## Claims

1. A transgenic, oil-producing plant of the genus Brassica, wherein the plant, under control of seed-specific promoters or promoters active in plant embryo or endosperm, exhibits

   a) a delta-6 desaturase,
   b) a delta-6 elongase,
   c) a delta-5 desaturase,
   d) a delta-12 desaturase, and
   e) a delta-5 elongase

   activity, the plant producing oil with DHA and/or EPA in the seeds.

2. The transgenic plant according to claim 1, wherein one or more desaturases are acyl-CoA-dependent desaturases.

3. The transgenic plant according to claim 1 or 2, wherein the oil contains less than 15% of EPA.

4. The transgenic plant according to any of claims 1 to 3, wherein the oil has an erucic acid content of less than 2% by weight, based on the total fatty acid content of the plant.

5. The transgenic plant according to any of claims 1 to 4, wherein the plant in its seed produces unsaturated C18-, C20- and/or C22-alkylcarbonyl acids having at least three, four, five or six double bonds with a content of at least 15% by weight, based on the total fatty acids in the seed of the plant.

6. The transgenic plant according to any of claims 1 to 5, wherein the plant, under control of seed-specific promoters or promoters active in plant embryo or endosperm, further exhibits delta-4 desaturase and/or omega-3 desaturase activity.

7. The transgenic plant according to any of claims 1 to 6, expressing a nucleic acid sequence which codes for polypeptides having omega-3 desaturase activity, selected from the group consisting of:

   a) a nucleic acid sequence having the sequence shown in SEQ ID NO: 87 or SEQ ID NO: 105, or
   b) nucleic acid sequences which can be derived as a result of the degenerate genetic code from the amino acid sequence shown in SEQ ID NO: 88 or SEQ ID: NO 106, or
   c) nucleic acid sequences which code for polypeptides having at least 60% identity at amino acid level with SEQ ID NO: 88 or SEQ ID NO: 106 and which have an omega-3 desaturase activity.

8. The transgenic plant according to any of claims 1 to 7, further comprising a nucleic acid sequence which codes for polypeptides having delta-12 desaturase activity, selected from the group consisting of:

   a) a nucleic acid sequence having the sequence shown in SEQ ID NO: 107, SEQ ID NO: 109 or SEQ ID NO: 195, or
   b) nucleic acid sequences which can be derived as a result of the degenerate genetic code from the amino acid sequence shown in SEQ ID NO: 108, SEQ ID NO: 110 or SEQ ID: NO 196, or
   c) nucleic acid sequences which code for polypeptides having at least 60% identity at amino acid level with SEQ ID NO: 108, SEQ ID NO: 110 or SEQ ID NO: 196 and which have a delta-12 desaturase activity.

9. The transgenic plant according to any of claims 1 to 8, further comprising a nucleic acid sequence which codes for polypeptides having delta-6 desaturase activity, selected from the group consisting of

   a) nucleic acid sequence coding for an amino acid sequence according to SEQ ID NO: 202, or at least with 60% sequence identity to the amino acid sequence according to SEQ ID NO: 202, and having a delta-6 desaturase activity;
   b) nucleic acid sequences which can be derived as a result of the degenerate genetic code from the amino acid

sequence shown in SEQ ID NO: 202, and having a delta-6 desaturase activity; and
c) a nucleic acid sequence having the sequence shown in SEQ ID NO: 201 or at least 60% sequence identity to the nucleic acid sequence according to SEQ ID NO: 201.

**10.** A seed of a transgenic, oil-producing plant according to any of claims 1 to 9, where the seed, under control of seed-specific promoters or promoters active in plant embryo or endosperm, exhibits

a) a delta-6 desaturase,
b) a delta-6 elongase,
c) a delta-5 desaturase,
d) a delta-12 desaturase, and
e) a delta-5 elongase

activity and where the seed contains oil having a fraction of DHA of at least 0.2% by weight.

**11.** The transgenic plant according to any of claims 1 to 9 or seed according to claim 10, wherein the EPA content is at least 5% and/or the docosahexaenoic acid content is at least 0.2% by weight, based on the total lipid content in the seeds of the transgenic plant.

**12.** The transgenic plant according to any of claims 1 to 11 or seed according to claim 10 or 11, **characterized in that** the content of docosahexaenoic acid in the seed of the transgenic plant is at least 0.5% by weight, based on the total lipid content.

**13.** The plant according to any of claims 1 to 12 or seed according to any of claims 10 to 12, wherein the EPA content is at least 5% and/or the docosahexaenoic acid content is at least 0.5% by weight, based on the total lipid content in the seeds of the transgenic plant.

**14.** The seed of the plant according to any of claims 1 to 13, wherein the seed, under control of seed-specific promoters or promoters active in plant embryo or endosperm, has

a) a delta-6 desaturase,
b) a delta-6 elongase,
c) a delta-5 desaturase,
d) a delta-12 desaturase, and
e) a delta-5 elongase

gene, where the plant produces oil with DHA and/or EPA in the seeds.

**15.** A process for producing a transgenic plant according to any of claims 1 to 14, comprising the introduction of the stated enzymes by transformation into an oil-producing plant.

**16.** The use of a transgenic plant according to any of claims 1 to 13 or of the seed according to any of claims 10 to 14 for producing an oil, lipid or free fatty acid, comprising the recovery of the oils, lipids or free fatty acids from the plant or seed according to any of the preceding claims.

**17.** The use of a transgenic plant according to any of claims 1 to 13 or seed according to any of claims 10 to 14 for producing oils, lipids or free fatty acid from an oil-producing plant according to any of claims 1 to 14 or from seed according to any of claims 10 to 14, comprising the comminution, steaming or roasting of seeds of the oil-producing plant according to any of claims 1 to 14 or of the seeds.

**18.** The use of a transgenic plant according to any of claims 1 to 14 or seed according to any of claims 10 to 14 for producing oils, lipids or free fatty acid from an oil-producing plant according to any of claims 1 to 16 or from seed according to any of claims 10 to 14, comprising the pressing of seeds of the oil-producing plant according to any of claims 1 to 16.

**19.** The use according to any of claims 16 to 18, wherein the oil produced contains a fraction of DHA of at least 0.2 wt% and less than 15% of EPA and/or ARA.

20. The use according to any of claims 16 to 19, wherein arachidonic acid, eicosapentaenoic acid, docosapentaenoic acid or docosahexaenoic acid, eicosapentaenoic acid and/or docosahexaenoic acid are produced and are subsequently used for various purposes in foodstuff, feed, cosmetic or pharmaceutical applications.

## Revendications

1. Plante transgénique produisant de l'huile du genre Brassica, la plante présentant, sous le contrôle de promoteurs spécifiques aux graines ou de promoteurs actifs dans l'embryon de la plante ou l'endosperme, une activité

   a) de delta-6 désaturase,
   b) de delta-6 élongase,
   c) de delta-5 désaturase,
   d) de delta-12 désaturase et
   e) de delta-5 élongase,

   la plante fabriquant dans les graines une huile avec du DHA et/ou de l'EPA.

2. Plante transgénique selon la revendication 1, dans laquelle une ou plusieurs désaturases sont des désaturases dépendant d'acyl-CoA.

3. Plante transgénique selon la revendication 1 ou 2, dans laquelle l'huile contient moins de 15 % d'EPA.

4. Plante transgénique selon l'une des revendications 1 à 3, dans laquelle l'huile présente une teneur en acide érucasique inférieure à 2 % en poids par rapport à la teneur totale en acides gras des plantes.

5. Plante transgénique selon l'une des revendications 1 à 4, la plante fabriquant dans ses graines des acides (alkyle en C18, en C20 et/ou en C22) carboxyliques insaturés ayant au moins trois, quatre, cinq ou six doubles liaisons, en une teneur d'au moins 15 % en poids par rapport à la totalité des acides gras dans la graine de la plante.

6. Plante transgénique selon l'une des revendications 1 à 5, la plante présentant en outre, sous le contrôle de promoteurs spécifiques aux graines ou de promoteurs actifs dans l'embryon de la plante ou dans l'endosperme, une activité de delta-4-désaturase et/ou d'oméga-3-désaturase.

7. Plante transgénique selon l'une des revendications 1 à 6, exprimant une séquence d'acides nucléiques qui code pour des polypeptides ayant une activité d'oméga-3-désaturase, choisie dans le groupe consistant en :

   a) une séquence d'acides nucléiques ayant la séquence présentée dans SEQ ID NO : 87 ou SEQ ID NO : 105, ou
   b) les séquences d'acides nucléiques qui, en tant que résultat du code génétique dégénéré, peuvent dériver de la séquence d'acides nucléiques présentée dans SEQ ID NO : 88 ou SEQ ID NO : 106, ou
   c) les séquences d'acides aminés qui codent pour des polypeptides ayant une identité d'au moins 60 %, sur le plan des acides aminés, avec SEQ ID NO : 88 ou SEQ ID NO : 106, et présentent une activité d'oméga-3-désaturase.

8. Plante transgénique selon l'une des revendications 1 à 7, contenant en outre une séquence d'acides nucléiques qui code pour des polypeptides ayant une activité de delta-12-désaturase, choisie dans le groupe consistant en :

   a) une séquence d'acides nucléiques ayant la séquence présentée dans SEQ ID NO : 107, SEQ ID NO : 109 ou SEQ ID NO : 195, ou
   b) les séquences d'acides nucléiques qui, en tant que résultat du code génétique dégénéré, peuvent dériver de la séquence d'acides aminés présentée dans SEQ ID NO : 108, SEQ ID NO : 110 ou SEQ ID NO : 196, ou
   c) les séquences d'acides nucléiques qui codent pour des polypeptides ayant une identité d'au moins 60 %, sur le plan des acides aminés, avec SEQ ID NO : 108, SEQ ID NO : 110 ou SEQ ID NO : 196, et présentent une activité de delta-12-désaturase.

9. Plante transgénique selon l'une des revendications 1 à 8, contenant en outre une séquence d'acides nucléiques qui code pour des polypeptides ayant une activité de delta-6-désaturase, choisie dans le groupe consistant en

a) la séquence d'acides nucléiques codant pour une séquence d'acides aminés selon SEQ ID NO : 202 ou présentant une identité de séquence d'au moins 60 % avec la séquence d'acides aminés selon SEQ ID NO : 202 et une activité de delta-6-désaturase ;
b) les séquences d'acides nucléiques qui, en tant que résultat du code génétique dégénéré, peuvent dériver de la séquence d'acides aminés présentée dans SEQ ID NO : 202, et présentant une activité de delta-6-désaturase, et
c) une séquence d'acides nucléiques ayant la séquence présentée dans SEQ ID NO : 201 ou une identité de séquence d'au moins 60 % avec la séquence d'acides nucléiques selon SEQ ID NO : 201.

**10.** Graine d'une plante transgénique produisant de l'huile selon l'une des revendications 1 à 9, la graine présentant, sous le contrôle de promoteurs spécifiques aux graines ou de promoteurs actifs dans l'embryon de la plante ou dans l'endosperme, une activité

a) de delta-6 désaturase,
b) de delta-6 élongase,
c) de delta-5 désaturase,
d) de delta-12 désaturase et
e) de delta-5 élongase,

la graine contenant une huile présentant une proportion de DHA d'au moins 0,2 % en poids.

**11.** Plante transgénique selon l'une des revendications 1 à 9, ou graine selon la revendication 10, la teneur en EPA étant d'au moins 5 % et/ou la teneur en acide docosahexaénoïque étant d'au moins 0,2 % en poids par rapport à la totalité de la teneur en lipides des graines de la plante transgénique.

**12.** Plante transgénique selon l'une des revendications 1 à 11 ou graine selon la revendication 10 ou 11, **caractérisée en ce que** la teneur de la graine de la plante transgénique en acide docosahexaénoïque est d'au moins 0,5 % en poids par rapport à la teneur totale en lipides.

**13.** Plante selon l'une des revendications 1 à 12 ou graine selon l'une des revendications 10 à 12, la teneur en EPA étant d'au moins 5 % et/ou la teneur en acide docosahexaénoïque étant d'au moins 0,5 % en poids par rapport à la totalité de la teneur en lipides des graines de la plante transgénique.

**14.** Graine de la plante selon l'une des revendications 1 à 13, la graine présentant, sous le contrôle de promoteurs spécifiques aux graines ou de promoteurs actifs dans l'embryon de la plante ou dans l'endosperme, un gène

a) de delta-6 désaturase,
b) de delta-6 élongase,
c) de delta-5 désaturase,
d) de delta-12 désaturase et
e) de delta-5 élongase,

la plante fabriquant dans les graines de l'huile contenant du DHA et/ou de l'EPA.

**15.** Procédé de fabrication d'une plante transgénique selon l'une des revendications 1 à 14, comprenant l'insertion par transformation, dans une plante produisant de l'huile, des enzymes mentionnées.

**16.** Utilisation d'une plante transgénique selon l'une des revendications 1 à 13 ou de la graine selon l'une des revendications 10 à 14 pour la fabrication d'une huile, d'un lipide ou d'un acide gras libre, comprenant l'extraction des huiles, des lipides ou des acides gras libres à partir de la plante ou de la graine selon l'une des revendications précédentes.

**17.** Utilisation d'une plante transgénique selon l'une des revendications 1 à 13 ou de la graine selon l'une des revendications 10 à 14 pour la fabrication d'huiles, de lipides ou d'un acide gras, à partir d'une plante produisant de l'huile selon l'une des revendications 1 à 14 ou de graines selon l'une des revendications 10 à 14, comprenant le broyage, l'étuvage ou la torréfaction de graines de la plante produisant de l'huile selon l'une des revendications 1 à 14 ou des graines.

**18.** Utilisation d'une plante transgénique selon l'une des revendications 1 à 14 ou de la graine selon l'une des revendications 10 à 14 pour la fabrication d'huiles, de lipides ou d'un acide gras libre à partir d'une plante produisant de l'huile selon l'une des revendications 1 à 16 ou de graines selon l'une des revendications 10 à 14, comprenant le pressage de graines de la plante produisant de l'huile selon l'une des revendications 1 à 16.

**19.** Utilisation selon l'une des revendications 16 à 18, l'huile fabriquée contenant une proportion de DHA d'au moins 0,2 % en poids et moins de 15 % d'EPA et/ou d'ARA.

**20.** Utilisation selon l'une des revendications 16 à 19, par laquelle on fabrique de l'acide arachidonique, de l'acide éicosapentaénoïque, de l'acide docosapentaénoïque ou de l'acide docosahexaénoïque, de l'acide éicosapentaénoïque et/ou de l'acide docosahexaénoïque, puis on les utilise à diverses fins dans des applications alimentaires, fourragères, cosmétiques ou pharmaceutiques.

Figur 1: Verschiedene Synthese-Wege zur Biosynthese von DHA (Docosahexaensäure)

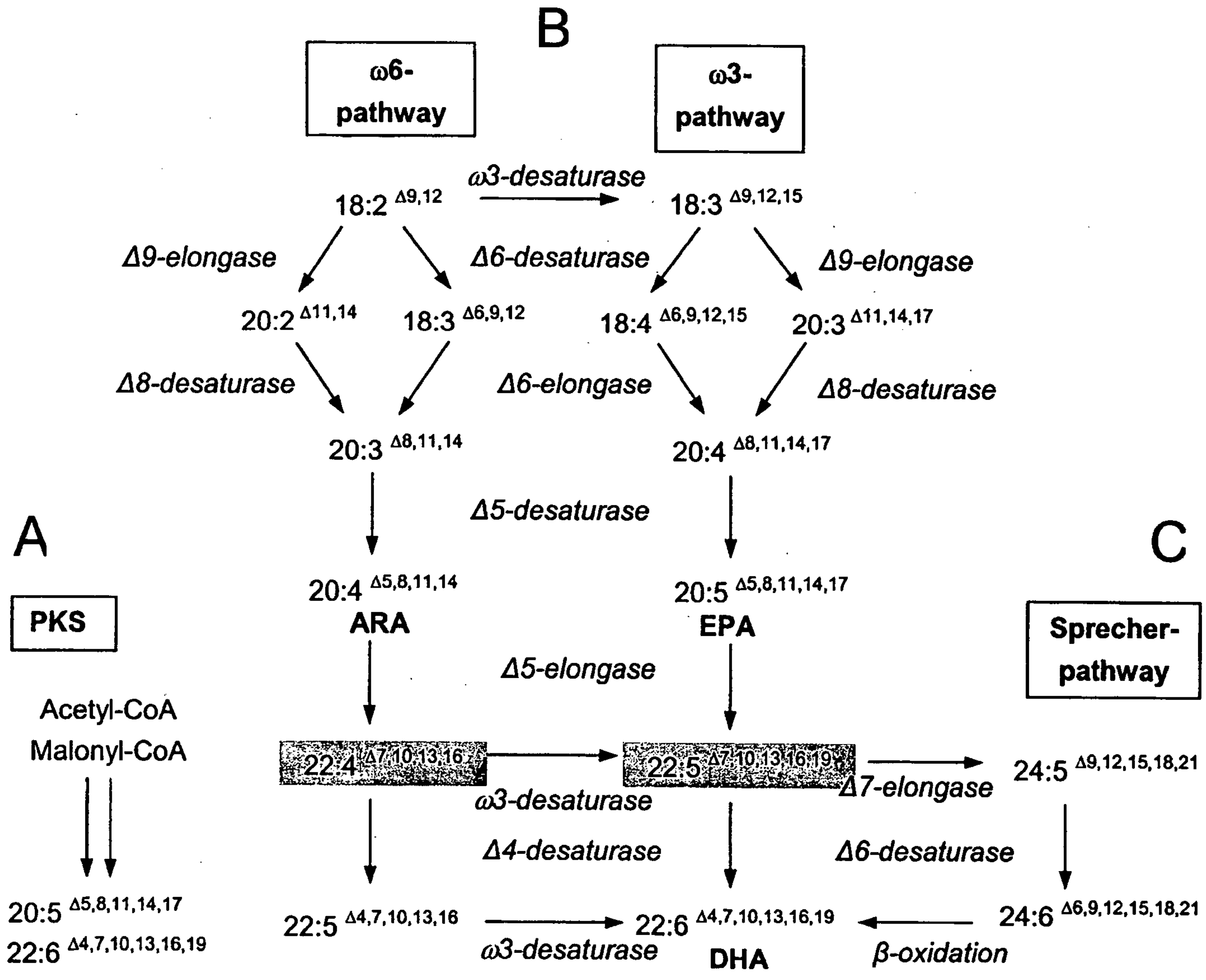

Figur 2: Substratspezifität der Δ-5-Elongase (SEQ ID NO: 53) gegenüber verschiedenen Fettsäuren

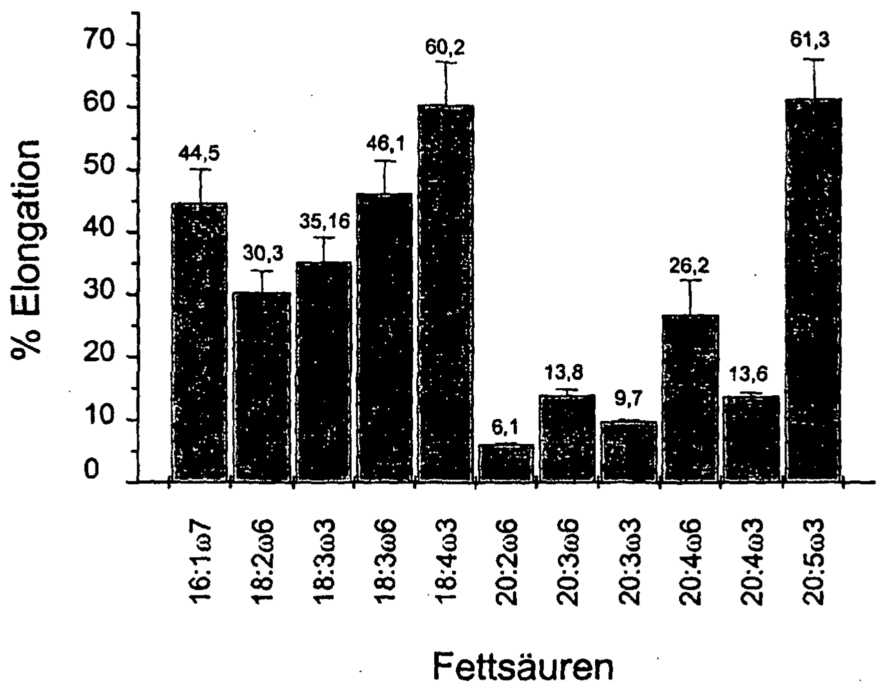

Figur 3: Rekonstitution der DHA-Biosynthese in Hefe ausgehend von 20:5ω3.

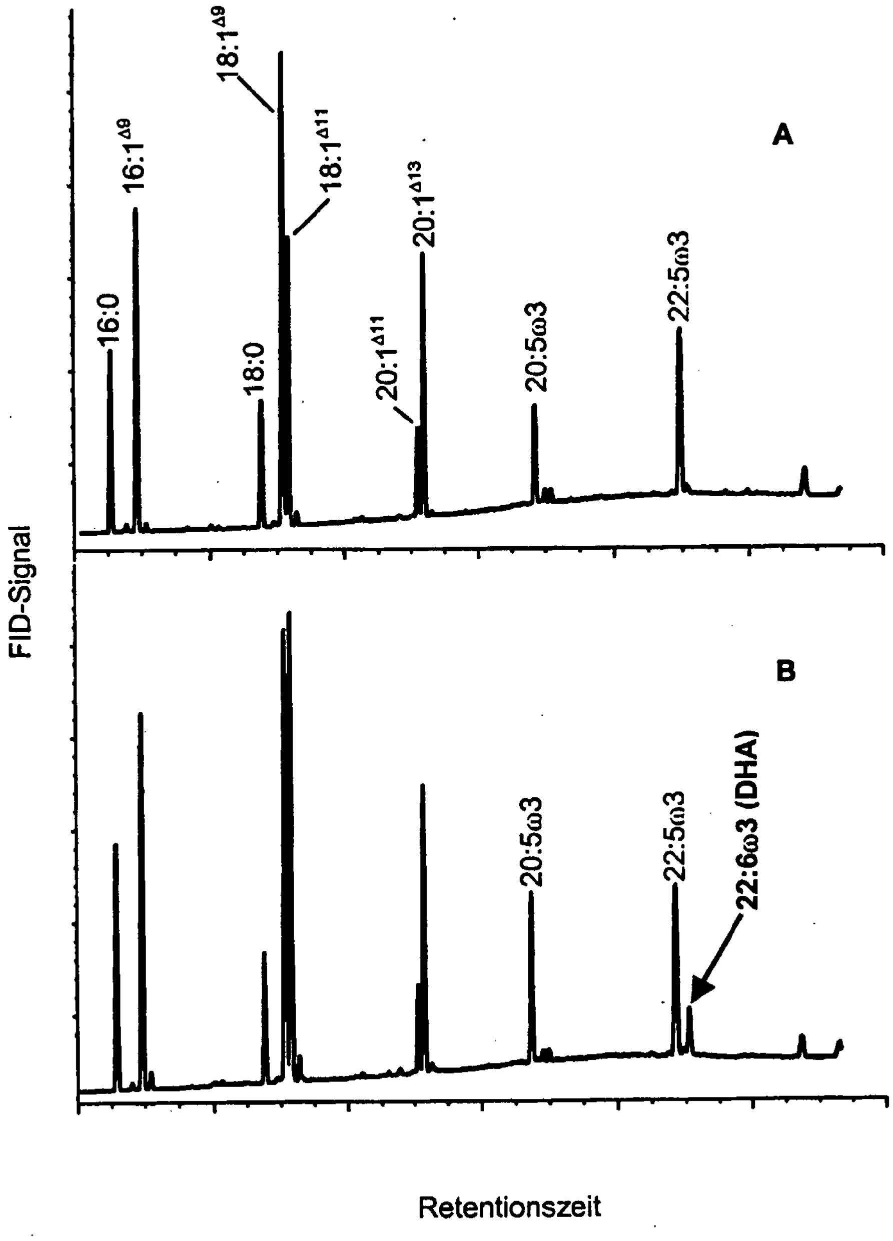

Figur 4: Rekonstitution der DHA-Biosynthese in Hefe ausgehend von 18:4ω3.

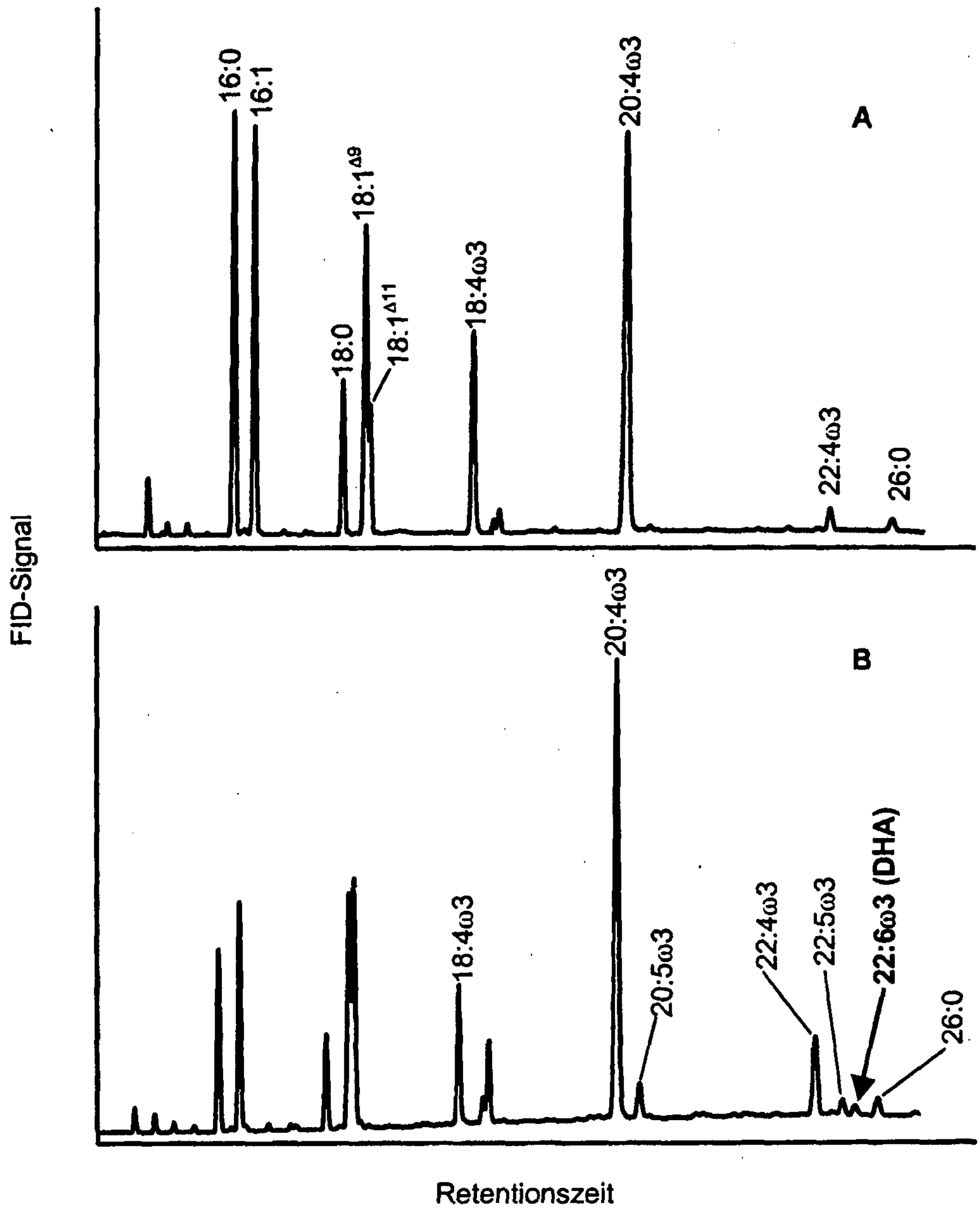

Figur 5: Fettsäure-Zusammensetzung (in Mol %) transgener Hefen, die mit den Vektoren pYes3-OmELO3/pYes2-EgD4 oder pYes3-OmELO3/pYes2-EgD4+pESCLeu-PtD5 transformiert worden waren. Die Hefezellen wurden in Minimalmedium ohne Tryptophan und Uracil / und Leucin in Gegenwart von 250 $\mu$M $20{:}5^{\Delta 5,8,11,14,17}$ bzw. $18{:}4^{\Delta 6,9,12,15}$ kultiviert. Die Fettsäuremethylester wurden durch saure Methanolyse aus Zellsedimenten gewonnen und über GLC analysiert. Jeder Wert gibt den Mittelwert (n=4) ± Standardabweichung wieder.

| | pYes3-OmELO/pYes2-EgD4 | pYes3-OmELO/pYes2-EgD4 EgD4 + pESCLeu-PtD5 |
|---|---|---|
| Fettsäuren | Fütterung mit $20{:}5^{\Delta 5,8,11,14,17}$ | Fütterung mit $18{:}4^{\Delta 6,9,12,15}$ |
| 16:0 | 9,35 ± 1,61 | 7,35 ± 1,37 |
| $16{:}1^{\Delta 9}$ | 14,70 ± 2,72 | 10,02 ± 1,81 |
| 18:0 | 5,11 ± 1,09 | 4,27 ± 1,21 |
| $18{:}1^{\Delta 9}$ | 19,49 ± 3,01 | 10,81 ± 1,95 |
| **$18{:}1^{\Delta 11}$** | **18,93 ± 2,71** | **11,61 ± 1,48** |
| **$18{:}4^{\Delta 6,9,12,15}$** | - | **7,79 ± 1,29** |
| **$20{:}1^{\Delta 11}$** | **3,24 ± 0,41** | **1,56 ± 0,23** |
| **$20{:}1^{\Delta 13}$** | **11,13± 2,07** | **4,40 ± 0,78** |
| **$20{:}4^{\Delta 8,11,14,17}$** | - | **30,05 ± 3,16** |
| **$20{:}5^{\Delta 5,8,11,14,17}$** | **6,91± 1,10** | **3,72 ± 0,59** |
| **$22{:}4^{\Delta 10,13,16,17}$** | - | **5,71 ± 1,30** |
| **$22{:}5^{\Delta 7,10,13,16,19}$** | **8,77 ± 1,32** | **1,10 ± 0,27** |
| **$22{:}6^{\Delta 4,7,10,13,16,19}$** | **2,73 ± 0,39** | **0,58 ± 0,10** |

Figur 6: Fütterungsexperiment zur Bestimmung der Funktionalität und Substratspezifität mit Hefestämmen

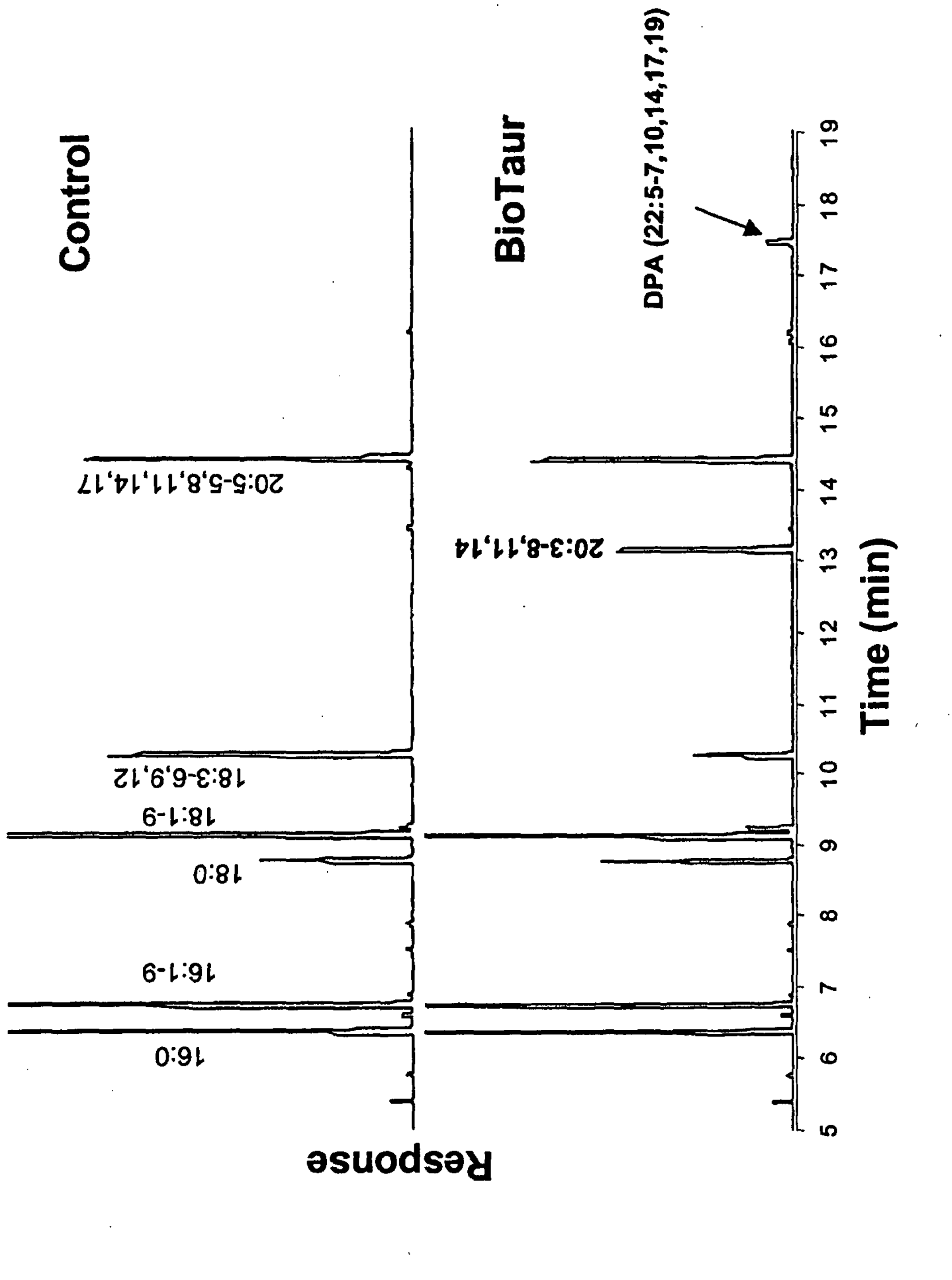

Figur 7: Elongation von Eicosapentaensäure durch OtElo1

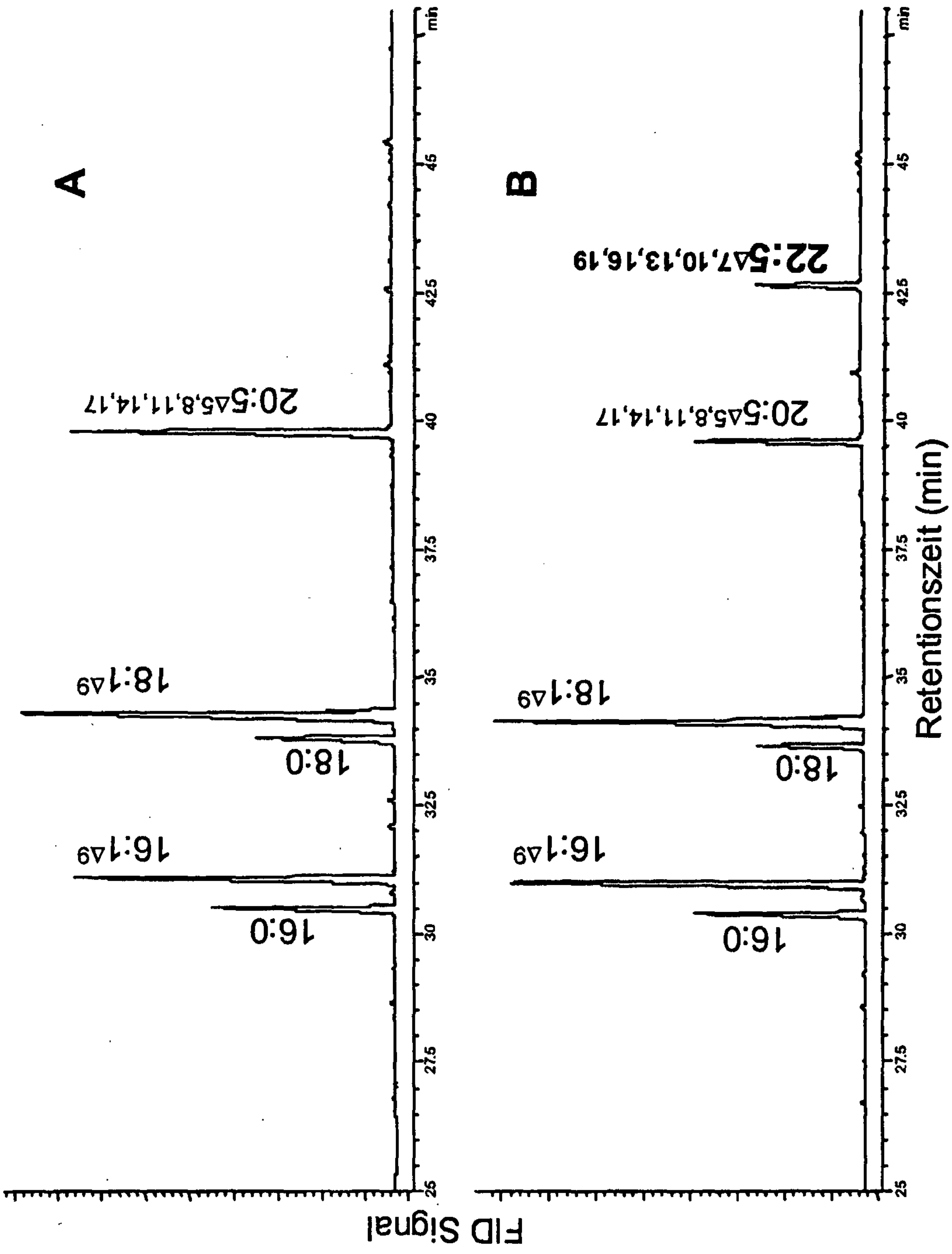

Figur 8: Elongation von Arachidonsäure durch OtElo1

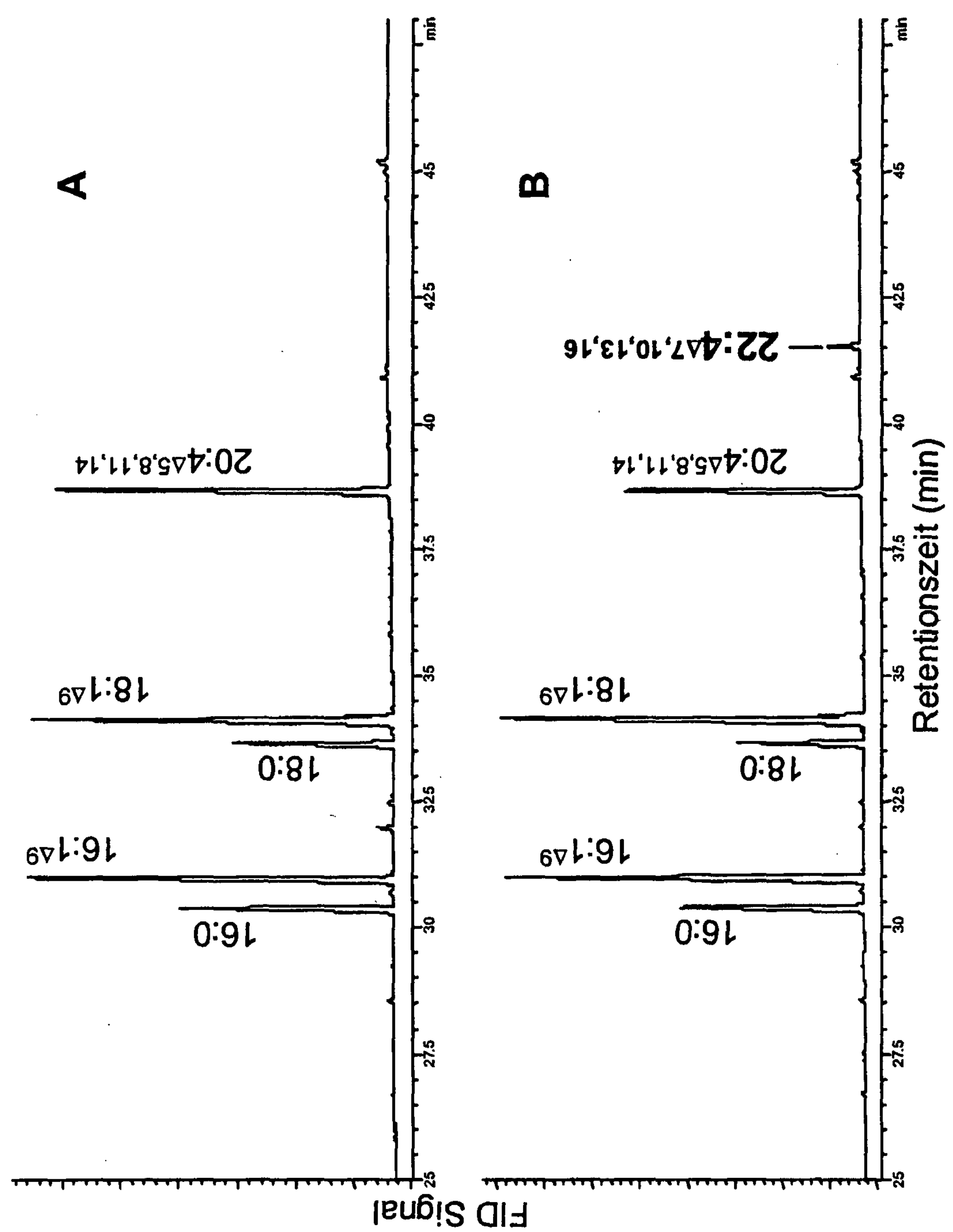

Figur 9: Expression von TpELO1 in Hefe

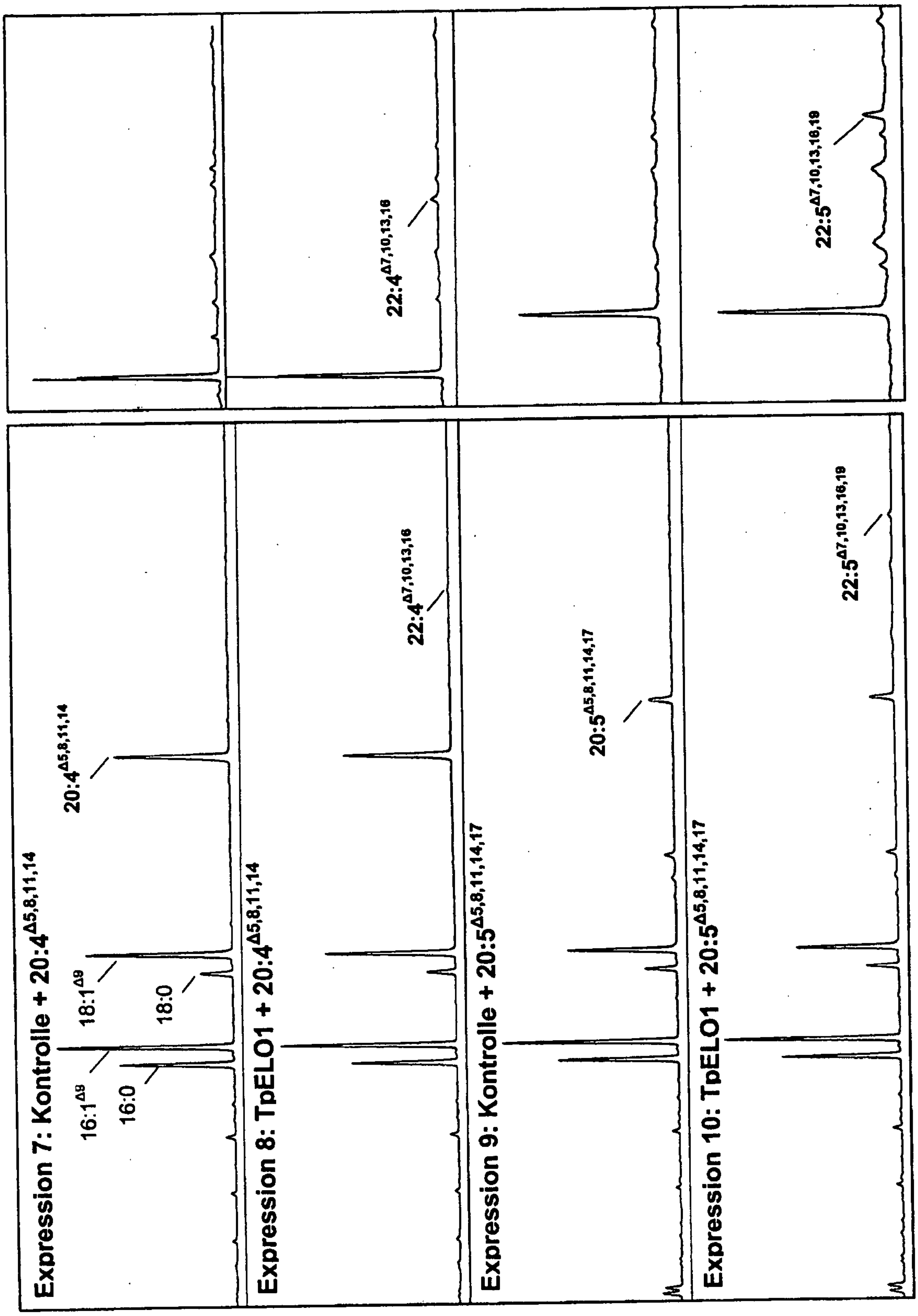

Figur 10: Expression von TpELO3 in Hefe.

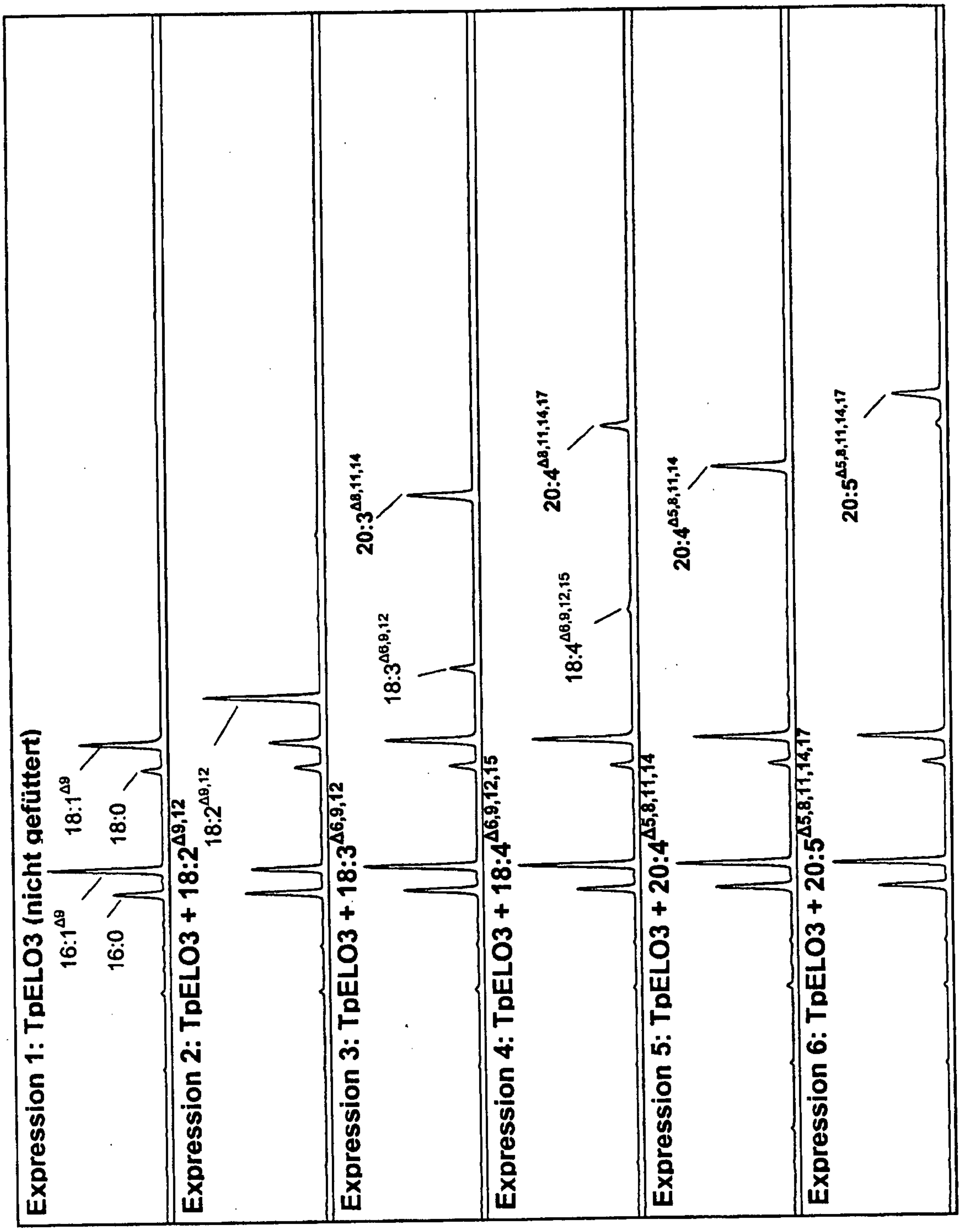

Figur 11: Expression von Thraustochytrium Δ5-Elongase TL16/pYES2.1 in Hefe.

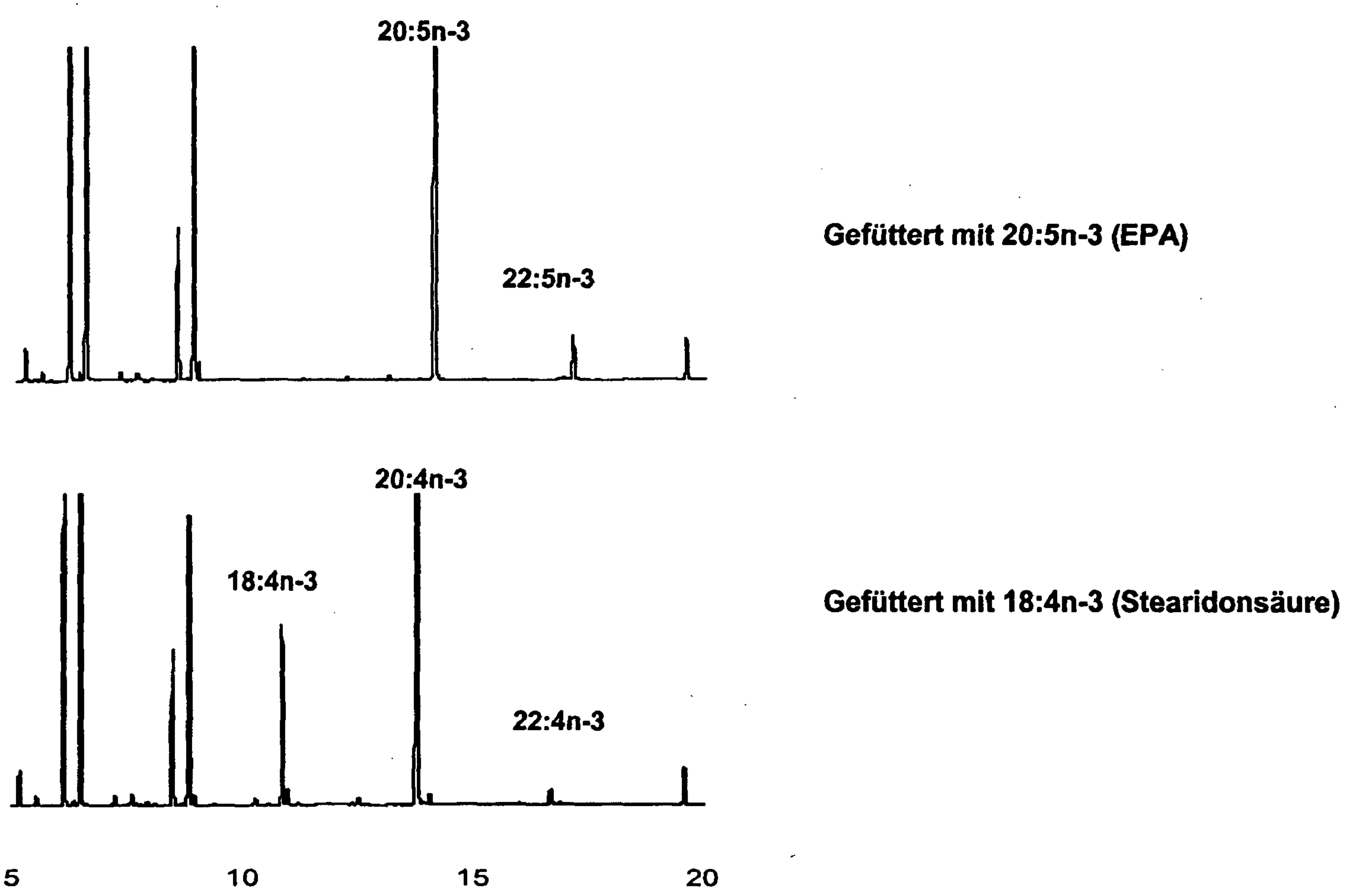

Figur 12: Desaturierung von Linolsäure (18:2 ω-6-Fettsäure) zu α-Linolensäure (18:3 ω-3-Fettsäure) durch Pi-omega3Des.

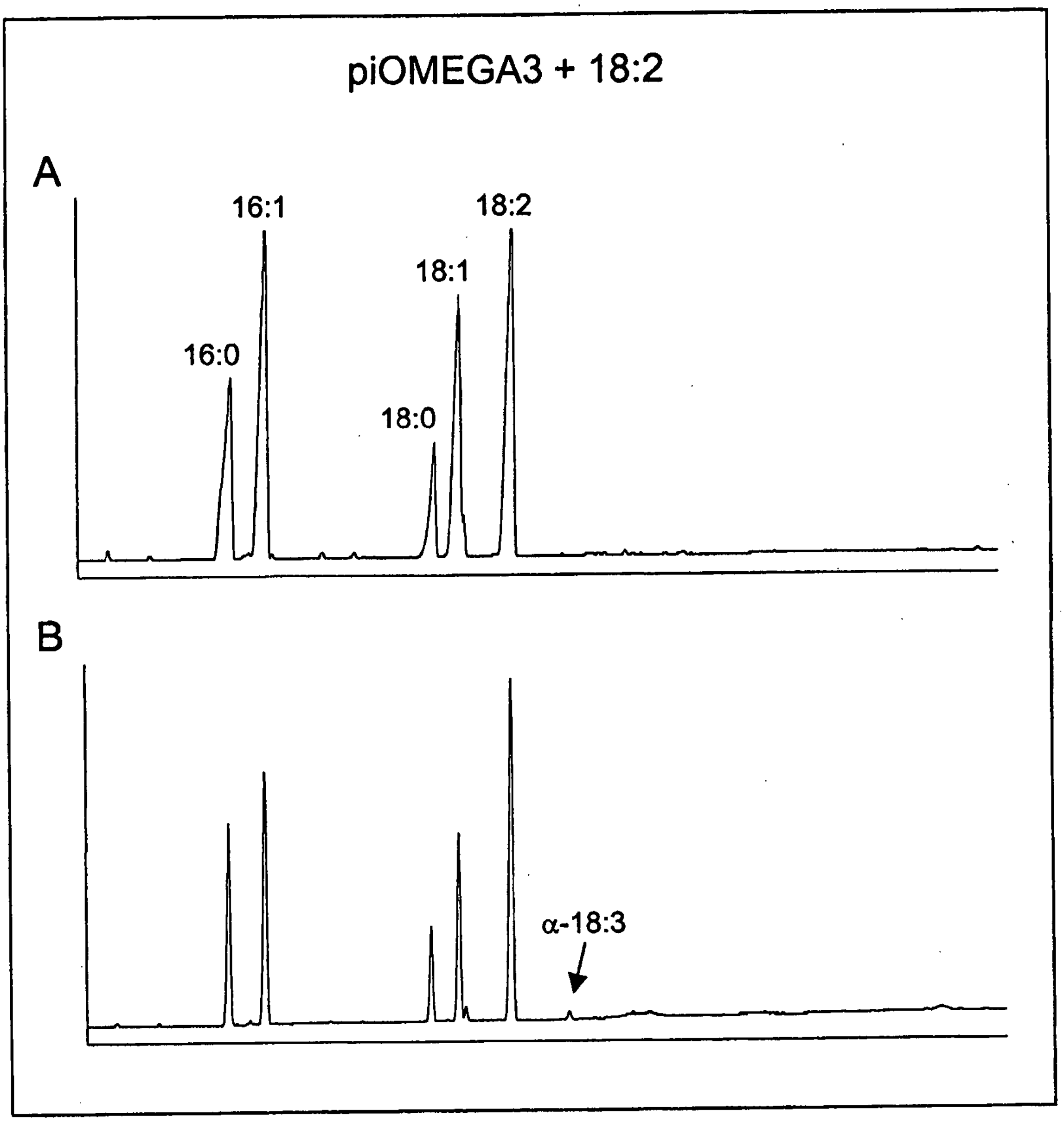

Figur 13: Desaturierung von γ-Linolensäure (18:3 ω-6-Fettsäure) zu Stearidonsäure (18:4 ω-3-Fettsäure) durch Pi-omega3Des.

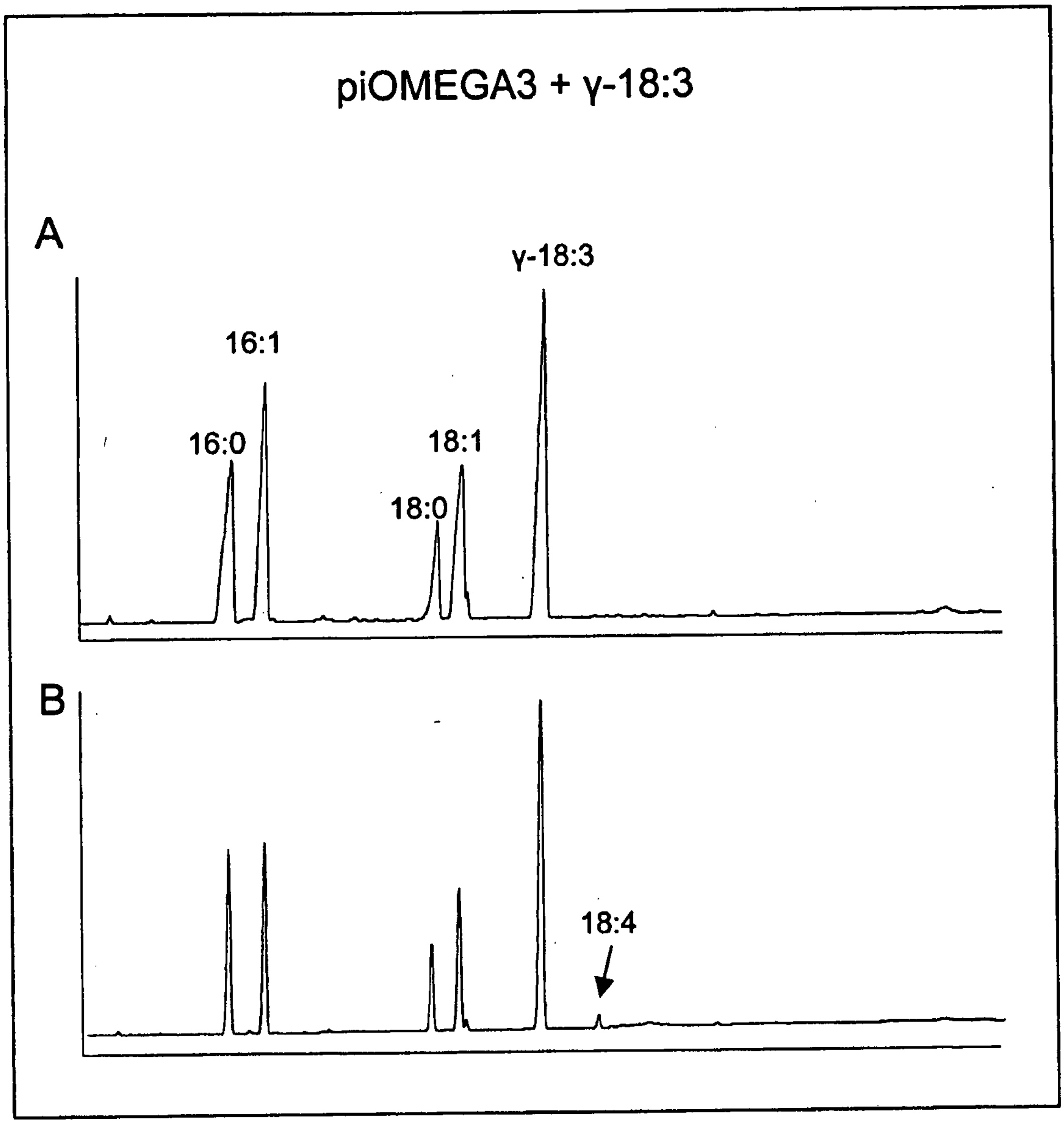

Figur 14: Desaturierung von C20:2 ω-6-Fettsäure zu C20:3 ω-3-Fettsäure durch Pi-omega3Des.

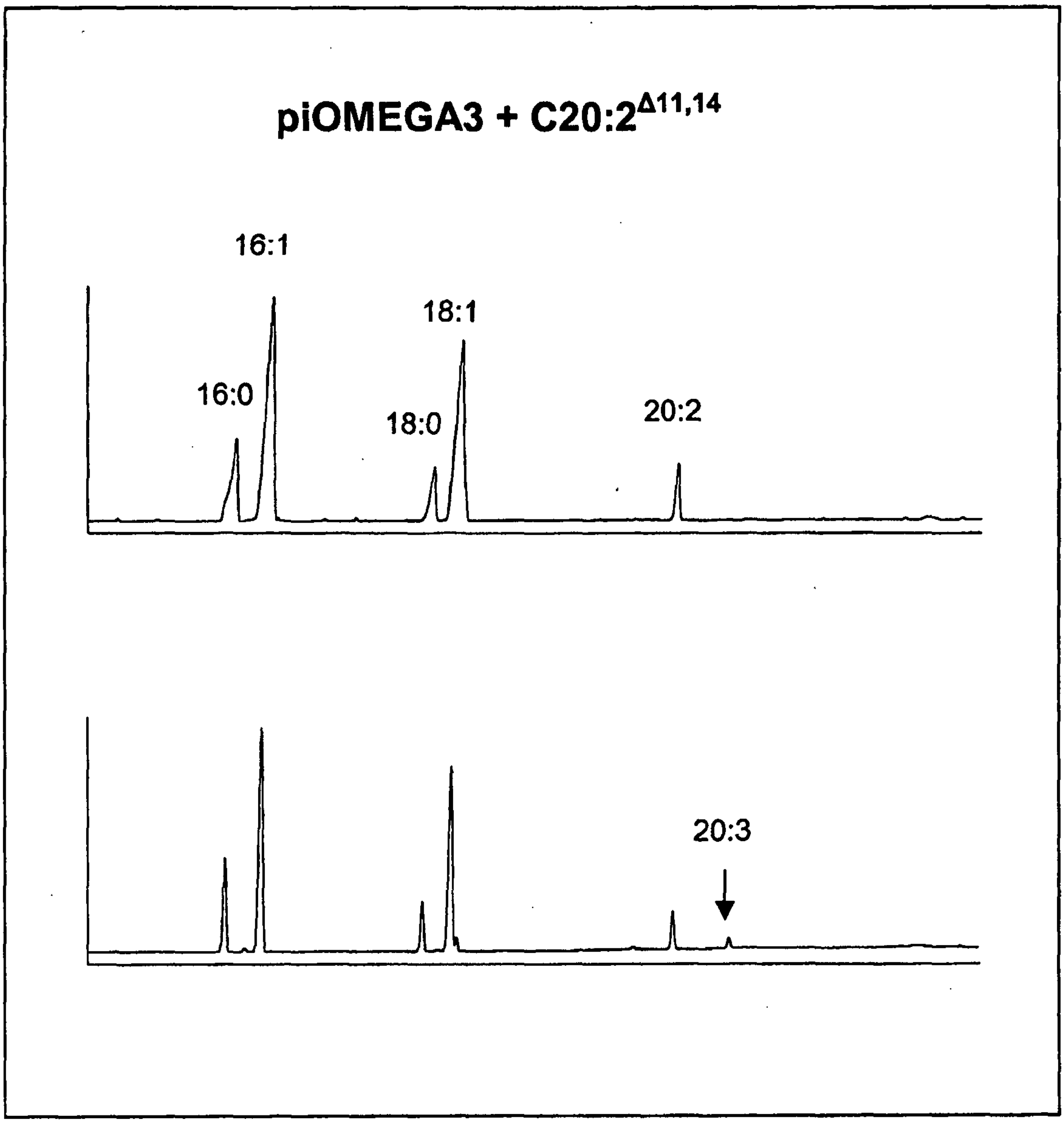

Figur 15: Desaturierung von C20:3-ω-6-Fettsäure zu C20:4-ω-3-Fettsäure durch Pi-omega3Des.

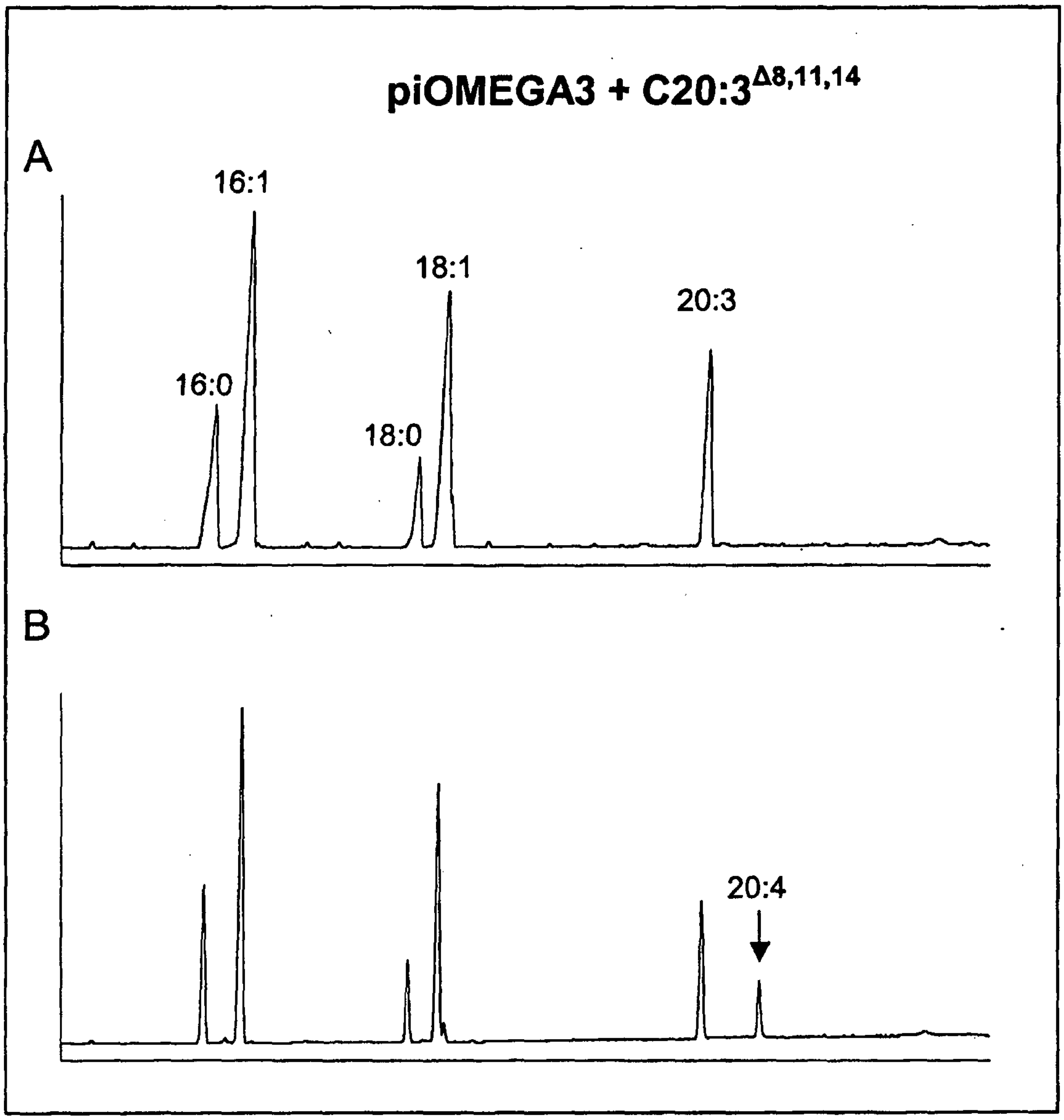

Figur 16: Desaturierung von Arachidonsäure (C20:4-ω-6-Fettsäure) zu Eicosapentaensäure (C20:5-ω-3-Fettsäure) durch die Pi-omega3Des.

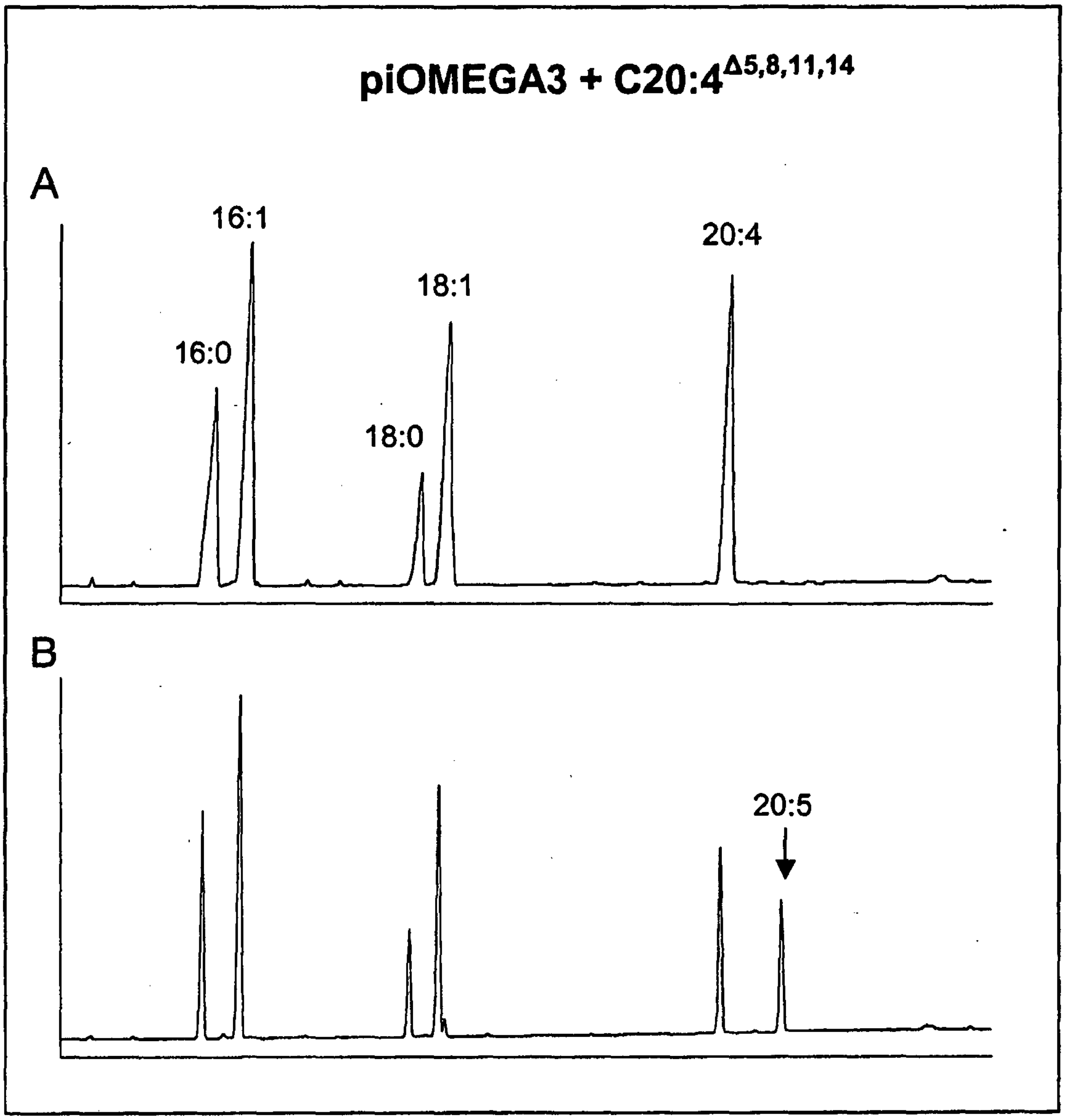

Figur 17: Desaturierung von Docosatetraensäure (C22:4-ω-6-Fettsäure) zu Docosapentaensäure (C22:5-ω-3-Fettsäure) durch Pi-omega3Des.

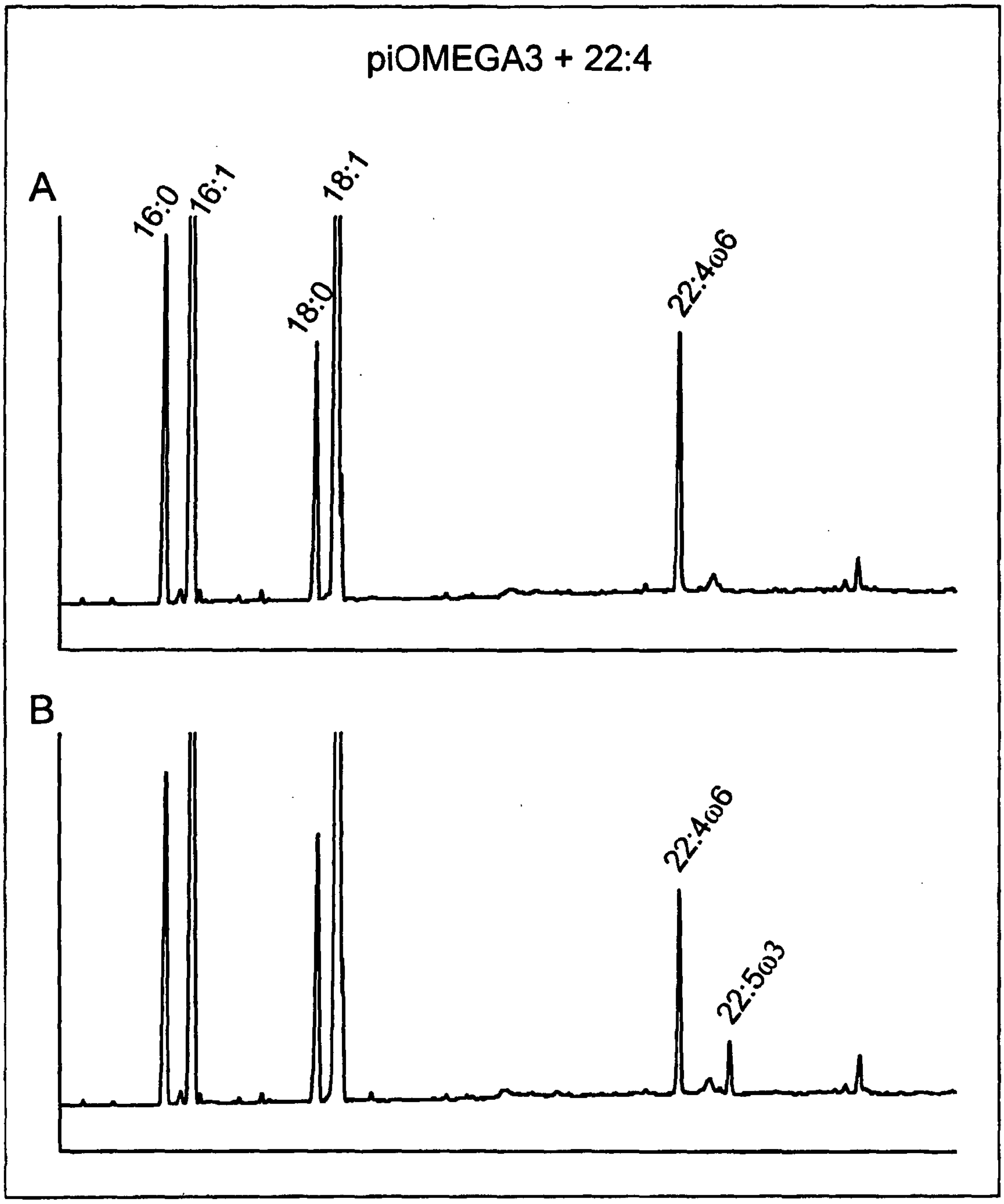

Figur 18: Substratspezifität der Pi-omega3Des gegenüber verschiedenen Fettsäuren

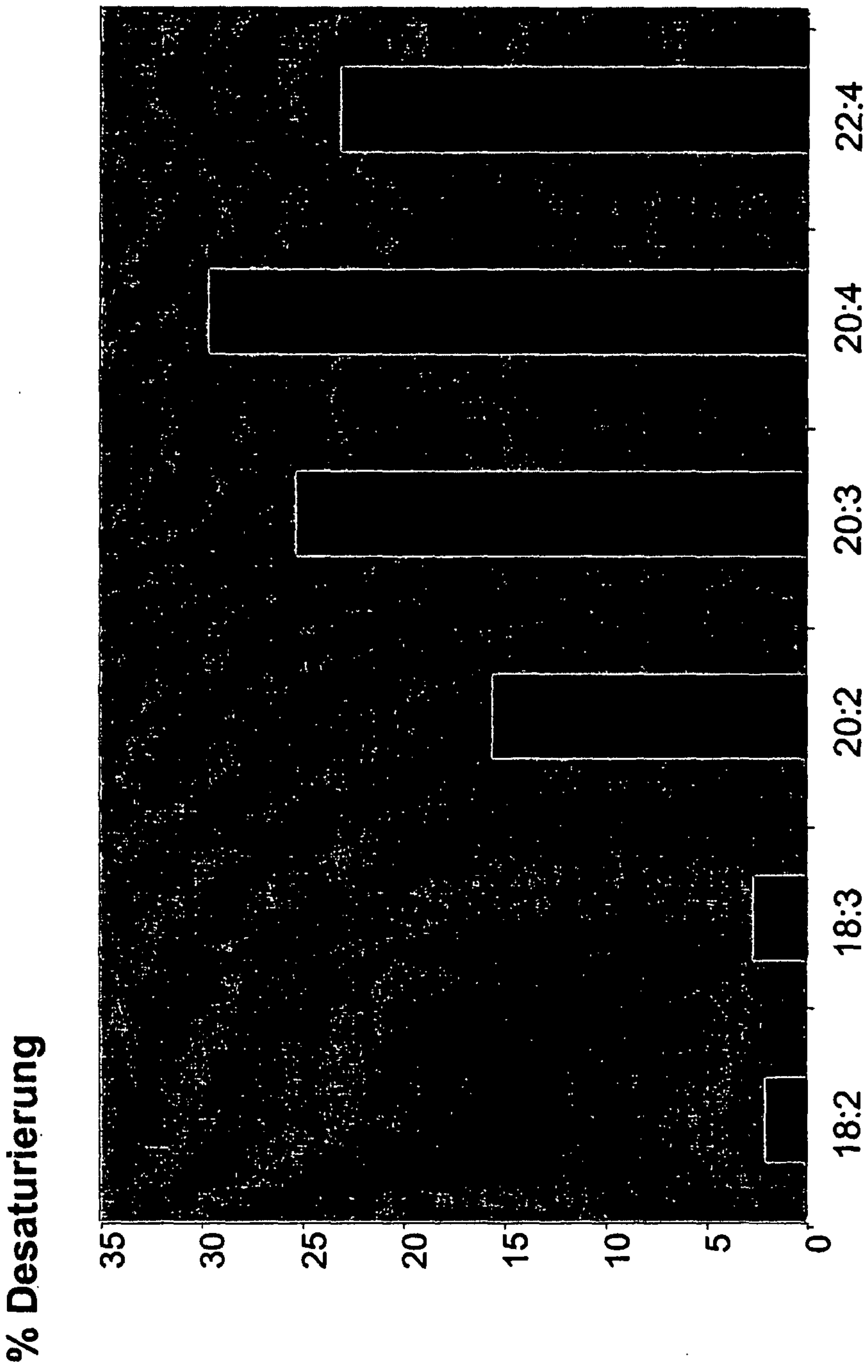

Figur 19: Desaturierung von Phospholipid gebundener Arachidonsäure zu EPA durch die Pi-Omega3Des

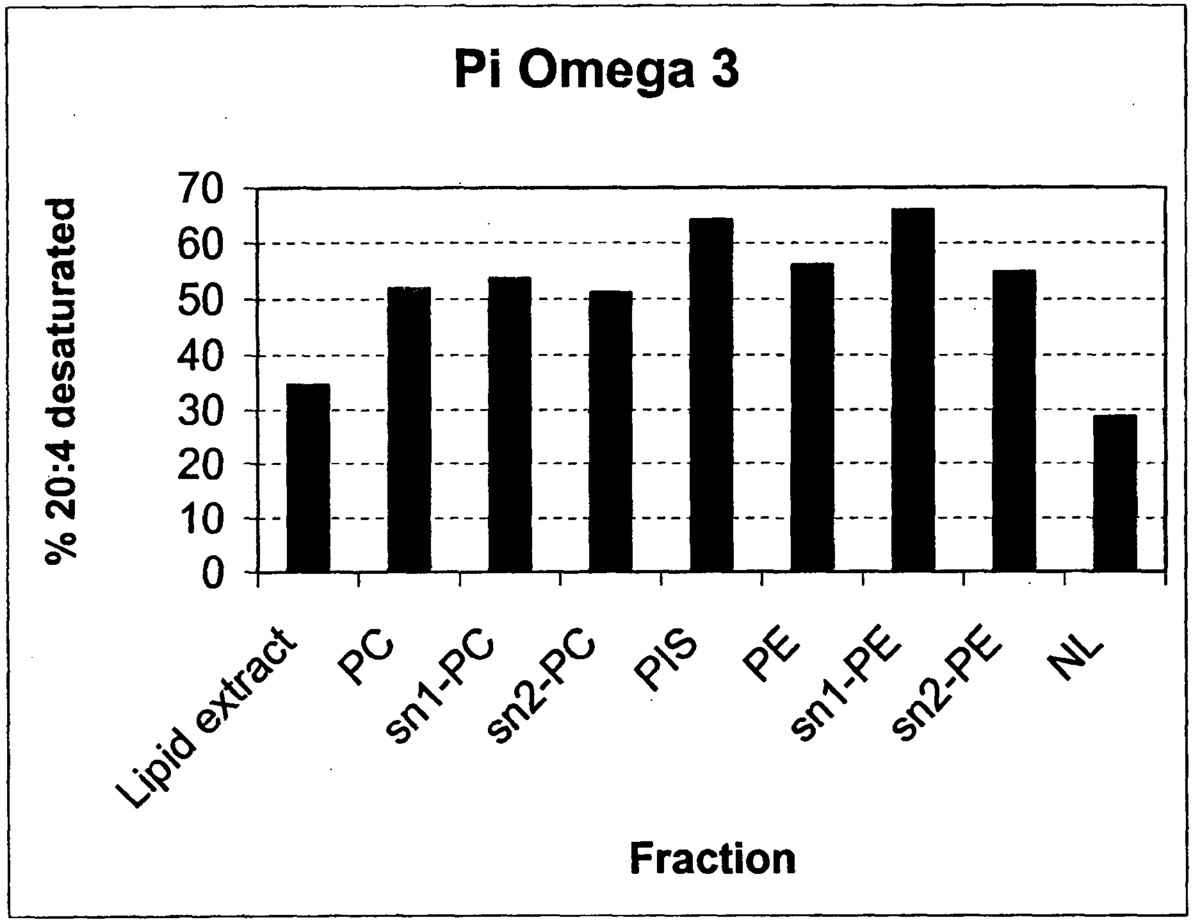

Figur 20: Umsetzung von Linolsäure (Pfeil) zu γ-Linolensäure (γ-18:3) durch Ot-Des6.1.

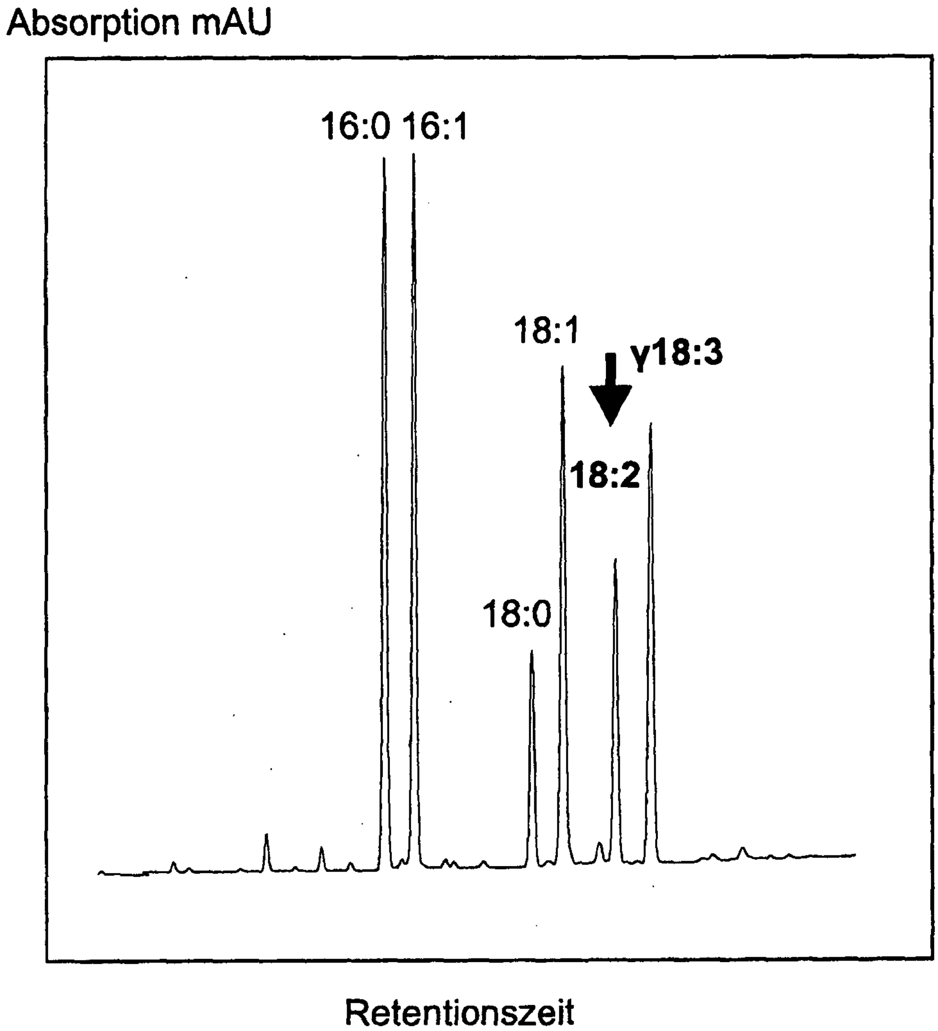

Figur 21: Umsetzung von Linolsäure und α-Linolensäure (A und C), sowie Rekonstitution des ARA- bzw. EPA-Syntheseweges in Hefe (B und D) in Gegenwart von OtD6.1.

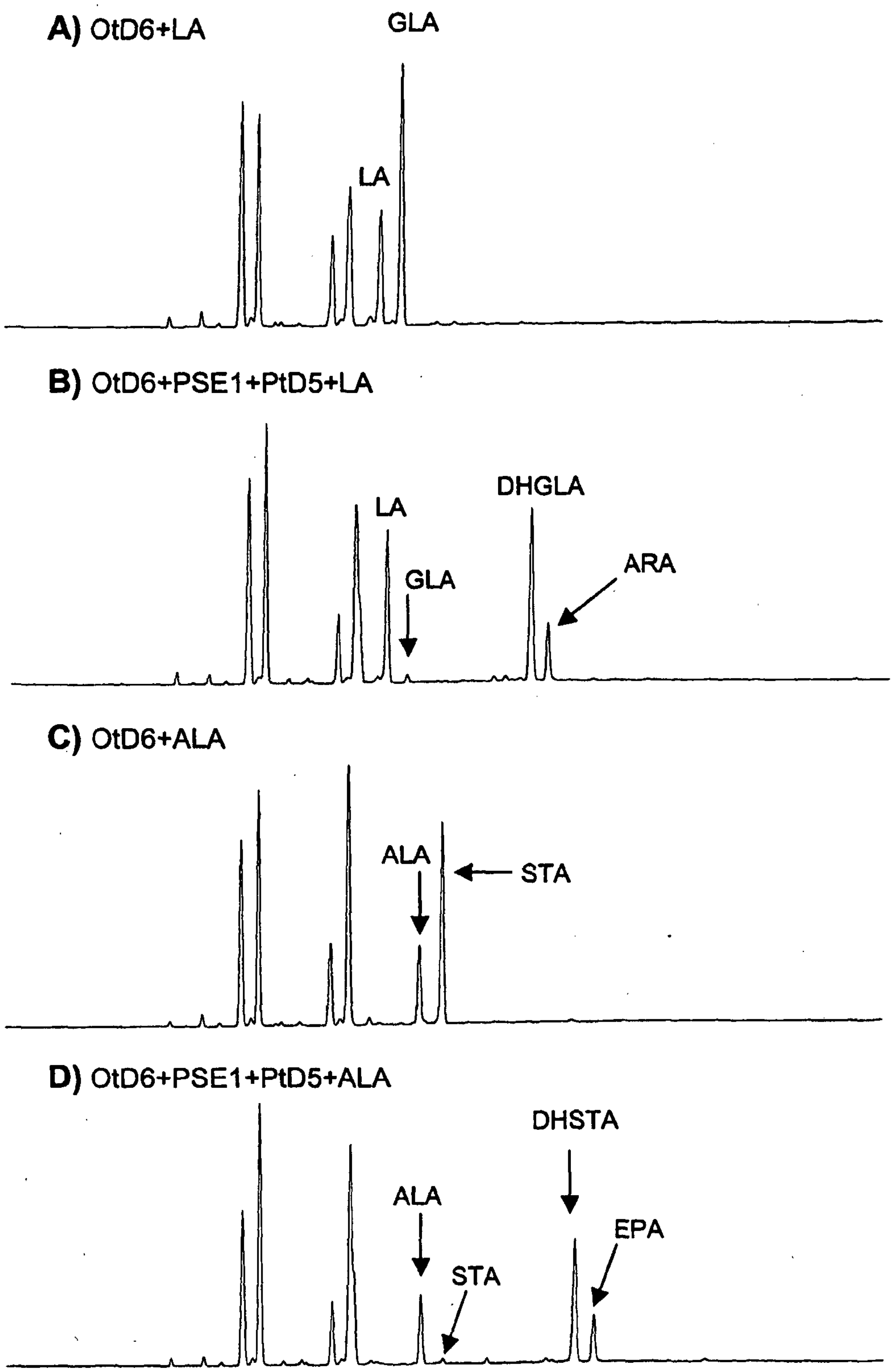

Figur 22: Expression von ELO(XI) in Hefe.

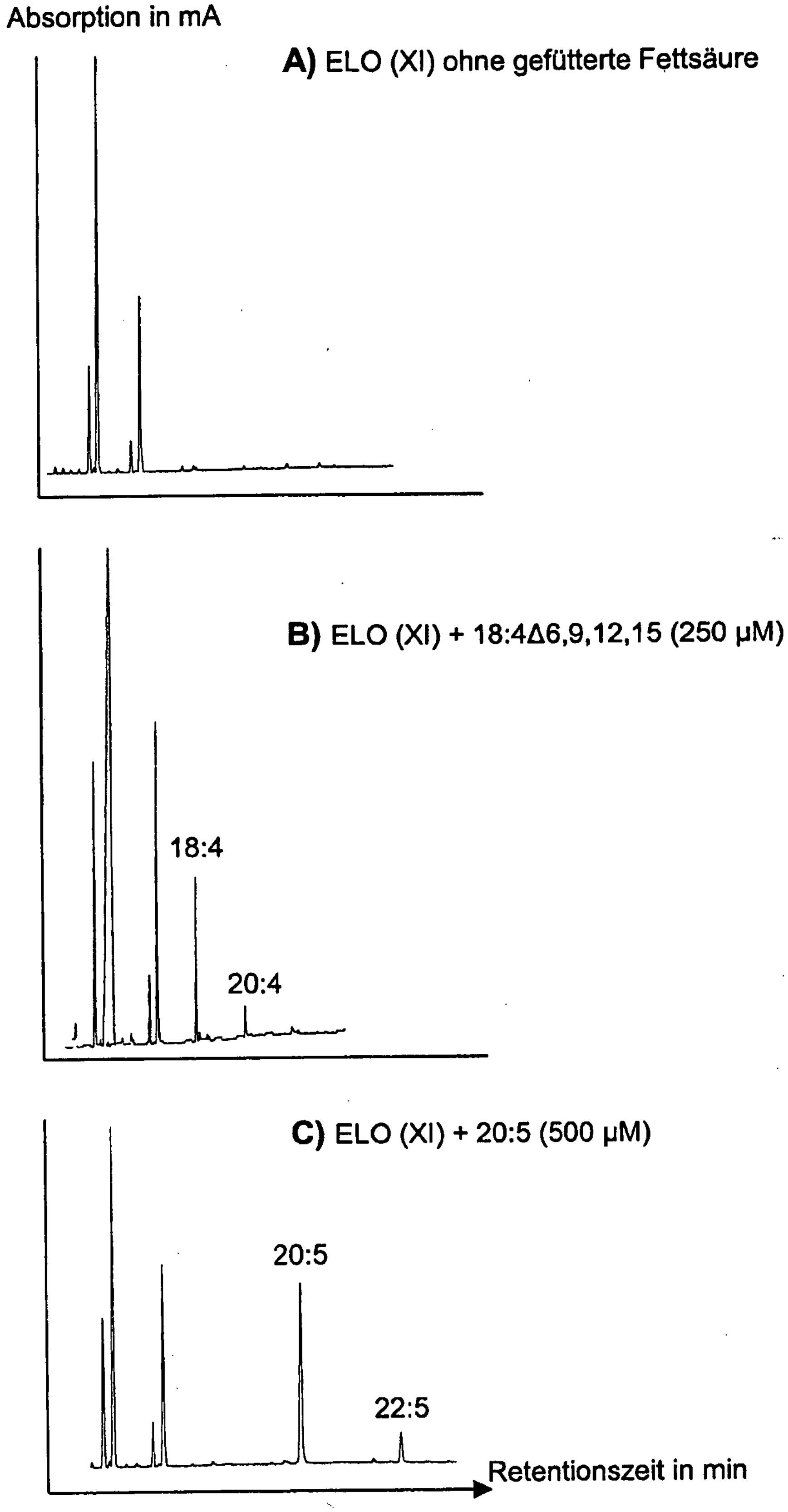

Figur 23:

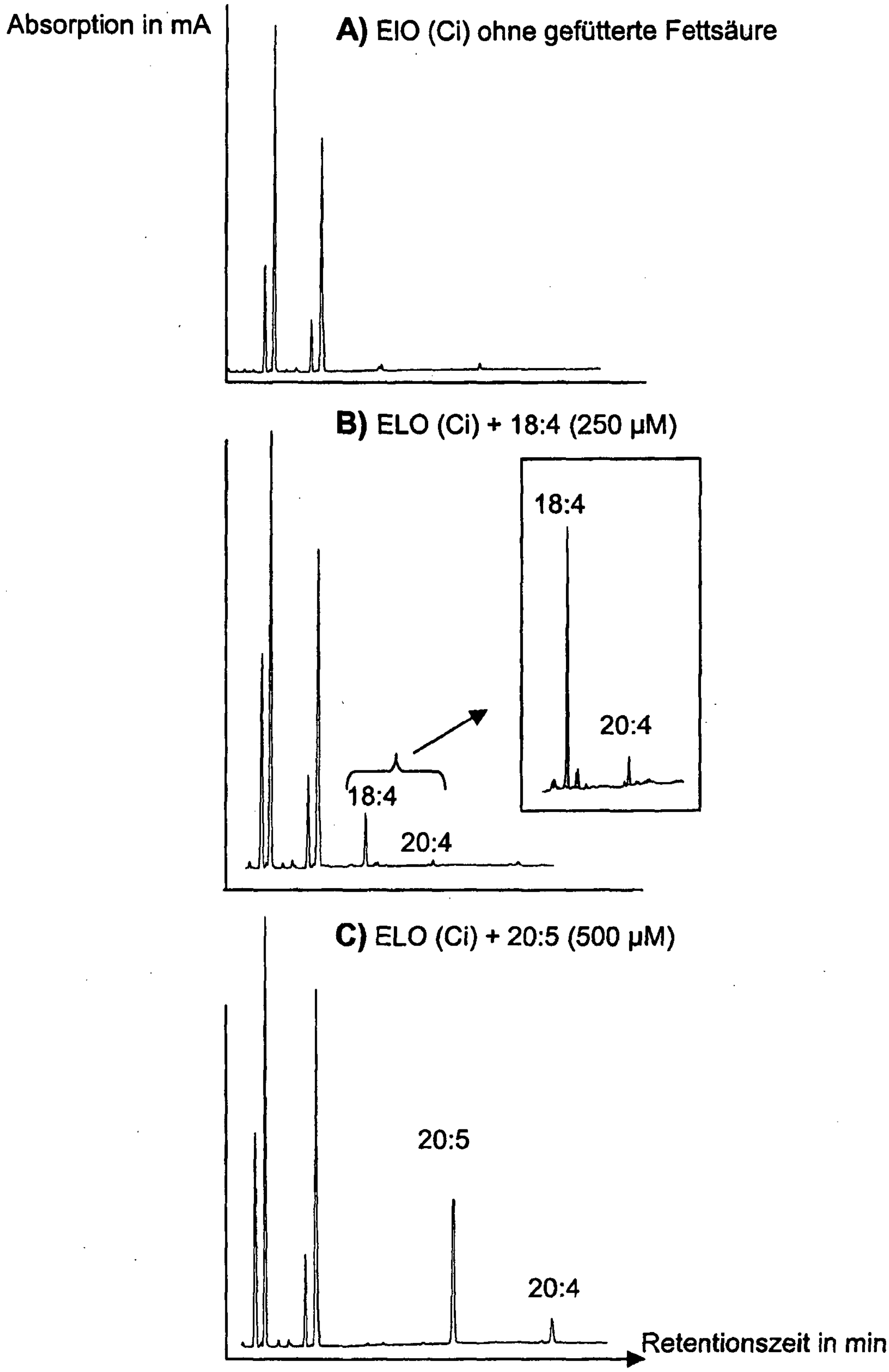
Absorption in mA
A) ElO (Ci) ohne gefütterte Fettsäure
B) ELO (Ci) + 18:4 (250 µM)
18:4
20:4
18:4
20:4
C) ELO (Ci) + 20:5 (500 µM)
20:5
20:4
Retentionszeit in min

Figur 24: Elongation von Eicosapentaensäure durch OtElo1 (B) bzw. OtElo1.2 (D). Die Kontrollen (A, C) zeigen nicht das Produkt der Elongation (22:5ω3).

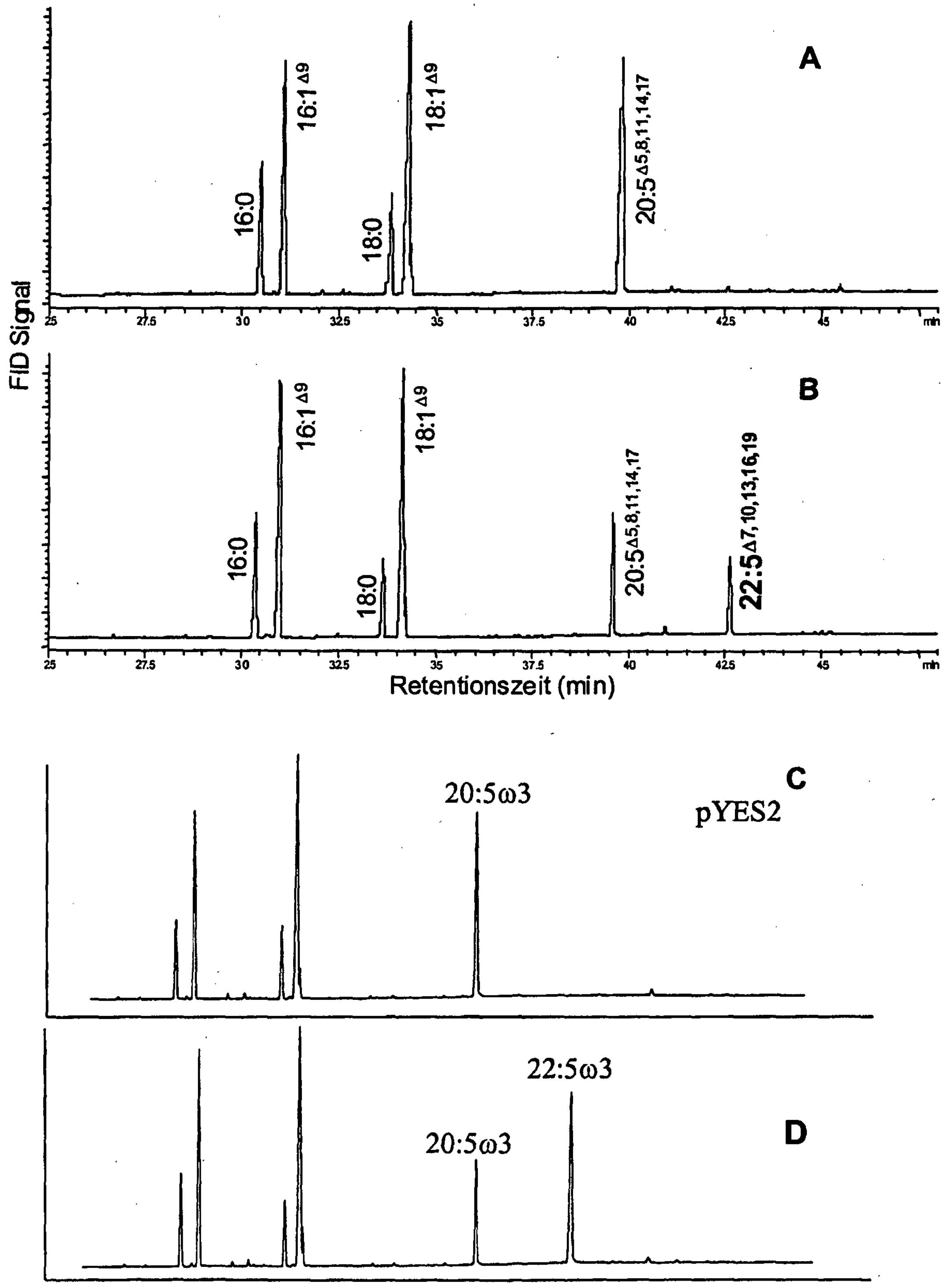

Figur 25: Elongation von Arachidonsäure durch OtElo1 (B) bzw. OtElo1.2 (D). Die Kontrollen (A, C) zeigen nicht das Produkt der Elongation (22:4ω6).

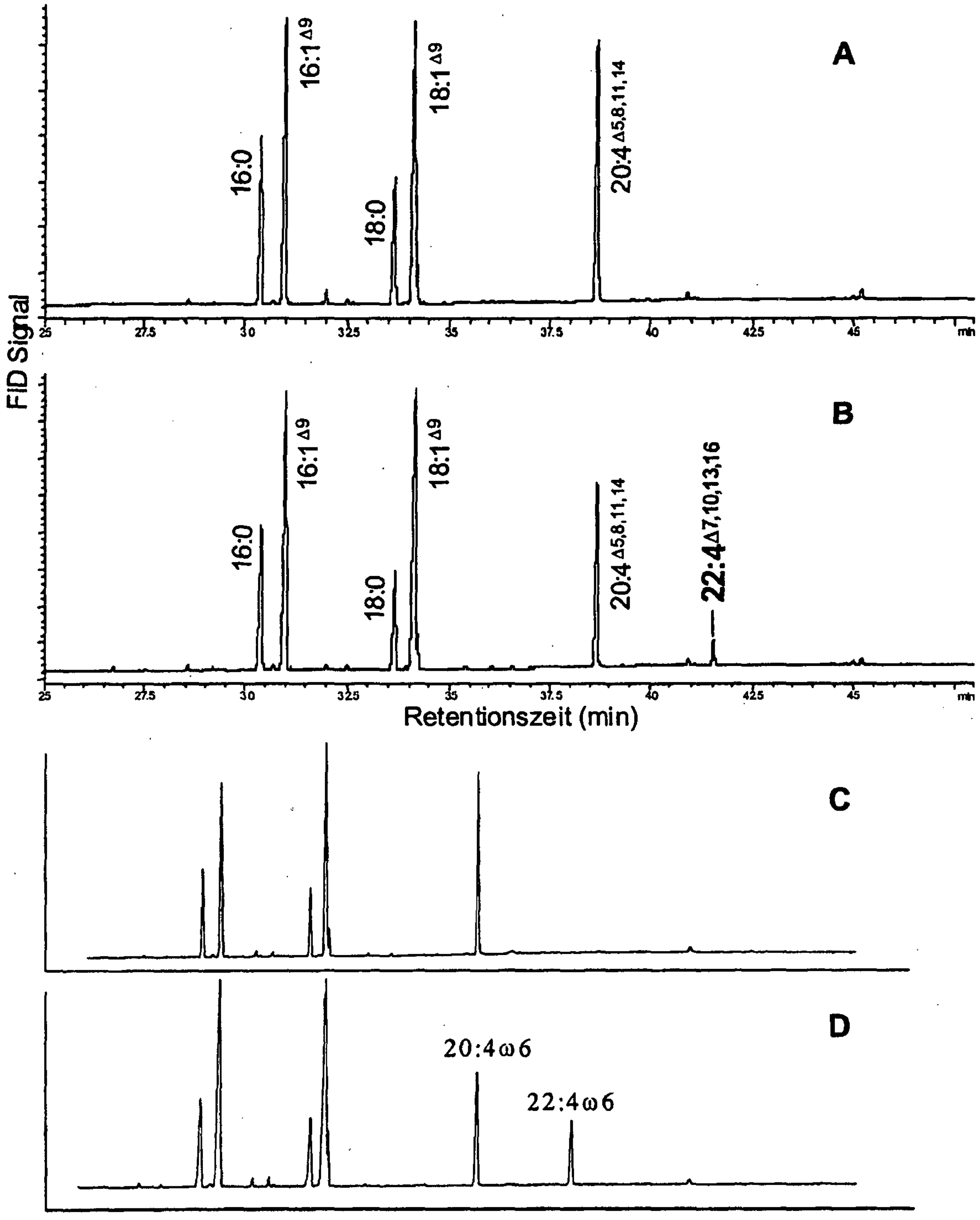

Figur 26: Elongation von 20:5n-3 durch die Elongasen At3g06470.

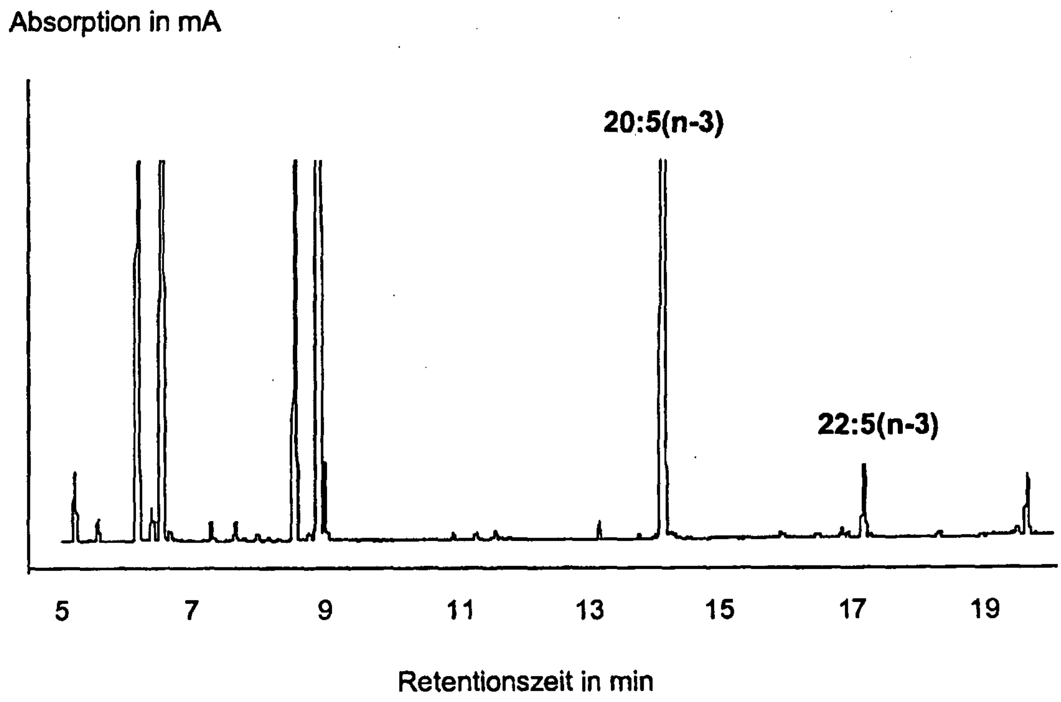

Figur 27: Substratspezifität der Xenopus Elongase (A), Ciona Elongase (B) und Oncorhynchus Elongase (C)

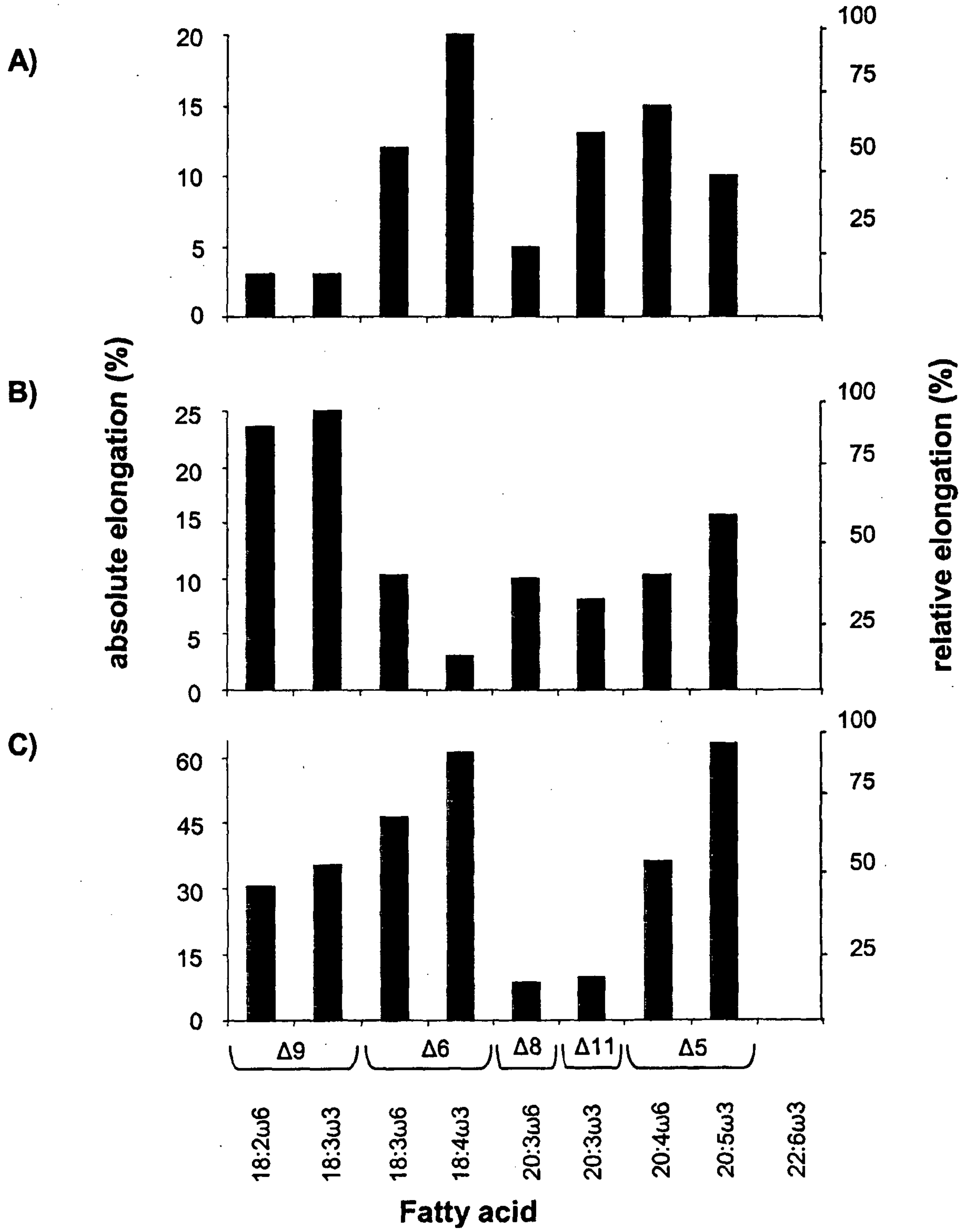

Figur 28: Substratspezifität der Ostreococcus Δ-5-Elongase (A), der Ostreococcus Δ-6-Elongase (B), der Thalassiosira Δ-5-Elongase (C) und Thalassiosira Ostreococcus Δ-6-Elongase (D)

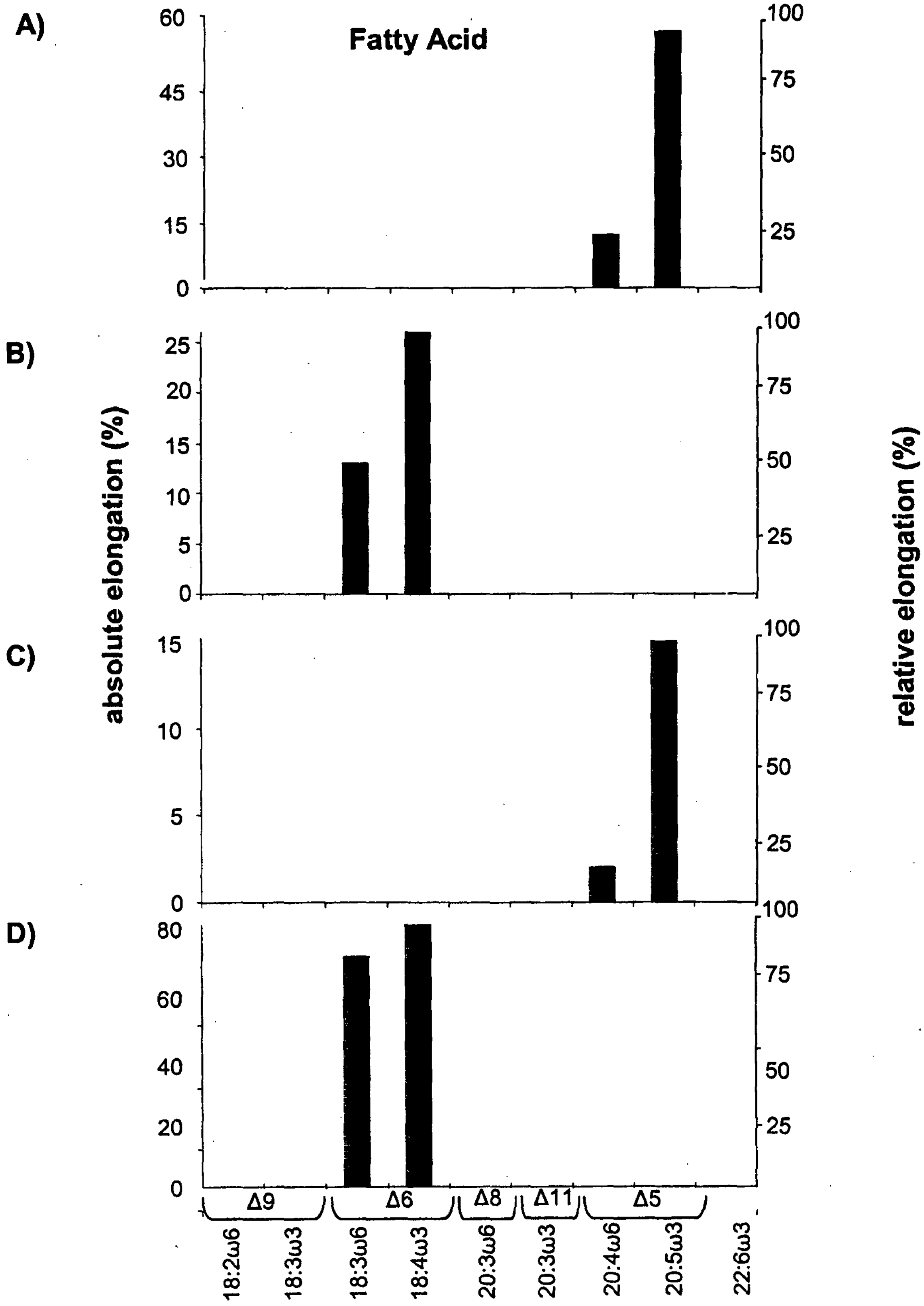

Figur 29: Expression der Phaeodactylum tricornutum Δ-6-Elongase (PtELO6) in Hefe. A) zeigt die Elongation der $C18{:}3^{\Delta 6,9,12}$ Fettsäure und B) die Elongation der $C18{:}4^{\Delta 6,9,12,15}$ Fettsäure

A)

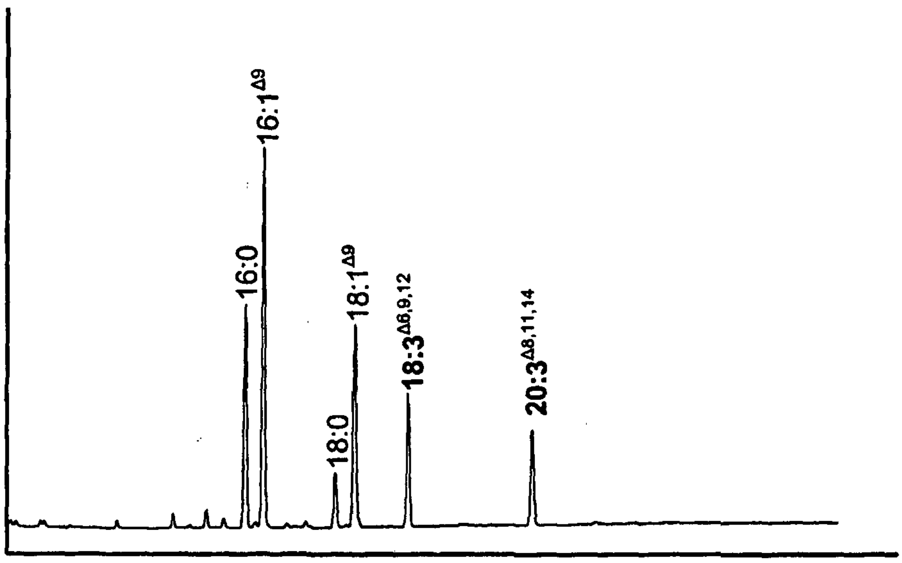


B)

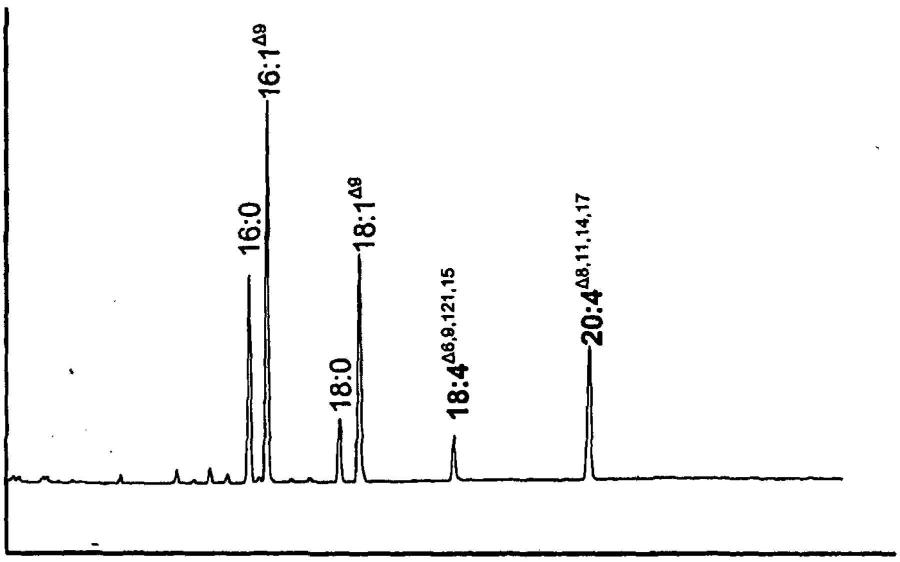

Figur 30: Figur 30 zeigt die Substratspezifität von PtELO6 in Bezug auf die gefütterten Substrate.

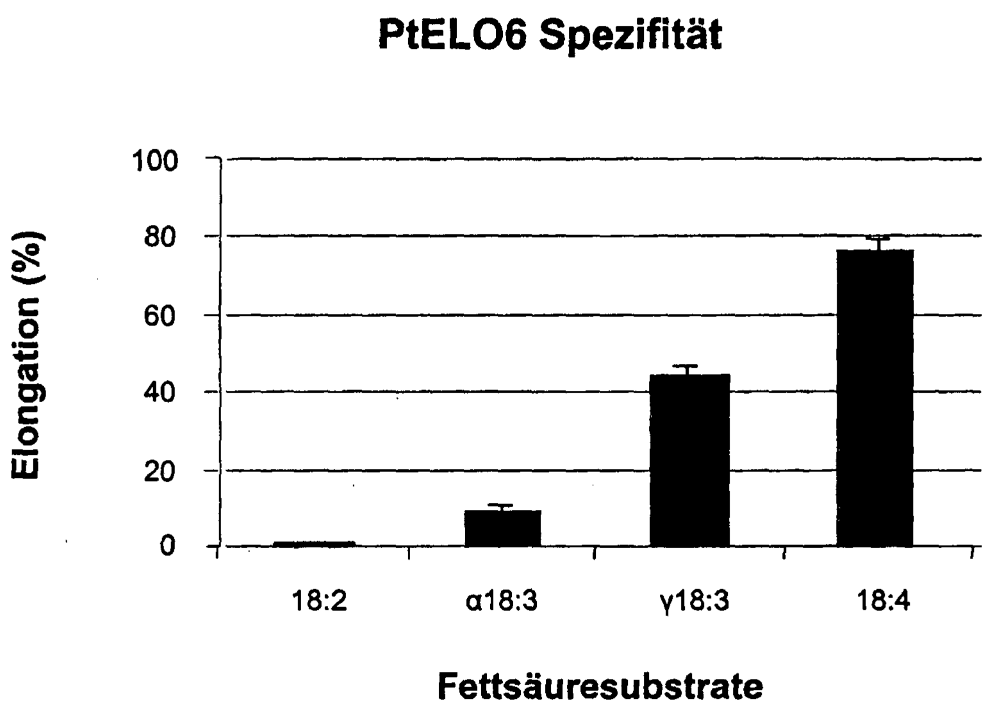

Figur 31: Gaschromatographische Analyse des Samens einer transgenen Pflanze, transformiert mit pSUN-5G.

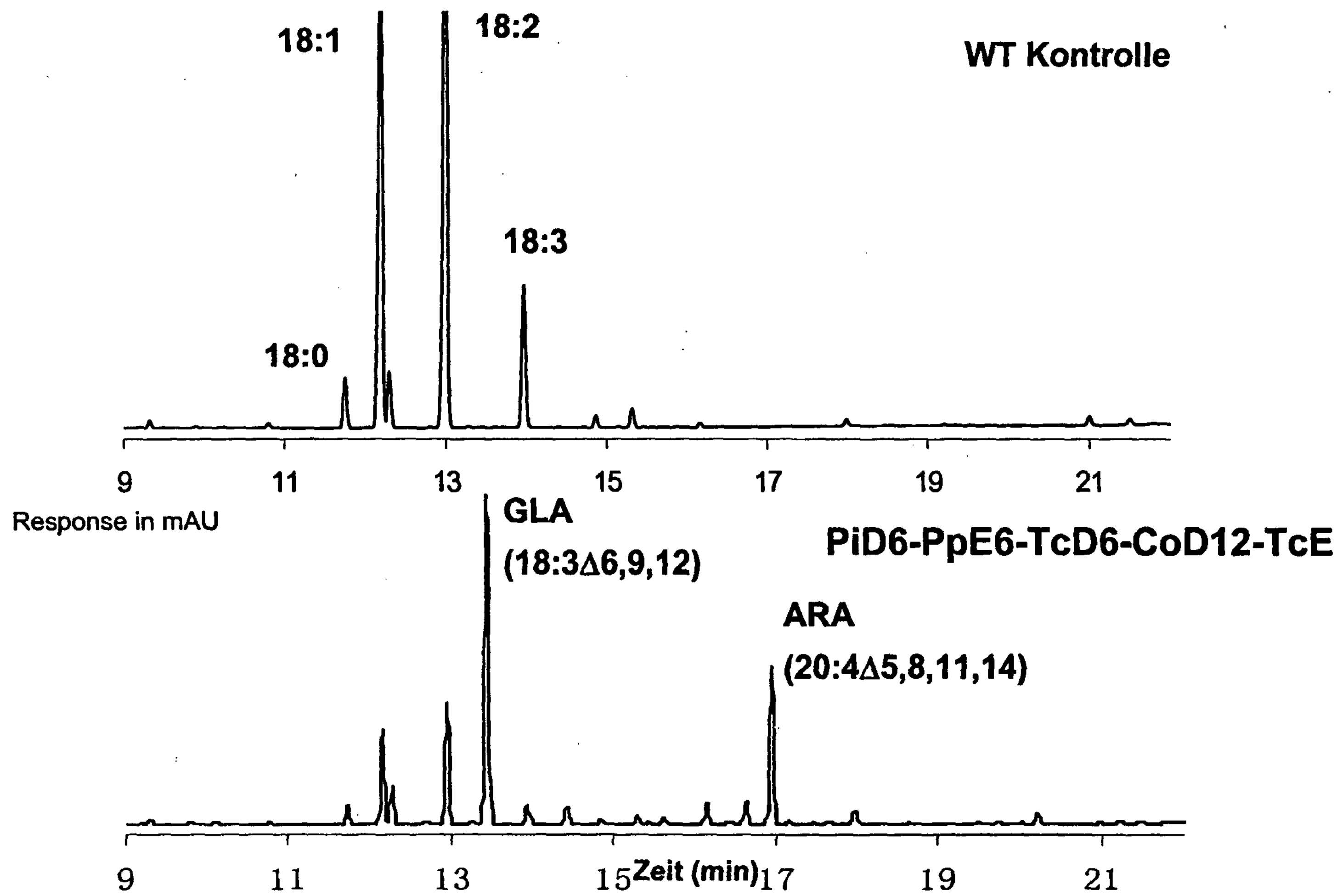

Figur 32: Gaschromatographische Analyse des Samens einer transgenen Pflanze, transformiert mit pGPTV-D6Des(Pir)_D5Des(Tc)_D6Elo(PP)_12Des(Co).

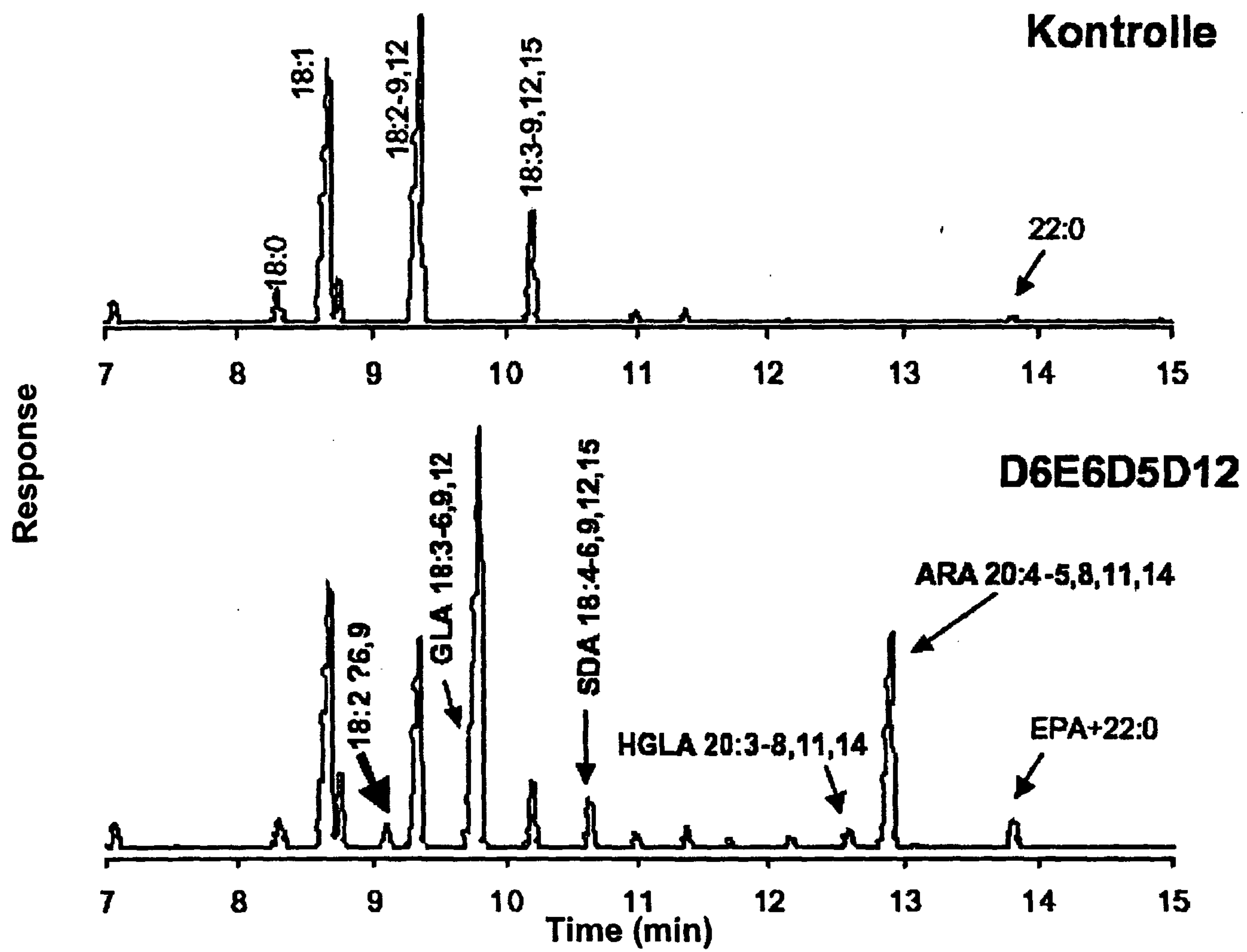

Figur 33: DHA in transgenen Samen von Brassica juncea. Die Pflanzen wurden mit dem Konstrukt pSUN-8G transformiert.

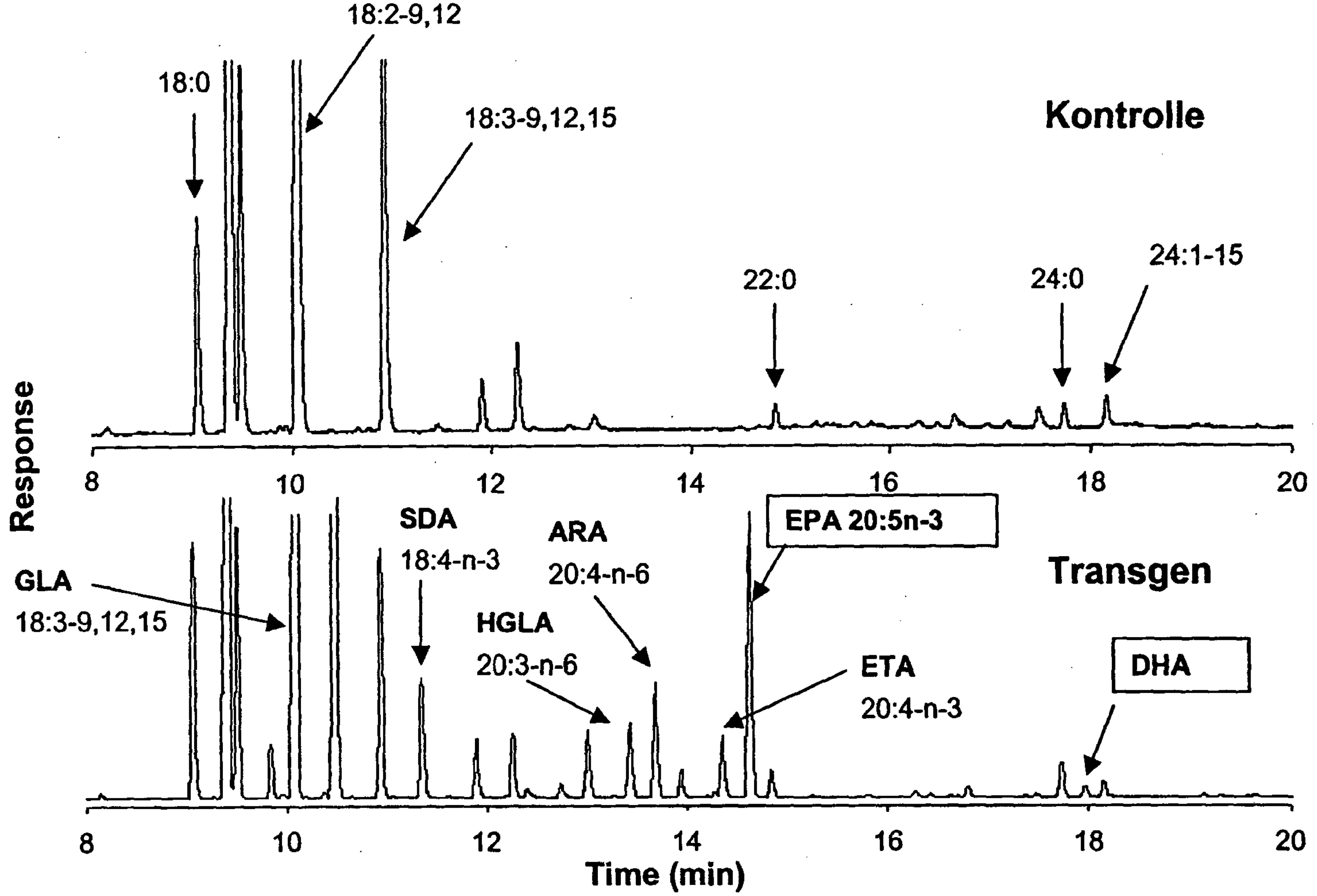

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9113972 A
- WO 9311245 A
- WO 9411516 A
- EP 0550162 A
- WO 9418337 A
- WO 9730582 A
- WO 9721340 A
- WO 9518222 A
- EP 0794250 A
- WO 9306712 A
- US 5614393 A
- WO 9621022 A
- WO 0021557 A
- WO 9927111 A
- WO 9846763 A
- WO 9846764 A
- WO 9846765 A
- WO 9964616 A
- WO 9846776 A
- WO 0159128 A
- WO 0012720 A
- WO 02077213 A
- WO 0208401 A
- WO 0244320 A
- DE 10219203
- WO 2004071467 A
- DE 10219203 A
- WO 0226946 A
- WO 0185968 A
- WO 9916890 A
- US 5608152 A
- WO 02102970 A
- US 5315001 A
- WO 9218634 A
- WO 9845461 A
- WO 9320216 A
- US 5504200 A
- WO 9113980 A
- WO 9515389 A
- WO 9523230 A
- US 5677474 A
- US 5530149 A
- EP 571741 A
- JP 6062870 A
- WO 9808962 A
- US 5689040 A
- EP 781849 A
- WO 9519443 A
- DE 10102337
- DE 10102338
- US 5352605 A
- WO 8402913 A
- US 4962028 A
- US 5187267 A
- WO 9612814 A
- EP 0375091 A
- WO 9516783 A
- WO 9706250 A
- WO 9946394 A
- US 5565350 A
- WO 0015815 A
- EP 0388186 A
- EP 0335528 A
- WO 9321334 A
- EP 0249676 A
- WO 9801572 A
- DE 10205607


### In der Beschreibung aufgeführte Nicht-Patentliteratur


- **F.C. NEIDHARDT et al.** E. coli und Salmonella. ASM Press, 1996, 612-636
- Biology of Procaryotes. Thieme, 1999
- **MAGNUSON, K. et al.** *Microbiological Reviews,* 1993, vol. 57, 522-542
- **FRENTZEN.** *Lipid,* 1998, vol. 100 (4-5), 161-166
- **KINNEY.** Genetic Engeneering. 1997, vol. 19, 149-166
- **OHLROGGE ; BROWSE.** *Plant Cell,* 1995, vol. 7, 957-970
- **SHANKLIN ; CAHOON.** *Annu. Rev. Plant Physiol. Plant Mol. Biol.,* 1998, vol. 49, 611-641
- **VOELKER.** Genetic Engeneering. 1996, vol. 18, 111-13
- **GERHARDT.** *Prog. Lipid R.,* 1992, vol. 31, 397-417
- **GÜHNEMANN-SCHÄFER ; KINDL.** *Biochim. Biophys Acta,* 1995, vol. 1256, 181-186
- **KUNAU et al.** *Prog. Lipid Res.,* 1995, vol. 34, 267-342

- **STYMNE et al.** Biochemistry and Molecular Biology of Membrane and Storage Lipids of Plants. Society of Plant Physiologists, 1993, 150-158
- **MURPHY ; ROSS.** *Plant Journal,* 1998, vol. 13 (1), 1-16
- **SHIMIKAWA.** *World Rev. Nutr. Diet.,* 2001, vol. 88, 100-108
- **CALDER.** *Proc. Nutr. Soc.,* 2002, vol. 61, 345-358
- **CLELAND ; JAMES.** *J. Rheumatol.,* 2000, vol. 27, 2305-2307
- **POULOS, A.** *Lipids,* 1995, vol. 30, 1-14
- **HORROCKS, LA ; YEO YK.** *Pharmacol Res,* 1999, vol. 40, 211-225
- **STUKEY et al.** *J. Biol. Chem.,* 1990, vol. 265, 20144-20149
- **WADA et al.** *Nature,* 1990, vol. 347, 200-203
- **HUANG et al.** *Lipids,* 1999, vol. 34, 649-659
- **MCKEON et al.** *Methods in Enzymol.,* 1981, vol. 71, 12141-12147
- **WANG et al.** *Plant Physiol. Biochem.,* 1988, vol. 26, 777-792
- **MILLAR ; KUNST.** *Plant Journal,* 1997, vol. 12, 121-131
- **MILLAR et al.** *Plant Cell,* 1999, vol. 11, 825-838
- **TVRDIK et al.** *J. Cell Biol.,* 2000, vol. 149, 707-718
- **R. VAZHAPPILLY ; F. CHEN.** *Botanica Marina,* 1998, vol. 41, 553-558
- **K. TOTANI ; K. OBA.** *Lipids,* 1987, vol. 22, 1060-1062
- **M. AKIMOTO et al.** *Appl. Biochemistry and Biotechnology,* 1998, vol. 73, 269-278
- **E. UCCIANI.** *Nouveau Dictionnaire des Huiles Végétales. Technique & Documentation - Lavoisier,* 1995, ISBN 2-7430-0009-0
- **YU, R. et al.** *Lipids,* 2000, vol. 35, 1061-1064
- **TAKEYAMA, H. et al.** *Microbiology,* 1997, vol. 143, 2725-2731
- **ZANK, T.K. et al.** *Plant Journal,* 2002, vol. 31, 255-268
- **SAKURADANI, E. et al.** *Gene,* 1999, vol. 238, 445-453
- **SPRECHER.** *Biochim. Biophys. Acta,* 2000, vol. 1486, 219-231
- **TOCHER et al.** *Prog. Lipid Res.,* 1998, vol. 37, 73-117
- **DOMERGUE et al.** *Eur. J. Biochem.,* 2002, vol. 269, 4105-4113
- **MIKOKLAJCZAK et al.** *Journal of the American Oil Chemical Society,* 1961, vol. 38, 678-681
- *J. Mol. Evolution.,* 1987, vol. 25, 351-360
- **HIGGINS et al.** *CABIOS,* 1989, vol. 5, 151-153
- **NEEDLEMAN ; WUNSCH.** *J. Mol. Biol.,* 1970, vol. 48, 443-453
- **SMITH ; WATERMAN.** *Adv. Appl. Math.,* 1981, vol. 2, 482-489
- Current Protocols in Molecular Biology. John Wiley & Sons, 1989
- **SAMBROOK et al.** Molecular Cloning. Cold Spring Harbor Laboratory, 1989
- Nucleic Acids Hybridization: A Practical Approach. IRL Press at Oxford University Press, 1985
- Essential Molecular Biology: A Practical Approach. IRL Press at Oxford University Press, 1991
- **HELLENS et al.** *Trends in Plant Science,* 2000, vol. 5, 446-451
- Plant Molecular Biology and Biotechnology. CRC Press, 1993, 71-119
- Vectors for Gene Transfer in Higher Plants. **F.F. WHITE.** Transgenic Plants. Academic Press, 1993, vol. 1, 15-38
- Techniques for Gene Transfer. **B. JENES et al.** Transgenic Plants. Academic Press, 1993, vol. 1, 128-143
- **POTRYKUS.** *Annu. Rev. Plant Physiol. Plant Molec. Biol.,* 1991, vol. 42, 205-225
- **BÄUMLEIN et al.** *Mol. Gen Genet.,* 1991, vol. 225 (3
- **BÄUMLEIN et al.** *Plant J.,* 1992, vol. 2, 2
- **GATZ.** *Annu. Rev. Plant Physiol. Plant Mol. Biol.,* 1997, vol. 48, 89-108
- **GATZ et al.** *Plant J.,* 1992, vol. 2, 397-404
- **GOEDDEL.** Gene Expression Technology: Methods in Enzymology. Academic Press, 1990, vol. 185
- **GRUBER ; CROSBY.** Methods in Plant Molecular Biology and Biotechnolgy. CRC Press, 89-108
- **FALCIATORE et al.** *Marine Biotechnology,* 1999, vol. 1 (3), 239-251
- **BECKER, D. ; KEMPER, E. ; SCHELL, J. ; MASTERSON, R.** New plant binary vectors with selectable markers located proximal to the left border. *Plant Mol. Biol.,* 1992, vol. 20, 1195-1197
- **BEVAN, M.W.** Binary Agrobacterium vectors for plant transformation. *Nucl. Acids Res.,* 1984, vol. 12, 8711-8721
- Vectors for Gene Transfer in Higher Plants. Transgenic Plants. Academic Press, 1993, vol. 1, 15-38
- **GIELEN et al.** *EMBO J.,* 1984, vol. 3, 835
- **GALLIE et al.** *Nucl. Acids Research,* 1987, vol. 15, 8693-8711
- **ENFEY et al.** *EMBO J.,* 1989, vol. 8, 2195-2202
- **FRANCK et al.** *Cell,* 1980, vol. 21, 285-294
- **WARD et al.** *Plant. Mol. Biol.,* 1993, vol. 22, 361-366

- **BAEUMLEIN et al.** *Mol Gen Genet,* 1991, vol. 225 (3), 459-67
- **BAEUMLEIN et al.** *Plant Journal,* 1992, vol. 2 (2), 233-9
- **KERMODE.** *Crit. Rev. Plant Sci.,* 1996, vol. 15 (4), 285-423
- **GOTTESMAN, S.** Gene Expression Technology: Methods in Enzymology. Academic Press, 1990, vol. 185, 119-128
- **WARD et al.** *Plant. Mol. Biol.,* 1993, vol. 22
- **GATZ.** *Plant J.,* 1992, vol. 2, 397-404
- **STOCKHAUS et al.** *EMBO J.,* 1989, vol. 8, 2445
- **VON BAEUMLEIN et al.** *Plant J.,* 1992, vol. 2 (2), 233-239
- **ROMANOS, M.A. et al.** Foreign gene expression in yeast: a review. *Yeast,* 1992, vol. 8, 423-488
- Heterologous gene expression in filamentous fungi. **VAN DEN HONDEL, C.A.M.J.J. et al.** More Gene Manipulations in Fungi. Academic Press, 1991, 396-428
- Gene transfer systems and vector development for filamentous fungi. **UND VAN DEN HONDEL, C.A.M.J.J. ; PUNT, P.J. et al.** Applied Molecular Genetics of Fungi. Cambridge University Press, 1991, 1-28
- **FALCIATORE et al.** *Marine Biotechnology,* 1999, vol. 3, 239-251
- **SCHMIDT, R. ; WILLMITZER, L.** High efficiency Agrobacterium tumefaciens-mediated transformation of Arabidopsis thaliana leaf and cotyledon explants. *Plant Cell Rep.,* 1988, 583-586
- Plant Molecular Biology and Biotechnology. C Press, 1993, 71-119
- Techniques for Gene Transfer. **F.F. WHITE ; B. JENES et al.** Transgenic Plants. Academic Press, 1993, vol. 1, 128-43
- **SMITH, D.B. ; JOHNSON, K.S.** *Gene,* 1988, vol. 67, 31-40
- **AMANN et al.** *Gene,* 1988, vol. 69, 301-315
- **STUDIER et al.** Gene Expression Technology: Methods in Enzymology. Academic Press, 1990, vol. 185, 60-89
- **BALDARI et al.** *Embo J.,* 1987, vol. 6, 229-234
- **KURJAN ; HERSKOWITZ.** *Cell,* 1982, vol. 30, 933-943
- **SCHULTZ et al.** *Gene,* 1987, vol. 54, 113-123
- Gene transfer systems and vector development for filamentous fungi. **VAN DEN HONDEL, C.A.M.J.J. ; PUNT, P.J. et al.** Applied Molecular Genetics of fungi. Cambridge University Press, 1991, 1-28
- More Gene Manipulations in Fungi. Academic Press, 396-428
- **SMITH et al.** *Mol. Cell Biol.,* 1983, vol. 3, 2156-2165
- **LUCKLOW ; SUMMERS.** *Virology,* 1989, vol. 170, 31-39
- Cloning Vectors. Elsevier, 1985
- **SAMBROOK, J. ; FRITSCH, E.F. ; MANIATIS, T.** Molecular Cloning: A Laboratory Manual. Cold Spring Harbor Laboratory, Cold Spring Harbor Laboratory Press, 1989
- **BENFEY et al.** *EMBO J.,* 1989, vol. 8, 2195-2202
- **SAMBROOK et al.** Molecular Cloning: A Laboratory Manual. Cold Spring Harbor Laboratory, Cold Spring Harbor Laboratory Press, 1989
- Agrobacterium protocols. Methods in Molecular Biology. Humana Press, 1995, vol. 44
- **SAMBROOK et al.** Molecular Cloning: A Laboratory Manual. Cold Spring Harbor Laboratory, Cold Spring Harbor Laboratory Press, 1989
- **CHIRGWIN et al.** Biochemistry. 1979, vol. 18, 5294-5299
- **SANGER et al.** *Proc. Natl. Acad. Sci. USA,* 1977, vol. 74, 5463-5467
- **HAJDUKIEWICZ,P ; SVAB, Z ; MALIGA, P.** The small versatile pPZP family of Agrobacterium binary vectors forplant transformation. *Plant Mol Biol,* 1994, vol. 25, 989-994
- **DE GREVE,H. ; DHAESE,P. ; SEURINCK,J. ; LEMMERS,M. ; VAN MONTAGU,M. ; SCHELL,J.** Nucleotide sequence and transcript map of the Agrobacterium tumefaciens Ti plasmid-encoded octopine synthase gene. *J. Mol. Appl. Genet.,* 1982, vol. 1 (6), 499-511
- **ULLMAN.** Encyclopedia of Industrial Chemistry. VCH, 1985, vol. A2, 89-90, 443-613
- **FALLON, A. et al.** Applications of HPLC in Biochemistry. *Laboratory Techniques in Biochemistry and Molecular Biology,* 1987, vol. 17
- Product recovery and purification. **REHM et al.** Biotechnology. VCH, 1993, vol. 3, 469-714
- **BELTER, P.A. et al.** Bioseparations: downstream processing for Biotechnology. John Wiley and Sons, 1988
- **KENNEDY, J.F. ; CABRAL, J.M.S.** Recovery processes for biological Materials. John Wiley and Sons, 1992
- Biochemical Separations. **SHAEIWITZ, J.A. ; HENRY, J.D.** Ullmann's Encyclopedia of Industrial Chemistry. VCH, 1988, vol. B3, 1-27
- **DECHOW, F.J.** Separation and purification techniques in biotechnology. Noyes Publications, 1989
- **CAHOON et al.** *Proc. Natl. Acad. Sci. USA,* 1999, vol. 96 (22), 12935-12940
- **BROWSE et al.** *Analytic Biochemistry,* 1986, vol. 152, 141-145
- **CHRISTIE, WILLIAM W.** Advances in Lipid Methodology. Oily Press (Oily Press Lipid Library, 2
- **CHRISTIE, WILLIAM W.** Gas Chromatography and Lipids. A Practical Guide. Oily Press, 1989

- Repr. Oily Press Lipid Library, 1992, vol. IX, 307
- Progress in Lipid Research. Progress in the Chemistry of Fats and Other Lipids CODEN. Pergamon Press, 1952, vol. 1, 16
- Applied Microbial Physiology; A Practical Approach. IRL Press, 103-129, 131-163, 165-192
- **1997.** Advances on Lipid Methodology. Oily Press, 119-169
- **1998.** *Gaschromatographie-Massenspektrometrie-Verfahren, Lipide,* vol. 33, 343-353
- **NAPIER ; MICHAELSON.** *Lipids,* 2001, vol. 36 (8), 761-766
- **SAYANOVA et al.** *Journal of Experimental Botany.,* 2001, vol. 52 (360), 1581-1585
- **SPERLING et al.** *Arch. Biochem. Biophys.,* 2001, vol. 388 (2), 293-298
- **MICHAELSON et al.** *FEBS Letters,* 1998, vol. 439 (3), 215-218
- **DEBLAERE et al.** *Nucl. Acids. Res.,* 1984, vol. 13, 4777-4788
- **MURASHIGE ; SKOOG.** *Physiol. Plant.,* 1962, vol. 15, 473
- **BELL et al.** *In Vitro Cell. Dev. Biol.-Plant.,* 1999, vol. 35 (6), 456-465
- **MLYNAROVA et al.** *Plant Cell Report,* 1994, vol. 13, 282-285
- **ALTSCHUL et al.** *J. Mol. Biol.,* 1990, vol. 215, 403-410
- **KOZAK.** *Cell,* 1986, vol. 44, 283-292
- **HAJDUKIEWICZ, P ; SVAB, Z ; MALIGA, P.** The small versatile pPZP family of Agrobacterium binary vectors for plant transformation. *Plant Mol Biol,* 1994, vol. 25, 989-994
- **MICHAELSON et al.** *FEBS Letters.,* 1998, vol. 439 (3), 215-218
- Culture of phytoplankton for feeding marine invertebrates. **GUILLARD, R.R.L.** Culture of Marine Invertebrate Animals. Plenum Press, 1975, 29-60
- **HAJDUKIEWICZ, P ; SVAB, Z ; MALIGA, P.** The small versatile pPZP family of Agrobacterium binary vectors forplant transformation. *Plant Mol Biol,* 1994, vol. 25, 989-994
- **DOMERGUE et al.** *Eur. J. Biochem.,* 2002, vol. 269, 4105-4103
- **HAJDUKIEWICZ,P ; SVAB, Z ; MALIGA, P.** The small versatile pPZP family of Agrobacterium binary vectors for plant transformation. *Plant Mol Biol,* 1994, vol. 25, 989-994
- **QIU et al.** *J. Biol. Chem.,* 2001, vol. 276, 31561-31566
- **HONG et al.** *Lipids,* 2002, vol. 37, 863-868
- **ZANK et al.** *Plant J.,* 2002, vol. 31, 255-268
- Genetic and genomic tools for Xenopus research: The NIH Xenopus initiative. *Dev. Dyn.,* 2002, vol. 225 (4), 384-391
- **SATOU,Y. ; YAMADA,L. ; MOCHIZUKI,Y. ; TAKATORI,N. ; KAWASHIMA,T. ; SASAKI,A. ; HAMAGUCHI,M. ; AWAZU,S. ; YAGI,K. ; SASAKURA,Y.** A cDNA resource from the basal chordate Ciona intestinalis. *JOURNAL Genesis,* 2002, vol. 33 (4), 153-154
- **ALTSCHUL et al.** *J. Mol. Biol.,* 1990, vol. 215, 403-410
- **CALVAYRAC R ; DOUCE R.** *FEBS Letters,* 1970, vol. 7, 259-262
- **DE GREVE, H. ; DHAESE, P. ; SEURINCK, J. ; LEMMERS, M. ; VAN MONTAGU, M. ; SCHELL, J.** Nucleotide sequence and transcript map of the Agrobacterium tumefaciens Ti plasmid-encoded octopine synthase gene. *J. Mol. Appl. Genet.,* 1982, vol. 1 (6), 499-511
- **CHRISTIE, WILLIAM W.** Advances in Lipid Methodology. Oily Press (Oily Press Lipid Library
- Repr. Oily Press Lipid Library, 1992, vol. IX, 307
- Progress in the Chemistry of Fats and Other Lipids CODEN. Progress in Lipid Research. Pergamon Press, 1952, vol. 1, 16
- Applied Microbial Physiology; A Practical Approach. IRL Press, 103-129
- Advances on Lipid Methodology. Oily Press, 1997, 119-169
- Gaschromatographie-Massenspektrometrie-Verfahren. *Lipide,* 1998, vol. 33, 343-353